# EUROPEAN PATENT APPLICATION

(11) **EP 3 567 371 A1**
(43) Date of publication of application: **13.11.2019**
(21) Application number: 19166832.6
(22) Date of filing: 14.03.2014
(51) Int. Cl.: G01N 33/68, A61K 38/04

(54) **BIOMARKERS AND METHODS FOR PREDICTING PREECLAMPSIA**

(30) Priority: 15.03.2013 US 201361798413 P
(62) Divisional of application: 14762389.6
(71) Applicant: Sera Prognostics, Inc., Salt Lake City, UT 84109 (US)
(72) Inventor: HICKOK, Durlin Edward, Salt Lake City, UT 84109 (US); BONIFACE, John Jay, Salt Lake City, UT 84109 (US); CRITCHFIELD, Gregory Charles, Salt Lake City, UT 84109 (US); FLEISCHER, Tracey Cristine, Salt Lake City, UT 84109 (US)
(74) Representative: Jones Day

(57) **Abstract**

The disclosure provides biomarkers useful in diagnostic methods related to pregnancy.

SEQUENCE LISTING

<110> SERA PROGNOSTICS, INC.

<120> BIOMARKERS AND METHODS FOR PREDICTING PREECLAMPSIA

<130> 103911PCEPT1

<150> 61/798,413
<151> 2013-03-15

<160> 964

<170> PatentIn version 3.5

<210> 1
<211> 7
<212> PRT
<213> Homo sapiens

<400> 1
Phe Ser Val Val Tyr Ala Lys
1               5

<210> 2
<211> 9
<212> PRT
<213> Homo sapiens

<400> 2
Ser Pro Glu Leu Gln Ala Glu Ala Lys
1               5

<210> 3
<211> 10
<212> PRT
<213> Homo sapiens

<400> 3
Val Asn His Val Thr Leu Ser Gln Pro Lys
1               5                   10

<210> 4
<211> 19
<212> PRT
<213> Homo sapiens

<400> 4
Ser Ser Asn Asn Pro His Ser Pro Ile Val Glu Glu Phe Gln Val Pro
1               5                   10                  15

Tyr Asn Lys

<210> 5
<211> 9
<212> PRT
<213> Homo sapiens

<400> 5
Val Val Gly Gly Leu Val Ala Leu Arg
1               5

<210> 6
<211> 9
<212> PRT
<213> Homo sapiens

<400> 6
Leu Asp Phe His Phe Ser Ser Asp Arg
1               5

<210> 7
<211> 10
<212> PRT
<213> Homo sapiens

<400> 7
Thr Val Gln Ala Val Leu Thr Val Pro Lys
1               5                   10

<210> 8
<211> 7
<212> PRT
<213> Homo sapiens

<400> 8
Gly Pro Gly Glu Asp Phe Arg
1               5

<210> 9
<211> 8
<212> PRT
<213> Homo sapiens

<400> 9
Glu Thr Leu Leu Gln Asp Phe Arg
1               5

<210> 10
<211> 9
<212> PRT
<213> Homo sapiens

<400> 10
Ala Thr Val Val Tyr Gln Gly Glu Arg
1               5

<210> 11
<211> 12
<212> PRT
<213> Homo sapiens

<400> 11
Gly Phe Gln Ala Leu Gly Asp Ala Ala Asp Ile Arg

1                   5                       10


<210> 12
<211> 18
<212> PRT
<213> Homo sapiens

<400> 12
Ser Ser Asn Asn Pro His Ser Pro Ile Val Glu Glu Phe Gln Val Pro
1                   5                       10                      15


Tyr Asn




<210> 13
<211> 11
<212> PRT
<213> Homo sapiens

<400> 13
Thr Ser Asp Gln Ile His Phe Phe Phe Ala Lys
1                   5                       10


<210> 14
<211> 11
<212> PRT
<213> Homo sapiens

<400> 14
Asp Pro Asn Gly Leu Pro Pro Glu Ala Gln Lys
1                   5                       10


<210> 15
<211> 13
<212> PRT
<213> Homo sapiens

<400> 15
Ser Val Ser Leu Pro Ser Leu Asp Pro Ala Ser Ala Lys
1                   5                       10


<210> 16
<211> 15
<212> PRT
<213> Homo sapiens

<400> 16
Ile Glu Gly Asn Leu Ile Phe Asp Pro Asn Asn Tyr Leu Pro Lys
1                   5                       10                      15


<210> 17
<211> 10
<212> PRT
<213> Homo sapiens

<400> 17

Tyr Trp Gly Val Ala Ser Phe Leu Gln Lys
1               5                   10

<210> 18
<211> 14
<212> PRT
<213> Homo sapiens

<400> 18
Ile Thr Glu Asn Asp Ile Gln Ile Ala Leu Asp Asp Ala Lys
1               5                   10

<210> 19
<211> 11
<212> PRT
<213> Homo sapiens

<400> 19
Gly Trp Val Thr Asp Gly Phe Ser Ser Leu Lys
1               5                   10

<210> 20
<211> 10
<212> PRT
<213> Homo sapiens

<400> 20
Thr Gly Ile Ser Pro Leu Ala Leu Ile Lys
1               5                   10

<210> 21
<211> 10
<212> PRT
<213> Homo sapiens

<400> 21
Ile Ile Gly Gly Ser Asp Ala Asp Ile Lys
1               5                   10

<210> 22
<211> 10
<212> PRT
<213> Homo sapiens

<400> 22
Thr Leu Leu Ile Ala Asn Glu Thr Leu Arg
1               5                   10

<210> 23
<211> 15
<212> PRT
<213> Homo sapiens

<400> 23
Val Ser Ala Leu Leu Thr Pro Ala Glu Gln Thr Gly Thr Trp Lys
1               5                   10                  15

<210> 24
<211> 16
<212> PRT
<213> Homo sapiens

<400> 24
Asp Ala Leu Ser Ser Val Gln Glu Ser Gln Val Ala Gln Gln Ala Arg
1               5                   10                  15


<210> 25
<211> 9
<212> PRT
<213> Homo sapiens

<400> 25
Ile Ala Gln Tyr Tyr Tyr Thr Phe Lys
1               5


<210> 26
<211> 7
<212> PRT
<213> Homo sapiens

<400> 26
Ile Glu Glu Ile Ala Ala Lys
1               5


<210> 27
<211> 22
<212> PRT
<213> Homo sapiens

<400> 27
Val Ile Leu Gly Ala His Gln Glu Val Asn Leu Glu Pro His Val Gln
1               5                   10                  15


Glu Ile Glu Val Ser Arg
            20


<210> 28
<211> 7
<212> PRT
<213> Homo sapiens

<400> 28
Asp Tyr Trp Ser Thr Val Lys
1               5


<210> 29
<211> 10
<212> PRT
<213> Homo sapiens

<400> 29
Val Pro Leu Ala Leu Phe Ala Leu Asn Arg
1               5                   10

<210> 30
<211> 9
<212> PRT
<213> Homo sapiens

<400> 30
Val Ile Ala Val Asn Glu Val Gly Arg
1               5


<210> 31
<211> 8
<212> PRT
<213> Homo sapiens

<400> 31
Leu Leu Glu Val Pro Glu Gly Arg
1               5


<210> 32
<211> 20
<212> PRT
<213> Homo sapiens

<400> 32
Val Glu Pro Leu Tyr Glu Leu Val Thr Ala Thr Asp Phe Ala Tyr Ser
1               5                   10                  15


Ser Thr Val Arg
            20


<210> 33
<211> 9
<212> PRT
<213> Homo sapiens

<400> 33
His His Gly Pro Thr Ile Thr Ala Lys
1               5


<210> 34
<211> 24
<212> PRT
<213> Homo sapiens

<400> 34
Ala Leu Asn Phe Gly Gly Ile Gly Val Val Val Gly His Glu Leu Thr
1               5                   10                  15


His Ala Phe Asp Asp Gln Gly Arg
            20


<210> 35
<211> 14
<212> PRT

<213> Homo sapiens

<400> 35
Gly Gly Glu Gly Thr Gly Tyr Phe Val Asp Phe Ser Val Arg
1               5                   10

<210> 36
<211> 16
<212> PRT
<213> Homo sapiens

<400> 36
Ser Pro Glu Gln Gln Glu Thr Val Leu Asp Gly Asn Leu Ile Ile Arg
1               5                   10                  15

<210> 37
<211> 11
<212> PRT
<213> Homo sapiens

<400> 37
Glu Pro Gly Leu Cys Thr Trp Gln Ser Leu Arg
1               5                   10

<210> 38
<211> 24
<212> PRT
<213> Homo sapiens

<400> 38
Val Val Leu Ser Ser Gly Ser Gly Pro Gly Leu Asp Leu Pro Leu Val
1               5                   10                  15

Leu Gly Leu Pro Leu Gln Leu Lys
            20

<210> 39
<211> 7
<212> PRT
<213> Homo sapiens

<400> 39
Tyr Thr Thr Glu Ile Ile Lys
1               5

<210> 40
<211> 12
<212> PRT
<213> Homo sapiens

<400> 40
Glu Asp Thr Pro Asn Ser Val Trp Glu Pro Ala Lys
1               5                   10

<210> 41
<211> 18

<212> PRT
<213> Homo sapiens

<400> 41
Leu His Glu Ala Phe Ser Pro Val Ser Tyr Gln His Asp Leu Ala Leu
1               5                   10                  15

Leu Arg

<210> 42
<211> 6
<212> PRT
<213> Homo sapiens

<400> 42
Ala His Tyr Asp Leu Arg
1               5

<210> 43
<211> 16
<212> PRT
<213> Homo sapiens

<400> 43
Gly Asp Thr Tyr Pro Ala Glu Leu Tyr Ile Thr Gly Ser Ile Leu Arg
1               5                   10                  15

<210> 44
<211> 22
<212> PRT
<213> Homo sapiens

<400> 44
Thr Ser Glu Ser Gly Glu Leu His Gly Leu Thr Thr Glu Glu Glu Phe
1               5                   10                  15

Val Glu Gly Ile Tyr Lys
            20

<210> 45
<211> 11
<212> PRT
<213> Homo sapiens

<400> 45
Ala Leu Glu Gln Asp Leu Pro Val Asn Ile Lys
1               5                   10

<210> 46
<211> 9
<212> PRT
<213> Homo sapiens

<400> 46
Leu Pro Asn Asn Val Leu Gln Glu Lys

1                   5


<210> 47
<211> 7
<212> PRT
<213> Homo sapiens

<400> 47
Phe Gln Leu Pro Gly Gln Lys
1                   5


<210> 48
<211> 10
<212> PRT
<213> Homo sapiens

<400> 48
His Thr Leu Asn Gln Ile Asp Glu Val Lys
1                   5                   10


<210> 49
<211> 10
<212> PRT
<213> Homo sapiens

<400> 49
Asp Ala Asp Pro Asp Thr Phe Phe Ala Lys
1                   5                   10


<210> 50
<211> 7
<212> PRT
<213> Homo sapiens

<400> 50
His Phe Gln Asn Leu Gly Lys
1                   5


<210> 51
<211> 18
<212> PRT
<213> Homo sapiens

<400> 51
Phe Asn Ala Val Leu Thr Asn Pro Gln Gly Asp Tyr Asp Thr Ser Thr
1                   5                   10                  15


Gly Lys



<210> 52
<211> 16
<212> PRT
<213> Homo sapiens

<400> 52

Ala Leu Asn His Leu Pro Leu Glu Tyr Asn Ser Ala Leu Tyr Ser Arg
1               5                   10                  15

<210> 53
<211> 6
<212> PRT
<213> Homo sapiens

<400> 53
Ala Trp Val Ala Trp Arg
1               5

<210> 54
<211> 12
<212> PRT
<213> Homo sapiens

<400> 54
Glu Thr Ala Ala Ser Leu Leu Gln Ala Gly Tyr Lys
1               5                   10

<210> 55
<211> 14
<212> PRT
<213> Homo sapiens

<400> 55
Ile Ala Leu Gly Gly Leu Leu Phe Pro Ala Ser Asn Leu Arg
1               5                   10

<210> 56
<211> 16
<212> PRT
<213> Homo sapiens

<400> 56
Ile Ala Pro Gln Leu Ser Thr Glu Glu Leu Val Ser Leu Gly Glu Lys
1               5                   10                  15

<210> 57
<211> 18
<212> PRT
<213> Homo sapiens

<400> 57
Val Ala Pro Glu Glu His Pro Val Leu Leu Thr Glu Ala Pro Leu Asn
1               5                   10                  15

Pro Lys

<210> 58
<211> 15
<212> PRT
<213> Homo sapiens

<400> 58
Ile Pro Gly Ile Phe Glu Leu Gly Ile Ser Ser Gln Ser Asp Arg
1               5                   10                  15

<210> 59
<211> 9
<212> PRT
<213> Homo sapiens

<400> 59
Ile Gln Thr His Ser Thr Thr Tyr Arg
1               5

<210> 60
<211> 10
<212> PRT
<213> Homo sapiens

<400> 60
Leu Leu Asp Ser Leu Pro Ser Asp Thr Arg
1               5                   10

<210> 61
<211> 19
<212> PRT
<213> Homo sapiens

<400> 61
Gln Leu Gly Leu Pro Gly Pro Pro Asp Val Pro Asp His Ala Ala Tyr
1               5                   10                  15

His Pro Phe

<210> 62
<211> 16
<212> PRT
<213> Homo sapiens

<400> 62
Ser Tyr Glu Leu Pro Asp Gly Gln Val Ile Thr Ile Gly Asn Glu Arg
1               5                   10                  15

<210> 63
<211> 7
<212> PRT
<213> Homo sapiens

<400> 63
Trp Gly Ala Ala Pro Tyr Arg
1               5

<210> 64
<211> 14
<212> PRT
<213> Homo sapiens

<400> 64
Asp Phe His Ile Asn Leu Phe Gln Val Leu Pro Trp Leu Lys
1               5                   10

<210> 65
<211> 22
<212> PRT
<213> Homo sapiens

<400> 65
Val Pro Thr Ala Asp Leu Glu Asp Val Leu Pro Leu Ala Glu Asp Ile
1               5                   10                  15

Thr Asn Ile Leu Ser Lys
            20

<210> 66
<211> 11
<212> PRT
<213> Homo sapiens

<400> 66
Leu Ile Glu Asn Gly Tyr Phe His Pro Val Lys
1               5                   10

<210> 67
<211> 17
<212> PRT
<213> Homo sapiens

<400> 67
Asn Lys Pro Gly Val Tyr Thr Asp Val Ala Tyr Tyr Leu Ala Trp Ile
1               5                   10                  15

Arg

<210> 68
<211> 11
<212> PRT
<213> Homo sapiens

<400> 68
Asn Thr Val Ile Ser Val Asn Pro Ser Thr Lys
1               5                   10

<210> 69
<211> 7
<212> PRT
<213> Homo sapiens

<400> 69
Leu Pro Asp Ala Thr Pro Lys
1               5

<210> 70
<211> 14
<212> PRT
<213> Homo sapiens

<400> 70
Leu Glu Gln Gly Glu Asn Val Phe Leu Gln Ala Thr Asp Lys
1               5                   10


<210> 71
<211> 8
<212> PRT
<213> Homo sapiens

<400> 71
Thr Leu Tyr Ser Ser Ser Pro Arg
1               5


<210> 72
<211> 20
<212> PRT
<213> Homo sapiens

<400> 72
Asp Gly Ser Pro Asp Val Thr Thr Ala Asp Ile Gly Ala Asn Thr Pro
1               5                   10                  15


Asp Ala Thr Lys
            20


<210> 73
<211> 15
<212> PRT
<213> Homo sapiens

<400> 73
Glu Trp Val Ala Ile Glu Ser Asp Ser Val Gln Pro Val Pro Arg
1               5                   10                  15


<210> 74
<211> 8
<212> PRT
<213> Homo sapiens

<400> 74
Leu Tyr Tyr Gly Asp Asp Glu Lys
1               5


<210> 75
<211> 24
<212> PRT
<213> Homo sapiens

<400> 75
Ala Asp Ser Gln Ala Gln Leu Leu Leu Ser Thr Val Val Gly Val Phe
1               5                   10                  15

Thr Ala Pro Gly Leu His Leu Lys
            20

<210> 76
<211> 7
<212> PRT
<213> Homo sapiens

<400> 76
Ala Tyr Ser Asp Leu Ser Arg
1               5

<210> 77
<211> 10
<212> PRT
<213> Homo sapiens

<400> 77
Asp Leu His Leu Ser Asp Val Phe Leu Lys
1               5                   10

<210> 78
<211> 10
<212> PRT
<213> Homo sapiens

<400> 78
Leu Ser Ser Pro Ala Val Ile Thr Asp Lys
1               5                   10

<210> 79
<211> 8
<212> PRT
<213> Homo sapiens

<400> 79
Ser Gly Phe Ser Phe Gly Phe Lys
1               5

<210> 80
<211> 16
<212> PRT
<213> Homo sapiens

<400> 80
Ile Ile Glu Val Glu Glu Glu Gln Glu Asp Pro Tyr Leu Asn Asp Arg
1               5                   10                  15

<210> 81
<211> 8
<212> PRT
<213> Homo sapiens

<400> 81
Ala Val Tyr Glu Ala Val Leu Arg

1                   5


<210> 82
<211> 22
<212> PRT
<213> Homo sapiens

<400> 82
Phe Thr Phe Thr Leu His Leu Glu Thr Pro Lys Pro Ser Ile Ser Ser
1               5                   10                  15


Ser Asn Leu Asn Pro Arg
            20


<210> 83
<211> 10
<212> PRT
<213> Homo sapiens

<400> 83
Asp Ala Gln Tyr Ala Pro Gly Tyr Asp Lys
1               5                   10


<210> 84
<211> 11
<212> PRT
<213> Homo sapiens

<400> 84
Tyr Gly Leu Val Thr Tyr Ala Thr Tyr Pro Lys
1               5                   10


<210> 85
<211> 9
<212> PRT
<213> Homo sapiens

<400> 85
Asp Ile Ser Glu Val Val Thr Pro Arg
1               5


<210> 86
<211> 13
<212> PRT
<213> Homo sapiens

<400> 86
Ile Val Leu Gly Gln Glu Gln Asp Ser Tyr Gly Gly Lys
1               5                   10


<210> 87
<211> 9
<212> PRT
<213> Homo sapiens

<400> 87

Thr Leu Glu Ala Gln Leu Thr Pro Arg
1               5

<210> 88
<211> 22
<212> PRT
<213> Homo sapiens

<400> 88
Val Pro Val Ala Val Gln Gly Glu Asp Thr Val Gln Ser Leu Thr Gln
1               5                   10                  15

Gly Asp Gly Val Ala Lys
            20

<210> 89
<211> 13
<212> PRT
<213> Homo sapiens

<400> 89
Ala Glu Ala Gln Ala Gln Tyr Ser Ala Ala Val Ala Lys
1               5                   10

<210> 90
<211> 10
<212> PRT
<213> Homo sapiens

<400> 90
Glu Leu Leu Glu Ser Tyr Ile Asp Gly Arg
1               5                   10

<210> 91
<211> 11
<212> PRT
<213> Homo sapiens

<400> 91
Thr Asn Leu Glu Ser Ile Leu Ser Tyr Pro Lys
1               5                   10

<210> 92
<211> 9
<212> PRT
<213> Homo sapiens

<400> 92
Leu Pro Thr Ala Val Val Pro Leu Arg
1               5

<210> 93
<211> 14
<212> PRT
<213> Homo sapiens

<400> 93
Asp Ser Pro Val Leu Ile Asp Phe Phe Glu Asp Thr Glu Arg
1               5                   10

<210> 94
<211> 7
<212> PRT
<213> Homo sapiens

<400> 94
Phe Ala Phe Asn Leu Tyr Arg
1               5

<210> 95
<211> 12
<212> PRT
<213> Homo sapiens

<400> 95
Phe Val Phe Gly Thr Thr Pro Glu Asp Ile Leu Arg
1               5                   10

<210> 96
<211> 15
<212> PRT
<213> Homo sapiens

<400> 96
Gly Asp Ser Gly Gly Ala Phe Ala Val Gln Asp Pro Asn Asp Lys
1               5                   10                  15

<210> 97
<211> 13
<212> PRT
<213> Homo sapiens

<400> 97
Ser Leu Asp Phe Thr Glu Leu Asp Val Ala Ala Glu Lys
1               5                   10

<210> 98
<211> 7
<212> PRT
<213> Homo sapiens

<400> 98
Ser Leu Leu Gln Pro Asn Lys
1               5

<210> 99
<211> 16
<212> PRT
<213> Homo sapiens

<400> 99
Val Gln Glu Ala His Leu Thr Glu Asp Gln Ile Phe Tyr Phe Pro Lys
1               5                   10                  15

<210> 100
<211> 11
<212> PRT
<213> Homo sapiens

<400> 100
Asp Asp Leu Tyr Val Ser Asp Ala Phe His Lys
1               5                   10


<210> 101
<211> 22
<212> PRT
<213> Homo sapiens

<400> 101
Asp Pro Asp Gln Thr Asp Gly Leu Gly Leu Ser Tyr Leu Ser Ser His
1               5                   10                  15


Ile Ala Asn Val Glu Arg
            20


<210> 102
<211> 10
<212> PRT
<213> Homo sapiens

<400> 102
Glu Ser Asp Thr Ser Tyr Val Ser Leu Lys
1               5                   10


<210> 103
<211> 8
<212> PRT
<213> Homo sapiens

<400> 103
Ile Leu Asp Asp Leu Ser Pro Arg
1               5


<210> 104
<211> 15
<212> PRT
<213> Homo sapiens

<400> 104
Ser Leu Pro Val Ser Asp Ser Val Leu Ser Gly Phe Glu Gln Arg
1               5                   10                  15


<210> 105
<211> 16
<212> PRT
<213> Homo sapiens

<400> 105
Thr Trp Asp Pro Glu Gly Val Ile Phe Tyr Gly Asp Thr Asn Pro Lys

1               5                   10                  15


<210> 106
<211> 10
<212> PRT
<213> Homo sapiens

<400> 106
Val Gly Glu Tyr Ser Leu Tyr Ile Gly Arg
1               5                   10


<210> 107
<211> 15
<212> PRT
<213> Homo sapiens

<400> 107
Val Pro Gly Thr Ser Thr Ser Ala Thr Leu Thr Gly Leu Thr Arg
1               5                   10                  15


<210> 108
<211> 15
<212> PRT
<213> Homo sapiens

<400> 108
Tyr Glu Val Gln Gly Glu Val Phe Thr Lys Pro Gln Leu Trp Pro
1               5                   10                  15


<210> 109
<211> 13
<212> PRT
<213> Homo sapiens

<400> 109
Ala Phe Thr Glu Cys Cys Val Val Ala Ser Gln Leu Arg
1               5                   10


<210> 110
<211> 8
<212> PRT
<213> Homo sapiens

<400> 110
Ala Pro Leu Thr Lys Pro Leu Lys
1               5


<210> 111
<211> 8
<212> PRT
<213> Homo sapiens

<400> 111
Phe Leu Gln Glu Gln Gly His Arg
1               5

<210> 112
<211> 14
<212> PRT
<213> Homo sapiens

<400> 112
Ile Ser Leu Leu Leu Ile Glu Ser Trp Leu Glu Pro Val Arg
1               5                   10


<210> 113
<211> 10
<212> PRT
<213> Homo sapiens

<400> 113
Leu Gln Gly Thr Leu Pro Val Glu Ala Arg
1               5                   10


<210> 114
<211> 14
<212> PRT
<213> Homo sapiens

<400> 114
Ser Tyr Thr Ile Thr Gly Leu Gln Pro Gly Thr Asp Tyr Lys
1               5                   10


<210> 115
<211> 8
<212> PRT
<213> Homo sapiens

<400> 115
Thr Ala Ser Asp Phe Ile Thr Lys
1               5


<210> 116
<211> 17
<212> PRT
<213> Homo sapiens

<400> 116
Val Leu Ser Ala Leu Gln Ala Val Gln Gly Leu Leu Val Ala Gln Gly
1               5                   10                  15


Arg


<210> 117
<211> 8
<212> PRT
<213> Homo sapiens

<400> 117
Val Thr Gly Trp Gly Asn Leu Lys
1               5

<210> 118
<211> 12
<212> PRT
<213> Homo sapiens

<400> 118
Ala Phe Ile Gln Leu Trp Ala Phe Asp Ala Val Lys
1               5                   10


<210> 119
<211> 11
<212> PRT
<213> Homo sapiens

<400> 119
Gly Tyr Val Ile Ile Lys Pro Leu Val Trp Val
1               5                   10


<210> 120
<211> 18
<212> PRT
<213> Homo sapiens

<400> 120
Ile Ile Thr Gly Leu Leu Glu Phe Glu Val Tyr Leu Glu Tyr Leu Gln
1               5                   10                  15


Asn Arg




<210> 121
<211> 13
<212> PRT
<213> Homo sapiens

<400> 121
Thr Asp Ala Pro Asp Leu Pro Glu Glu Asn Gln Ala Arg
1               5                   10


<210> 122
<211> 8
<212> PRT
<213> Homo sapiens

<400> 122
Thr Phe Thr Leu Leu Asp Pro Lys
1               5


<210> 123
<211> 7
<212> PRT
<213> Homo sapiens

<400> 123
Val Leu Glu Pro Thr Leu Lys
1               5

<210> 124
<211> 7
<212> PRT
<213> Homo sapiens

<400> 124
Tyr Glu Phe Leu Asn Gly Arg
1               5


<210> 125
<211> 23
<212> PRT
<213> Homo sapiens

<400> 125
Ala Ile Thr Pro Pro His Pro Ala Ser Gln Ala Asn Ile Ile Phe Asp
1               5                   10                  15


Ile Thr Glu Gly Asn Leu Arg
            20


<210> 126
<211> 12
<212> PRT
<213> Homo sapiens

<400> 126
Arg Ile Val Gln Ile Tyr Lys Asp Leu Leu Arg Asn
1               5                   10


<210> 127
<211> 10
<212> PRT
<213> Homo sapiens

<400> 127
Lys Val Met Asn His Ile Cys Ser Lys Gln
1               5                   10


<210> 128
<211> 14
<212> PRT
<213> Homo sapiens

<400> 128
Arg Arg His Pro Asp Leu Ser Ile Pro Glu Leu Leu Arg Ile
1               5                   10


<210> 129
<211> 9
<212> PRT
<213> Homo sapiens

<400> 129
Lys His Phe Gln Asn Leu Gly Lys Asp

1                   5


<210> 130
<211> 13
<212> PRT
<213> Homo sapiens

<400> 130
Lys Ile Thr Leu Leu Ser Ala Leu Val Glu Thr Arg Thr
1               5                   10


<210> 131
<211> 20
<212> PRT
<213> Homo sapiens

<400> 131
Arg Leu Tyr Gly Ser Glu Ala Phe Ala Thr Asp Phe Gln Asp Ser Ala
1               5                   10                  15


Ala Ala Lys Lys
            20


<210> 132
<211> 12
<212> PRT
<213> Homo sapiens

<400> 132
Arg Asn Leu Ala Val Ser Gln Val Val His Lys Ala
1               5                   10


<210> 133
<211> 16
<212> PRT
<213> Homo sapiens

<400> 133
Arg Cys Glu Gly Pro Ile Pro Asp Val Thr Phe Glu Leu Leu Arg Glu
1               5                   10                  15


<210> 134
<211> 39
<212> PRT
<213> Homo sapiens

<400> 134
Arg Leu His Asp Asn Gln Asn Gly Trp Ser Gly Asp Ser Ala Pro Val
1               5                   10                  15


Glu Leu Ile Leu Ser Asp Glu Thr Leu Pro Ala Pro Glu Phe Ser Pro
            20                  25                  30


Glu Pro Glu Ser Gly Arg Ala
        35

<210> 135
<211> 24
<212> PRT
<213> Homo sapiens

<400> 135
Arg Thr Pro Gly Ala Ala Ala Asn Leu Glu Leu Ile Phe Val Gly Pro
1               5                   10                  15

Gln His Ala Gly Asn Tyr Arg Cys
            20

<210> 136
<211> 10
<212> PRT
<213> Homo sapiens

<400> 136
Lys Leu Leu Glu Leu Thr Gly Pro Lys Ser
1               5                   10

<210> 137
<211> 13
<212> PRT
<213> Homo sapiens

<400> 137
Arg Ala Thr Trp Ser Gly Ala Val Leu Ala Gly Arg Asp
1               5                   10

<210> 138
<211> 26
<212> PRT
<213> Homo sapiens

<400> 138
Lys His Gln Met Asp Leu Val Ala Thr Leu Ser Gln Leu Gly Leu Gln
1               5                   10                  15

Glu Leu Phe Gln Ala Pro Asp Leu Arg Gly
            20                  25

<210> 139
<211> 15
<212> PRT
<213> Homo sapiens

<400> 139
Lys Leu Gly Asn Gln Glu Pro Gly Gly Gln Thr Ala Leu Lys Ser
1               5                   10                  15

<210> 140
<211> 28
<212> PRT

<213> Homo sapiens

<400> 140
Lys Gly Phe Pro Ile Lys Glu Asp Phe Leu Glu Gln Ser Glu Gln Leu
1               5                   10                  15

Phe Gly Ala Lys Pro Val Ser Leu Thr Gly Lys Gln
            20                  25

<210> 141
<211> 31
<212> PRT
<213> Homo sapiens

<400> 141
Arg Gln Pro Asn Cys Asp Asp Pro Glu Thr Glu Glu Ala Ala Leu Val
1               5                   10                  15

Ala Ile Asp Tyr Ile Asn Gln Asn Leu Pro Trp Gly Tyr Lys His
            20                  25                  30

<210> 142
<211> 34
<212> PRT
<213> Homo sapiens

<400> 142
Lys Val Trp Pro Gln Gln Pro Ser Gly Glu Leu Phe Glu Ile Glu Ile
1               5                   10                  15

Asp Thr Leu Glu Thr Thr Cys His Val Leu Asp Pro Thr Pro Val Ala
            20                  25                  30

Arg Cys

<210> 143
<211> 16
<212> PRT
<213> Homo sapiens

<400> 143
Lys Glu His Ala Val Glu Gly Asp Cys Asp Phe Gln Leu Leu Lys Leu
1               5                   10                  15

<210> 144
<211> 12
<212> PRT
<213> Homo sapiens

<400> 144
Lys His Thr Leu Asn Gln Ile Asp Glu Val Lys Val
1               5                   10

<210> 145
<211> 23
<212> PRT
<213> Homo sapiens

<400> 145
Arg Thr Val Val Gln Pro Ser Val Gly Ala Ala Ala Gly Pro Val Val
1               5                   10                  15

Pro Pro Cys Pro Gly Arg Ile
            20

<210> 146
<211> 27
<212> PRT
<213> Homo sapiens

<400> 146
Lys Thr Gly Cys Ser Leu Met Gly Ala Ser Val Asp Ser Thr Leu Ala
1               5                   10                  15

Phe Asn Thr Tyr Val His Phe Gln Gly Lys Met
            20                  25

<210> 147
<211> 16
<212> PRT
<213> Homo sapiens

<400> 147
Arg Ala Ala Met Val Gly Met Leu Ala Asn Phe Leu Gly Phe Arg Ile
1               5                   10                  15

<210> 148
<211> 19
<212> PRT
<213> Homo sapiens

<400> 148
Lys Gln Pro Phe Val Gln Gly Leu Ala Leu Tyr Thr Pro Val Val Leu
1               5                   10                  15

Pro Arg Ser

<210> 149
<211> 19
<212> PRT
<213> Homo sapiens

<400> 149
Lys Val Leu Ser Ala Leu Gln Ala Val Gln Gly Leu Leu Val Ala Gln
1               5                   10                  15

Gly Arg Ala

<210> 150
<211> 26
<212> PRT
<213> Homo sapiens

<400> 150
Arg Ala Asp Ser Gln Ala Gln Leu Leu Leu Ser Thr Val Val Gly Val
1               5                   10                  15

Phe Thr Ala Pro Gly Leu His Leu Lys Gln
            20                  25

<210> 151
<211> 27
<212> PRT
<213> Homo sapiens

<400> 151
Arg Ile Thr Asp Val Ile Pro Ser Glu Ala Ile Asn Glu Leu Thr Val
1               5                   10                  15

Leu Val Leu Val Asn Thr Ile Tyr Phe Lys Gly
            20                  25

<210> 152
<211> 23
<212> PRT
<213> Homo sapiens

<400> 152
Lys Asn Asp Asn Asp Asn Ile Phe Leu Ser Pro Leu Ser Ile Ser Thr
1               5                   10                  15

Ala Phe Ala Met Thr Lys Leu
            20

<210> 153
<211> 14
<212> PRT
<213> Homo sapiens

<400> 153
Arg Glu Val Pro Leu Asn Thr Ile Ile Phe Met Gly Arg Val
1               5                   10

<210> 154
<211> 30
<212> PRT
<213> Homo sapiens

<400> 154
Arg Val Ala Glu Gly Thr Gln Val Leu Glu Leu Pro Phe Lys Gly Asp
1               5                   10                  15

Asp Ile Thr Met Val Leu Ile Leu Pro Lys Pro Glu Lys Ser
            20                  25                  30

<210> 155
<211> 18
<212> PRT
<213> Homo sapiens

<400> 155
Lys Glu Gln Leu Gln Asp Met Gly Leu Val Asp Leu Phe Ser Pro Glu
1               5                   10                  15

Lys Ser

<210> 156
<211> 13
<212> PRT
<213> Homo sapiens

<400> 156
Arg Asp Ile Pro Met Asn Pro Met Cys Ile Tyr Arg Ser
1               5                   10

<210> 157
<211> 22
<212> PRT
<213> Homo sapiens

<400> 157
Lys Glu Pro Cys Val Glu Ser Leu Val Ser Gln Tyr Phe Gln Thr Val
1               5                   10                  15

Thr Asp Tyr Gly Lys Asp
            20

<210> 158
<211> 19
<212> PRT
<213> Homo sapiens

<400> 158
Lys Ser Leu Ala Glu Leu Gly Gly His Leu Asp Gln Gln Val Glu Glu
1               5                   10                  15

Phe Arg Arg

<210> 159
<211> 11
<212> PRT
<213> Homo sapiens

<400> 159
Arg Leu Ala Pro Leu Ala Glu Asp Val Arg Gly
1               5                   10

<210> 160
<211> 23
<212> PRT
<213> Homo sapiens

<400> 160
Arg Val Leu Arg Glu Asn Ala Asp Ser Leu Gln Ala Ser Leu Arg Pro
1               5                   10                  15

His Ala Asp Glu Leu Lys Ala
            20

<210> 161
<211> 27
<212> PRT
<213> Homo sapiens

<400> 161
Arg Ser Leu Ala Pro Tyr Ala Gln Asp Thr Gln Glu Lys Leu Asn His
1               5                   10                  15

Gln Leu Glu Gly Leu Thr Phe Gln Met Lys Lys
            20                  25

<210> 162
<211> 19
<212> PRT
<213> Homo sapiens

<400> 162
Lys Leu Gly Pro His Ala Gly Asp Val Glu Gly His Leu Ser Phe Leu
1               5                   10                  15

Glu Lys Asp

<210> 163
<211> 29
<212> PRT
<213> Homo sapiens

<400> 163
Lys Ser Glu Leu Thr Gln Gln Leu Asn Ala Leu Phe Gln Asp Lys Leu
1               5                   10                  15

Gly Glu Val Asn Thr Tyr Ala Gly Asp Leu Gln Lys Lys
            20                  25

<210> 164
<211> 13

<212> PRT
<213> Homo sapiens

<400> 164
Arg Leu Leu Pro His Ala Asn Glu Val Ser Gln Lys Ile
1               5                   10


<210> 165
<211> 20
<212> PRT
<213> Homo sapiens

<400> 165
Lys Ser Leu Ala Glu Leu Gly Gly His Leu Asp Gln Gln Val Glu Glu
1               5                   10                  15


Phe Arg Arg Arg
            20


<210> 166
<211> 16
<212> PRT
<213> Homo sapiens

<400> 166
Lys Ser Glu Leu Thr Gln Gln Leu Asn Ala Leu Phe Gln Asp Lys Leu
1               5                   10                  15


<210> 167
<211> 14
<212> PRT
<213> Homo sapiens

<400> 167
Lys Gly Phe Glu Pro Thr Leu Glu Ala Leu Phe Gly Lys Gln
1               5                   10


<210> 168
<211> 17
<212> PRT
<213> Homo sapiens

<400> 168
Lys Ala Leu Tyr Trp Val Asn Gly Gln Val Pro Asp Gly Val Ser Lys
1               5                   10                  15


Val



<210> 169
<211> 9
<212> PRT
<213> Homo sapiens

<400> 169
Lys Phe Ile Ile Pro Ser Pro Lys Arg

1               5


<210> 170
<211> 9
<212> PRT
<213> Homo sapiens

<400> 170
Arg Thr Pro Ala Leu His Phe Lys Ser
1               5


<210> 171
<211> 13
<212> PRT
<213> Homo sapiens

<400> 171
Lys Thr Glu Val Ile Pro Pro Leu Ile Glu Asn Arg Gln
1               5                   10


<210> 172
<211> 17
<212> PRT
<213> Homo sapiens

<400> 172
Arg Asn Leu Gln Asn Asn Ala Glu Trp Val Tyr Gln Gly Ala Ile Arg
1               5                   10                  15


Gln




<210> 173
<211> 18
<212> PRT
<213> Homo sapiens

<400> 173
Lys Leu Pro Gln Gln Ala Asn Asp Tyr Leu Asn Ser Phe Asn Trp Glu
1               5                   10                  15


Arg Gln




<210> 174
<211> 11
<212> PRT
<213> Homo sapiens

<400> 174
Arg Leu Ala Ala Tyr Leu Met Leu Met Arg Ser
1               5                   10


<210> 175
<211> 19

<212> PRT
<213> Homo sapiens

<400> 175
Arg Val Ile Gly Asn Met Gly Gln Thr Met Glu Gln Leu Thr Pro Glu
1               5                   10                  15

Leu Lys Ser

<210> 176
<211> 13
<212> PRT
<213> Homo sapiens

<400> 176
Lys Leu Ile Val Ala Met Ser Ser Trp Leu Gln Lys Ala
1               5                   10

<210> 177
<211> 17
<212> PRT
<213> Homo sapiens

<400> 177
Arg Thr Ser Ser Phe Ala Leu Asn Leu Pro Thr Leu Pro Glu Val Lys
1               5                   10                  15

Phe

<210> 178
<211> 24
<212> PRT
<213> Homo sapiens

<400> 178
Lys Ile Ala Asp Phe Glu Leu Pro Thr Ile Ile Val Pro Glu Gln Thr
1               5                   10                  15

Ile Glu Ile Pro Ser Ile Lys Phe
            20

<210> 179
<211> 17
<212> PRT
<213> Homo sapiens

<400> 179
Lys Ile Glu Gly Asn Leu Ile Phe Asp Pro Asn Asn Tyr Leu Pro Lys
1               5                   10                  15

Glu

<210> 180
<211> 26
<212> PRT
<213> Homo sapiens

<400> 180
Arg Thr Ser Ser Phe Ala Leu Asn Leu Pro Thr Leu Pro Glu Val Lys
1               5                   10                  15

Phe Pro Glu Val Asp Val Leu Thr Lys Tyr
            20                  25

<210> 181
<211> 26
<212> PRT
<213> Homo sapiens

<400> 181
Arg Leu Glu Leu Glu Leu Arg Pro Thr Gly Glu Ile Glu Gln Tyr Ser
1               5                   10                  15

Val Ser Ala Thr Tyr Glu Leu Gln Arg Glu
            20                  25

<210> 182
<211> 23
<212> PRT
<213> Homo sapiens

<400> 182
Lys Ser Thr Ala Ala Met Ser Thr Tyr Thr Gly Ile Phe Thr Asp Gln
1               5                   10                  15

Val Leu Ser Val Leu Lys Gly
            20

<210> 183
<211> 22
<212> PRT
<213> Homo sapiens

<400> 183
Arg Gly Trp Val Thr Asp Gly Phe Ser Ser Leu Lys Asp Tyr Trp Ser
1               5                   10                  15

Thr Val Lys Asp Lys Phe
            20

<210> 184
<211> 9
<212> PRT
<213> Homo sapiens

<400> 184

Arg Trp Glu Leu Ala Leu Gly Arg Phe
1               5

<210> 185
<211> 11
<212> PRT
<213> Homo sapiens

<400> 185
Arg Leu Ala Val Tyr Gln Ala Gly Ala Arg Glu
1               5                   10

<210> 186
<211> 14
<212> PRT
<213> Homo sapiens

<400> 186
Lys Ser Trp Phe Glu Pro Leu Val Glu Asp Met Gln Arg Gln
1               5                   10

<210> 187
<211> 17
<212> PRT
<213> Homo sapiens

<400> 187
Arg Ala Ala Thr Val Gly Ser Leu Ala Gly Gln Pro Leu Gln Glu Arg
1               5                   10                  15

Ala

<210> 188
<211> 18
<212> PRT
<213> Homo sapiens

<400> 188
Arg Thr Pro Glu Tyr Tyr Pro Asn Ala Gly Leu Ile Met Asn Tyr Cys
1               5                   10                  15

Arg Asn

<210> 189
<211> 22
<212> PRT
<213> Homo sapiens

<400> 189
Lys Thr Phe Tyr Glu Pro Gly Glu Glu Ile Thr Tyr Ser Cys Lys Pro
1               5                   10                  15

Gly Tyr Val Ser Arg Gly

            20

<210> 190
<211> 17
<212> PRT
<213> Homo sapiens

<400> 190
Lys Phe Ile Cys Pro Leu Thr Gly Leu Trp Pro Ile Asn Thr Leu Lys
1               5                   10                  15

Cys

<210> 191
<211> 15
<212> PRT
<213> Homo sapiens

<400> 191
Arg Ser Leu Gln Thr Phe Ser Gln Ala Trp Phe Thr Cys Arg Arg
1               5                   10                  15

<210> 192
<211> 23
<212> PRT
<213> Homo sapiens

<400> 192
Lys His Tyr Tyr Ile Gly Ile Ile Glu Thr Thr Trp Asp Tyr Ala Ser
1               5                   10                  15

Asp His Gly Glu Lys Lys Leu
            20

<210> 193
<211> 13
<212> PRT
<213> Homo sapiens

<400> 193
Arg Glu Tyr Thr Asp Ala Ser Phe Thr Asn Arg Lys Glu
1               5                   10

<210> 194
<211> 23
<212> PRT
<213> Homo sapiens

<400> 194
Lys Met Tyr Tyr Ser Ala Val Asp Pro Thr Lys Asp Ile Phe Thr Gly
1               5                   10                  15

Leu Ile Gly Pro Met Lys Ile
            20

<210> 195
<211> 39
<212> PRT
<213> Homo sapiens

<400> 195
Arg Ser Gly Ala Gly Thr Glu Asp Ser Ala Cys Ile Pro Trp Ala Tyr
1               5                   10                  15

Tyr Ser Thr Val Asp Gln Val Lys Asp Leu Tyr Ser Gly Leu Ile Gly
            20                  25                  30

Pro Leu Ile Val Cys Arg Arg
        35

<210> 196
<211> 25
<212> PRT
<213> Homo sapiens

<400> 196
Arg Lys Ala Glu Glu Glu His Leu Gly Ile Leu Gly Pro Gln Leu His
1               5                   10                  15

Ala Asp Val Gly Asp Lys Val Lys Ile
            20                  25

<210> 197
<211> 16
<212> PRT
<213> Homo sapiens

<400> 197
Lys Glu Val Gly Pro Thr Asn Ala Asp Pro Val Cys Leu Ala Lys Met
1               5                   10                  15

<210> 198
<211> 24
<212> PRT
<213> Homo sapiens

<400> 198
Arg Met Tyr Ser Val Asn Gly Tyr Thr Phe Gly Ser Leu Pro Gly Leu
1               5                   10                  15

Ser Met Cys Ala Glu Asp Arg Val
            20

<210> 199
<211> 16
<212> PRT
<213> Homo sapiens

<400> 199
Lys Asp Ile Ala Ser Gly Leu Ile Gly Pro Leu Ile Ile Cys Lys Lys
1               5                   10                  15

<210> 200
<211> 30
<212> PRT
<213> Homo sapiens

<400> 200
Arg Gln Lys Asp Val Asp Lys Glu Phe Tyr Leu Phe Pro Thr Val Phe
1               5                   10                  15

Asp Glu Asn Glu Ser Leu Leu Leu Glu Asp Asn Ile Arg Met
            20                  25                  30

<210> 201
<211> 25
<212> PRT
<213> Homo sapiens

<400> 201
Arg Gly Pro Glu Glu Glu His Leu Gly Ile Leu Gly Pro Val Ile Trp
1               5                   10                  15

Ala Glu Val Gly Asp Thr Ile Arg Val
            20                  25

<210> 202
<211> 15
<212> PRT
<213> Homo sapiens

<400> 202
Lys Gly Ala Tyr Pro Leu Ser Ile Glu Pro Ile Gly Val Arg Phe
1               5                   10                  15

<210> 203
<211> 25
<212> PRT
<213> Homo sapiens

<400> 203
Arg Gly Val Tyr Ser Ser Asp Val Phe Asp Ile Phe Pro Gly Thr Tyr
1               5                   10                  15

Gln Thr Leu Glu Met Phe Pro Arg Thr
            20                  25

<210> 204
<211> 17
<212> PRT
<213> Homo sapiens

<400> 204

Lys Asp Ile Ala Ser Gly Leu Ile Gly Pro Leu Ile Ile Cys Lys Lys
1               5                   10                  15

Asp

<210> 205
<211> 25
<212> PRT
<213> Homo sapiens

<400> 205
Arg Ser Gly Ala Gly Thr Glu Asp Ser Ala Cys Ile Pro Trp Ala Tyr
1               5                   10                  15

Tyr Ser Thr Val Asp Gln Val Lys Asp
            20                  25

<210> 206
<211> 12
<212> PRT
<213> Homo sapiens

<400> 206
Arg Ile Tyr His Ser His Ile Asp Ala Pro Lys Asp
1               5                   10

<210> 207
<211> 35
<212> PRT
<213> Homo sapiens

<400> 207
Arg Ala Asp Asp Lys Val Tyr Pro Gly Glu Gln Tyr Thr Tyr Met Leu
1               5                   10                  15

Leu Ala Thr Glu Glu Gln Ser Pro Gly Glu Gly Asp Gly Asn Cys Val
            20                  25                  30

Thr Arg Ile
        35

<210> 208
<211> 16
<212> PRT
<213> Homo sapiens

<400> 208
Lys Asp Leu Tyr Ser Gly Leu Ile Gly Pro Leu Ile Val Cys Arg Arg
1               5                   10                  15

<210> 209
<211> 19
<212> PRT

<213> Homo sapiens

<400> 209
Arg Thr Thr Ile Glu Lys Pro Val Trp Leu Gly Phe Leu Gly Pro Ile
1               5                   10                  15

Ile Lys Ala

<210> 210
<211> 13
<212> PRT
<213> Homo sapiens

<400> 210
Lys Ile Phe Phe Pro Gly Val Ser Glu Phe Gly Lys Glu
1               5                   10

<210> 211
<211> 23
<212> PRT
<213> Homo sapiens

<400> 211
Arg Ala Ile Leu Gln Ser Gly Ser Phe Asn Ala Pro Trp Ala Val Thr
1               5                   10                  15

Ser Leu Tyr Glu Ala Arg Asn
            20

<210> 212
<211> 20
<212> PRT
<213> Homo sapiens

<400> 212
Arg Leu His Glu Ala Phe Ser Pro Val Ser Tyr Gln His Asp Leu Ala
1               5                   10                  15

Leu Leu Arg Leu
            20

<210> 213
<211> 18
<212> PRT
<213> Homo sapiens

<400> 213
Arg Gly Asp Thr Tyr Pro Ala Glu Leu Tyr Ile Thr Gly Ser Ile Leu
1               5                   10                  15

Arg Met

<210> 214
<211> 14
<212> PRT
<213> Homo sapiens

<400> 214
Lys Val Leu His Gly Asp Leu Ile Asp Phe Val Cys Lys Gln
1               5                   10


<210> 215
<211> 22
<212> PRT
<213> Homo sapiens

<400> 215
Lys Leu Val Phe Gln Gln Phe Asp Leu Glu Pro Ser Glu Gly Cys Phe
1               5                   10                  15


Tyr Asp Tyr Val Lys Ile
            20


<210> 216
<211> 12
<212> PRT
<213> Homo sapiens

<400> 216
Arg Val Lys Asn Tyr Val Asp Trp Ile Met Lys Thr
1               5                   10


<210> 217
<211> 18
<212> PRT
<213> Homo sapiens

<400> 217
Lys Ser Asn Ala Leu Asp Ile Ile Phe Gln Thr Asp Leu Thr Gly Gln
1               5                   10                  15


Lys Lys


<210> 218
<211> 10
<212> PRT
<213> Homo sapiens

<400> 218
Arg Asp Phe His Ile Asn Leu Phe Arg Met
1               5                   10


<210> 219
<211> 19
<212> PRT
<213> Homo sapiens

<400> 219
Arg Gly Ala Leu Ile Ser Asp Gln Trp Val Leu Thr Ala Ala His Cys
1               5                   10                  15

Phe Arg Asp

<210> 220
<211> 19
<212> PRT
<213> Homo sapiens

<400> 220
Lys Lys Asn Gln Gly Ile Leu Glu Phe Tyr Gly Asp Asp Ile Ala Leu
1               5                   10                  15

Leu Lys Leu

<210> 221
<211> 12
<212> PRT
<213> Homo sapiens

<400> 221
Arg Ile His Trp Glu Ser Ala Ser Leu Leu Arg Ser
1               5                   10

<210> 222
<211> 11
<212> PRT
<213> Homo sapiens

<400> 222
Arg Val His Tyr Thr Val Cys Ile Trp Arg Asn
1               5                   10

<210> 223
<211> 15
<212> PRT
<213> Homo sapiens

<400> 223
Lys Ala Glu Met Ala Asp Gln Ala Ala Ala Trp Leu Thr Arg Gln
1               5                   10                  15

<210> 224
<211> 21
<212> PRT
<213> Homo sapiens

<400> 224
Lys Met Arg Pro Ser Thr Asp Thr Ile Thr Val Met Val Glu Asn Ser
1               5                   10                  15

His Gly Leu Arg Val
            20

<210> 225
<211> 24
<212> PRT
<213> Homo sapiens

<400> 225
Arg Val Gln Gln Pro Asp Cys Arg Glu Pro Phe Leu Ser Cys Cys Gln
1               5                   10                  15

Phe Ala Glu Ser Leu Arg Lys Lys
            20

<210> 226
<211> 14
<212> PRT
<213> Homo sapiens

<400> 226
Lys Leu Val Asn Gly Gln Ser His Ile Ser Leu Ser Lys Ala
1               5                   10

<210> 227
<211> 13
<212> PRT
<213> Homo sapiens

<400> 227
Arg Gly Gln Ile Val Phe Met Asn Arg Glu Pro Lys Arg
1               5                   10

<210> 228
<211> 23
<212> PRT
<213> Homo sapiens

<400> 228
Lys Val Gly Leu Ser Gly Met Ala Ile Ala Asp Val Thr Leu Leu Ser
1               5                   10                  15

Gly Phe His Ala Leu Arg Ala
            20

<210> 229
<211> 19
<212> PRT
<213> Homo sapiens

<400> 229
Arg Gly His Leu Phe Leu Gln Thr Asp Gln Pro Ile Tyr Asn Pro Gly
1               5                   10                  15

Gln Arg Val

<210> 230
<211> 11
<212> PRT
<213> Homo sapiens

<400> 230
Lys Ser His Ala Leu Gln Leu Asn Asn Arg Gln
1               5                   10


<210> 231
<211> 25
<212> PRT
<213> Homo sapiens

<400> 231
Arg Tyr Val Ser His Phe Glu Thr Glu Gly Pro His Val Leu Leu Tyr
1               5                   10                  15


Phe Asp Ser Val Pro Thr Ser Arg Glu
            20                  25


<210> 232
<211> 14
<212> PRT
<213> Homo sapiens

<400> 232
Arg Gly Ser Ser Thr Trp Leu Thr Ala Phe Val Leu Lys Val
1               5                   10


<210> 233
<211> 16
<212> PRT
<213> Homo sapiens

<400> 233
Arg Tyr Ile Tyr Gly Lys Pro Val Gln Gly Val Ala Tyr Val Arg Phe
1               5                   10                  15


<210> 234
<211> 11
<212> PRT
<213> Homo sapiens

<400> 234
Lys Ser Cys Gly Leu His Gln Leu Leu Arg Gly
1               5                   10


<210> 235
<211> 16
<212> PRT
<213> Homo sapiens

<400> 235

Arg Gly Pro Glu Val Gln Leu Val Ala His Ser Pro Trp Leu Lys Asp
1               5                   10                  15

<210> 236
<211> 29
<212> PRT
<213> Homo sapiens

<400> 236
Arg Lys Lys Glu Val Tyr Met Pro Ser Ser Ile Phe Gln Asp Asp Phe
1               5                   10                  15

Val Ile Pro Asp Ile Ser Glu Pro Gly Thr Trp Lys Ile
            20                  25

<210> 237
<211> 23
<212> PRT
<213> Homo sapiens

<400> 237
Arg Val Gln Gln Pro Asp Cys Arg Glu Pro Phe Leu Ser Cys Cys Gln
1               5                   10                  15

Phe Ala Glu Ser Leu Arg Lys
            20

<210> 238
<211> 24
<212> PRT
<213> Homo sapiens

<400> 238
Lys Ala Ser Ala Gly Leu Leu Gly Ala His Ala Ala Ala Ile Thr Ala
1               5                   10                  15

Tyr Ala Leu Thr Leu Thr Lys Ala
            20

<210> 239
<211> 13
<212> PRT
<213> Homo sapiens

<400> 239
Lys Ile Thr His Tyr Asn Tyr Leu Ile Leu Ser Lys Gly
1               5                   10

<210> 240
<211> 12
<212> PRT
<213> Homo sapiens

<400> 240
Arg Lys Ala Phe Asp Ile Cys Pro Leu Val Lys Ile

1               5                   10


<210> 241
<211> 10
<212> PRT
<213> Homo sapiens

<400> 241
Arg Ile Pro Leu Asp Leu Val Pro Lys Thr
1               5                   10


<210> 242
<211> 28
<212> PRT
<213> Homo sapiens

<400> 242
Arg Met Val Glu Thr Thr Ala Tyr Ala Leu Leu Thr Ser Leu Asn Leu
1               5                   10                  15


Lys Asp Ile Asn Tyr Val Asn Pro Val Ile Lys Trp
            20                  25


<210> 243
<211> 17
<212> PRT
<213> Homo sapiens

<400> 243
Lys Ala Leu Leu Val Gly Glu His Leu Asn Ile Ile Val Thr Pro Lys
1               5                   10                  15


Ser




<210> 244
<211> 14
<212> PRT
<213> Homo sapiens

<400> 244
Lys Leu Lys Glu Gly Met Leu Ser Ile Met Ser Tyr Arg Asn
1               5                   10


<210> 245
<211> 18
<212> PRT
<213> Homo sapiens

<400> 245
Arg Tyr Ile Tyr Pro Leu Asp Ser Leu Thr Trp Ile Glu Tyr Trp Pro
1               5                   10                  15


Arg Asp

<210> 246
<211> 16
<212> PRT
<213> Homo sapiens

<400> 246
Lys Gly Gly Ser Ala Ser Thr Trp Leu Thr Ala Phe Ala Leu Arg Val
1               5                   10                  15


<210> 247
<211> 28
<212> PRT
<213> Homo sapiens

<400> 247
Arg Tyr Gly Gly Gly Phe Tyr Ser Thr Gln Asp Thr Ile Asn Ala Ile
1               5                   10                  15


Glu Gly Leu Thr Glu Tyr Ser Leu Leu Val Lys Gln
            20                  25


<210> 248
<211> 14
<212> PRT
<213> Homo sapiens

<400> 248
Lys Ala Lys Asp Leu His Leu Ser Asp Val Phe Leu Lys Ala
1               5                   10


<210> 249
<211> 18
<212> PRT
<213> Homo sapiens

<400> 249
Lys Ala Leu Asn His Leu Pro Leu Glu Tyr Asn Ser Ala Leu Tyr Ser
1               5                   10                  15


Arg Ile



<210> 250
<211> 15
<212> PRT
<213> Homo sapiens

<400> 250
Arg Leu Ser Gly Asn Val Leu Ser Tyr Thr Phe Gln Val Lys Ile
1               5                   10                  15


<210> 251
<211> 30
<212> PRT

<213> Homo sapiens

<400> 251
Arg Lys Asp Asp Ile Met Leu Asp Glu Gly Met Leu Gln Ser Leu Met
1               5                   10                  15

Glu Leu Pro Asp Gln Tyr Asn Tyr Gly Met Tyr Ala Lys Phe
            20                  25                  30

<210> 252
<211> 26
<212> PRT
<213> Homo sapiens

<400> 252
Arg Asp Phe Gly Thr His Tyr Ile Thr Glu Ala Val Leu Gly Gly Ile
1               5                   10                  15

Tyr Glu Tyr Thr Leu Val Met Asn Lys Glu
            20                  25

<210> 253
<211> 14
<212> PRT
<213> Homo sapiens

<400> 253
Arg Asp Thr Met Val Glu Asp Leu Val Val Leu Val Arg Gly
1               5                   10

<210> 254
<211> 17
<212> PRT
<213> Homo sapiens

<400> 254
Arg Tyr Tyr Ala Gly Gly Cys Ser Pro His Tyr Ile Leu Asn Thr Arg
1               5                   10                  15

Phe

<210> 255
<211> 17
<212> PRT
<213> Homo sapiens

<400> 255
Arg Ser Leu Pro Val Ser Asp Ser Val Leu Ser Gly Phe Glu Gln Arg
1               5                   10                  15

Val

<210> 256
<211> 18
<212> PRT
<213> Homo sapiens

<400> 256
Arg Val Gln Glu Ala His Leu Thr Glu Asp Gln Ile Phe Tyr Phe Pro
1               5                   10                  15

Lys Tyr

<210> 257
<211> 31
<212> PRT
<213> Homo sapiens

<400> 257
Arg Thr Ala Gly Tyr Gly Ile Asn Ile Leu Gly Met Asp Pro Leu Ser
1               5                   10                  15

Thr Pro Phe Asp Asn Glu Phe Tyr Asn Gly Leu Cys Asn Arg Asp
            20                  25                  30

<210> 258
<211> 15
<212> PRT
<213> Homo sapiens

<400> 258
Arg Arg Pro Trp Asn Val Ala Ser Leu Ile Tyr Glu Thr Lys Gly
1               5                   10                  15

<210> 259
<211> 15
<212> PRT
<213> Homo sapiens

<400> 259
Arg Ala Ile Glu Asp Tyr Ile Asn Glu Phe Ser Val Arg Lys Cys
1               5                   10                  15

<210> 260
<211> 14
<212> PRT
<213> Homo sapiens

<400> 260
Arg Ala Ile Glu Asp Tyr Ile Asn Glu Phe Ser Val Arg Lys
1               5                   10

<210> 261
<211> 13
<212> PRT
<213> Homo sapiens

<400> 261
Arg Leu Glu Asp Ser Val Thr Tyr His Cys Ser Arg Gly
1               5                   10

<210> 262
<211> 18
<212> PRT
<213> Homo sapiens

<400> 262
Arg Phe Ile Gln Val Gly Val Ile Ser Trp Gly Val Val Asp Val Cys
1               5                   10                  15

Lys Asn

<210> 263
<211> 16
<212> PRT
<213> Homo sapiens

<400> 263
Arg Asp Phe His Ile Asn Leu Phe Gln Val Leu Pro Trp Leu Lys Glu
1               5                   10                  15

<210> 264
<211> 20
<212> PRT
<213> Homo sapiens

<400> 264
Lys Tyr Gly Gln Thr Ile Arg Pro Ile Cys Leu Pro Cys Thr Glu Gly
1               5                   10                  15

Thr Thr Arg Ala
            20

<210> 265
<211> 26
<212> PRT
<213> Homo sapiens

<400> 265
Arg Leu Leu Gln Glu Gly Gln Ala Leu Glu Tyr Val Cys Pro Ser Gly
1               5                   10                  15

Phe Tyr Pro Tyr Pro Val Gln Thr Arg Thr
            20                  25

<210> 266
<211> 12
<212> PRT
<213> Homo sapiens

<400> 266

Arg Arg Pro Tyr Phe Pro Val Ala Val Gly Lys Tyr
1               5                   10

<210> 267
<211> 13
<212> PRT
<213> Homo sapiens

<400> 267
Lys Cys Thr Ser Thr Gly Trp Ile Pro Ala Pro Arg Cys
1               5                   10

<210> 268
<211> 11
<212> PRT
<213> Homo sapiens

<400> 268
Lys Cys Leu His Pro Cys Val Ile Ser Arg Glu
1               5                   10

<210> 269
<211> 13
<212> PRT
<213> Homo sapiens

<400> 269
Arg Glu Ile Met Glu Asn Tyr Asn Ile Ala Leu Arg Trp
1               5                   10

<210> 270
<211> 20
<212> PRT
<213> Homo sapiens

<400> 270
Lys Ala Val Tyr Thr Cys Asn Glu Gly Tyr Gln Leu Leu Gly Glu Ile
1               5                   10                  15

Asn Tyr Arg Glu
            20

<210> 271
<211> 22
<212> PRT
<213> Homo sapiens

<400> 271
Arg Ser Ile Thr Cys Ile His Gly Val Trp Thr Gln Leu Pro Gln Cys
1               5                   10                  15

Val Ala Ile Asp Lys Leu
            20

<210> 272

<211> 12
<212> PRT
<213> Homo sapiens

<400> 272
Arg Trp Gln Ser Ile Pro Leu Cys Val Glu Lys Ile
1               5                   10


<210> 273
<211> 20
<212> PRT
<213> Homo sapiens

<400> 273
Lys Thr Asp Cys Leu Ser Leu Pro Ser Phe Glu Asn Ala Ile Pro Met
1               5                   10                  15


Gly Glu Lys Lys
            20


<210> 274
<211> 16
<212> PRT
<213> Homo sapiens

<400> 274
Lys Cys Phe Glu Gly Phe Gly Ile Asp Gly Pro Ala Ile Ala Lys Cys
1               5                   10                  15


<210> 275
<211> 11
<212> PRT
<213> Homo sapiens

<400> 275
Lys Ile Asp Val His Leu Val Pro Asp Arg Lys
1               5                   10


<210> 276
<211> 14
<212> PRT
<213> Homo sapiens

<400> 276
Lys Ser Ser Asn Leu Ile Ile Leu Glu Glu His Leu Lys Asn
1               5                   10


<210> 277
<211> 15
<212> PRT
<213> Homo sapiens

<400> 277
Arg Ala Gln Leu Gly Asp Leu Pro Trp Gln Val Ala Ile Lys Asp
1               5                   10                  15

<210> 278
<211> 12
<212> PRT
<213> Homo sapiens

<400> 278
Arg Val Phe Ser Leu Gln Trp Gly Glu Val Lys Leu
1               5                   10


<210> 279
<211> 18
<212> PRT
<213> Homo sapiens

<400> 279
Arg Trp Ser Ala Gly Leu Thr Ser Ser Gln Val Asp Leu Tyr Ile Pro
1               5                   10                  15


Lys Val


<210> 280
<211> 22
<212> PRT
<213> Homo sapiens

<400> 280
Lys Thr Phe Pro Gly Phe Phe Ser Pro Met Leu Gly Glu Phe Val Ser
1               5                   10                  15


Glu Thr Glu Ser Arg Gly
            20


<210> 281
<211> 32
<212> PRT
<213> Homo sapiens

<400> 281
Arg Ile Glu Gly Ser Asn Lys Val Pro Val Asp Pro Ala Thr Tyr Gly
1               5                   10                  15


Gln Phe Tyr Gly Gly Asp Ser Tyr Ile Ile Leu Tyr Asn Tyr Arg His
            20                  25                  30


<210> 282
<211> 24
<212> PRT
<213> Homo sapiens

<400> 282
Arg Ala Gln Pro Val Gln Val Ala Glu Gly Ser Glu Pro Asp Gly Phe
1               5                   10                  15


Trp Glu Ala Leu Gly Gly Lys Ala

            20

<210> 283
<211> 28
<212> PRT
<213> Homo sapiens

<400> 283
Lys Thr Pro Ser Ala Ala Tyr Leu Trp Val Gly Thr Gly Ala Ser Glu
1               5                   10                  15

Ala Glu Lys Thr Gly Ala Gln Glu Leu Leu Arg Val
            20                  25

<210> 284
<211> 29
<212> PRT
<213> Homo sapiens

<400> 284
Arg Val Glu Lys Phe Asp Leu Val Pro Val Pro Thr Asn Leu Tyr Gly
1               5                   10                  15

Asp Phe Phe Thr Gly Asp Ala Tyr Val Ile Leu Lys Thr
            20                  25

<210> 285
<211> 17
<212> PRT
<213> Homo sapiens

<400> 285
Arg Glu Val Gln Gly Phe Glu Ser Ala Thr Phe Leu Gly Tyr Phe Lys
1               5                   10                  15

Ser

<210> 286
<211> 27
<212> PRT
<213> Homo sapiens

<400> 286
Lys Asn Trp Arg Asp Pro Asp Gln Thr Asp Gly Leu Gly Leu Ser Tyr
1               5                   10                  15

Leu Ser Ser His Ile Ala Asn Val Glu Arg Val
            20                  25

<210> 287
<211> 20
<212> PRT
<213> Homo sapiens

<400> 287
Lys Thr Pro Ser Ala Ala Tyr Leu Trp Val Gly Thr Gly Ala Ser Glu
1               5                   10                  15

Ala Glu Lys Thr
            20

<210> 288
<211> 14
<212> PRT
<213> Homo sapiens

<400> 288
Lys Phe Leu Val Gly Pro Asp Gly Ile Pro Ile Met Arg Trp
1               5                   10

<210> 289
<211> 29
<212> PRT
<213> Homo sapiens

<400> 289
Arg Leu Glu Lys Glu Val Gly Thr Pro His Gly Ile Ile Leu Asp Ser
1               5                   10                  15

Val Asp Ala Ala Phe Ile Cys Pro Gly Ser Ser Arg Leu
            20                  25

<210> 290
<211> 15
<212> PRT
<213> Homo sapiens

<400> 290
Arg Trp Lys Asn Phe Pro Ser Pro Val Asp Ala Ala Phe Arg Gln
1               5                   10                  15

<210> 291
<211> 29
<212> PRT
<213> Homo sapiens

<400> 291
Arg Gly Glu Cys Gln Ala Glu Gly Val Leu Phe Phe Gln Gly Asp Arg
1               5                   10                  15

Glu Trp Phe Trp Asp Leu Ala Thr Gly Thr Met Lys Glu
            20                  25

<210> 292
<211> 26
<212> PRT
<213> Homo sapiens

<400> 292
Lys Glu Val Gly Thr Pro His Gly Ile Ile Leu Asp Ser Val Asp Ala
1               5                   10                  15

Ala Phe Ile Cys Pro Gly Ser Ser Arg Leu
            20                  25

<210> 293
<211> 9
<212> PRT
<213> Homo sapiens

<400> 293
Arg Leu Trp Trp Leu Asp Leu Lys Ser
1               5

<210> 294
<211> 13
<212> PRT
<213> Homo sapiens

<400> 294
Lys Asn Phe Pro Ser Pro Val Asp Ala Ala Phe Arg Gln
1               5                   10

<210> 295
<211> 14
<212> PRT
<213> Homo sapiens

<400> 295
Arg Glu Trp Phe Trp Asp Leu Ala Thr Gly Thr Met Lys Glu
1               5                   10

<210> 296
<211> 13
<212> PRT
<213> Homo sapiens

<400> 296
Lys Gly Gly Tyr Thr Leu Val Ser Gly Tyr Pro Lys Arg
1               5                   10

<210> 297
<211> 17
<212> PRT
<213> Homo sapiens

<400> 297
Lys Leu Tyr Leu Val Gln Gly Thr Gln Val Tyr Val Phe Leu Thr Lys
1               5                   10                  15

Gly

<210> 298
<211> 22
<212> PRT
<213> Homo sapiens

<400> 298
Arg Glu Tyr Tyr Phe Ala Glu Ala Gln Ile Ala Asp Phe Ser Asp Pro
1               5                   10                  15

Ala Phe Ile Ser Lys Thr
            20

<210> 299
<211> 11
<212> PRT
<213> Homo sapiens

<400> 299
Lys Gln Phe Pro Ile Leu Leu Asp Phe Lys Thr
1               5                   10

<210> 300
<211> 9
<212> PRT
<213> Homo sapiens

<400> 300
Lys Phe Ala Phe Asn Leu Tyr Arg Val
1               5

<210> 301
<211> 11
<212> PRT
<213> Homo sapiens

<400> 301
Arg Asp Gly Tyr Leu Phe Gln Leu Leu Arg Ile
1               5                   10

<210> 302
<211> 24
<212> PRT
<213> Homo sapiens

<400> 302
Arg Ser Phe Glu Gly Leu Gly Gln Leu Glu Val Leu Thr Leu Asp His
1               5                   10                  15

Asn Gln Leu Gln Glu Val Lys Ala
            20

<210> 303
<211> 13
<212> PRT
<213> Homo sapiens

<400> 303
Arg Thr Phe Thr Pro Gln Pro Pro Gly Leu Glu Arg Leu
1               5                   10

<210> 304
<211> 12
<212> PRT
<213> Homo sapiens

<400> 304
Arg Ala Phe Trp Leu Asp Val Ser His Asn Arg Leu
1               5                   10

<210> 305
<211> 16
<212> PRT
<213> Homo sapiens

<400> 305
Arg Leu Ala Glu Leu Pro Ala Asp Ala Leu Gly Pro Leu Gln Arg Ala
1               5                   10                  15

<210> 306
<211> 16
<212> PRT
<213> Homo sapiens

<400> 306
Arg Leu Glu Ala Leu Pro Asn Ser Leu Leu Ala Pro Leu Gly Arg Leu
1               5                   10                  15

<210> 307
<211> 17
<212> PRT
<213> Homo sapiens

<400> 307
Arg Asn Leu Ile Ala Ala Val Ala Pro Gly Ala Phe Leu Gly Leu Lys
1               5                   10                  15

Ala

<210> 308
<211> 15
<212> PRT
<213> Homo sapiens

<400> 308
Arg Gln Ala Val Asp Thr Ala Val Asp Gly Val Phe Ile Arg Ser
1               5                   10                  15

<210> 309
<211> 23
<212> PRT
<213> Homo sapiens

<400> 309
Lys Thr Ala Phe Ile Ser Asp Phe Ala Val Thr Ala Asp Gly Asn Ala
1               5                   10                  15

Phe Ile Gly Asp Ile Lys Asp
            20

<210> 310
<211> 12
<212> PRT
<213> Homo sapiens

<400> 310
Arg Gly His Met Leu Glu Asn His Val Glu Arg Leu
1               5                   10

<210> 311
<211> 30
<212> PRT
<213> Homo sapiens

<400> 311
Lys Thr Ala Phe Ile Ser Asp Phe Ala Val Thr Ala Asp Gly Asn Ala
1               5                   10                  15

Phe Ile Gly Asp Ile Lys Asp Lys Val Thr Ala Trp Lys Gln
            20                  25                  30

<210> 312
<211> 39
<212> PRT
<213> Homo sapiens

<400> 312
Arg Gly Ile Glu Ile Leu Asn Gln Val Gln Glu Ser Leu Pro Glu Leu
1               5                   10                  15

Ser Asn His Ala Ser Ile Leu Ile Met Leu Thr Asp Gly Asp Pro Thr
            20                  25                  30

Glu Gly Val Thr Asp Arg Ser
        35

<210> 313
<211> 21
<212> PRT
<213> Homo sapiens

<400> 313
Lys Ile Leu Gly Asp Met Gln Pro Gly Asp Tyr Phe Asp Leu Val Leu
1               5                   10                  15

Phe Gly Thr Arg Val

            20

<210> 314
<211> 12
<212> PRT
<213> Homo sapiens

<400> 314
Lys Leu Asp Ala Gln Ala Ser Phe Leu Pro Lys Glu
1               5                   10

<210> 315
<211> 18
<212> PRT
<213> Homo sapiens

<400> 315
Arg Gly Phe Ser Leu Asp Glu Ala Thr Asn Leu Asn Gly Gly Leu Leu
1               5                   10                  15

Arg Gly

<210> 316
<211> 25
<212> PRT
<213> Homo sapiens

<400> 316
Lys Thr Ala Phe Ile Ser Asp Phe Ala Val Thr Ala Asp Gly Asn Ala
1               5                   10                  15

Phe Ile Gly Asp Ile Lys Asp Lys Val
            20                  25

<210> 317
<211> 21
<212> PRT
<213> Homo sapiens

<400> 317
Lys Gly Ser Leu Val Gln Ala Ser Glu Ala Asn Leu Gln Ala Ala Gln
1               5                   10                  15

Asp Phe Val Arg Gly
            20

<210> 318
<211> 15
<212> PRT
<213> Homo sapiens

<400> 318
Arg Leu Trp Ala Tyr Leu Thr Ile Gln Glu Leu Leu Ala Lys Arg
1               5                   10                  15

<210> 319
<211> 12
<212> PRT
<213> Homo sapiens

<400> 319
Arg Glu Val Ala Phe Asp Leu Glu Ile Pro Lys Thr
1               5                   10


<210> 320
<211> 30
<212> PRT
<213> Homo sapiens

<400> 320
Arg Ser Ile Leu Gln Met Ser Leu Asp His His Ile Val Thr Pro Leu
1               5                   10                  15


Thr Ser Leu Val Ile Glu Asn Glu Ala Gly Asp Glu Arg Met
            20                  25                  30


<210> 321
<211> 27
<212> PRT
<213> Homo sapiens

<400> 321
Lys Ala Gly Glu Leu Glu Val Phe Asn Gly Tyr Phe Val His Phe Phe
1               5                   10                  15


Ala Pro Asp Asn Leu Asp Pro Ile Pro Lys Asn
            20                  25


<210> 322
<211> 17
<212> PRT
<213> Homo sapiens

<400> 322
Arg Glu Thr Ala Val Asp Gly Glu Leu Val Val Leu Tyr Asp Val Lys
1               5                   10                  15


Arg




<210> 323
<211> 35
<212> PRT
<213> Homo sapiens

<400> 323
Arg Asn Val Gln Phe Asn Tyr Pro His Thr Ser Val Thr Asp Val Thr
1               5                   10                  15

Gln Asn Asn Phe His Asn Tyr Phe Gly Gly Ser Glu Ile Val Val Ala
            20                  25                  30

Gly Lys Phe
        35

<210> 324
<211> 22
<212> PRT
<213> Homo sapiens

<400> 324
Arg Phe Leu His Val Pro Asp Thr Phe Glu Gly His Phe Asp Gly Val
1               5                   10                  15

Pro Val Ile Ser Lys Gly
            20

<210> 325
<211> 11
<212> PRT
<213> Homo sapiens

<400> 325
Lys Tyr Ile Phe His Asn Phe Met Glu Arg Leu
1               5                   10

<210> 326
<211> 35
<212> PRT
<213> Homo sapiens

<400> 326
Arg Ser Phe Ala Ala Gly Ile Gln Ala Leu Gly Gly Thr Asn Ile Asn
1               5                   10                  15

Asp Ala Met Leu Met Ala Val Gln Leu Leu Asp Ser Ser Asn Gln Glu
            20                  25                  30

Glu Arg Leu
        35

<210> 327
<211> 18
<212> PRT
<213> Homo sapiens

<400> 327
Arg Asn Met Glu Gln Phe Gln Val Ser Val Ser Val Ala Pro Asn Ala
1               5                   10                  15

Lys Ile

<210> 328
<211> 12
<212> PRT
<213> Homo sapiens

<400> 328
Arg Val Gln Gly Asn Asp His Ser Ala Thr Arg Glu
1               5                   10


<210> 329
<211> 15
<212> PRT
<213> Homo sapiens

<400> 329
Lys Trp Lys Glu Thr Leu Phe Ser Val Met Pro Gly Leu Lys Met
1               5                   10                  15


<210> 330
<211> 13
<212> PRT
<213> Homo sapiens

<400> 330
Lys Ala Gly Phe Ser Trp Ile Glu Val Thr Phe Lys Asn
1               5                   10


<210> 331
<211> 31
<212> PRT
<213> Homo sapiens

<400> 331
Arg Asp Gln Phe Asn Leu Ile Val Phe Ser Thr Glu Ala Thr Gln Trp
1               5                   10                  15


Arg Pro Ser Leu Val Pro Ala Ser Ala Glu Asn Val Asn Lys Ala
            20                  25                  30


<210> 332
<211> 28
<212> PRT
<213> Homo sapiens

<400> 332
Arg Leu Trp Ala Tyr Leu Thr Ile Gln Gln Leu Leu Glu Gln Thr Val
1               5                   10                  15


Ser Ala Ser Asp Ala Asp Gln Gln Ala Leu Arg Asn
            20                  25


<210> 333
<211> 17
<212> PRT

<213> Homo sapiens

<400> 333
Lys Phe Ser Ile Ser Gly Ser Tyr Val Leu Asp Gln Ile Leu Pro Arg
1               5                   10                  15

Leu

<210> 334
<211> 15
<212> PRT
<213> Homo sapiens

<400> 334
Lys Ala Ala Thr Gly Glu Cys Thr Ala Thr Val Gly Lys Arg Ser
1               5                   10                  15

<210> 335
<211> 13
<212> PRT
<213> Homo sapiens

<400> 335
Lys Glu Asn Phe Leu Phe Leu Thr Pro Asp Cys Lys Ser
1               5                   10

<210> 336
<211> 21
<212> PRT
<213> Homo sapiens

<400> 336
Arg Asp Ile Pro Thr Asn Ser Pro Glu Leu Glu Glu Thr Leu Thr His
1               5                   10                  15

Thr Ile Thr Lys Leu
            20

<210> 337
<211> 19
<212> PRT
<213> Homo sapiens

<400> 337
Lys Ile Tyr Pro Thr Val Asn Cys Gln Pro Leu Gly Met Ile Ser Leu
1               5                   10                  15

Met Lys Arg

<210> 338
<211> 15
<212> PRT
<213> Homo sapiens

<400> 338
Arg Ile Gly Glu Ile Lys Glu Glu Thr Thr Ser His Leu Arg Ser
1               5                   10                  15

<210> 339
<211> 17
<212> PRT
<213> Homo sapiens

<400> 339
Lys Tyr Asn Ser Gln Asn Gln Ser Asn Asn Gln Phe Val Leu Tyr Arg
1               5                   10                  15

Ile

<210> 340
<211> 13
<212> PRT
<213> Homo sapiens

<400> 340
Lys Thr Val Gly Ser Asp Thr Phe Tyr Ser Phe Lys Tyr
1               5                   10

<210> 341
<211> 23
<212> PRT
<213> Homo sapiens

<400> 341
Arg Asp Gly Phe Asp Ile Ser Gly Asn Pro Trp Ile Cys Asp Gln Asn
1               5                   10                  15

Leu Ser Asp Leu Tyr Arg Trp
            20

<210> 342
<211> 25
<212> PRT
<213> Homo sapiens

<400> 342
Arg Asn Ala Leu Thr Gly Leu Pro Pro Gly Leu Phe Gln Ala Ser Ala
1               5                   10                  15

Thr Leu Asp Thr Leu Val Leu Lys Glu
            20                  25

<210> 343
<211> 13
<212> PRT
<213> Homo sapiens

<400> 343
Lys Ala Leu Gly His Leu Asp Leu Ser Gly Asn Arg Leu
1               5                   10

<210> 344
<211> 12
<212> PRT
<213> Homo sapiens

<400> 344
Arg Val Ala Ala Gly Ala Phe Gln Gly Leu Arg Gln
1               5                   10

<210> 345
<211> 20
<212> PRT
<213> Homo sapiens

<400> 345
Arg Ser Pro Val Thr Leu Leu Ala Ala Val Met Ser Leu Pro Glu Glu
1               5                   10                  15

His Asn Lys Met
            20

<210> 346
<211> 16
<212> PRT
<213> Homo sapiens

<400> 346
Lys Ser Leu Glu Tyr Leu Asp Leu Ser Phe Asn Gln Ile Ala Arg Leu
1               5                   10                  15

<210> 347
<211> 20
<212> PRT
<213> Homo sapiens

<400> 347
Arg Leu Thr Val Gly Ala Ala Gln Val Pro Ala Gln Leu Leu Val Gly
1               5                   10                  15

Ala Leu Arg Val
            20

<210> 348
<211> 34
<212> PRT
<213> Homo sapiens

<400> 348
Arg Glu Gly Lys Glu Tyr Gly Val Val Leu Ala Pro Asp Gly Ser Thr
1               5                   10                  15

Val Ala Val Glu Pro Leu Leu Ala Gly Leu Glu Ala Gly Leu Gln Gly
            20                  25                  30

Arg Arg

<210> 349
<211> 17
<212> PRT
<213> Homo sapiens

<400> 349
Lys Glu Phe Thr Glu Ala Phe Leu Gly Cys Pro Ala Ile His Pro Arg
1               5                   10                  15

Cys

<210> 350
<211> 15
<212> PRT
<213> Homo sapiens

<400> 350
Arg Thr Asp Cys Pro Gly Asp Ala Leu Phe Asp Leu Leu Arg Thr
1               5                   10                  15

<210> 351
<211> 17
<212> PRT
<213> Homo sapiens

<400> 351
Lys Val Ala Phe Leu Thr Val Thr Leu His Gln Gly Gly Ala Thr Arg
1               5                   10                  15

Met

<210> 352
<211> 30
<212> PRT
<213> Homo sapiens

<400> 352
Arg Ala Leu Tyr Tyr Asp Leu Ile Ser Ser Pro Asp Ile His Gly Thr
1               5                   10                  15

Tyr Lys Glu Leu Leu Asp Thr Val Thr Ala Pro Gln Lys Asn
            20                  25                  30

<210> 353
<211> 12
<212> PRT

<213> Homo sapiens

<400> 353
Lys Thr Val Gln Ala Val Leu Thr Val Pro Lys Leu
1               5                   10


<210> 354
<211> 17
<212> PRT
<213> Homo sapiens

<400> 354
Arg Leu Asp Leu Gln Glu Ile Asn Asn Trp Val Gln Ala Gln Met Lys
1               5                   10                  15


Gly


<210> 355
<211> 15
<212> PRT
<213> Homo sapiens

<400> 355
Arg Leu Val Gly Ile Thr Ser Trp Gly Glu Gly Cys Ala Arg Arg
1               5                   10                  15


<210> 356
<211> 23
<212> PRT
<213> Homo sapiens

<400> 356
Lys Thr Asn Leu Glu Ser Ile Leu Ser Tyr Pro Lys Asp Phe Thr Cys
1               5                   10                  15


Val His Gln Ala Leu Lys Gly
            20


<210> 357
<211> 14
<212> PRT
<213> Homo sapiens

<400> 357
Arg Leu Val Leu Leu Asn Ala Ile Tyr Leu Ser Ala Lys Trp
1               5                   10


<210> 358
<211> 12
<212> PRT
<213> Homo sapiens

<400> 358
Lys Phe Gln Pro Thr Leu Leu Thr Leu Pro Arg Ile
1               5                   10

<210> 359
<211> 13
<212> PRT
<213> Homo sapiens

<400> 359
Arg His Ser Ile Phe Thr Pro Glu Thr Asn Pro Arg Ala
1               5                   10

<210> 360
<211> 12
<212> PRT
<213> Homo sapiens

<400> 360
Arg Phe Val Thr Trp Ile Glu Gly Val Met Arg Asn
1               5                   10

<210> 361
<211> 10
<212> PRT
<213> Homo sapiens

<400> 361
Arg Gly Gln Ile Val Phe Met Asn Arg Glu
1               5                   10

<210> 362
<211> 14
<212> PRT
<213> Homo sapiens

<400> 362
Arg Asp Ser Ser Thr Trp Leu Thr Ala Phe Val Leu Lys Val
1               5                   10

<210> 363
<211> 18
<212> PRT
<213> Homo sapiens

<400> 363
Arg Tyr Leu Asp Lys Thr Glu Gln Trp Ser Thr Leu Pro Pro Glu Thr
1               5                   10                  15

Lys Asp

<210> 364
<211> 14
<212> PRT
<213> Homo sapiens

<400> 364
Arg Asp Phe Ala Leu Leu Ser Leu Gln Val Pro Leu Lys Asp

1               5                   10


<210> 365
<211> 25
<212> PRT
<213> Homo sapiens

<400> 365
Arg Thr Leu Glu Ile Pro Gly Asn Ser Asp Pro Asn Met Ile Pro Asp
1               5                   10                  15


Gly Asp Phe Asn Ser Tyr Val Arg Val
            20                  25


<210> 366
<211> 15
<212> PRT
<213> Homo sapiens

<400> 366
Arg Glu Met Ser Gly Ser Pro Ala Ser Gly Ile Pro Val Lys Val
1               5                   10                  15


<210> 367
<211> 28
<212> PRT
<213> Homo sapiens

<400> 367
Lys Leu His Leu Glu Thr Asp Ser Leu Ala Leu Val Ala Leu Gly Ala
1               5                   10                  15


Leu Asp Thr Ala Leu Tyr Ala Ala Gly Ser Lys Ser
            20                  25


<210> 368
<211> 20
<212> PRT
<213> Homo sapiens

<400> 368
Arg Gly Cys Gly Glu Gln Thr Met Ile Tyr Leu Ala Pro Thr Leu Ala
1               5                   10                  15


Ala Ser Arg Tyr
            20


<210> 369
<211> 15
<212> PRT
<213> Homo sapiens

<400> 369
Arg Asn Glu Leu Ile Pro Leu Ile Tyr Leu Glu Asn Pro Arg Arg
1               5                   10                  15

<210> 370
<211> 24
<212> PRT
<213> Homo sapiens

<400> 370
Lys Leu Glu Ala Gly Ile Asn Gln Leu Ser Phe Pro Leu Ser Ser Glu
1               5                   10                  15

Pro Ile Gln Gly Ser Tyr Arg Val
            20

<210> 371
<211> 15
<212> PRT
<213> Homo sapiens

<400> 371
Arg Asn Gln Gly Asn Thr Trp Leu Thr Ala Phe Val Leu Lys Thr
1               5                   10                  15

<210> 372
<211> 19
<212> PRT
<213> Homo sapiens

<400> 372
Arg Ala Phe Gln Pro Phe Phe Val Glu Leu Thr Met Pro Tyr Ser Val
1               5                   10                  15

Ile Arg Gly

<210> 373
<211> 16
<212> PRT
<213> Homo sapiens

<400> 373
Arg Ile Gln His Pro Phe Thr Val Glu Glu Phe Val Leu Pro Lys Phe
1               5                   10                  15

<210> 374
<211> 14
<212> PRT
<213> Homo sapiens

<400> 374
Lys Ala Leu Leu Ala Tyr Ala Phe Ser Leu Leu Gly Lys Gln
1               5                   10

<210> 375
<211> 11
<212> PRT

<213> Homo sapiens

<400> 375
Arg Thr Leu Phe Leu Leu Gly Val Thr Lys Tyr
1               5                   10


<210> 376
<211> 13
<212> PRT
<213> Homo sapiens

<400> 376
Arg Thr Val Ala Ala Cys Asn Leu Pro Ile Val Arg Gly
1               5                   10


<210> 377
<211> 16
<212> PRT
<213> Homo sapiens

<400> 377
Lys Trp Tyr Asn Leu Ala Ile Gly Ser Thr Cys Pro Trp Leu Lys Lys
1               5                   10                  15


<210> 378
<211> 12
<212> PRT
<213> Homo sapiens

<400> 378
Lys Leu Ile Leu Val Asp Tyr Ile Leu Phe Lys Gly
1               5                   10


<210> 379
<211> 18
<212> PRT
<213> Homo sapiens

<400> 379
Arg Lys Ser Pro Gln Glu Leu Leu Cys Gly Ala Ser Leu Ile Ser Asp
1               5                   10                  15


Arg Trp




<210> 380
<211> 15
<212> PRT
<213> Homo sapiens

<400> 380
Arg Thr Ala Thr Ser Glu Tyr Gln Thr Phe Phe Asn Pro Arg Thr
1               5                   10                  15


<210> 381
<211> 19

<212> PRT
<213> Homo sapiens

<400> 381
Arg Val Thr Gly Trp Gly Asn Leu Lys Glu Thr Trp Thr Ala Asn Val
1               5                   10                  15

Gly Lys Gly

<210> 382
<211> 22
<212> PRT
<213> Homo sapiens

<400> 382
Arg Ile Val Glu Gly Ser Asp Ala Glu Ile Gly Met Ser Pro Trp Gln
1               5                   10                  15

Val Met Leu Phe Arg Lys
            20

<210> 383
<211> 18
<212> PRT
<213> Homo sapiens

<400> 383
Lys His Gln Asp Phe Asn Ser Ala Val Gln Leu Val Glu Asn Phe Cys
1               5                   10                  15

Arg Asn

<210> 384
<211> 23
<212> PRT
<213> Homo sapiens

<400> 384
Arg Arg Gln Glu Cys Ser Ile Pro Val Cys Gly Gln Asp Gln Val Thr
1               5                   10                  15

Val Ala Met Thr Pro Arg Ser
            20

<210> 385
<211> 21
<212> PRT
<213> Homo sapiens

<400> 385
Arg Leu Ala Val Thr Thr His Gly Leu Pro Cys Leu Ala Trp Ala Ser
1               5                   10                  15

Ala Gln Ala Lys Ala
            20

<210> 386
<211> 22
<212> PRT
<213> Homo sapiens

<400> 386
Lys Gly Gln Pro Ser Val Leu Gln Val Val Asn Leu Pro Ile Val Glu
1               5                   10                  15

Arg Pro Val Cys Lys Asp
            20

<210> 387
<211> 20
<212> PRT
<213> Homo sapiens

<400> 387
Arg Leu Leu Asn Leu Asp Gly Thr Cys Ala Asp Ser Tyr Ser Phe Val
1               5                   10                  15

Phe Ser Arg Asp
            20

<210> 388
<211> 28
<212> PRT
<213> Homo sapiens

<400> 388
Arg Leu Phe Leu Gly Ala Leu Pro Gly Glu Asp Ser Ser Thr Ser Phe
1               5                   10                  15

Cys Leu Asn Gly Leu Trp Ala Gln Gly Gln Arg Leu
            20                  25

<210> 389
<211> 27
<212> PRT
<213> Homo sapiens

<400> 389
Arg Thr Trp Asp Pro Glu Gly Val Ile Phe Tyr Gly Asp Thr Asn Pro
1               5                   10                  15

Lys Asp Asp Trp Phe Met Leu Gly Leu Arg Asp
            20                  25

<210> 390
<211> 16

<212> PRT
<213> Homo sapiens

<400> 390
Arg Ile Ala Leu Gly Gly Leu Leu Phe Pro Ala Ser Asn Leu Arg Leu
1               5                   10                  15


<210> 391
<211> 26
<212> PRT
<213> Homo sapiens

<400> 391
Lys Val Val Leu Ser Ser Gly Ser Gly Pro Gly Leu Asp Leu Pro Leu
1               5                   10                  15


Val Leu Gly Leu Pro Leu Gln Leu Lys Leu
            20                  25


<210> 392
<211> 10
<212> PRT
<213> Homo sapiens

<400> 392
Lys Ala Val Leu His Ile Gly Glu Lys Gly
1               5                   10


<210> 393
<211> 11
<212> PRT
<213> Homo sapiens

<400> 393
Lys Gly Trp Val Asp Leu Phe Val Pro Lys Phe
1               5                   10


<210> 394
<211> 17
<212> PRT
<213> Homo sapiens

<400> 394
Lys Phe Ser Ile Ser Ala Thr Tyr Asp Leu Gly Ala Thr Leu Leu Lys
1               5                   10                  15


Met



<210> 395
<211> 9
<212> PRT
<213> Homo sapiens

<400> 395
Arg Ser Ile Leu Phe Leu Gly Lys Val

1                   5


<210> 396
<211> 14
<212> PRT
<213> Homo sapiens

<400> 396
Arg Leu Thr Leu Leu Ala Pro Leu Asn Ser Val Phe Lys Asp
1               5                   10


<210> 397
<211> 29
<212> PRT
<213> Homo sapiens

<400> 397
Lys Glu Tyr Ala Asn Gln Phe Met Trp Glu Tyr Ser Thr Asn Tyr Gly
1               5                   10                  15


Gln Ala Pro Leu Ser Leu Leu Val Ser Tyr Thr Lys Ser
            20                  25


<210> 398
<211> 10
<212> PRT
<213> Homo sapiens

<400> 398
Lys Glu Leu Pro Glu His Thr Val Lys Leu
1               5                   10


<210> 399
<211> 13
<212> PRT
<213> Homo sapiens

<400> 399
Arg Arg Thr His Leu Pro Glu Val Phe Leu Ser Lys Val
1               5                   10


<210> 400
<211> 31
<212> PRT
<213> Homo sapiens

<400> 400
Lys Thr Ala Met Asp Val Phe Val Cys Thr Tyr Phe Met Pro Ala Ala
1               5                   10                  15


Gln Leu Pro Glu Leu Pro Asp Val Glu Leu Pro Thr Asn Lys Asp
            20                  25                  30


<210> 401
<211> 13

<212> PRT
<213> Homo sapiens

<400> 401
Lys Leu Pro Asp Ala Thr Pro Thr Glu Leu Ala Lys Leu
1               5                   10


<210> 402
<211> 28
<212> PRT
<213> Homo sapiens

<400> 402
Lys Glu Leu Ser Ser Phe Ile Asp Lys Gly Gln Glu Leu Cys Ala Asp
1               5                   10                  15


Tyr Ser Glu Asn Thr Phe Thr Glu Tyr Lys Lys Lys
            20                  25


<210> 403
<211> 15
<212> PRT
<213> Homo sapiens

<400> 403
Lys Glu Asp Phe Thr Ser Leu Ser Leu Val Leu Tyr Ser Arg Lys
1               5                   10                  15


<210> 404
<211> 25
<212> PRT
<213> Homo sapiens

<400> 404
Lys His Gln Pro Gln Glu Phe Pro Thr Tyr Val Glu Pro Thr Asn Asp
1               5                   10                  15


Glu Ile Cys Glu Ala Phe Arg Lys Asp
            20                  25


<210> 405
<211> 24
<212> PRT
<213> Homo sapiens

<400> 405
Lys His Gln Pro Gln Glu Phe Pro Thr Tyr Val Glu Pro Thr Asn Asp
1               5                   10                  15


Glu Ile Cys Glu Ala Phe Arg Lys
            20


<210> 406
<211> 17
<212> PRT

<213> Homo sapiens

<400> 406
Arg Lys Phe Pro Ser Gly Thr Phe Glu Gln Val Ser Gln Leu Val Lys
1               5                   10                  15

Glu

<210> 407
<211> 27
<212> PRT
<213> Homo sapiens

<400> 407
Lys Glu Leu Ser Ser Phe Ile Asp Lys Gly Gln Glu Leu Cys Ala Asp
1               5                   10                  15

Tyr Ser Glu Asn Thr Phe Thr Glu Tyr Lys Lys
            20                  25

<210> 408
<211> 22
<212> PRT
<213> Homo sapiens

<400> 408
Lys Glu Phe Ser His Leu Gly Lys Glu Asp Phe Thr Ser Leu Ser Leu
1               5                   10                  15

Val Leu Tyr Ser Arg Lys
            20

<210> 409
<211> 23
<212> PRT
<213> Homo sapiens

<400> 409
Lys Ser Tyr Leu Ser Met Val Gly Ser Cys Cys Thr Ser Ala Ser Pro
1               5                   10                  15

Thr Val Cys Phe Leu Lys Glu
            20

<210> 410
<211> 16
<212> PRT
<213> Homo sapiens

<400> 410
Arg Ile Tyr Ile Ser Gly Met Ala Pro Arg Pro Ser Leu Ala Lys Lys
1               5                   10                  15

<210> 411
<211> 20
<212> PRT
<213> Homo sapiens

<400> 411
Lys Leu Ile Arg Asp Val Trp Gly Ile Glu Gly Pro Ile Asp Ala Ala
1               5                   10                  15

Phe Thr Arg Ile
            20

<210> 412
<211> 20
<212> PRT
<213> Homo sapiens

<400> 412
Arg Ile Gly Trp Pro Asn Ala Pro Ile Leu Ile Gln Asp Phe Glu Thr
1               5                   10                  15

Leu Pro Arg Glu
            20

<210> 413
<211> 10
<212> PRT
<213> Homo sapiens

<400> 413
Arg Leu Cys Phe Phe Tyr Asn Lys Lys Ser
1               5                   10

<210> 414
<211> 10
<212> PRT
<213> Homo sapiens

<400> 414
Arg Arg Pro Cys Phe Glu Ser Leu Lys Ala
1               5                   10

<210> 415
<211> 8
<212> PRT
<213> Homo sapiens

<400> 415
Arg Ile Val Gln Ile Tyr Lys Asp
1               5

<210> 416
<211> 9
<212> PRT
<213> Homo sapiens

<400> 416
Arg Phe Leu Val Asn Leu Val Lys Leu
1               5

<210> 417
<211> 11
<212> PRT
<213> Homo sapiens

<400> 417
Lys Leu Pro Asn Asn Val Leu Gln Glu Lys Ile
1               5                   10

<210> 418
<211> 21
<212> PRT
<213> Homo sapiens

<400> 418
Arg Leu Tyr Gly Ser Glu Ala Phe Ala Thr Asp Phe Gln Asp Ser Ala
1               5                   10                  15

Ala Ala Lys Lys Leu
            20

<210> 419
<211> 17
<212> PRT
<213> Homo sapiens

<400> 419
Lys Glu Gln Leu Ser Leu Leu Asp Arg Phe Thr Glu Asp Ala Lys Arg
1               5                   10                  15

Leu

<210> 420
<211> 11
<212> PRT
<213> Homo sapiens

<400> 420
Arg Glu Ile Gly Glu Leu Tyr Leu Pro Lys Phe
1               5                   10

<210> 421
<211> 10
<212> PRT
<213> Homo sapiens

<400> 421
Arg Trp Arg Asp Ser Leu Glu Phe Arg Glu
1               5                   10

<210> 422
<211> 21
<212> PRT
<213> Homo sapiens

<400> 422
Lys Arg Leu Tyr Gly Ser Glu Ala Phe Ala Thr Asp Phe Gln Asp Ser
1               5                   10                  15

Ala Ala Ala Lys Lys
            20

<210> 423
<211> 7
<212> PRT
<213> Homo sapiens

<400> 423
Arg Phe Ala Leu Val Arg Glu
1               5

<210> 424
<211> 10
<212> PRT
<213> Homo sapiens

<400> 424
Arg Gly Val Thr Phe Leu Leu Arg Arg Glu
1               5                   10

<210> 425
<211> 12
<212> PRT
<213> Homo sapiens

<400> 425
Arg Arg Gly Glu Lys Glu Leu Leu Val Pro Arg Ser
1               5                   10

<210> 426
<211> 8
<212> PRT
<213> Homo sapiens

<400> 426
Lys Glu Leu Leu Val Pro Arg Ser
1               5

<210> 427
<211> 18
<212> PRT
<213> Homo sapiens

<400> 427
Lys Asn Gly Val Ala Gln Glu Pro Val His Leu Asp Ser Pro Ala Ile
1               5                   10                  15

Lys His

<210> 428
<211> 12
<212> PRT
<213> Homo sapiens

<400> 428
Arg Asn Lys Phe Asp Pro Ser Leu Thr Gln Arg Asp
1               5                   10

<210> 429
<211> 20
<212> PRT
<213> Homo sapiens

<400> 429
Arg Gln Leu Thr Ser Gly Pro Asn Gln Glu Gln Val Ser Pro Leu Thr
1               5                   10                  15

Leu Leu Lys Leu
            20

<210> 430
<211> 11
<212> PRT
<213> Homo sapiens

<400> 430
Arg Phe Met Gln Ala Val Thr Gly Trp Lys Thr
1               5                   10

<210> 431
<211> 16
<212> PRT
<213> Homo sapiens

<400> 431
Lys Pro Lys Asp Pro Thr Phe Ile Pro Ala Pro Ile Gln Ala Lys Thr
1               5                   10                  15

<210> 432
<211> 15
<212> PRT
<213> Homo sapiens

<400> 432
Arg Ser Leu Asp Phe Thr Glu Leu Asp Val Ala Ala Glu Lys Ile
1               5                   10                  15

<210> 433
<211> 8
<212> PRT
<213> Homo sapiens

<400> 433
Arg Lys Asn Leu Val Pro Arg Asp
1               5


<210> 434
<211> 9
<212> PRT
<213> Homo sapiens

<400> 434
Arg Arg Val Trp Glu Leu Ser Lys Ala
1               5


<210> 435
<211> 14
<212> PRT
<213> Homo sapiens

<400> 435
Lys Val Lys Ile Asp Gln Thr Val Glu Glu Leu Arg Arg Ser
1               5                   10


<210> 436
<211> 16
<212> PRT
<213> Homo sapiens

<400> 436
Lys Asp Leu Arg Asp Lys Val Asn Ser Phe Phe Ser Thr Phe Lys Glu
1               5                   10                  15


<210> 437
<211> 13
<212> PRT
<213> Homo sapiens

<400> 437
Lys Leu Val Pro Phe Ala Thr Glu Leu His Glu Arg Leu
1               5                   10


<210> 438
<211> 13
<212> PRT
<213> Homo sapiens

<400> 438
Arg Arg Val Glu Pro Tyr Gly Glu Asn Phe Asn Lys Ala
1               5                   10


<210> 439
<211> 11
<212> PRT
<213> Homo sapiens

<400> 439
Lys Val Asn Ser Phe Phe Ser Thr Phe Lys Glu

1                   5                   10

<210> 440
<211> 17
<212> PRT
<213> Homo sapiens

<400> 440
Lys Ala Val Ser Met Pro Ser Phe Ser Ile Leu Gly Ser Asp Val Arg
1               5                   10                  15

Val

<210> 441
<211> 23
<212> PRT
<213> Homo sapiens

<400> 441
Lys Val Asn Trp Glu Glu Glu Ala Ala Ser Gly Leu Leu Thr Ser Leu
1               5                   10                  15

Lys Asp Asn Val Pro Lys Ala
            20

<210> 442
<211> 13
<212> PRT
<213> Homo sapiens

<400> 442
Arg Asp Leu Lys Val Glu Asp Ile Pro Leu Ala Arg Ile
1               5                   10

<210> 443
<211> 13
<212> PRT
<213> Homo sapiens

<400> 443
Lys Met Arg Glu Trp Phe Ser Glu Thr Phe Gln Lys Val
1               5                   10

<210> 444
<211> 25
<212> PRT
<213> Homo sapiens

<400> 444
Lys Ser Thr Ala Ala Met Ser Thr Tyr Thr Gly Ile Phe Thr Asp Gln
1               5                   10                  15

Val Leu Ser Val Leu Lys Gly Glu Glu
            20                  25

<210> 445
<211> 13
<212> PRT
<213> Homo sapiens

<400> 445
Arg Ala Lys Leu Glu Glu Gln Ala Gln Gln Ile Arg Leu
1               5                   10


<210> 446
<211> 8
<212> PRT
<213> Homo sapiens

<400> 446
Arg Phe Trp Asp Tyr Leu Arg Trp
1               5


<210> 447
<211> 16
<212> PRT
<213> Homo sapiens

<400> 447
Arg Leu Lys Ser Trp Phe Glu Pro Leu Val Glu Asp Met Gln Arg Gln
1               5                   10                  15


<210> 448
<211> 8
<212> PRT
<213> Homo sapiens

<400> 448
Lys Val Ser Phe Phe Cys Lys Asn
1               5


<210> 449
<211> 16
<212> PRT
<213> Homo sapiens

<400> 449
Arg Val Cys Pro Phe Ala Gly Ile Leu Glu Asn Gly Ala Val Arg Tyr
1               5                   10                  15


<210> 450
<211> 24
<212> PRT
<213> Homo sapiens

<400> 450
Lys Ser Asn Phe Leu Asn Cys Tyr Val Ser Gly Phe His Pro Ser Asp
1               5                   10                  15


Ile Glu Val Asp Leu Leu Lys Asn

            20

<210> 451
<211> 15
<212> PRT
<213> Homo sapiens

<400> 451
Arg Leu Ser Ser Gly Leu Val Thr Ala Ala Leu Tyr Gly Arg Leu
1               5                   10                  15

<210> 452
<211> 9
<212> PRT
<213> Homo sapiens

<400> 452
Lys Ile Ala Trp His Val Ile Arg Asn
1               5

<210> 453
<211> 18
<212> PRT
<213> Homo sapiens

<400> 453
Lys Leu Ser Asn Asn Ala Leu Ser Gly Leu Pro Gln Gly Val Phe Gly
1               5                   10                  15

Lys Leu

<210> 454
<211> 16
<212> PRT
<213> Homo sapiens

<400> 454
Arg Asp His Leu Gly Phe Gln Val Thr Trp Pro Asp Glu Ser Lys Ala
1               5                   10                  15

<210> 455
<211> 8
<212> PRT
<213> Homo sapiens

<400> 455
Lys Val Tyr Val His Leu Lys Asn
1               5

<210> 456
<211> 21
<212> PRT
<213> Homo sapiens

<400> 456

Lys Leu Ile Ser Val Asp Thr Glu His Ser Asn Ile Tyr Leu Gln Asn
1               5                   10                  15

Gly Pro Asp Arg Ile
            20

<210> 457
<211> 28
<212> PRT
<213> Homo sapiens

<400> 457
Lys Asp Val Asp Lys Glu Phe Tyr Leu Phe Pro Thr Val Phe Asp Glu
1               5                   10                  15

Asn Glu Ser Leu Leu Leu Glu Asp Asn Ile Arg Met
            20                  25

<210> 458
<211> 13
<212> PRT
<213> Homo sapiens

<400> 458
Lys Asp Ile Phe Thr Gly Leu Ile Gly Pro Met Lys Ile
1               5                   10

<210> 459
<211> 25
<212> PRT
<213> Homo sapiens

<400> 459
Arg Ser Val Pro Pro Ser Ala Ser His Val Ala Pro Thr Glu Thr Phe
1               5                   10                  15

Thr Tyr Glu Trp Thr Val Pro Lys Glu
            20                  25

<210> 460
<211> 15
<212> PRT
<213> Homo sapiens

<400> 460
Lys Val Asn Lys Asp Asp Glu Glu Phe Ile Glu Ser Asn Lys Met
1               5                   10                  15

<210> 461
<211> 9
<212> PRT
<213> Homo sapiens

<400> 461
Arg Lys Tyr Asn Glu Leu Leu Lys Ser

1                   5


<210> 462
<211> 20
<212> PRT
<213> Homo sapiens

<400> 462
Arg Thr Thr Leu Ser Gly Ala Pro Cys Gln Pro Trp Ala Ser Glu Ala
1               5                   10                  15


Thr Tyr Arg Asn
            20


<210> 463
<211> 23
<212> PRT
<213> Homo sapiens

<400> 463
Lys Gly His Ile Tyr Gln Gly Ser Glu Ala Asp Ser Val Phe Ser Gly
1               5                   10                  15


Phe Leu Ile Phe Pro Ser Ala
            20


<210> 464
<211> 11
<212> PRT
<213> Homo sapiens

<400> 464
Lys Phe Gln Ser Val Phe Thr Val Thr Arg Gln
1               5                   10


<210> 465
<211> 14
<212> PRT
<213> Homo sapiens

<400> 465
Arg Trp Ile Leu Thr Ala Ala His Thr Leu Tyr Pro Lys Glu
1               5                   10


<210> 466
<211> 12
<212> PRT
<213> Homo sapiens

<400> 466
Lys Val Leu Asn Tyr Val Asp Trp Ile Lys Lys Glu
1               5                   10


<210> 467
<211> 10

<212> PRT
<213> Homo sapiens

<400> 467
Arg Leu Pro Val Ala Pro Leu Arg Lys Cys
1               5                   10


<210> 468
<211> 17
<212> PRT
<213> Homo sapiens

<400> 468
Arg Pro Ile Cys Leu Pro Cys Thr Met Glu Ala Asn Leu Ala Leu Arg
1               5                   10                  15


Arg




<210> 469
<211> 13
<212> PRT
<213> Homo sapiens

<400> 469
Arg Gln His Leu Gly Asp Val Leu Asn Phe Leu Pro Leu
1               5                   10


<210> 470
<211> 13
<212> PRT
<213> Homo sapiens

<400> 470
Lys Leu Gly Gln Tyr Ala Ser Pro Thr Ala Lys Arg Cys
1               5                   10


<210> 471
<211> 15
<212> PRT
<213> Homo sapiens

<400> 471
Lys Glu Phe Pro Tyr Arg Ile Pro Leu Asp Leu Val Pro Lys Thr
1               5                   10                  15


<210> 472
<211> 9
<212> PRT
<213> Homo sapiens

<400> 472
Arg Val Phe Gln Phe Leu Glu Lys Ser
1               5


<210> 473

<211> 18
<212> PRT
<213> Homo sapiens

<400> 473
Arg Met Val Glu Thr Thr Ala Tyr Ala Leu Leu Thr Ser Leu Asn Leu
1               5                   10                  15

Lys Asp

<210> 474
<211> 17
<212> PRT
<213> Homo sapiens

<400> 474
Arg Glu Asn Ser Leu Tyr Leu Thr Ala Phe Thr Val Ile Gly Ile Arg
1               5                   10                  15

Lys

<210> 475
<211> 20
<212> PRT
<213> Homo sapiens

<400> 475
Lys Tyr Asn Pro Val Val Ile Asp Phe Glu Met Gln Pro Ile His Glu
1               5                   10                  15

Val Leu Arg His
            20

<210> 476
<211> 17
<212> PRT
<213> Homo sapiens

<400> 476
Lys Ile Pro Gly Ile Phe Glu Leu Gly Ile Ser Ser Gln Ser Asp Arg
1               5                   10                  15

Gly

<210> 477
<211> 14
<212> PRT
<213> Homo sapiens

<400> 477
Arg Arg Pro Ala Ser Pro Ile Ser Thr Ile Gln Pro Lys Ala
1               5                   10

<210> 478
<211> 10
<212> PRT
<213> Homo sapiens

<400> 478
Arg Phe Leu Gln Glu Gln Gly His Arg Ala
1               5                   10


<210> 479
<211> 10
<212> PRT
<213> Homo sapiens

<400> 479
Lys Val Ser Val Gly Gly Glu Lys Arg Asp
1               5                   10


<210> 480
<211> 10
<212> PRT
<213> Homo sapiens

<400> 480
Lys Cys Leu Val Asn Leu Ile Glu Lys Val
1               5                   10


<210> 481
<211> 11
<212> PRT
<213> Homo sapiens

<400> 481
Lys Lys Asp Asn Glu Gln His Val Phe Lys Val
1               5                   10


<210> 482
<211> 10
<212> PRT
<213> Homo sapiens

<400> 482
Lys Ile Ser Val Ile Arg Pro Ser Lys Gly
1               5                   10


<210> 483
<211> 11
<212> PRT
<213> Homo sapiens

<400> 483
Lys Lys Cys Leu Val Asn Leu Ile Glu Lys Val
1               5                   10


<210> 484

<211> 23
<212> PRT
<213> Homo sapiens

<400> 484
Arg Leu Pro Pro Thr Thr Thr Cys Gln Gln Gln Lys Glu Glu Leu Leu
1               5                   10                  15

Pro Ala Gln Asp Ile Lys Ala
            20

<210> 485
<211> 10
<212> PRT
<213> Homo sapiens

<400> 485
Lys Leu Gln Asp Glu Asp Leu Gly Phe Leu
1               5                   10

<210> 486
<211> 13
<212> PRT
<213> Homo sapiens

<400> 486
Lys Ser Cys Asp Ile Pro Val Phe Met Asn Ala Arg Thr
1               5                   10

<210> 487
<211> 12
<212> PRT
<213> Homo sapiens

<400> 487
Lys His Gly Gly Leu Tyr His Glu Asn Met Arg Arg
1               5                   10

<210> 488
<211> 10
<212> PRT
<213> Homo sapiens

<400> 488
Lys Ile Ile Tyr Lys Glu Asn Glu Arg Phe
1               5                   10

<210> 489
<211> 16
<212> PRT
<213> Homo sapiens

<400> 489
Lys Arg Ala Gln Leu Gly Asp Leu Pro Trp Gln Val Ala Ile Lys Asp
1               5                   10                  15

<210> 490
<211> 8
<212> PRT
<213> Homo sapiens

<400> 490
Lys Ile Ser Asn Leu Leu Lys Phe
1               5


<210> 491
<211> 16
<212> PRT
<213> Homo sapiens

<400> 491
Arg Arg Tyr Ala Arg Thr Glu Gly Asn Cys Thr Ala Leu Thr Arg Gly
1               5                   10                  15


<210> 492
<211> 19
<212> PRT
<213> Homo sapiens

<400> 492
Arg Val Gln Leu Gly Pro Tyr Gln Pro Gly Arg Pro Ala Ala Cys Asp
1               5                   10                  15


Leu Arg Glu



<210> 493
<211> 20
<212> PRT
<213> Homo sapiens

<400> 493
Arg Val Pro Glu Ala Arg Pro Asn Ser Met Val Val Glu His Pro Glu
1               5                   10                  15


Phe Leu Lys Ala
            20


<210> 494
<211> 13
<212> PRT
<213> Homo sapiens

<400> 494
Lys Ala Gly Lys Glu Pro Gly Leu Gln Ile Trp Arg Val
1               5                   10


<210> 495
<211> 22
<212> PRT
<213> Homo sapiens

<400> 495
Lys Val Trp Ser Glu Val Asn Gln Ala Val Leu Asp Tyr Glu Asn Arg
1               5                   10                  15

Glu Ser Thr Pro Lys Leu
            20

<210> 496
<211> 18
<212> PRT
<213> Homo sapiens

<400> 496
Arg Met Leu Trp Ala Leu Leu Ser Gly Pro Gly Arg Arg Gly Ser Thr
1               5                   10                  15

Arg Gly

<210> 497
<211> 8
<212> PRT
<213> Homo sapiens

<400> 497
Arg Glu Leu Ile Ser Glu Arg Trp
1               5

<210> 498
<211> 14
<212> PRT
<213> Homo sapiens

<400> 498
Arg Asp Val Arg Asp Tyr Phe Met Pro Cys Pro Gly Arg Gly
1               5                   10

<210> 499
<211> 14
<212> PRT
<213> Homo sapiens

<400> 499
Lys Gly Asp Lys Val Trp Val Tyr Pro Pro Glu Lys Lys Glu
1               5                   10

<210> 500
<211> 13
<212> PRT
<213> Homo sapiens

<400> 500
Arg Tyr Tyr Cys Phe Gln Gly Asn Gln Phe Leu Arg Phe
1               5                   10

<210> 501
<211> 10
<212> PRT
<213> Homo sapiens

<400> 501
Arg Arg Leu Trp Trp Leu Asp Leu Lys Ser
1               5                   10


<210> 502
<211> 9
<212> PRT
<213> Homo sapiens

<400> 502
Arg Leu Asn Ile Leu Asn Ala Lys Phe
1               5


<210> 503
<211> 9
<212> PRT
<213> Homo sapiens

<400> 503
Arg Asn Phe Gly Tyr Thr Leu Arg Ser
1               5


<210> 504
<211> 17
<212> PRT
<213> Homo sapiens

<400> 504
Lys Leu Leu Pro Pro Pro Pro Ile Met Ser Ala Arg Val Leu Pro Arg
1               5                   10                  15


Pro




<210> 505
<211> 12
<212> PRT
<213> Homo sapiens

<400> 505
Lys Arg Pro Gly Val Tyr Thr Gln Val Thr Lys Phe
1               5                   10


<210> 506
<211> 8
<212> PRT
<213> Homo sapiens

<400> 506
Lys Phe Leu Asn Trp Ile Lys Ala
1               5

<210> 507
<211> 23
<212> PRT
<213> Homo sapiens

<400> 507
Met Glu Cys Ala Leu Asp Ala Gln Ser Leu Ile Ser Ile Ser Leu Arg
1               5                   10                  15

Lys Ile His Ser Ser Arg Thr
            20

<210> 508
<211> 23
<212> PRT
<213> Homo sapiens

<400> 508
Lys Ala Gly Ala Asp Thr His Gly Arg Leu Leu Gln Gly Asn Ile Cys
1               5                   10                  15

Asn Asp Ala Val Thr Lys Ala
            20

<210> 509
<211> 11
<212> PRT
<213> Homo sapiens

<400> 509
Lys Ala Asn Val Phe Val Gln Leu Pro Arg Leu
1               5                   10

<210> 510
<211> 11
<212> PRT
<213> Homo sapiens

<400> 510
Lys Glu Leu Ala Ala Gln Thr Ile Lys Lys Ser
1               5                   10

<210> 511
<211> 15
<212> PRT
<213> Homo sapiens

<400> 511
Lys Val Thr Phe Gln Leu Thr Tyr Glu Glu Val Leu Lys Arg Asn
1               5                   10                  15

<210> 512
<211> 18
<212> PRT
<213> Homo sapiens

<400> 512
Arg Thr Met Glu Gln Phe Thr Ile His Leu Thr Val Asn Pro Gln Ser
1               5                   10                  15

Lys Val

<210> 513
<211> 20
<212> PRT
<213> Homo sapiens

<400> 513
Arg Phe Ala His Tyr Val Val Thr Ser Gln Val Val Asn Thr Ala Asn
1               5                   10                  15

Glu Ala Arg Glu
            20

<210> 514
<211> 22
<212> PRT
<213> Homo sapiens

<400> 514
Arg Ser Ser Ala Leu Asp Met Glu Asn Phe Arg Thr Glu Val Asn Val
1               5                   10                  15

Leu Pro Gly Ala Lys Val
            20

<210> 515
<211> 11
<212> PRT
<213> Homo sapiens

<400> 515
Lys Met Lys Gln Thr Val Glu Ala Met Lys Thr
1               5                   10

<210> 516
<211> 9
<212> PRT
<213> Homo sapiens

<400> 516
Arg Ile Tyr Leu Gln Pro Gly Arg Leu
1               5

<210> 517
<211> 17
<212> PRT
<213> Homo sapiens

<400> 517
Lys His Leu Glu Val Asp Val Trp Val Ile Glu Pro Gln Gly Leu Arg
1               5                   10                  15

Phe

<210> 518
<211> 13
<212> PRT
<213> Homo sapiens

<400> 518
Lys Phe Tyr Asn Gln Val Ser Thr Pro Leu Leu Arg Asn
1               5                   10

<210> 519
<211> 10
<212> PRT
<213> Homo sapiens

<400> 519
Arg Lys Leu Gly Ser Tyr Glu His Arg Ile
1               5                   10

<210> 520
<211> 11
<212> PRT
<213> Homo sapiens

<400> 520
Lys Gly Ser Glu Met Val Val Ala Gly Lys Leu
1               5                   10

<210> 521
<211> 13
<212> PRT
<213> Homo sapiens

<400> 521
Arg Met Asn Phe Arg Pro Gly Val Leu Ser Ser Arg Gln
1               5                   10

<210> 522
<211> 13
<212> PRT
<213> Homo sapiens

<400> 522
Lys Glu Thr Leu Phe Ser Val Met Pro Gly Leu Lys Met
1               5                   10

<210> 523
<211> 12
<212> PRT
<213> Homo sapiens

<400> 523
Arg Phe Lys Pro Thr Leu Ser Gln Gln Gln Lys Ser
1               5                   10

<210> 524
<211> 12
<212> PRT
<213> Homo sapiens

<400> 524
Arg Arg Leu Gly Val Tyr Glu Leu Leu Leu Lys Val
1               5                   10

<210> 525
<211> 35
<212> PRT
<213> Homo sapiens

<400> 525
Arg Asp Thr Asp Arg Phe Ser Ser His Val Gly Gly Thr Leu Gly Gln
1               5                   10                  15

Phe Tyr Gln Glu Val Leu Trp Gly Ser Pro Ala Ala Ser Asp Asp Gly
            20                  25                  30

Arg Arg Thr
        35

<210> 526
<211> 10
<212> PRT
<213> Homo sapiens

<400> 526
Lys Val Arg Pro Gln Gln Leu Val Lys His
1               5                   10

<210> 527
<211> 13
<212> PRT
<213> Homo sapiens

<400> 527
Arg Asn Val His Ser Ala Gly Ala Ala Gly Ser Arg Met
1               5                   10

<210> 528
<211> 11
<212> PRT
<213> Homo sapiens

<400> 528
Arg Leu Gly Phe Thr Asp Leu Phe Ser Lys Trp
1               5                   10

<210> 529
<211> 14
<212> PRT
<213> Homo sapiens

<400> 529
Arg Val Gly Ser Ala Leu Phe Leu Ser His Asn Leu Lys Phe
1               5                   10


<210> 530
<211> 10
<212> PRT
<213> Homo sapiens

<400> 530
Arg Val Gln Val Val Ala Gly Lys Lys Tyr
1               5                   10


<210> 531
<211> 15
<212> PRT
<213> Homo sapiens

<400> 531
Arg Leu His Leu Glu Gly Asn Lys Leu Gln Val Leu Gly Lys Asp
1               5                   10                  15


<210> 532
<211> 10
<212> PRT
<213> Homo sapiens

<400> 532
Arg Phe Asn Ala Leu Gln Tyr Leu Arg Leu
1               5                   10


<210> 533
<211> 21
<212> PRT
<213> Homo sapiens

<400> 533
Arg Ile Val Phe Glu Asn Pro Asp Pro Ser Asp Gly Phe Val Leu Ile
1               5                   10                  15


Pro Asp Leu Lys Trp
            20


<210> 534
<211> 14
<212> PRT
<213> Homo sapiens

<400> 534
Lys Ile Leu Glu Ile Glu Asp Leu Phe Ser Ser Leu Lys His
1               5                   10

<210> 535
<211> 8
<212> PRT
<213> Homo sapiens

<400> 535
Lys Trp Phe Asp Tyr Leu Arg Glu
1               5


<210> 536
<211> 34
<212> PRT
<213> Homo sapiens

<400> 536
Arg Cys Glu Leu Gln Ile Arg Gly Leu Ala Val Glu Asp Thr Gly Glu
1               5                   10                  15


Tyr Leu Cys Val Cys Gly Gln Glu Arg Thr Ser Ala Thr Leu Thr Val
            20                  25                  30


Arg Ala




<210> 537
<211> 14
<212> PRT
<213> Homo sapiens

<400> 537
Lys Asp Met Lys Gln Gly Leu Ala Lys Leu Met His Arg Met
1               5                   10


<210> 538
<211> 19
<212> PRT
<213> Homo sapiens

<400> 538
Lys Gly Ile Val Ala Ala Phe Tyr Ser Gly Pro Ser Leu Ser Asp Lys
1               5                   10                  15


Glu Lys Leu




<210> 539
<211> 13
<212> PRT
<213> Homo sapiens

<400> 539
Arg Thr Leu Leu Leu Val Gly Ser Pro Thr Trp Lys Asn
1               5                   10

<210> 540
<211> 16
<212> PRT
<213> Homo sapiens

<400> 540
Arg Trp Tyr Val Pro Val Lys Asp Leu Leu Gly Ile Tyr Glu Lys Leu
1               5                   10                  15


<210> 541
<211> 32
<212> PRT
<213> Homo sapiens

<400> 541
Arg Ser Ser Thr Ser Pro Thr Thr Asn Val Leu Leu Ser Pro Leu Ser
1               5                   10                  15


Val Ala Thr Ala Leu Ser Ala Leu Ser Leu Gly Ala Glu Gln Arg Thr
            20                  25                  30


<210> 542
<211> 19
<212> PRT
<213> Homo sapiens

<400> 542
Lys Gly Val Thr Ser Val Ser Gln Ile Phe His Ser Pro Asp Leu Ala
1               5                   10                  15


Ile Arg Asp




<210> 543
<211> 11
<212> PRT
<213> Homo sapiens

<400> 543
Arg Asp Lys Gly Gln Ala Gly Leu Gln Arg Ala
1               5                   10


<210> 544
<211> 11
<212> PRT
<213> Homo sapiens

<400> 544
Lys Ser His Lys Pro Leu Asn Met Gly Lys Val
1               5                   10


<210> 545
<211> 12
<212> PRT
<213> Homo sapiens

<400> 545
Arg Phe Gly Leu Leu Asp Glu Asp Gly Lys Lys Thr
1               5                   10


<210> 546
<211> 13
<212> PRT
<213> Homo sapiens

<400> 546
Lys Gly Leu Cys Val Ala Thr Pro Val Gln Leu Arg Val
1               5                   10


<210> 547
<211> 11
<212> PRT
<213> Homo sapiens

<400> 547
Arg Tyr Arg Val Phe Ala Leu Asp Gln Lys Met
1               5                   10


<210> 548
<211> 23
<212> PRT
<213> Homo sapiens

<400> 548
Lys Ala Glu Phe Gln Asp Ala Leu Glu Lys Leu Asn Met Gly Ile Thr
1               5                   10                  15


Asp Leu Gln Gly Leu Arg Leu
            20


<210> 549
<211> 34
<212> PRT
<213> Homo sapiens

<400> 549
Arg Glu Cys Val Gly Phe Glu Ala Val Gln Glu Val Pro Val Gly Leu
1               5                   10                  15


Val Gln Pro Ala Ser Ala Thr Leu Tyr Asp Tyr Tyr Asn Pro Glu Arg
            20                  25                  30


Arg Cys



<210> 550
<211> 28
<212> PRT
<213> Homo sapiens

<400> 550
Arg Val Thr Ala Ser Asp Pro Leu Asp Thr Leu Gly Ser Glu Gly Ala
1               5                   10                  15

Leu Ser Pro Gly Gly Val Ala Ser Leu Leu Arg Leu
            20                  25

<210> 551
<211> 16
<212> PRT
<213> Homo sapiens

<400> 551
Arg Asn Glu Leu Ile Pro Leu Ile Tyr Leu Glu Asn Pro Arg Arg Asn
1               5                   10                  15

<210> 552
<211> 13
<212> PRT
<213> Homo sapiens

<400> 552
Lys Ala Val Gly Tyr Leu Ile Thr Gly Tyr Gln Arg Gln
1               5                   10

<210> 553
<211> 14
<212> PRT
<213> Homo sapiens

<400> 553
Arg Ala Phe Ile Gln Leu Trp Ala Phe Asp Ala Val Lys Gly
1               5                   10

<210> 554
<211> 28
<212> PRT
<213> Homo sapiens

<400> 554
Arg Leu Thr Glu Arg Glu Trp Ala Asp Glu Trp Lys His Leu Asp His
1               5                   10                  15

Ala Leu Asn Cys Ile Met Glu Met Val Glu Lys Thr
            20                  25

<210> 555
<211> 24
<212> PRT
<213> Homo sapiens

<400> 555
Lys Ala Leu Met Asp Leu Leu Ala Gly Lys Gly Ser Gln Gly Ser Gln
1               5                   10                  15

Ala Pro Gln Ala Leu Asp Arg Thr
            20

<210> 556
<211> 19
<212> PRT
<213> Homo sapiens

<400> 556
Arg Thr Phe Gly Ser Gly Glu Ala Asp Cys Gly Leu Arg Pro Leu Phe
1               5                   10                  15

Glu Lys Lys

<210> 557
<211> 8
<212> PRT
<213> Homo sapiens

<400> 557
Lys Ile Ser Asn Ile Ile Lys Gln
1               5

<210> 558
<211> 12
<212> PRT
<213> Homo sapiens

<400> 558
Arg Phe Ser Gly Thr Trp Tyr Ala Met Ala Lys Lys
1               5                   10

<210> 559
<211> 27
<212> PRT
<213> Homo sapiens

<400> 559
Arg Leu Leu Asn Asn Trp Asp Val Cys Ala Asp Met Val Gly Thr Phe
1               5                   10                  15

Thr Asp Thr Glu Asp Pro Ala Lys Phe Lys Met
            20                  25

<210> 560
<211> 12
<212> PRT
<213> Homo sapiens

<400> 560
Lys Tyr Trp Gly Val Ala Ser Phe Leu Gln Lys Gly
1               5                   10

<210> 561

<211> 12
<212> PRT
<213> Homo sapiens

<400> 561
Arg Gly Tyr Val Ile Ile Lys Pro Leu Val Trp Val
1               5                   10


<210> 562
<211> 14
<212> PRT
<213> Homo sapiens

<400> 562
Arg Leu Pro Leu Val Pro Ala Leu Asp Gly Cys Leu Arg Arg
1               5                   10


<210> 563
<211> 16
<212> PRT
<213> Homo sapiens

<400> 563
Arg Asn Glu Leu Ile Arg Gln Glu Lys Leu Glu Gln Leu Ala Arg Arg
1               5                   10                  15


<210> 564
<211> 19
<212> PRT
<213> Homo sapiens

<400> 564
Lys Glu Glu Leu Ala Thr Arg Leu Asn Ser Ser Glu Thr Ala Asp Leu
1               5                   10                  15


Leu Lys Glu


<210> 565
<211> 16
<212> PRT
<213> Homo sapiens

<400> 565
Arg Gln Cys Leu Leu Asn Arg Pro Phe Ser Asp Asn Ser Ala Arg Asp
1               5                   10                  15


<210> 566
<211> 12
<212> PRT
<213> Homo sapiens

<400> 566
Lys Asn Ala Leu Ala Leu Phe Val Leu Pro Lys Glu
1               5                   10

<210> 567
<211> 11
<212> PRT
<213> Homo sapiens

<400> 567
Arg Ser Phe Met Leu Leu Ile Leu Glu Arg Ser
1               5                   10


<210> 568
<211> 14
<212> PRT
<213> Homo sapiens

<400> 568
Lys Thr Glu Pro Lys Ala Pro Glu Pro Ile Ser Ser Lys Pro
1               5                   10


<210> 569
<211> 15
<212> PRT
<213> Homo sapiens

<400> 569
Arg Gly Ser Pro Ala Ile Asn Val Ala Val His Val Phe Arg Lys
1               5                   10                  15


<210> 570
<211> 28
<212> PRT
<213> Homo sapiens

<400> 570
Arg Glu Leu Ile Ser Asp Leu Glu His Arg Leu Gln Gly Ser Val Met
1               5                   10                  15


Glu Leu Leu Gln Gly Val Asp Gly Val Ile Lys Arg
            20                  25


<210> 571
<211> 40
<212> PRT
<213> Homo sapiens

<400> 571
Lys Thr Ala Met Asp Val Phe Val Cys Thr Tyr Phe Met Pro Ala Ala
1               5                   10                  15


Gln Leu Pro Glu Leu Pro Asp Val Glu Leu Pro Thr Asn Lys Asp Val
            20                  25                  30


Cys Asp Pro Gly Asn Thr Lys Val
        35                  40


<210> 572

<211> 13
<212> PRT
<213> Homo sapiens

<400> 572
Lys Val Met Asp Lys Tyr Thr Phe Glu Leu Ser Arg Arg
1               5                   10


<210> 573
<211> 28
<212> PRT
<213> Homo sapiens

<400> 573
Lys Leu Ala Gln Lys Val Pro Thr Ala Asp Leu Glu Asp Val Leu Pro
1               5                   10                  15


Leu Ala Glu Asp Ile Thr Asn Ile Leu Ser Lys Cys
            20                  25


<210> 574
<211> 22
<212> PRT
<213> Homo sapiens

<400> 574
Lys Ser Cys Glu Ser Asn Ser Pro Phe Pro Val His Pro Gly Thr Ala
1               5                   10                  15


Glu Cys Cys Thr Lys Glu
            20


<210> 575
<211> 10
<212> PRT
<213> Homo sapiens

<400> 575
Arg Lys Leu Cys Met Ala Ala Leu Lys His
1               5                   10


<210> 576
<211> 10
<212> PRT
<213> Homo sapiens

<400> 576
Lys Leu Cys Asp Asn Leu Ser Thr Lys Asn
1               5                   10


<210> 577
<211> 11
<212> PRT
<213> Homo sapiens

<400> 577

Arg Ile Tyr Ile Ser Gly Met Ala Pro Arg Pro
1               5                   10

<210> 578
<211> 9
<212> PRT
<213> Homo sapiens

<400> 578
Arg Glu Arg Val Tyr Phe Phe Lys Gly
1               5

<210> 579
<211> 10
<212> PRT
<213> Homo sapiens

<400> 579
Lys Ala Val Arg Pro Gly Tyr Pro Lys Leu
1               5                   10

<210> 580
<211> 8
<212> PRT
<213> Homo sapiens

<400> 580
Lys Thr Arg Phe Leu Leu Arg Thr
1               5

<210> 581
<211> 30
<212> PRT
<213> Homo sapiens

<400> 581
Lys Thr Tyr Val Pro Pro Pro Phe Ser Gln Asp Leu Phe Thr Phe His
1               5                   10                  15

Ala Asp Met Cys Gln Ser Gln Asn Glu Glu Leu Gln Arg Lys
            20                  25                  30

<210> 582
<211> 20
<212> PRT
<213> Homo sapiens

<400> 582
Lys Lys Ser Asp Val Gly Phe Leu Pro Pro Phe Pro Thr Leu Asp Pro
1               5                   10                  15

Glu Glu Lys Cys
            20

<210> 583

<211> 23
<212> PRT
<213> Homo sapiens

<400> 583
Arg Gly Thr His Val Asp Leu Gly Leu Ala Ser Ala Asn Val Asp Phe
1               5                   10                  15

Ala Phe Ser Leu Tyr Lys Gln
            20

<210> 584
<211> 22
<212> PRT
<213> Homo sapiens

<400> 584
Lys Ser Leu Pro Ala Pro Trp Leu Ser Met Ala Pro Val Ser Trp Ile
1               5                   10                  15

Thr Pro Gly Leu Lys Thr
            20

<210> 585
<211> 11
<212> PRT
<213> Homo sapiens

<400> 585
Arg Cys Leu Ala Pro Leu Glu Gly Ala Arg Phe
1               5                   10

<210> 586
<211> 19
<212> PRT
<213> Homo sapiens

<400> 586
Arg Trp Phe Leu Leu Glu Gln Pro Glu Ile Gln Val Ala His Phe Pro
1               5                   10                  15

Phe Lys Asn

<210> 587
<211> 13
<212> PRT
<213> Homo sapiens

<400> 587
Arg Leu Cys Gln Asp Leu Gly Pro Gly Ala Phe Arg Leu
1               5                   10

<210> 588
<211> 19

<212> PRT
<213> Homo sapiens

<400> 588
Arg Gln Leu Lys Glu His Ala Val Glu Gly Asp Cys Asp Phe Gln Leu
1               5                   10                  15

Leu Lys Leu

<210> 589
<211> 9
<212> PRT
<213> Homo sapiens

<400> 589
Lys Cys Asn Leu Leu Ala Glu Lys Gln
1               5

<210> 590
<211> 18
<212> PRT
<213> Homo sapiens

<400> 590
Arg Ser Leu Asp Phe Thr Glu Leu Asp Val Ala Ala Glu Lys Ile Asp
1               5                   10                  15

Arg Phe

<210> 591
<211> 14
<212> PRT
<213> Homo sapiens

<400> 591
Lys Asp Pro Thr Phe Ile Pro Ala Pro Ile Gln Ala Lys Thr
1               5                   10

<210> 592
<211> 27
<212> PRT
<213> Homo sapiens

<400> 592
Lys Glu Pro Cys Val Glu Ser Leu Val Ser Gln Tyr Phe Gln Thr Val
1               5                   10                  15

Thr Asp Tyr Gly Lys Asp Leu Met Glu Lys Val
            20                  25

<210> 593
<211> 20
<212> PRT

<213> Homo sapiens

<400> 593
Lys Phe Ser Val Pro Ala Gly Ile Val Ile Pro Ser Phe Gln Ala Leu
1               5                   10                  15

Thr Ala Arg Phe
            20

<210> 594
<211> 13
<212> PRT
<213> Homo sapiens

<400> 594
Lys Glu Gln His Leu Phe Leu Pro Phe Ser Tyr Lys Asn
1               5                   10

<210> 595
<211> 18
<212> PRT
<213> Homo sapiens

<400> 595
Arg Gly Ile Ile Ser Ala Leu Leu Val Pro Pro Glu Thr Glu Glu Ala
1               5                   10                  15

Lys Gln

<210> 596
<211> 14
<212> PRT
<213> Homo sapiens

<400> 596
Lys Cys Phe Lys Glu His Ser Ser Leu Ala Phe Trp Lys Thr
1               5                   10

<210> 597
<211> 11
<212> PRT
<213> Homo sapiens

<400> 597
Lys Glu His Ser Ser Leu Ala Phe Trp Lys Thr
1               5                   10

<210> 598
<211> 21
<212> PRT
<213> Homo sapiens

<400> 598
Arg Phe Asn Lys Asn Asn Glu Gly Thr Tyr Tyr Ser Pro Asn Tyr Asn
1               5                   10                  15

Pro Gln Ser Arg Ser
            20

<210> 599
<211> 22
<212> PRT
<213> Homo sapiens

<400> 599
Lys His Tyr Tyr Ile Gly Ile Ile Glu Thr Thr Trp Asp Tyr Ala Ser
1               5                   10                  15

Asp His Gly Glu Lys Lys
            20

<210> 600
<211> 11
<212> PRT
<213> Homo sapiens

<400> 600
Arg Val Val Gly Gly Leu Val Ala Leu Arg Gly
1               5                   10

<210> 601
<211> 24
<212> PRT
<213> Homo sapiens

<400> 601
Lys Lys Gly His Ile Tyr Gln Gly Ser Glu Ala Asp Ser Val Phe Ser
1               5                   10                  15

Gly Phe Leu Ile Phe Pro Ser Ala
            20

<210> 602
<211> 15
<212> PRT
<213> Homo sapiens

<400> 602
Arg Gln Thr His Gln Pro Pro Ala Pro Asn Ser Leu Ile Arg Phe
1               5                   10                  15

<210> 603
<211> 21
<212> PRT
<213> Homo sapiens

<400> 603
Arg Gln Phe Gly Pro Tyr Cys Gly His Gly Phe Pro Gly Pro Leu Asn
1               5                   10                  15

Ile Glu Thr Lys Ser
                20

<210> 604
<211> 33
<212> PRT
<213> Homo sapiens

<400> 604
Arg Gln Pro Tyr Ser Tyr Asp Phe Pro Glu Asp Val Ala Pro Ala Leu
1               5                   10                  15

Gly Thr Ser Phe Ser His Met Leu Gly Ala Thr Asn Pro Thr Gln Lys
                20                  25                  30

Thr

<210> 605
<211> 15
<212> PRT
<213> Homo sapiens

<400> 605
Arg Leu Leu Gly Met Glu Thr Met Ala Trp Gln Glu Ile Arg His
1               5                   10                  15

<210> 606
<211> 20
<212> PRT
<213> Homo sapiens

<400> 606
Arg Ala Val Gly Ser Gly Ala Thr Phe Ser His Tyr Tyr Tyr Met Ile
1               5                   10                  15

Leu Ser Arg Gly
                20

<210> 607
<211> 16
<212> PRT
<213> Homo sapiens

<400> 607
Arg Phe Gly Leu Leu Asp Glu Asp Gly Lys Lys Thr Phe Phe Arg Gly
1               5                   10                  15

<210> 608
<211> 11
<212> PRT
<213> Homo sapiens

<400> 608

Lys Ile Thr Gln Val Leu His Phe Thr Lys Asp
1               5                   10

<210> 609
<211> 13
<212> PRT
<213> Homo sapiens

<400> 609
Arg Ile Val Ala Cys Ala Ser Tyr Lys Pro Ser Arg Glu
1               5                   10

<210> 610
<211> 18
<212> PRT
<213> Homo sapiens

<400> 610
Arg Ser Tyr Phe Pro Glu Ser Trp Leu Trp Glu Val His Leu Val Pro
1               5                   10                  15

Arg Arg

<210> 611
<211> 21
<212> PRT
<213> Homo sapiens

<400> 611
Lys Gln Leu Pro Gly Gly Gln Asn Pro Val Ser Tyr Val Tyr Leu Glu
1               5                   10                  15

Val Val Ser Lys His
            20

<210> 612
<211> 13
<212> PRT
<213> Homo sapiens

<400> 612
Lys Thr Leu Leu Pro Val Ser Lys Pro Glu Ile Arg Ser
1               5                   10

<210> 613
<211> 38
<212> PRT
<213> Homo sapiens

<400> 613
Arg Gly Gly Ala Ser Glu His Ile Thr Thr Leu Ala Tyr Gln Glu Leu
1               5                   10                  15

Pro Thr Ala Asp Leu Met Gln Glu Trp Gly Asp Ala Val Gln Tyr Asn

20                  25                  30

Pro Ala Ile Ile Lys Val
        35

<210> 614
<211> 14
<212> PRT
<213> Homo sapiens

<400> 614
Lys Gly Thr Asp Tyr His Lys Gln Pro Trp Gln Ala Lys Ile
1               5                   10

<210> 615
<211> 13
<212> PRT
<213> Homo sapiens

<400> 615
Lys Val Lys Asp Ile Ser Glu Val Val Thr Pro Arg Phe
1               5                   10

<210> 616
<211> 9
<212> PRT
<213> Homo sapiens

<400> 616
Lys Gln Val Pro Ala His Ala Arg Asp
1               5

<210> 617
<211> 14
<212> PRT
<213> Homo sapiens

<400> 617
Arg Gly Asp Ser Gly Gly Pro Leu Ile Val His Lys Arg Ser
1               5                   10

<210> 618
<211> 19
<212> PRT
<213> Homo sapiens

<400> 618
Arg Phe Leu Cys Thr Gly Gly Val Ser Pro Tyr Ala Asp Pro Asn Thr
1               5                   10                  15

Cys Arg Gly

<210> 619
<211> 19

<212> PRT
<213> Homo sapiens

<400> 619
Lys Lys Glu Ala Gly Ile Pro Glu Phe Tyr Asp Tyr Asp Val Ala Leu
1               5                   10                  15

Ile Lys Leu

<210> 620
<211> 13
<212> PRT
<213> Homo sapiens

<400> 620
Arg Tyr Gly Leu Val Thr Tyr Ala Thr Tyr Pro Lys Ile
1               5                   10

<210> 621
<211> 11
<212> PRT
<213> Homo sapiens

<400> 621
Lys Glu Phe Asp His Asn Ser Asn Ile Arg Tyr
1               5                   10

<210> 622
<211> 15
<212> PRT
<213> Homo sapiens

<400> 622
Lys Trp Ser Ser Pro Pro Gln Cys Glu Gly Leu Pro Cys Lys Ser
1               5                   10                  15

<210> 623
<211> 13
<212> PRT
<213> Homo sapiens

<400> 623
Arg Lys Gly Glu Trp Val Ala Leu Asn Pro Leu Arg Lys
1               5                   10

<210> 624
<211> 12
<212> PRT
<213> Homo sapiens

<400> 624
Lys Ser Leu Glu Cys Leu His Pro Gly Thr Lys Phe
1               5                   10

<210> 625

<211> 17
<212> PRT
<213> Homo sapiens

<400> 625
Arg Gly Leu Ala Ser Ala Asn Val Asp Phe Ala Phe Ser Leu Tyr Lys
1               5                   10                  15

His

<210> 626
<211> 10
<212> PRT
<213> Homo sapiens

<400> 626
Lys Leu Val Val Leu Pro Phe Pro Lys Glu
1               5                   10

<210> 627
<211> 20
<212> PRT
<213> Homo sapiens

<400> 627
Arg Ala Ser Ser Gln Trp Val Val Gly Pro Ser Tyr Phe Val Glu Tyr
1               5                   10                  15

Leu Ile Lys Glu
            20

<210> 628
<211> 16
<212> PRT
<213> Homo sapiens

<400> 628
Arg Leu Leu Gly Glu Val Asp His Tyr Gln Leu Ala Leu Gly Lys Phe
1               5                   10                  15

<210> 629
<211> 19
<212> PRT
<213> Homo sapiens

<400> 629
Lys Gln Thr Gln Val Ser Val Leu Pro Glu Gly Gly Glu Thr Pro Leu
1               5                   10                  15

Phe Lys Gln

<210> 630
<211> 11

<212> PRT
<213> Homo sapiens

<400> 630
Lys Val Asp Gly Ala Leu Cys Met Glu Lys Ser
1               5                   10


<210> 631
<211> 27
<212> PRT
<213> Homo sapiens

<400> 631
Lys Ser Gly Ala Gln Ala Thr Trp Thr Glu Leu Pro Trp Pro His Glu
1               5                   10                  15


Lys Val Asp Gly Ala Leu Cys Met Glu Lys Ser
            20                  25


<210> 632
<211> 12
<212> PRT
<213> Homo sapiens

<400> 632
Arg Gln Gly His Asn Ser Val Phe Leu Ile Lys Gly
1               5                   10


<210> 633
<211> 11
<212> PRT
<213> Homo sapiens

<400> 633
Lys Thr Leu Glu Ala Gln Leu Thr Pro Arg Val
1               5                   10


<210> 634
<211> 16
<212> PRT
<213> Homo sapiens

<400> 634
Lys Asp Ser Pro Val Leu Ile Asp Phe Phe Glu Asp Thr Glu Arg Tyr
1               5                   10                  15


<210> 635
<211> 17
<212> PRT
<213> Homo sapiens

<400> 635
Lys Ala Leu Arg Asp Phe Ala Leu Gln Asn Pro Ser Ala Val Pro Arg
1               5                   10                  15


Phe

<210> 636
<211> 17
<212> PRT
<213> Homo sapiens

<400> 636
Arg Leu Trp Leu Glu Gly Asn Pro Trp Asp Cys Gly Cys Pro Leu Lys
1               5                   10                  15

Ala

<210> 637
<211> 12
<212> PRT
<213> Homo sapiens

<400> 637
Arg Ser Ser Ala Leu Asp Met Glu Asn Phe Arg Thr
1               5                   10

<210> 638
<211> 11
<212> PRT
<213> Homo sapiens

<400> 638
Arg Ser Leu Ala Pro Thr Ala Ala Ala Lys Arg
1               5                   10

<210> 639
<211> 13
<212> PRT
<213> Homo sapiens

<400> 639
Arg Leu Ser Asn Glu Asn His Gly Ile Ala Gln Arg Ile
1               5                   10

<210> 640
<211> 15
<212> PRT
<213> Homo sapiens

<400> 640
Arg Ile Tyr Gly Asn Gln Asp Thr Ser Ser Gln Leu Lys Lys Phe
1               5                   10                  15

<210> 641
<211> 24
<212> PRT
<213> Homo sapiens

<400> 641

Lys Thr Gly Leu Leu Leu Leu Ser Asp Pro Asp Lys Val Thr Ile Gly
1               5                   10                  15

Leu Leu Phe Trp Asp Gly Arg Gly
            20

<210> 642
<211> 13
<212> PRT
<213> Homo sapiens

<400> 642
Lys Ile Pro Lys Pro Glu Ala Ser Phe Ser Pro Arg Arg
1               5                   10

<210> 643
<211> 24
<212> PRT
<213> Homo sapiens

<400> 643
Arg Gln Gly Pro Val Asn Leu Leu Ser Asp Pro Glu Gln Gly Val Glu
1               5                   10                  15

Val Thr Gly Gln Tyr Glu Arg Glu
            20

<210> 644
<211> 17
<212> PRT
<213> Homo sapiens

<400> 644
Arg Ala Asn Thr Val Gln Glu Ala Thr Phe Gln Met Glu Leu Pro Lys
1               5                   10                  15

Lys

<210> 645
<211> 21
<212> PRT
<213> Homo sapiens

<400> 645
Arg Arg Leu Asp Tyr Gln Glu Gly Pro Pro Gly Val Glu Ile Ser Cys
1               5                   10                  15

Trp Ser Val Glu Leu
            20

<210> 646
<211> 18
<212> PRT

<213> Homo sapiens

<400> 646
Lys Ser Pro Glu Gln Gln Glu Thr Val Leu Asp Gly Asn Leu Ile Ile
1               5                   10                  15

Arg Tyr

<210> 647
<211> 13
<212> PRT
<213> Homo sapiens

<400> 647
Lys Ala Leu Trp Glu Lys Pro Phe Ile Ser Ser Arg Thr
1               5                   10

<210> 648
<211> 11
<212> PRT
<213> Homo sapiens

<400> 648
Arg Gln Val Val Ala Gly Leu Asn Phe Arg Ile
1               5                   10

<210> 649
<211> 20
<212> PRT
<213> Homo sapiens

<400> 649
Lys Leu Gly Gln Ser Leu Asp Cys Asn Ala Glu Val Tyr Val Val Pro
1               5                   10                  15

Trp Glu Lys Lys
            20

<210> 650
<211> 24
<212> PRT
<213> Homo sapiens

<400> 650
Arg Ile Ala Ser Phe Ser Gln Asn Cys Asp Ile Tyr Pro Gly Lys Asp
1               5                   10                  15

Phe Val Gln Pro Pro Thr Lys Ile
            20

<210> 651
<211> 19
<212> PRT
<213> Homo sapiens

<400> 651
Arg Cys Ala Gly Pro Glu Ala Val Lys Gly Gln Thr Leu Leu Ala Val
1               5                   10                  15

Ala Lys Ser

<210> 652
<211> 11
<212> PRT
<213> Homo sapiens

<400> 652
Lys Gly Gln Thr Leu Leu Ala Val Ala Lys Ser
1               5                   10

<210> 653
<211> 12
<212> PRT
<213> Homo sapiens

<400> 653
Lys Asp Leu Leu Leu Pro Gln Pro Asp Leu Arg Tyr
1               5                   10

<210> 654
<211> 11
<212> PRT
<213> Homo sapiens

<400> 654
Lys Ile Leu Gly Pro Leu Ser Tyr Ser Lys Ile
1               5                   10

<210> 655
<211> 11
<212> PRT
<213> Homo sapiens

<400> 655
Arg Gln Gly Ser Phe Gln Gly Gly Phe Arg Ser
1               5                   10

<210> 656
<211> 11
<212> PRT
<213> Homo sapiens

<400> 656
Lys Tyr Val Leu Pro Asn Phe Glu Val Lys Ile
1               5                   10

<210> 657
<211> 19
<212> PRT

<213> Homo sapiens

<400> 657
Arg Leu Leu Ala Thr Leu Cys Ser Ala Glu Val Cys Gln Cys Ala Glu
1               5                   10                  15

Gly Lys Cys

<210> 658
<211> 12
<212> PRT
<213> Homo sapiens

<400> 658
Arg Val Gly Asp Thr Leu Asn Leu Asn Leu Arg Ala
1               5                   10

<210> 659
<211> 16
<212> PRT
<213> Homo sapiens

<400> 659
Arg Glu Pro Phe Leu Ser Cys Cys Gln Phe Ala Glu Ser Leu Arg Lys
1               5                   10                  15

<210> 660
<211> 15
<212> PRT
<213> Homo sapiens

<400> 660
Arg Glu Glu Leu Val Tyr Glu Leu Asn Pro Leu Asp His Arg Gly
1               5                   10                  15

<210> 661
<211> 15
<212> PRT
<213> Homo sapiens

<400> 661
Arg Gly Ser Phe Glu Phe Pro Val Gly Asp Ala Val Ser Lys Val
1               5                   10                  15

<210> 662
<211> 20
<212> PRT
<213> Homo sapiens

<400> 662
Lys Gly Ser Phe Ala Leu Ser Phe Pro Val Glu Ser Asp Val Ala Pro
1               5                   10                  15

Ile Ala Arg Met
            20

<210> 663
<211> 38
<212> PRT
<213> Homo sapiens

<400> 663
Arg Val Val Ala Gln Gly Val Gly Ile Pro Glu Asp Ser Ile Phe Thr
1               5                   10                  15

Met Ala Asp Arg Gly Glu Cys Val Pro Gly Glu Gln Glu Pro Glu Pro
            20                  25                  30

Ile Leu Ile Pro Arg Val
        35

<210> 664
<211> 11
<212> PRT
<213> Homo sapiens

<400> 664
Arg Ser Gly Ile Glu Cys Gln Leu Trp Arg Ser
1               5                   10

<210> 665
<211> 16
<212> PRT
<213> Homo sapiens

<400> 665
Lys Met Ser Ser Ile Asn Ala Asp Phe Ala Phe Asn Leu Tyr Arg Arg
1               5                   10                  15

<210> 666
<211> 27
<212> PRT
<213> Homo sapiens

<400> 666
Arg Met Asp Trp Leu Val Pro Ala Thr Cys Glu Pro Ile Gln Ser Val
1               5                   10                  15

Phe Phe Phe Ser Gly Asp Lys Tyr Tyr Arg Val
            20                  25

<210> 667
<211> 17
<212> PRT
<213> Homo sapiens

<400> 667
Arg Asp Val Trp Gly Ile Glu Gly Pro Ile Asp Ala Ala Phe Thr Arg
1               5                   10                  15

Ile

<210> 668
<211> 9
<212> PRT
<213> Homo sapiens

<400> 668
Arg Ser Cys Pro Asp Asn Pro Lys Gly
1               5

<210> 669
<211> 6
<212> PRT
<213> Homo sapiens

<400> 669
Leu Gln Val Leu Gly Lys
1               5

<210> 670
<211> 14
<212> PRT
<213> Homo sapiens

<400> 670
Lys Ala Ala Ile Ser Gly Glu Asn Ala Gly Leu Val Arg Ala
1               5                   10

<210> 671
<211> 9
<212> PRT
<213> Homo sapiens

<400> 671
Lys Val Thr Tyr Asp Val Ser Arg Asp
1               5

<210> 672
<211> 8
<212> PRT
<213> Homo sapiens

<400> 672
Lys Thr Ala Gly Leu Val Arg Ser
1               5

<210> 673
<211> 12
<212> PRT
<213> Homo sapiens

<400> 673
Arg Ser Leu Ala Pro Thr Ala Ala Ala Lys Arg Arg
1               5                   10

<210> 674
<211> 9
<212> PRT
<213> Homo sapiens

<400> 674
Lys Phe Gln Leu Pro Gly Gln Lys Leu
1               5


<210> 675
<211> 27
<212> PRT
<213> Homo sapiens

<400> 675
Arg Asp Leu Tyr His Tyr Ile Thr Ser Tyr Val Val Asp Gly Glu Ile
1               5                   10                  15


Ile Ile Tyr Gly Pro Ala Tyr Ser Gly Arg Glu
            20                  25


<210> 676
<211> 9
<212> PRT
<213> Homo sapiens

<400> 676
Thr Leu Phe Ile Phe Gly Val Thr Lys
1               5


<210> 677
<211> 18
<212> PRT
<213> Homo sapiens

<400> 677
Asn Tyr Thr Tyr Ile Trp Trp Leu Asn Gly Gln Ser Leu Pro Val Ser
1               5                   10                  15


Pro Arg



<210> 678
<211> 8
<212> PRT
<213> Homo sapiens

<400> 678
Leu Gln Leu Ser Glu Thr Asn Arg
1               5


<210> 679
<211> 9
<212> PRT

<213> Homo sapiens

<400> 679
Ile Leu Ile Leu Pro Ser Val Thr Arg
1               5


<210> 680
<211> 16
<212> PRT
<213> Homo sapiens

<400> 680
Lys Glu Leu Glu Leu Gln Ile Gly Asn Ala Leu Phe Ile Gly Lys His
1               5                   10                  15


<210> 681
<211> 13
<212> PRT
<213> Homo sapiens

<400> 681
Lys Ala Gln Trp Ala Asn Pro Phe Asp Pro Ser Lys Thr
1               5                   10


<210> 682
<211> 13
<212> PRT
<213> Homo sapiens

<400> 682
Lys Thr Glu Asp Ser Ser Ser Phe Leu Ile Asp Lys Thr
1               5                   10


<210> 683
<211> 8
<212> PRT
<213> Homo sapiens

<400> 683
Lys Phe Leu Asn Asp Val Lys Thr
1               5


<210> 684
<211> 9
<212> PRT
<213> Homo sapiens

<400> 684
Lys Leu Ser Asn Ala Ala His Lys Ala
1               5


<210> 685
<211> 8
<212> PRT
<213> Homo sapiens

<400> 685

Arg Val Leu Asp Leu Thr Arg Asn
1               5

<210> 686
<211> 13
<212> PRT
<213> Homo sapiens

<400> 686
Lys Ala Leu Gly His Leu Asp Leu Ser Gly Asn Arg Leu
1               5                   10

<210> 687
<211> 15
<212> PRT
<213> Homo sapiens

<400> 687
Lys Leu Pro Pro Gly Leu Leu Ala Asn Phe Thr Leu Leu Arg Thr
1               5                   10                  15

<210> 688
<211> 20
<212> PRT
<213> Homo sapiens

<400> 688
Arg Thr Leu Asp Leu Gly Glu Asn Gln Leu Glu Thr Leu Pro Pro Asp
1               5                   10                  15

Leu Leu Arg Gly
            20

<210> 689
<211> 9
<212> PRT
<213> Homo sapiens

<400> 689
Arg Gly Pro Leu Gln Leu Glu Arg Leu
1               5

<210> 690
<211> 9
<212> PRT
<213> Homo sapiens

<400> 690
Arg Leu His Leu Glu Gly Asn Lys Leu
1               5

<210> 691
<211> 8
<212> PRT
<213> Homo sapiens

<400> 691
Lys Leu Gln Val Leu Gly Lys Asp
1               5

<210> 692
<211> 12
<212> PRT
<213> Homo sapiens

<400> 692
Lys Asp Leu Leu Leu Pro Gln Pro Asp Leu Arg Tyr
1               5                   10

<210> 693
<211> 12
<212> PRT
<213> Homo sapiens

<400> 693
Arg Val Ala Ala Gly Ala Phe Gln Gly Leu Arg Gln
1               5                   10

<210> 694
<211> 8
<212> PRT
<213> Homo sapiens

<400> 694
Arg Trp Leu Gln Ala Gln Lys Asp
1               5

<210> 695
<211> 11
<212> PRT
<213> Homo sapiens

<400> 695
Lys Gly Gln Thr Leu Leu Ala Val Ala Lys Ser
1               5                   10

<210> 696
<211> 10
<212> PRT
<213> Homo sapiens

<400> 696
Arg Tyr Leu Phe Leu Asn Gly Asn Lys Leu
1               5                   10

<210> 697
<211> 14
<212> PRT
<213> Homo sapiens

<400> 697
Lys His Gln Phe Leu Leu Thr Gly Asp Thr Gln Gly Arg Tyr
1               5                   10

<210> 698
<211> 14
<212> PRT
<213> Homo sapiens

<400> 698
Arg Ser Gly Leu Ser Thr Gly Trp Thr Gln Leu Ser Lys Leu
1               5                   10


<210> 699
<211> 10
<212> PRT
<213> Homo sapiens

<400> 699
Lys Leu Leu Glu Leu Thr Gly Pro Lys Ser
1               5                   10


<210> 700
<211> 9
<212> PRT
<213> Homo sapiens

<400> 700
Arg Gly Val Thr Phe Leu Leu Arg Arg
1               5


<210> 701
<211> 8
<212> PRT
<213> Homo sapiens

<400> 701
Lys Glu Leu Leu Val Pro Arg Ser
1               5


<210> 702
<211> 9
<212> PRT
<213> Homo sapiens

<400> 702
Arg Ser Ser Thr Ser Pro Asp Arg Ile
1               5


<210> 703
<211> 17
<212> PRT
<213> Homo sapiens

<400> 703
Arg Leu Glu Leu His Val Asp Gly Pro Pro Pro Arg Pro Gln Leu Arg
1               5                   10                  15


Ala

<210> 704
<211> 13
<212> PRT
<213> Homo sapiens

<400> 704
Arg Ala Thr Trp Ser Gly Ala Val Leu Ala Gly Arg Asp
1               5                   10

<210> 705
<211> 24
<212> PRT
<213> Homo sapiens

<400> 705
Arg Thr Pro Gly Ala Ala Ala Asn Leu Glu Leu Ile Phe Val Gly Pro
1               5                   10                  15

Gln His Ala Gly Asn Tyr Arg Cys
            20

<210> 706
<211> 24
<212> PRT
<213> Homo sapiens

<400> 706
Arg Ser Leu Ala Leu Gly Thr Phe Ala His Thr Pro Ala Leu Ala Ser
1               5                   10                  15

Leu Gly Leu Ser Asn Asn Arg Leu
            20

<210> 707
<211> 10
<212> PRT
<213> Homo sapiens

<400> 707
Arg Glu Leu Val Leu Ala Gly Asn Arg Leu
1               5                   10

<210> 708
<211> 18
<212> PRT
<213> Homo sapiens

<400> 708
Arg Leu Ala Tyr Leu Gln Pro Ala Leu Phe Ser Gly Leu Ala Glu Leu
1               5                   10                  15

Arg Glu

<210> 709
<211> 8
<212> PRT
<213> Homo sapiens

<400> 709
Arg Glu Leu Asp Leu Ser Arg Asn
1               5


<210> 710
<211> 11
<212> PRT
<213> Homo sapiens

<400> 710
Lys Ala Asn Val Phe Val Gln Leu Pro Arg Leu
1               5                   10


<210> 711
<211> 17
<212> PRT
<213> Homo sapiens

<400> 711
Arg Asn Leu Ile Ala Ala Val Ala Pro Gly Ala Phe Leu Gly Leu Lys
1               5                   10                  15


Ala


<210> 712
<211> 18
<212> PRT
<213> Homo sapiens

<400> 712
Arg Val Ala Gly Leu Leu Glu Asp Thr Phe Pro Gly Leu Leu Gly Leu
1               5                   10                  15


Arg Val


<210> 713
<211> 24
<212> PRT
<213> Homo sapiens

<400> 713
Arg Ser Phe Glu Gly Leu Gly Gln Leu Glu Val Leu Thr Leu Asp His
1               5                   10                  15


Asn Gln Leu Gln Glu Val Lys Ala
            20

<210> 714
<211> 10
<212> PRT
<213> Homo sapiens

<400> 714
Arg Asn Leu Pro Glu Gln Val Phe Arg Gly
1               5                   10


<210> 715
<211> 15
<212> PRT
<213> Homo sapiens

<400> 715
Arg Ile Arg Pro His Thr Phe Thr Gly Leu Ser Gly Leu Arg Arg
1               5                   10                  15


<210> 716
<211> 10
<212> PRT
<213> Homo sapiens

<400> 716
Lys Leu Glu Tyr Leu Leu Leu Ser Arg Asn
1               5                   10


<210> 717
<211> 16
<212> PRT
<213> Homo sapiens

<400> 717
Arg Leu Ala Glu Leu Pro Ala Asp Ala Leu Gly Pro Leu Gln Arg Ala
1               5                   10                  15


<210> 718
<211> 16
<212> PRT
<213> Homo sapiens

<400> 718
Arg Leu Glu Ala Leu Pro Asn Ser Leu Leu Ala Pro Leu Gly Arg Leu
1               5                   10                  15


<210> 719
<211> 13
<212> PRT
<213> Homo sapiens

<400> 719
Arg Thr Phe Thr Pro Gln Pro Pro Gly Leu Glu Arg Leu
1               5                   10


<210> 720
<211> 14

<212> PRT
<213> Homo sapiens

<400> 720
Arg Asp Phe Ala Leu Gln Asn Pro Ser Ala Val Pro Arg Phe
1               5                   10

<210> 721
<211> 17
<212> PRT
<213> Homo sapiens

<400> 721
Lys Phe Ile Cys Pro Leu Thr Gly Leu Trp Pro Ile Asn Thr Leu Lys
1               5                   10                  15

Cys

<210> 722
<211> 16
<212> PRT
<213> Homo sapiens

<400> 722
Arg Val Cys Pro Phe Ala Gly Ile Leu Glu Asn Gly Ala Val Arg Tyr
1               5                   10                  15

<210> 723
<211> 8
<212> PRT
<213> Homo sapiens

<400> 723
Lys Cys Thr Glu Glu Gly Lys Trp
1               5

<210> 724
<211> 27
<212> PRT
<213> Homo sapiens

<400> 724
Lys Trp Ser Pro Glu Leu Pro Val Cys Ala Pro Ile Ile Cys Pro Pro
1               5                   10                  15

Pro Ser Ile Pro Thr Phe Ala Thr Leu Arg Val
            20                  25

<210> 725
<211> 23
<212> PRT
<213> Homo sapiens

<400> 725
Lys Ala Thr Phe Gly Cys His Asp Gly Tyr Ser Leu Asp Gly Pro Glu

1               5                   10                  15

Glu Ile Glu Cys Thr Lys Leu
            20

<210> 726
<211> 11
<212> PRT
<213> Homo sapiens

<400> 726
Lys Ala Thr Val Val Tyr Gln Gly Glu Arg Val
1               5                   10

<210> 727
<211> 8
<212> PRT
<213> Homo sapiens

<400> 727
Lys Val Ser Phe Phe Cys Lys Asn
1               5

<210> 728
<211> 20
<212> PRT
<213> Homo sapiens

<400> 728
Lys Cys Ser Tyr Thr Glu Asp Ala Gln Cys Ile Asp Gly Thr Ile Glu
1               5                   10                  15

Val Pro Lys Cys
            20

<210> 729
<211> 11
<212> PRT
<213> Homo sapiens

<400> 729
Lys Glu His Ser Ser Leu Ala Phe Trp Lys Thr
1               5                   10

<210> 730
<211> 10
<212> PRT
<213> Homo sapiens

<400> 730
Lys Thr Asp Ala Ser Asp Val Lys Pro Cys
1               5                   10

<210> 731
<211> 13

<212> PRT
<213> Homo sapiens

<400> 731
Lys Ser Pro Tyr Gln Leu Val Leu Gln His Ser Arg Leu
1               5                   10


<210> 732
<211> 9
<212> PRT
<213> Homo sapiens

<400> 732
Arg Val Leu Thr Asp Glu Leu Lys His
1               5


<210> 733
<211> 25
<212> PRT
<213> Homo sapiens

<400> 733
Lys Val Ile Ser Thr Ile Thr Asn Asn Ile Gln Gln Ile Ile Glu Ile
1               5                   10                  15


Glu Asp Thr Phe Glu Thr Leu Arg Ala
            20                  25


<210> 734
<211> 10
<212> PRT
<213> Homo sapiens

<400> 734
Lys Ile Pro Ser Glu Thr Leu Asn Arg Ile
1               5                   10


<210> 735
<211> 11
<212> PRT
<213> Homo sapiens

<400> 735
Arg Ile Leu Gly Asp Pro Glu Ala Leu Arg Asp
1               5                   10


<210> 736
<211> 11
<212> PRT
<213> Homo sapiens

<400> 736
Arg Asp Leu Leu Asn Asn His Ile Leu Lys Ser
1               5                   10


<210> 737

<211> 8
<212> PRT
<213> Homo sapiens

<400> 737
Lys Ala Ile Ile Ser Asn Lys Asp
1               5


<210> 738
<211> 24
<212> PRT
<213> Homo sapiens

<400> 738
Lys Asp Ile Leu Ala Thr Asn Gly Val Ile His Tyr Ile Asp Glu Leu
1               5                   10                  15


Leu Ile Pro Asp Ser Ala Lys Thr
            20


<210> 739
<211> 21
<212> PRT
<213> Homo sapiens

<400> 739
Lys Thr Leu Phe Glu Leu Ala Ala Glu Ser Asp Val Ser Thr Ala Ile
1               5                   10                  15


Asp Leu Phe Arg Gln
            20


<210> 740
<211> 15
<212> PRT
<213> Homo sapiens

<400> 740
Arg Gln Ala Gly Leu Gly Asn His Leu Ser Gly Ser Glu Arg Leu
1               5                   10                  15


<210> 741
<211> 14
<212> PRT
<213> Homo sapiens

<400> 741
Arg Leu Thr Leu Leu Ala Pro Leu Asn Ser Val Phe Lys Asp
1               5                   10


<210> 742
<211> 13
<212> PRT
<213> Homo sapiens

<400> 742

Lys Asp Gly Thr Pro Pro Ile Asp Ala His Thr Arg Asn
1               5                   10

<210> 743
<211> 16
<212> PRT
<213> Homo sapiens

<400> 743
Lys Tyr Leu Tyr His Gly Gln Thr Leu Glu Thr Leu Gly Gly Lys Lys
1               5                   10                  15

<210> 744
<211> 17
<212> PRT
<213> Homo sapiens

<400> 744
Arg Glu Gly Val Tyr Thr Val Phe Ala Pro Thr Asn Glu Ala Phe Arg
1               5                   10                  15

Ala

<210> 745
<211> 8
<212> PRT
<213> Homo sapiens

<400> 745
Arg Leu Leu Gly Asp Ala Lys Glu
1               5

<210> 746
<211> 9
<212> PRT
<213> Homo sapiens

<400> 746
Lys Glu Leu Ala Asn Ile Leu Lys Tyr
1               5

<210> 747
<211> 20
<212> PRT
<213> Homo sapiens

<400> 747
Lys Tyr His Ile Gly Asp Glu Ile Leu Val Ser Gly Gly Ile Gly Ala
1               5                   10                  15

Leu Val Arg Leu
            20

<210> 748

<211> 8
<212> PRT
<213> Homo sapiens

<400> 748
Lys Leu Glu Val Ser Leu Lys Asn
1               5


<210> 749
<211> 10
<212> PRT
<213> Homo sapiens

<400> 749
Lys Asn Asn Val Val Ser Val Asn Lys Glu
1               5                   10


<210> 750
<211> 15
<212> PRT
<213> Homo sapiens

<400> 750
Arg Gly Asp Glu Leu Ala Asp Ser Ala Leu Glu Ile Phe Lys Gln
1               5                   10                  15


<210> 751
<211> 9
<212> PRT
<213> Homo sapiens

<400> 751
Lys Gln Ala Ser Ala Phe Ser Arg Ala
1               5


<210> 752
<211> 9
<212> PRT
<213> Homo sapiens

<400> 752
Arg Leu Ala Pro Val Tyr Gln Lys Leu
1               5


<210> 753
<211> 21
<212> PRT
<213> Homo sapiens

<400> 753
Lys Leu Ile Ser Val Asp Thr Glu His Ser Asn Ile Tyr Leu Gln Asn
1               5                   10                  15


Gly Pro Asp Arg Ile
            20

<210> 754
<211> 14
<212> PRT
<213> Homo sapiens

<400> 754
Lys Ala Leu Tyr Leu Gln Tyr Thr Asp Glu Thr Phe Arg Thr
1               5                   10

<210> 755
<211> 19
<212> PRT
<213> Homo sapiens

<400> 755
Arg Thr Thr Ile Glu Lys Pro Val Trp Leu Gly Phe Leu Gly Pro Ile
1               5                   10                  15

Ile Lys Ala

<210> 756
<211> 8
<212> PRT
<213> Homo sapiens

<400> 756
Lys Val Tyr Val His Leu Lys Asn
1               5

<210> 757
<211> 12
<212> PRT
<213> Homo sapiens

<400> 757
Arg Ile Tyr His Ser His Ile Asp Ala Pro Lys Asp
1               5                   10

<210> 758
<211> 25
<212> PRT
<213> Homo sapiens

<400> 758
Arg His Tyr Tyr Ile Ala Ala Glu Glu Ile Ile Trp Asn Tyr Ala Pro
1               5                   10                  15

Ser Gly Ile Asp Ile Phe Thr Lys Glu
            20                  25

<210> 759
<211> 8
<212> PRT
<213> Homo sapiens

<400> 759
Arg Ile Gly Gly Ser Tyr Lys Lys
1               5

<210> 760
<211> 12
<212> PRT
<213> Homo sapiens

<400> 760
Arg Glu Tyr Thr Asp Ala Ser Phe Thr Asn Arg Lys
1               5                   10

<210> 761
<211> 25
<212> PRT
<213> Homo sapiens

<400> 761
Arg Gly Pro Glu Glu Glu His Leu Gly Ile Leu Gly Pro Val Ile Trp
1               5                   10                  15

Ala Glu Val Gly Asp Thr Ile Arg Val
            20                  25

<210> 762
<211> 8
<212> PRT
<213> Homo sapiens

<400> 762
Arg Val Thr Phe His Asn Lys Gly
1               5

<210> 763
<211> 15
<212> PRT
<213> Homo sapiens

<400> 763
Lys Gly Ala Tyr Pro Leu Ser Ile Glu Pro Ile Gly Val Arg Phe
1               5                   10                  15

<210> 764
<211> 18
<212> PRT
<213> Homo sapiens

<400> 764
Lys Asn Asn Glu Gly Thr Tyr Tyr Ser Pro Asn Tyr Asn Pro Gln Ser
1               5                   10                  15

Arg Ser

<210> 765
<211> 25
<212> PRT
<213> Homo sapiens

<400> 765
Arg Ser Val Pro Pro Ser Ala Ser His Val Ala Pro Thr Glu Thr Phe
1               5                   10                  15

Thr Tyr Glu Trp Thr Val Pro Lys Glu
            20                  25

<210> 766
<211> 10
<212> PRT
<213> Homo sapiens

<400> 766
Lys Gly Ser Leu His Ala Asn Gly Arg Gln
1               5                   10

<210> 767
<211> 14
<212> PRT
<213> Homo sapiens

<400> 767
Arg Gln Ser Glu Asp Ser Thr Phe Tyr Leu Gly Glu Arg Thr
1               5                   10

<210> 768
<211> 19
<212> PRT
<213> Homo sapiens

<400> 768
Arg Thr Tyr Tyr Ile Ala Ala Val Glu Val Glu Trp Asp Tyr Ser Pro
1               5                   10                  15

Gln Arg Glu

<210> 769
<211> 19
<212> PRT
<213> Homo sapiens

<400> 769
Lys Glu Leu His His Leu Gln Glu Gln Asn Val Ser Asn Ala Phe Leu
1               5                   10                  15

Asp Lys Gly

<210> 770

<211> 10
<212> PRT
<213> Homo sapiens

<400> 770
Lys Gly Glu Phe Tyr Ile Gly Ser Lys Tyr
1               5                   10


<210> 771
<211> 10
<212> PRT
<213> Homo sapiens

<400> 771
Arg Gln Tyr Thr Asp Ser Thr Phe Arg Val
1               5                   10


<210> 772
<211> 22
<212> PRT
<213> Homo sapiens

<400> 772
Lys Ala Glu Glu Glu His Leu Gly Ile Leu Gly Pro Gln Leu His Ala
1               5                   10                  15


Asp Val Gly Asp Lys Val
            20


<210> 773
<211> 25
<212> PRT
<213> Homo sapiens

<400> 773
Lys Leu Glu Phe Ala Leu Leu Phe Leu Val Phe Asp Glu Asn Glu Ser
1               5                   10                  15


Trp Tyr Leu Asp Asp Asn Ile Lys Thr
            20                  25


<210> 774
<211> 10
<212> PRT
<213> Homo sapiens

<400> 774
Lys Thr Tyr Ser Asp His Pro Glu Lys Val
1               5                   10


<210> 775
<211> 11
<212> PRT
<213> Homo sapiens

<400> 775

Arg Tyr Glu Tyr Leu Glu Gly Gly Asp Arg Trp
1               5                   10

<210> 776
<211> 21
<212> PRT
<213> Homo sapiens

<400> 776
Arg Val Gln Leu Ser Pro Asp Leu Leu Ala Thr Leu Pro Glu Pro Ala
1               5                   10                  15

Ser Pro Gly Arg Gln
            20

<210> 777
<211> 14
<212> PRT
<213> Homo sapiens

<400> 777
Arg Thr Thr Asp Val Thr Gln Thr Phe Gly Ile Glu Lys Tyr
1               5                   10

<210> 778
<211> 13
<212> PRT
<213> Homo sapiens

<400> 778
Arg Glu Ala Leu Val Pro Leu Val Ala Asp His Lys Cys
1               5                   10

<210> 779
<211> 11
<212> PRT
<213> Homo sapiens

<400> 779
Arg Leu His Lys Pro Gly Val Tyr Thr Arg Val
1               5                   10

<210> 780
<211> 13
<212> PRT
<213> Homo sapiens

<400> 780
Arg Val Ala Asn Tyr Val Asp Trp Ile Asn Asp Arg Ile
1               5                   10

<210> 781
<211> 22
<212> PRT
<213> Homo sapiens

<400> 781
Lys Phe Asn Leu Thr Glu Thr Ser Glu Ala Glu Ile His Gln Ser Phe
1               5                   10                  15

Gln His Leu Leu Arg Thr
            20

<210> 782
<211> 10
<212> PRT
<213> Homo sapiens

<400> 782
Lys Glu Gln Leu Ser Leu Leu Asp Arg Phe
1               5                   10

<210> 783
<211> 18
<212> PRT
<213> Homo sapiens

<400> 783
Lys Tyr Thr Gly Asn Ala Ser Ala Leu Phe Ile Leu Pro Asp Gln Asp
1               5                   10                  15

Lys Met

<210> 784
<211> 11
<212> PRT
<213> Homo sapiens

<400> 784
Arg Glu Ile Gly Glu Leu Tyr Leu Pro Lys Phe
1               5                   10

<210> 785
<211> 22
<212> PRT
<213> Homo sapiens

<400> 785
Arg Asp Tyr Asn Leu Asn Asp Ile Leu Leu Gln Leu Gly Ile Glu Glu
1               5                   10                  15

Ala Phe Thr Ser Lys Ala
            20

<210> 786
<211> 12
<212> PRT
<213> Homo sapiens

<400> 786

Lys Ala Asp Leu Ser Gly Ile Thr Gly Ala Arg Asn
1               5                   10

<210> 787
<211> 21
<212> PRT
<213> Homo sapiens

<400> 787
Lys Ala Val Leu Asp Val Phe Glu Glu Gly Thr Glu Ala Ser Ala Ala
1               5                   10                  15

Thr Ala Val Lys Ile
            20

<210> 788
<211> 16
<212> PRT
<213> Homo sapiens

<400> 788
Lys Leu Ala Ala Ala Val Ser Asn Phe Gly Tyr Asp Leu Tyr Arg Val
1               5                   10                  15

<210> 789
<211> 19
<212> PRT
<213> Homo sapiens

<400> 789
Arg Ala Leu Tyr Tyr Asp Leu Ile Ser Ser Pro Asp Ile His Gly Thr
1               5                   10                  15

Tyr Lys Glu

<210> 790
<211> 13
<212> PRT
<213> Homo sapiens

<400> 790
Lys Glu Leu Leu Asp Thr Val Thr Ala Pro Gln Lys Asn
1               5                   10

<210> 791
<211> 11
<212> PRT
<213> Homo sapiens

<400> 791
Lys Ser Ser Phe Val Ala Pro Leu Glu Lys Ser
1               5                   10

<210> 792

<211> 19
<212> PRT
<213> Homo sapiens

<400> 792
Lys Glu Ile Pro Asp Glu Ile Ser Ile Leu Leu Leu Gly Val Ala His
1               5                   10                  15

Phe Lys Gly

<210> 793
<211> 14
<212> PRT
<213> Homo sapiens

<400> 793
Lys Thr Ser Leu Glu Asp Phe Tyr Leu Asp Glu Glu Arg Thr
1               5                   10

<210> 794
<211> 24
<212> PRT
<213> Homo sapiens

<400> 794
Lys Val Thr Gln Asn Leu Thr Leu Ile Glu Glu Ser Leu Thr Ser Glu
1               5                   10                  15

Phe Ile His Asp Ile Asp Arg Glu
            20

<210> 795
<211> 12
<212> PRT
<213> Homo sapiens

<400> 795
Lys Thr Val Gln Ala Val Leu Thr Val Pro Lys Leu
1               5                   10

<210> 796
<211> 11
<212> PRT
<213> Homo sapiens

<400> 796
Lys Leu Ser Tyr Glu Gly Glu Val Thr Lys Ser
1               5                   10

<210> 797
<211> 14
<212> PRT
<213> Homo sapiens

<400> 797

Lys Leu Gln Ser Leu Phe Asp Ser Pro Asp Phe Ser Lys Ile
1               5                   10

<210> 798
<211> 14
<212> PRT
<213> Homo sapiens

<400> 798
Arg Asp Thr Asp Thr Gly Ala Leu Leu Phe Ile Gly Lys Ile
1               5                   10

<210> 799
<211> 12
<212> PRT
<213> Homo sapiens

<400> 799
Asp Thr Asp Thr Gly Ala Leu Leu Phe Ile Gly Lys
1               5                   10

<210> 800
<211> 10
<212> PRT
<213> Homo sapiens

<400> 800
Val Glu His Ser Asp Leu Ser Phe Ser Lys
1               5                   10

<210> 801
<211> 22
<212> PRT
<213> Homo sapiens

<400> 801
Ala Leu Ala Leu Pro Pro Leu Gly Leu Ala Pro Leu Leu Asn Leu Trp
1               5                   10                  15

Ala Lys Pro Gln Gly Arg
            20

<210> 802
<211> 5
<212> PRT
<213> Homo sapiens

<400> 802
Phe Leu Tyr His Lys
1               5

<210> 803
<211> 14
<212> PRT
<213> Homo sapiens

<400> 803
Glu Val Phe Ser Lys Pro Ile Ser Trp Glu Glu Leu Leu Gln
1               5                   10

<210> 804
<211> 15
<212> PRT
<213> Homo sapiens

<400> 804
Phe Ile Cys Pro Leu Thr Gly Leu Trp Pro Ile Asn Thr Leu Lys
1               5                   10                  15

<210> 805
<211> 20
<212> PRT
<213> Homo sapiens

<400> 805
Asp Val Leu Leu Leu Val His Asn Leu Pro Gln Asn Leu Thr Gly His
1               5                   10                  15

Ile Trp Tyr Lys
            20

<210> 806
<211> 6
<212> PRT
<213> Homo sapiens

<400> 806
Thr Leu Ala Phe Val Arg
1               5

<210> 807
<211> 14
<212> PRT
<213> Homo sapiens

<400> 807
Glu Leu Ile Glu Glu Leu Val Asn Ile Thr Gln Asn Gln Lys
1               5                   10

<210> 808
<211> 9
<212> PRT
<213> Homo sapiens

<400> 808
Glu Leu Pro Gln Ser Ile Val Tyr Lys
1               5

<210> 809
<211> 7
<212> PRT
<213> Homo sapiens

<400> 809
Val Val Glu Ser Leu Ala Lys
1               5


<210> 810
<211> 8
<212> PRT
<213> Homo sapiens

<400> 810
Tyr Gly Ile Glu Glu His Gly Lys
1               5


<210> 811
<211> 13
<212> PRT
<213> Homo sapiens

<400> 811
Leu Pro Asp Thr Pro Gln Gly Leu Leu Gly Glu Ala Arg
1               5                   10


<210> 812
<211> 12
<212> PRT
<213> Homo sapiens

<400> 812
Thr Asn Thr Asn Glu Phe Leu Ile Asp Val Asp Lys
1               5                   10


<210> 813
<211> 11
<212> PRT
<213> Homo sapiens

<400> 813
Ala Ile Gly Leu Pro Glu Glu Leu Ile Gln Lys
1               5                   10


<210> 814
<211> 16
<212> PRT
<213> Homo sapiens

<400> 814
Ala Glu His Pro Thr Trp Gly Asp Glu Gln Leu Phe Gln Thr Thr Arg
1               5                   10                  15


<210> 815
<211> 11
<212> PRT
<213> Homo sapiens

<400> 815
Thr Phe Leu Thr Val Tyr Trp Thr Pro Glu Arg

1               5                   10


<210> 816
<211> 10
<212> PRT
<213> Homo sapiens

<400> 816
Ser Glu Pro Arg Pro Gly Val Leu Leu Arg
1               5                   10


<210> 817
<211> 7
<212> PRT
<213> Homo sapiens

<400> 817
Ala Leu Asp Leu Ser Leu Lys
1               5


<210> 818
<211> 13
<212> PRT
<213> Homo sapiens

<400> 818
Asn Cys Ser Phe Ser Ile Ile Tyr Pro Val Val Ile Lys
1               5                   10


<210> 819
<211> 8
<212> PRT
<213> Homo sapiens

<400> 819
Ile Pro Ser Asn Pro Ser His Arg
1               5


<210> 820
<211> 6
<212> PRT
<213> Homo sapiens

<400> 820
Thr Leu Pro Phe Ser Arg
1               5


<210> 821
<211> 22
<212> PRT
<213> Homo sapiens

<400> 821
Ala Ala Leu Ala Ala Phe Asn Ala Gln Asn Asn Gly Ser Asn Phe Gln
1               5                   10                  15

Leu Glu Glu Ile Ser Arg
            20

<210> 822
<211> 19
<212> PRT
<213> Homo sapiens

<400> 822
Asn Asn Gln Leu Val Ala Gly Tyr Leu Gln Gly Pro Asn Val Asn Leu
1               5                   10                  15

Glu Glu Lys

<210> 823
<211> 9
<212> PRT
<213> Homo sapiens

<400> 823
Glu His Ser Ser Leu Ala Phe Trp Lys
1               5

<210> 824
<211> 11
<212> PRT
<213> Homo sapiens

<400> 824
Asn Phe Pro Ser Pro Val Asp Ala Ala Phe Arg
1               5                   10

<210> 825
<211> 14
<212> PRT
<213> Homo sapiens

<400> 825
Trp Asn Phe Ala Tyr Trp Ala Ala His Gln Pro Trp Ser Arg
1               5                   10

<210> 826
<211> 10
<212> PRT
<213> Homo sapiens

<400> 826
Thr Ala Val Thr Ala Asn Leu Asp Ile Arg
1               5                   10

<210> 827
<211> 10
<212> PRT
<213> Homo sapiens

<400> 827
Tyr Asn Ser Gln Leu Leu Ser Phe Val Arg
1               5                   10

<210> 828
<211> 25
<212> PRT
<213> Homo sapiens

<400> 828
Asp Leu Tyr His Tyr Ile Thr Ser Tyr Val Val Asp Gly Glu Ile Ile
1               5                   10                  15

Ile Tyr Gly Pro Ala Tyr Ser Gly Arg
            20                  25

<210> 829
<211> 7
<212> PRT
<213> Homo sapiens

<400> 829
Tyr Gln Ile Ser Val Asn Lys
1               5

<210> 830
<211> 10
<212> PRT
<213> Homo sapiens

<400> 830
Leu Asn Ile Gly Tyr Ile Glu Asp Leu Lys
1               5                   10

<210> 831
<211> 18
<212> PRT
<213> Homo sapiens

<400> 831
Glu Asn Pro Ala Val Ile Asp Phe Glu Leu Ala Pro Ile Val Asp Leu
1               5                   10                  15

Val Arg

<210> 832
<211> 7
<212> PRT
<213> Homo sapiens

<400> 832
Tyr Tyr Leu Gln Gly Ala Lys
1               5

<210> 833
<211> 9
<212> PRT
<213> Homo sapiens

<400> 833
Ile Thr Gly Phe Leu Lys Pro Gly Lys
1               5

<210> 834
<211> 21
<212> PRT
<213> Homo sapiens

<400> 834
His Glu Leu Thr Asp Glu Glu Leu Gln Ser Leu Phe Thr Asn Phe Ala
1               5                   10                  15

Asn Val Val Asp Lys
            20

<210> 835
<211> 6
<212> PRT
<213> Homo sapiens

<400> 835
Phe Leu Asn Trp Ile Lys
1               5

<210> 836
<211> 8
<212> PRT
<213> Homo sapiens

<400> 836
Ala Glu Ile Glu Tyr Leu Glu Lys
1               5

<210> 837
<211> 7
<212> PRT
<213> Homo sapiens

<400> 837
Val Gln Glu Val Leu Leu Lys
1               5

<210> 838
<211> 10
<212> PRT
<213> Homo sapiens

<400> 838
Thr Gln Ile Asp Ser Pro Leu Ser Gly Lys
1               5                   10

<210> 839
<211> 10
<212> PRT
<213> Homo sapiens

<400> 839
Asp Phe Asn Gln Phe Ser Ser Gly Glu Lys
1               5                   10


<210> 840
<211> 8
<212> PRT
<213> Homo sapiens

<400> 840
Leu Leu Glu Leu Thr Gly Pro Lys
1               5


<210> 841
<211> 17
<212> PRT
<213> Homo sapiens

<400> 841
Asn Glu Ile Val Phe Pro Ala Gly Ile Leu Gln Ala Pro Phe Tyr Thr
1               5                   10                  15


Arg




<210> 842
<211> 17
<212> PRT
<213> Homo sapiens

<400> 842
Glu Phe Asp Asp Asp Thr Tyr Asp Asn Asp Ile Ala Leu Leu Gln Leu
1               5                   10                  15


Lys




<210> 843
<211> 13
<212> PRT
<213> Homo sapiens

<400> 843
Phe Ser Leu Val Ser Gly Trp Gly Gln Leu Leu Asp Arg
1               5                   10


<210> 844
<211> 19
<212> PRT
<213> Homo sapiens

<400> 844
Leu Ile Gln Asp Ala Val Thr Gly Leu Thr Val Asn Gly Gln Ile Thr
1               5                   10                  15

Gly Asp Lys

<210> 845
<211> 10
<212> PRT
<213> Homo sapiens

<400> 845
Gln Gly His Asn Ser Val Phe Leu Ile Lys
1               5                   10

<210> 846
<211> 7
<212> PRT
<213> Homo sapiens

<400> 846
Ile Leu Pro Ser Val Pro Lys
1               5

<210> 847
<211> 7
<212> PRT
<213> Homo sapiens

<400> 847
Ser Thr Leu Phe Val Pro Arg
1               5

<210> 848
<211> 13
<212> PRT
<213> Homo sapiens

<400> 848
Gly Val Thr Gly Tyr Phe Thr Phe Asn Leu Tyr Leu Lys
1               5                   10

<210> 849
<211> 11
<212> PRT
<213> Homo sapiens

<400> 849
Glu Ala Leu Val Pro Leu Val Ala Asp His Lys
1               5                   10

<210> 850
<211> 8
<212> PRT

<213> Homo sapiens

<400> 850
Ser Phe Arg Pro Phe Val Pro Arg
1               5

<210> 851
<211> 19
<212> PRT
<213> Homo sapiens

<400> 851
Gly Ser Leu Val Gln Ala Ser Glu Ala Asn Leu Gln Ala Ala Gln Asp
1               5                   10                  15

Phe Val Arg

<210> 852
<211> 11
<212> PRT
<213> Homo sapiens

<400> 852
Ile Thr Leu Pro Asp Phe Thr Gly Asp Leu Arg
1               5                   10

<210> 853
<211> 9
<212> PRT
<213> Homo sapiens

<400> 853
Asp Ile Ile Lys Pro Asp Pro Pro Lys
1               5

<210> 854
<211> 13
<212> PRT
<213> Homo sapiens

<400> 854
Gln Gly Phe Gly Asn Val Ala Thr Asn Thr Asp Gly Lys
1               5                   10

<210> 855
<211> 8
<212> PRT
<213> Homo sapiens

<400> 855
Ala Val Leu His Ile Gly Glu Lys
1               5

<210> 856
<211> 11

<212> PRT
<213> Homo sapiens

<400> 856
Tyr Glu Asn Tyr Thr Ser Ser Phe Phe Ile Arg
1               5                   10


<210> 857
<211> 19
<212> PRT
<213> Homo sapiens

<400> 857
Gly Leu Gln Tyr Ala Ala Gln Glu Gly Leu Leu Ala Leu Gln Ser Glu
1               5                   10                  15


Leu Leu Arg


<210> 858
<211> 14
<212> PRT
<213> Homo sapiens

<400> 858
Val Glu Leu Ala Pro Leu Pro Ser Trp Gln Pro Val Gly Lys
1               5                   10


<210> 859
<211> 12
<212> PRT
<213> Homo sapiens

<400> 859
Cys Arg Pro Ile Asn Ala Thr Leu Ala Val Glu Lys
1               5                   10


<210> 860
<211> 9
<212> PRT
<213> Homo sapiens

<400> 860
Gly Ile Val Glu Glu Cys Cys Phe Arg
1               5


<210> 861
<211> 7
<212> PRT
<213> Homo sapiens

<400> 861
Ser Ile Leu Phe Leu Gly Lys
1               5


<210> 862

<211> 6
<212> PRT
<213> Homo sapiens

<400> 862
His Val Val Gln Leu Arg
1               5


<210> 863
<211> 10
<212> PRT
<213> Homo sapiens

<400> 863
Thr Gln Ile Leu Glu Trp Ala Ala Glu Arg
1               5                   10


<210> 864
<211> 9
<212> PRT
<213> Homo sapiens

<400> 864
Asn Ser Asp Gln Glu Ile Asp Phe Lys
1               5


<210> 865
<211> 16
<212> PRT
<213> Homo sapiens

<400> 865
Ser Gly Ala Gln Ala Thr Trp Thr Glu Leu Pro Trp Pro His Glu Lys
1               5                   10                  15


<210> 866
<211> 8
<212> PRT
<213> Homo sapiens

<400> 866
Val Arg Pro Gln Gln Leu Val Lys
1               5


<210> 867
<211> 11
<212> PRT
<213> Homo sapiens

<400> 867
Ser Glu Tyr Gly Ala Ala Leu Ala Trp Glu Lys
1               5                   10


<210> 868
<211> 8
<212> PRT
<213> Homo sapiens

<400> 868
Leu Glu Glu His Tyr Glu Leu Arg
1               5

<210> 869
<211> 10
<212> PRT
<213> Homo sapiens

<400> 869
Ile His Trp Glu Ser Ala Ser Leu Leu Arg
1               5                   10

<210> 870
<211> 19
<212> PRT
<213> Homo sapiens

<400> 870
Asn Ala Val Val Gln Gly Leu Glu Gln Pro His Gly Leu Val Val His
1               5                   10                  15

Pro Leu Arg

<210> 871
<211> 18
<212> PRT
<213> Homo sapiens

<400> 871
Val Thr Gly Leu Asp Phe Ile Pro Gly Leu His Pro Ile Leu Thr Leu
1               5                   10                  15

Ser Lys

<210> 872
<211> 8
<212> PRT
<213> Homo sapiens

<400> 872
Ala Leu Val Leu Glu Leu Ala Lys
1               5

<210> 873
<211> 11
<212> PRT
<213> Homo sapiens

<400> 873
Ser Leu Gln Ala Phe Val Ala Val Ala Ala Arg
1               5                   10

<210> 874
<211> 10
<212> PRT
<213> Homo sapiens

<400> 874
Ala Gly Phe Ala Gly Asp Asp Ala Pro Arg
1               5                   10


<210> 875
<211> 6
<212> PRT
<213> Homo sapiens

<400> 875
Tyr Asn Gln Leu Leu Arg
1               5


<210> 876
<211> 11
<212> PRT
<213> Homo sapiens

<400> 876
Ala Gln Glu Thr Ser Gly Glu Glu Ile Ser Lys
1               5                   10


<210> 877
<211> 12
<212> PRT
<213> Homo sapiens

<400> 877
Asp Ser Pro Ser Val Trp Ala Ala Val Pro Gly Lys
1               5                   10


<210> 878
<211> 25
<212> PRT
<213> Homo sapiens

<400> 878
Glu Thr Pro Glu Gly Ala Glu Ala Lys Pro Trp Tyr Glu Pro Ile Tyr
1               5                   10                  15


Leu Gly Gly Val Phe Gln Leu Glu Lys
            20                  25


<210> 879
<211> 11
<212> PRT
<213> Homo sapiens

<400> 879
Ala Val Gly Tyr Leu Ile Thr Gly Tyr Gln Arg
1               5                   10

<210> 880
<211> 10
<212> PRT
<213> Homo sapiens

<400> 880
Asp Ile Pro His Trp Leu Asn Pro Thr Arg
1               5                   10


<210> 881
<211> 10
<212> PRT
<213> Homo sapiens

<400> 881
Gln Thr Leu Ser Trp Thr Val Thr Pro Lys
1               5                   10


<210> 882
<211> 14
<212> PRT
<213> Homo sapiens

<400> 882
Ile Gln His Pro Phe Thr Val Glu Glu Phe Val Leu Pro Lys
1               5                   10


<210> 883
<211> 6
<212> PRT
<213> Homo sapiens

<400> 883
Asn Glu Ile Trp Tyr Arg
1               5


<210> 884
<211> 12
<212> PRT
<213> Homo sapiens

<400> 884
Ala Phe Gln Val Trp Ser Asp Val Thr Pro Leu Arg
1               5                   10


<210> 885
<211> 8
<212> PRT
<213> Homo sapiens

<400> 885
His Tyr Ile Asn Leu Ile Thr Arg
1               5


<210> 886

<211> 13
<212> PRT
<213> Homo sapiens

<400> 886
Trp Trp Gly Gly Gln Pro Leu Trp Ile Thr Ala Thr Lys
1               5                   10


<210> 887
<211> 13
<212> PRT
<213> Homo sapiens

<400> 887
Leu Asp Gly Ser Thr His Leu Asn Ile Phe Phe Ala Lys
1               5                   10


<210> 888
<211> 11
<212> PRT
<213> Homo sapiens

<400> 888
Asn His Tyr Thr Glu Ser Ile Ser Val Ala Lys
1               5                   10


<210> 889
<211> 16
<212> PRT
<213> Homo sapiens

<400> 889
Val Ser Phe Ser Ser Pro Leu Val Ala Ile Ser Gly Val Ala Leu Arg
1               5                   10                  15


<210> 890
<211> 12
<212> PRT
<213> Homo sapiens

<400> 890
Ala Leu Gln Asp Gln Leu Val Leu Val Ala Ala Lys
1               5                   10


<210> 891
<211> 12
<212> PRT
<213> Homo sapiens

<400> 891
Asp Pro Thr Phe Ile Pro Ala Pro Ile Gln Ala Lys
1               5                   10


<210> 892
<211> 6
<212> PRT
<213> Homo sapiens

<400> 892
Leu Ser Glu Thr Asn Arg
1               5


<210> 893
<211> 8
<212> PRT
<213> Homo sapiens

<400> 893
Thr Gly Ala Gln Glu Leu Leu Arg
1               5


<210> 894
<211> 9
<212> PRT
<213> Homo sapiens

<400> 894
Ile His Pro Ser Tyr Thr Asn Tyr Arg
1               5


<210> 895
<211> 22
<212> PRT
<213> Homo sapiens

<400> 895
Ala Gln Pro Val Gln Val Ala Glu Gly Ser Glu Pro Asp Gly Phe Trp
1               5                   10                  15


Glu Ala Leu Gly Gly Lys
            20


<210> 896
<211> 22
<212> PRT
<213> Homo sapiens

<400> 896
Ser Phe Glu Gly Leu Gly Gln Leu Glu Val Leu Thr Leu Asp His Asn
1               5                   10                  15


Gln Leu Gln Glu Val Lys
            20


<210> 897
<211> 7
<212> PRT
<213> Homo sapiens

<400> 897
Gln Ile Asn Ser Tyr Val Lys
1               5

<210> 898
<211> 12
<212> PRT
<213> Homo sapiens

<400> 898
Trp Ile Leu Thr Ala Ala His Thr Leu Tyr Pro Lys
1               5                   10


<210> 899
<211> 9
<212> PRT
<213> Homo sapiens

<400> 899
Ala Lys Pro Ala Leu Glu Asp Leu Arg
1               5


<210> 900
<211> 14
<212> PRT
<213> Homo sapiens

<400> 900
Ala Val Asp Ile Pro Gly Leu Glu Ala Ala Thr Pro Tyr Arg
1               5                   10


<210> 901
<211> 10
<212> PRT
<213> Homo sapiens

<400> 901
Leu Ser Ile Thr Gly Thr Tyr Asp Leu Lys
1               5                   10


<210> 902
<211> 12
<212> PRT
<213> Homo sapiens

<400> 902
Phe Leu Ile Pro Asn Ala Ser Gln Ala Glu Ser Lys
1               5                   10


<210> 903
<211> 9
<212> PRT
<213> Homo sapiens

<400> 903
Phe Gln Ser Val Phe Thr Val Thr Arg
1               5


<210> 904
<211> 8

<212> PRT
<213> Homo sapiens

<400> 904
Ile Asn Pro Ala Ser Leu Asp Lys
1               5


<210> 905
<211> 11
<212> PRT
<213> Homo sapiens

<400> 905
Ile Pro Lys Pro Glu Ala Ser Phe Ser Pro Arg
1               5                   10


<210> 906
<211> 8
<212> PRT
<213> Homo sapiens

<400> 906
Ile Thr Gln Asp Ala Gln Leu Lys
1               5


<210> 907
<211> 8
<212> PRT
<213> Homo sapiens

<400> 907
Thr Ser Tyr Gln Val Tyr Ser Lys
1               5


<210> 908
<211> 20
<212> PRT
<213> Homo sapiens

<400> 908
Asp Val Leu Leu Leu Val His Asn Leu Pro Gln Asn Leu Pro Gly Tyr
1               5                   10                  15


Phe Trp Tyr Lys
            20


<210> 909
<211> 7
<212> PRT
<213> Homo sapiens

<400> 909
Ala Gly Pro Leu Gln Ala Arg
1               5


<210> 910

<211> 8
<212> PRT
<213> Homo sapiens

<400> 910
Phe Gln Leu Ser Glu Thr Asn Arg
1               5


<210> 911
<211> 10
<212> PRT
<213> Homo sapiens

<400> 911
Leu Ile Glu Ile Ala Asn His Val Asp Lys
1               5                   10


<210> 912
<211> 7
<212> PRT
<213> Homo sapiens

<400> 912
Val Phe Gln Phe Leu Glu Lys
1               5


<210> 913
<211> 14
<212> PRT
<213> Homo sapiens

<400> 913
Val Pro Gly Leu Tyr Tyr Phe Thr Tyr His Ala Ser Ser Arg
1               5                   10


<210> 914
<211> 16
<212> PRT
<213> Homo sapiens

<400> 914
Val Ser Ala Pro Ser Gly Thr Gly His Leu Pro Gly Leu Asn Pro Leu
1               5                   10                  15


<210> 915
<211> 7
<212> PRT
<213> Homo sapiens

<400> 915
Ala Gly Ile Thr Ile Pro Arg
1               5


<210> 916
<211> 8
<212> PRT
<213> Homo sapiens

<400> 916
Ile Arg Pro Phe Phe Pro Gln Gln
1               5

<210> 917
<211> 18
<212> PRT
<213> Homo sapiens

<400> 917
Ser Glu Thr Glu Ile His Gln Gly Phe Gln His Leu His Gln Leu Phe
1               5                   10                  15

Ala Lys

<210> 918
<211> 8
<212> PRT
<213> Homo sapiens

<400> 918
Thr Glu Gln Ala Ala Val Ala Arg
1               5

<210> 919
<211> 11
<212> PRT
<213> Homo sapiens

<400> 919
Thr Leu Leu Pro Val Ser Lys Pro Glu Ile Arg
1               5                   10

<210> 920
<211> 7
<212> PRT
<213> Homo sapiens

<400> 920
Tyr Ser His Tyr Asn Glu Arg
1               5

<210> 921
<211> 19
<212> PRT
<213> Homo sapiens

<400> 921
Ala Asn Asp Gln Tyr Leu Thr Ala Ala Ala Leu His Asn Leu Asp Glu
1               5                   10                  15

Ala Val Lys

<210> 922
<211> 11
<212> PRT
<213> Homo sapiens

<400> 922
Ala Thr Trp Ser Gly Ala Val Leu Ala Gly Arg
1               5                   10


<210> 923
<211> 10
<212> PRT
<213> Homo sapiens

<400> 923
Thr Tyr Leu His Thr Tyr Glu Ser Glu Ile
1               5                   10


<210> 924
<211> 9
<212> PRT
<213> Homo sapiens

<400> 924
Tyr Tyr Gly Tyr Thr Gly Ala Phe Arg
1               5


<210> 925
<211> 7
<212> PRT
<213> Homo sapiens

<400> 925
Glu Leu Ala Asn Thr Ile Lys
1               5


<210> 926
<211> 12
<212> PRT
<213> Homo sapiens

<400> 926
Gly Glu Val Thr Tyr Thr Thr Ser Gln Val Ser Lys
1               5                   10


<210> 927
<211> 11
<212> PRT
<213> Homo sapiens

<400> 927
Leu Ser Asn Glu Asn His Gly Ile Ala Gln Arg
1               5                   10


<210> 928
<211> 13

<212> PRT
<213> Homo sapiens

<400> 928
Ile Arg Pro His Thr Phe Thr Gly Leu Ser Gly Leu Arg
1               5                   10


<210> 929
<211> 10
<212> PRT
<213> Homo sapiens

<400> 929
Glu Ala Gln Leu Pro Val Ile Glu Asn Lys
1               5                   10


<210> 930
<211> 26
<212> PRT
<213> Homo sapiens

<400> 930
Gln Arg Pro Pro Asp Leu Asp Thr Ser Ser Asn Ala Val Asp Leu Leu
1               5                   10                  15


Phe Phe Thr Asp Glu Ser Gly Asp Ser Arg
            20                  25


<210> 931
<211> 7
<212> PRT
<213> Homo sapiens

<400> 931
Val Gln Thr Ala His Phe Lys
1               5


<210> 932
<211> 15
<212> PRT
<213> Homo sapiens

<400> 932
Ala Asn Leu Ile Asn Asn Ile Phe Glu Leu Ala Gly Leu Gly Lys
1               5                   10                  15


<210> 933
<211> 19
<212> PRT
<213> Homo sapiens

<400> 933
Ala His Gln Leu Ala Ile Asp Thr Tyr Gln Glu Phe Glu Glu Thr Tyr
1               5                   10                  15


Ile Pro Lys

<210> 934
<211> 9
<212> PRT
<213> Homo sapiens

<400> 934
Asp Thr Tyr Val Ser Ser Phe Pro Arg
1               5


<210> 935
<211> 18
<212> PRT
<213> Homo sapiens

<400> 935
Thr Pro Ser Ala Ala Tyr Leu Trp Val Gly Thr Gly Ala Ser Glu Ala
1               5                   10                  15


Glu Lys


<210> 936
<211> 14
<212> PRT
<213> Homo sapiens

<400> 936
Glu Gln Leu Gly Glu Phe Tyr Glu Ala Leu Asp Cys Leu Arg
1               5                   10


<210> 937
<211> 16
<212> PRT
<213> Homo sapiens

<400> 937
Ser Asn Pro Val Thr Leu Asn Val Leu Tyr Gly Pro Asp Leu Pro Arg
1               5                   10                  15


<210> 938
<211> 13
<212> PRT
<213> Homo sapiens

<400> 938
Leu Trp Ala Tyr Leu Thr Ile Gln Glu Leu Leu Ala Lys
1               5                   10


<210> 939
<211> 11
<212> PRT
<213> Homo sapiens

<400> 939

Val Ala Asn Tyr Val Asp Trp Ile Asn Asp Arg
1               5                   10

<210> 940
<211> 22
<212> PRT
<213> Homo sapiens

<400> 940
Gly Ala Val His Val Val Val Ala Glu Thr Asp Tyr Gln Ser Phe Ala
1               5                   10                  15

Val Leu Tyr Leu Glu Arg
            20

<210> 941
<211> 25
<212> PRT
<213> Homo sapiens

<400> 941
Tyr His Phe Glu Ala Leu Ala Asp Thr Gly Ile Ser Ser Glu Phe Tyr
1               5                   10                  15

Asp Asn Ala Asn Asp Leu Leu Ser Lys
            20                  25

<210> 942
<211> 16
<212> PRT
<213> Homo sapiens

<400> 942
Ser Cys Asp Leu Ala Leu Leu Glu Thr Tyr Cys Ala Thr Pro Ala Lys
1               5                   10                  15

<210> 943
<211> 12
<212> PRT
<213> Homo sapiens

<400> 943
Tyr Val Val Ile Ser Gln Gly Leu Asp Lys Pro Arg
1               5                   10

<210> 944
<211> 10
<212> PRT
<213> Homo sapiens

<400> 944
Leu Leu Asp Phe Glu Phe Ser Ser Gly Arg
1               5                   10

<210> 945

<211> 13
<212> PRT
<213> Homo sapiens

<400> 945
Thr Ala Thr Ser Glu Tyr Gln Thr Phe Phe Asn Pro Arg
1               5                   10


<210> 946
<211> 11
<212> PRT
<213> Homo sapiens

<400> 946
Asn Val Asn Gln Ser Leu Leu Glu Leu His Lys
1               5                   10


<210> 947
<211> 9
<212> PRT
<213> Homo sapiens

<400> 947
Leu His Lys Pro Gly Val Tyr Thr Arg
1               5


<210> 948
<211> 10
<212> PRT
<213> Homo sapiens

<400> 948
Ser Glu Arg Pro Pro Ile Phe Glu Ile Arg
1               5                   10


<210> 949
<211> 11
<212> PRT
<213> Homo sapiens

<400> 949
Ala Leu Asn Ser Ile Ile Asp Val Tyr His Lys
1               5                   10


<210> 950
<211> 9
<212> PRT
<213> Homo sapiens

<400> 950
Leu Ser Ile Pro Gln Ile Thr Thr Lys
1               5


<210> 951
<211> 9
<212> PRT
<213> Homo sapiens

<400> 951
Leu Phe Ile Pro Gln Ile Thr Pro Lys
1               5

<210> 952
<211> 7
<212> PRT
<213> Homo sapiens

<400> 952
Phe Ile Val Gly Phe Thr Arg
1               5

<210> 953
<211> 23
<212> PRT
<213> Homo sapiens

<400> 953
Ala Phe Leu Glu Val Asn Glu Glu Gly Ser Glu Ala Ala Ala Ser Thr
1               5                   10                  15

Ala Val Val Ile Ala Gly Arg
            20

<210> 954
<211> 8
<212> PRT
<213> Homo sapiens

<400> 954
Phe Phe Gln Tyr Asp Thr Trp Lys
1               5

<210> 955
<211> 18
<212> PRT
<213> Homo sapiens

<400> 955
Gly Pro Ile Thr Ser Ala Ala Glu Leu Asn Asp Pro Gln Ser Ile Leu
1               5                   10                  15

Leu Arg

<210> 956
<211> 14
<212> PRT
<213> Homo sapiens

<400> 956
Glu Gln Ser Leu Asn Val Ser Gln Asp Leu Asp Thr Ile Arg
1               5                   10

<210> 957
<211> 13
<212> PRT
<213> Homo sapiens

<400> 957
Asp Ala Val Val Tyr Pro Ile Leu Val Glu Phe Thr Arg
1               5                   10


<210> 958
<211> 11
<212> PRT
<213> Homo sapiens

<400> 958
Ser Gly Val Asp Leu Ala Asp Ser Asn Gln Lys
1               5                   10


<210> 959
<211> 23
<212> PRT
<213> Homo sapiens

<400> 959
Thr Glu Phe Leu Ser Asn Tyr Leu Thr Asn Val Asp Asp Ile Thr Leu
1               5                   10                  15


Val Pro Gly Thr Leu Gly Arg
            20


<210> 960
<211> 16
<212> PRT
<213> Homo sapiens

<400> 960
Thr Glu Leu Arg Pro Gly Glu Thr Leu Asn Val Asn Phe Leu Leu Arg
1               5                   10                  15


<210> 961
<211> 9
<212> PRT
<213> Homo sapiens

<400> 961
Tyr Gly Phe Tyr Thr His Val Phe Arg
1               5


<210> 962
<211> 13
<212> PRT
<213> Homo sapiens

<400> 962
Phe Gly Phe Gly Gly Ser Thr Asp Ser Gly Pro Ile Arg
1               5                   10

<210> 963
<211> 16
<212> PRT
<213> Homo sapiens

<400> 963
Thr Phe Val Asn Ile Thr Pro Ala Glu Val Gly Val Leu Val Gly Lys
1               5                   10                  15

<210> 964
<211> 14
<212> PRT
<213> Homo sapiens

<400> 964
Val Ser Glu Ala Asp Ser Ser Asn Ala Asp Trp Val Thr Lys
1               5                   10

## Description

This application claims the benefit of priority to U.S. provisional patent application number 61/798,413, filed March 15, 2013, which is herein incorporated by reference in its entirety.

The invention relates generally to the field of personalized medicine and, more specifically to compositions and methods for determining the probability for preeclampsia in a pregnant female.

### BACKGROUND

Preeclampsia (PE), a pregnancy-specific multi-system disorder characterized by hypertension and excess protein excretion in the urine, is a leading cause of maternal and fetal morbidity and mortality worldwide. Preeclampsia affects at least 5-8% of all pregnancies and accounts for nearly 18% of maternal deaths in the United States. The disorder is probably multifactorial, although most cases of preeclampsia are characterized by abnormal maternal uterine vascular remodeling by fetally derived placental trophoblast cells.

Complications of preeclampsia can include compromised placental blood flow, placental abruption, eclampsia, HELLP syndrome (hemolysis, elevated liver enzymes and low platelet count), acute renal failure, cerebral hemorrhage, hepatic failure or rupture, pulmonary edema, disseminated intravascular coagulation and future cardiovascular disease. Even a slight increase in blood pressure can be a sign of preeclampsia. While symptoms can include swelling, sudden weight gain, headaches and changes in vision, some women remain asymptomatic.

Management of preeclampsia consists of two options: delivery or observation. Management decisions depend on the gestational age at which preeclampsia is diagnosed and the relative state of health of the fetus . The only cure for preeclampsia is delivery of the fetus and placenta. However, the decision to deliver involves balancing the potential benefit to the fetus of further *in utero* development with fetal and maternal risk of progressive disease, including the development of eclampsia, which is preeclampsia complicated by maternal seizures.

There is a great need to identify women at risk for preeclampsia as most currently available tests fail to predict the majority of women who eventually develop preeclampsia. Women identified as high-risk can be scheduled for more intensive antenatal surveillance and prophylactic interventions. Reliable early detection of preeclampsia would enable planning appropriate monitoring and clinical management, potentially providing the early identification of disease complications. Such monitoring and management might include: more frequent assessment of blood pressure and urinary protein concentration, uterine artery doppler measurement, ultrasound assessment of fetal growth and prophylactic treatment with aspirin. Finally, reliable antenatal identification of preeclampsia also is crucial to cost-effective allocation of monitoring resources.

The present invention addresses this need by providing compositions and methods for determining whether a pregnant woman is at risk for developing preeclampsia. Related advantages are provided as well.

### SUMMARY

The present invention provides compositions and methods for predicting the probability of preeclampsia in a pregnant female.

In one aspect, the invention provides a panel of isolated biomarkers comprising N of the biomarkers listed in Tables 2, 3, 4, 5 and 7 through 22. In some embodiments, N is a number selected from the group consisting of 2 to 24. In additional embodiments, the biomarker panel comprises at least two of the isolated biomarkers selected from the group consisting of FSVVYAK, SPELQAEAK, VNHVTLSQPK, SSNNPHSPIVEEFQVPYNK, and VVGGLVALR. In additional embodiments, the biomarker panel comprises at least two of the isolated biomarkers selected from the group consisting of LDFHFSSDR, TVQAVLTVPK, GPGEDFR, ETLLQDFR, ATVVYQGER, GFQALGDAADIR. In additional embodiments, the biomarker panel comprises at least two of the isolated biomarkers selected from the group consisting of FSVVYAK, SPELQAEAK, VNHVTLSQPK, SSNNPHSPIVEEFQVPYNK, VVGGLVALR, LDFHFSSDR, TVQAVLTVPK, GPGEDFR, ETLLQDFR, ATVVYQGER, and GFQALGDAADIR.

In some embodiments, the invention provides a biomarker panel comprising at least two of the isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4). In additional embodiments, the invention provides a biomarker panel comprising at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (CIS), and retinol binding protein 4 (RBP4 or RET4).

In some embodiments, the invention provides a biomarker panel comprising at least two of the isolated biomarkers selected from the group consisting of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

In other embodiments, the invention provides a biomarker panel comprising alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, subcomponent (C1S), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), and plasminogen (PLMN). In another aspect, the invention provides a biomarker panel comprising at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (CIS), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), and plasminogen (PLMN).

Also provided by the invention is a method of determining probability for preeclampsia in a pregnant female comprising detecting a measurable feature of each of N biomarkers selected from the biomarkers listed in Tables 2, 3, 4, 5 and 7 through 22 in a biological sample obtained from the pregnant female, and analyzing the measurable feature to determine the probability for preeclampsia in the pregnant female. In some embodiments, a measurable feature comprises fragments or derivatives of each of the N biomarkers selected from the biomarkers listed in Tables 2, 3, 4, 5 and 7 through 22. In some embodiments of the disclosed methods detecting a measurable feature comprises quantifying an amount of each of N biomarkers selected from the biomarkers listed in Tables 2, 3, 4, 5 and 7 through 22, combinations or portions and/or derivatives thereof in a biological sample obtained from the pregnant female. In additional embodiments, the disclosed methods of determining probability for preeclampsia in a pregnant female further encompass detecting a measurable feature for one or more risk indicia associated with preeclampsia.

In some embodiments, the disclosed methods of determining probability for preeclampsia in a pregnant female comprises detecting a measurable feature of each of N biomarkers, wherein N is selected from the group consisting of 2 to 24. In further embodiments, the disclosed methods of determining probability for preeclampsia in a pregnant female comprises detecting a measurable feature of each of at least two isolated biomarkers selected from the group consisting of FSVVYAK, SPELQAEAK, VNHVTLSQPK, SSNNPHSPIVEEFQVPYNK, and VVGGLVALR.

In further embodiments, the disclosed methods of determining probability for preeclampsia in a pregnant female comprises detecting a measurable feature of each of at least two isolated biomarkers selected from the group consisting of LDFHFSSDR, TVQAVLTVPK, GPGEDFR, ETLLQDFR, ATVVYQGER, GFQALGDAADIR.

In additional embodiments, the disclosed methods of determining probability for preeclampsia in a pregnant female comprises detecting a measurable feature of each of at least two isolated biomarkers selected from the group consisting of FSVVYAK, SPELQAEAK, VNHVTLSQPK, SSNNPHSPIVEEFQVPYNK, VVGGLVALR, LDFHFSSDR, TVQAVLTVPK, GPGEDFR, ETLLQDFR, ATVVYQGER, and GFQALGDAADIR.

In other embodiments, the disclosed methods of determining probability for preeclampsia in a pregnant female comprise detecting a measurable feature of each of at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (CIS), and retinol binding protein 4 (RBP4 or RET4).

In some embodiments, the disclosed methods of determining probability for preeclampsia in a pregnant female comprise detecting a measurable feature of each of at least two isolated biomarkers selected from the group consisting of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

In further embodiments, the disclosed methods of determining probability for preeclampsia in a pregnant female comprise detecting a measurable feature of each of at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, subcomponent (CIS), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), and plasminogen (PLMN).

In some embodiments of the methods of determining probability for preeclampsia in a pregnant female, the probability for preeclampsia in the pregnant female is calculated based on the quantified amount of each of N biomarkers selected from the biomarkers listed in Tables 2, 3, 4, 5 and 7 through 22. In some embodiments, the disclosed methods for determining the probability of preeclampsia encompass detecting and/or quantifying one or more biomarkers using mass sprectrometry, a capture agent or a combination thereof.

In some embodiments, the disclosed methods of determining probability for preeclampsia in a pregnant female encompass an initial step of providing a biomarker panel comprising N of the biomarkers listed in Tables 2, 3, 4, 5 and 7 through 22. In additional embodiments, the disclosed methods of determining probability for preeclampsia in a pregnant female encompass an initial step of providing a biological sample from the pregnant female.

In some embodiments, the disclosed methods of determining probability for preeclampsia in a pregnant female encompass communicating the probability to a health care provider. In additional embodiments, the communication informs a subsequent treatment decision for the pregnant female. In further embodiments, the treatment decision comprises one or more selected from the group of consisting of more frequent assessment of blood pressure and urinary protein concentration, uterine artery doppler measurement, ultrasound assessment of fetal growth and prophylactic treatment with aspirin.

In further embodiments, the disclosed methods of determining probability for preeclampsia in a pregnant female encompass analyzing the measurable feature of one or more isolated biomarkers using a predictive model. In some embodiments of the disclosed methods, a measurable feature of one or more isolated biomarkers is compared with a reference feature.

In additional embodiments, the disclosed methods of determining probability for preeclampsia in a pregnant female encompass using one or more analyses selected from a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, and a combination thereof. In one embodiment, the disclosed methods of determining probability for preeclampsia in a pregnant female encompasses logistic regression.

In some embodiments, the invention provides a method of determining probability for preeclampsia in a pregnant female encompasses quantifying in a biological sample obtained from the pregnant female an amount of each of N biomarkers selected from the biomarkers listed in Tables 2, 3, 4, 5 and 7 through 22; multiplying the amount by a predetermined coefficient, and determining the probability for preeclampsia in the pregnant female comprising adding the individual products to obtain a total risk score that corresponds to the probability.

Other features and advantages of the invention will be apparent from the detailed description, and from the claims.

### DETAILED DESCRIPTION

The present disclosure is based, in part, on the discovery that certain proteins and peptides in biological samples obtained from a pregnant female are differentially expressed in pregnant females that have an increased risk of developing in the future or presently suffering from preeclampsia relative to matched controls. The present disclosure is further based, in part, on the unexepected discovery that panels combining one or more of these proteins and peptides can be utilized in methods of determining the probability for preeclampsia in a pregnant female with relatively high sensitivity and specificity. These proteins and peptides dislosed herein serve as biomarkers for classifying test samples, predicting a probability of preeclampsia, monitoring of progress of preeclampsia in a pregnant female, either individually or in a panel of biomarkers.

The disclosure provides biomarker panels, methods and kits for determining the probability for preeclampsia in a pregnant female. One major advantage of the present disclosure is that risk of developing preeclampsia can be assessed early during pregnancy so that management of the condition can be initiated in a timely fashion. Sibai, Hypertension. In: Gabbe et al., eds. Obstetrics: Normal and Problem Pregnancies. 6th ed. Philadelphia, Pa: Saunders Elsevier; 2012:chap 35. The present invention is of particular benefit to asymptomatic females who would not otherwise be identified and treated.

By way of example, the present disclosure includes methods for generating a result useful in determining probability for preeclampsia in a pregnant female by obtaining a dataset associated with a sample, where the dataset at least includes quantitative data about biomarkers and panels of biomarkers that have been identified as predictive of preeclampsia, and inputting the dataset into an analytic process that uses the dataset to generate a result useful in determining probability for preeclampsia in a pregnant female. As described further below, this quantitative data can include amino acids, peptides, polypeptides, proteins, nucleotides, nucleic acids, nucleosides, sugars, fatty acids, steroids, metabolites, carbohydrates, lipids, hormones, antibodies, regions of interest that serve as surrogates for biological macromolecules and combinations thereof.

In addition to the specific biomarkers identified in this disclosure, for example, by accession number, sequence, or reference, the invention also contemplates contemplates use of biomarker variants that are at least 90% or at least 95% or at least 97% identical to the exemplified sequences and that are now known or later discover and that have utility for the methods of the invention. These variants may represent polymorphisms, splice variants, mutations, and the like. In this regard, the instant specification discloses multiple art-known proteins in the context of the invention and provides exemplary accession numbers associated with one or more public databases as well as exemplary references to published journal articles relating to these art-known proteins. However, those skilled in the art appreciate that additional accession numbers and journal articles can easily be identified that can provide additional characteristics of the disclosed biomarkers and that the exemplified references are in no way limiting with regard to the disclosed biomarkers. As described herein, various techniques and reagents find use in the methods of the present invention. Suitable samples in the context of the present invention include, for example, blood, plasma, serum, amniotic fluid, vaginal secretions, saliva, and urine. In some embodiments, the biological sample is selected from the group consisting of whole blood, plasma, and serum. In a particular embodiment, the biological sample is serum. As described herein, biomarkers can be detected through a variety of assays and techniques known in the art. As further described herein, such assays include, without limitation, mass spectrometry (MS)-based assays, antibody-based assays as well as assays that combine aspects of the two.

Protein biomarkers associated with the probability for preeclampsia in a pregnant female include, but are not limited to, one or more of the isolated biomarkers listed in Tables 2, 3, 4, 5, and 7 through 22. In addition to the specific biomarkers, the disclosure further includes biomarker variants that are about 90%, about 95%, or about 97% identical to the exemplified sequences. Variants, as used herein, include polymorphisms, splice variants, mutations, and the like.

Additional markers can be selected from one or more risk indicia, including but not limited to, maternal age, race, ethnicity, medical history, past pregnancy history, and obstetrical history. Such additional markers can include, for example, age, prepregnancy weight, ethnicity, race; the presence, absence or severity of diabetes, hypertension, heart disease, kidney disease; the incidence and/or frequency of prior preeclampsia, prior preeclampsia; the presence, absence, frequency or severity of present or past smoking, illicit drug use, alcohol use; the presence, absence or severity of bleeding after the 12th gestational week; cervical cerclage and transvaginal cervical length. Additional risk indicia useful for as markers can be identified using learning algorithms known in the art, such as linear discriminant analysis, support vector machine classification, recursive feature elimination, prediction analysis of microarray, logistic regression, CART, FlexTree, LART, random forest, MART, and/or survival analysis regression, which are known to those of skill in the art and are further described herein.

Provided herein are panels of isolated biomarkers comprising N of the biomarkers selected from the group listed in Tables 2, 3, 4, 5, and 7 through 22. In the disclosed panels of biomarkers N can be a number selected from the group consisting of 2 to 24. In the disclosed methods, the number of biomarkers that are detected and whose levels are determined, can be 1, or more than 1, such as 2, 3, 4, 5, 6, 7, 8, 9, 10, 11, 12, 12, 14, 15, 16, 17, 18, 19, 20, 21, 22, 23, 24, 25 or more. In certain embodiments, the number of biomarkers that are detected, and whose levels are determined, can be 1, or more than 1, such as 2, 3, 4, 5, 6, 7, 8, 9, 10, or more. The methods of this disclosure are useful for determining the probability for preeclampsia in a pregnant female.

While certain of the biomarkers listed in Tables 2, 3, 4, 5, and 7 through 22 are useful alone for determining the probability for preeclampsia in a pregnant female, methods are also described herein for the grouping of multiple subsets of the biomarkers that are each useful as a panel of three or more biomarkers. In some embodiments, the invention provides panels comprising N biomarkers, wherein N is at least three biomarkers. In other embodiments, N is selected to be any number from 3-23 biomarkers.

In yet other embodiments, N is selected to be any number from 2-5, 2-10, 2-15, 2-20, or 2-23. In other embodiments, N is selected to be any number from 3-5, 3-10, 3-15, 3-20, or 3-23. In other embodiments, N is selected to be any number from 4-5, 4-10, 4-15, 4-20, or 4-23. In other embodiments, N is selected to be any number from 5-10, 5-15, 5-20, or 5-23. In other embodiments, N is selected to be any number from 6-10, 6-15, 6-20, or 6-23. In other embodiments, N is selected to be any number from 7-10, 7-15, 7-20, or 7-23. In other embodiments, N is selected to be any number from 8-10, 8-15, 8-20, or 8-23. In other embodiments, N is selected to be any number from 9-10, 9-15, 9-20, or 9-23. In other embodiments, N is selected to be any number from 10-15, 10-20, or 10-23. It will be appreciated that N can be selected to encompass similar, but higher order, ranges.

In certain embodiments, the panel of isolated biomarkers comprises one or more, two or more, three or more, four or more, or five isolated biomarkers comprising an amino acid sequence selected from SPELQAEAK, SSNNPHSPIVEEFQVPYN, VNHVTLSQPK, VVGGLVALR, and FSVVYAK. In some embodiments, the panel of isolated biomarkers comprises one or more, two or more, three or more, four or more, five of the isolated biomarkers consisting of an amino acid sequence selected from SPELQAEAK, SSNNPHSPIVEEFQVPYN, VNHVTLSQPK, VVGGLVALR, and FSVVYAK.

In certain embodiments, the panel of isolated biomarkers comprises one or more, two or more, three or more, four or more, or five isolated biomarkers comprising an amino acid sequence selected from LDFHFSSDR, TVQAVLTVPK, GPGEDFR, ETLLQDFR, ATVVYQGER, GFQALGDAADIR. In some embodiments, the panel of isolated biomarkers comprises one or more, two or more, three or more, four or more, five of the isolated biomarkers consisting of an amino acid sequence selected from LDFHFSSDR, TVQAVLTVPK, GPGEDFR, ETLLQDFR, ATVVYQGER, GFQALGDAADIR.

In certain embodiments, the panel of isolated biomarkers comprises one or more, two or more, three or more, four or more, or five isolated biomarkers comprising an amino acid sequence selected from FSVVYAK, SPELQAEAK, VNHVTLSQPK, SSNNPHSPIVEEFQVPYNK, VVGGLVALR, LDFHFSSDR, TVQAVLTVPK, GPGEDFR, ETLLQDFR, ATVVYQGER, and GFQALGDAADIR. In some embodiments, the panel of isolated biomarkers comprises one or more, two or more, three or more, four or more, five of the isolated biomarkers consisting of an amino acid sequence selected from FSVVYAK, SPELQAEAK, VNHVTLSQPK, SSNNPHSPIVEEFQVPYNK, VVGGLVALR, LDFHFSSDR, TVQAVLTVPK, GPGEDFR, ETLLQDFR, ATVVYQGER, and GFQALGDAADIR.

In some embodiments, the panel of isolated biomarkers comprises one or more peptides comprising a fragment from alpha-1-microglobulin (AMBP) Traboni and Cortese, Nucleic Acids Res. 14 (15), 6340 (1986); ADP/ATP translocase 3 (ANT3) Cozens et al., J. Mol. Biol. 206 (2), 261-280 (1989) (NCBI Reference Sequence: NP_001627.2); apolipoprotein A-II (APOA2) Fullerton et al., Hum. Genet. 111 (1), 75-87 (2002) GenBank: AY100524.1); apolipoprotein B (APOB) Knott et al., Nature 323, 734 - 738 (1986) (GenBank: EAX00803.1); apolipoprotein C-III (APOC3), Fullerton et al., Hum. Genet. 115 (1), 36-56 (2004)(GenBank: AAS68230.1); beta-2-microglobulin (B2MG) Cunningham et al., Biochemistry 12 (24), 4811-4822 (1973) (GenBank: AI686916.1); complement component 1, s subcomponent (C1S) Mackinnon et al., Eur. J. Biochem. 169 (3), 547-553 (1987), and retinol binding protein 4 (RBP4 or RET4) Rask et al., Ann. N. Y. Acad. Sci. 359, 79-90 (1981) (UniProtKB/Swiss-Prot: P02753.3).

In some embodiments, the panel of isolated biomarkers comprises one or more peptides comprising a fragment from cell adhesion molecule with homology to L1CAM (close homolog of L1) (CHL1) (GenBank: AAI43497.1), complement component C5 (C5 or CO5) Haviland, J. Immunol. 146 (1), 362-368 (1991)(GenBank: AAA51925.1); Complement component C8 beta chain (C8B or CO8B) Howard et al., Biochemistry 26 (12), 3565-3570 (1987) (NCBI Reference Sequence: NP_000057.1), endothelin-converting enzyme 1 (ECE1) Xu et al., Cell 78 (3), 473-485 (1994) (NCBI Reference Sequence: NM_001397.2; NP_001388.1); coagulation factor XIII, B polypeptide (F13B) Grundmann et al., Nucleic Acids Res. 18 (9), 2817-2818 (1990) (NCBI Reference Sequence: NP_001985.2); Interleukin 5 (IL5), Murata et al., J. Exp. Med. 175 (2), 341-351 (1992) (NCBI Reference Sequence: NP_000870.1), Peptidase D (PEPD) Endo et al., J. Biol. Chem. 264 (8), 4476-4481 (1989) (UniProtKB/Swiss-Prot: P12955.3); Plasminogen (PLMN) Petersen et al., J. Biol. Chem. 265 (11), 6104-6111 (1990), (NCBI Reference Sequences: NP_000292.1 NP_001161810.1).

In additional embodiments, the invention provides a panel of isolated biomarkers comprising N of the biomarkers listed in Tables 2, 3, 4, 5 and 7 through 22. In some embodiments, N is a number selected from the group consisting of 2 to 24. In additional embodiments, the biomarker panel comprises at least two of the isolated biomarkers selected from the group consisting of FSVVYAK, SPELQAEAK, VNHVTLSQPK, SSNNPHSPIVEEFQVPYNK, and VVGGLVALR.

In further embodiments, the biomarker panel comprises at least two of the isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (CIS), and retinol binding protein 4 (RBP4 or RET4). In another embodiment, the invention provides a biomarker panel comprising at least three isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (CIS), and retinol binding protein 4 (RBP4 or RET4).

In further embodiments, the biomarker panel comprises at least two of the isolated biomarkers selected from the group consisting Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG). In another embodiment, the invention provides a biomarker panel comprising at least three isolated biomarkers selected from the group consisting of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

In some embodiments, the invention provides a biomarker panel comprising alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, subcomponent (C1S), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), and plasminogen (PLMN). In another aspect, the invention provides a biomarker panel comprising at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (CIS), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), and plasminogen (PLMN).

In some embodiments, the invention provides a biomarker panel comprising Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG). In another aspect, the invention provides a biomarker panel comprising at least two isolated biomarkers selected from the group consisting of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

As used in this application, including the appended claims, the singular forms "a," "an," and "the" include plural references, unless the content clearly dictates otherwise, and are used interchangeably with "at least one" and "one or more."

The term "about," particularly in reference to a given quantity, is meant to encompass deviations of plus or minus five percent.

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

As used herein, the term "panel" refers to a composition, such as an array or a collection, comprising one or more biomarkers. The term can also refer to a profile or index of expression patterns of one or more biomarkers described herein. The number of biomarkers useful for a biomarker panel is based on the sensitivity and specificity value for the particular combination of biomarker values.

As used herein, and unless otherwise specified, the terms "isolated" and "purified" generally describes a composition of matter that has been removed from its native environment (*e.g.,* the natural environment if it is naturally occurring), and thus is altered by the hand of man from its natural state. An isolated protein or nucleic acid is distinct from the way it exists in nature.

The term "biomarker" refers to a biological molecule, or a fragment of a biological molecule, the change and/or the detection of which can be correlated with a particular physical condition or state. The terms "marker" and "biomarker" are used interchangeably throughout the disclosure. For example, the biomarkers of the present invention are correlated with an increased likelihood of preeclampsia. Such biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (*e.g.,* glycoproteins, ribonucleoproteins, lipoproteins). The term also encompasses portions or fragments of a biological molecule, for example, peptide fragment of a protein or polypeptide that comprises at least 5 consecutive amino acid residues, at least 6 consecutive amino acid residues, at least 7 consecutive amino acid residues, at least 8 consecutive amino acid residues, at least 9 consecutive amino acid residues, at least 10 consecutive amino acid residues, at least 11 consecutive amino acid residues, at least 12 consecutive amino acid residues, at least 13 consecutive amino acid residues, at least 14 consecutive amino acid residues, at least 15 consecutive amino acid residues, at least 5 consecutive amino acid residues, at least 16 consecutive amino acid residues, at least 17consecutive amino acid residues, at least 18 consecutive amino acid residues, at least 19 consecutive amino acid residues, at least 20 consecutive amino acid residues, at least 21 consecutive amino acid residues, at least 22 consecutive amino acid residues, at least 23 consecutive amino acid residues, at least 24 consecutive amino acid residues, at least 25 consecutive amino acid residues,or more consecutive amino acid residues.

The invention also provides a method of determining probability for preeclampsia in a pregnant female, the method comprising detecting a measurable feature of each of N biomarkers selected from the biomarkers listed in Tables 2, 3, 4, 5 and 7 through 22 in a biological sample obtained from the pregnant female, and analyzing the measurable feature to determine the probability for preeclampsia in the pregnant female. As disclosed herein, a measurable feature comprises fragments or derivatives of each of said N biomarkers selected from the biomarkers listed in Tables 2, 3, 4, 5 and 7 through 22. In some embodiments of the disclosed methods detecting a measurable feature comprises quantifying an amount of each of N biomarkers selected from the biomarkers listed in Tables 2, 3, 4, 5 and 7 through 22, combinations or portions and/or derivatives thereof in a biological sample obtained from said pregnant female.

In some embodiments, the present invention describes a method for predicting the time to onset of preeclamspsia in a pregnant female, the method comprising: (a) obtaining a biological sample from said pregnant female; (b) quantifying an amount of each of N biomarkers selected from the biomarkers listed in Tables 2, 3, 4, 5 and 7 through 22 in said biological sample; (c) multiplying or thresholding said amount by a predetermined coefficient, (d) determining predicted onset of of said preeclampsia in said pregnant female comprising adding said individual products to obtain a total risk score that corresponds to said predicted onset of said preeclampsia in said pregnant female. Although described and exemplified with reference to methods of determining probability for preeclampsia in a pregnant female, the present disclosure is similarly applicable to the method of predicting time to onset of in a pregnant female. It will be apparent to one skilled in the art that each of the aforementioned methods has specific and substantial utilities and benefits with regard maternal-fetal health considerations.

In some embodiments, the method of determining probability for preeclampsia in a pregnant female comprises detecting a measurable feature of each of N biomarkers, wherein N is selected from the group consisting of 2 to 24. In further embodiments, the disclosed methods of determining probability for preeclampsia in a pregnant female comprises detecting a measurable feature of each of at least two isolated biomarkers selected from the group consisting of FSVVYAK, SPELQAEAK, VNHVTLSQPK, SSNNPHSPIVEEFQVPYNK, and VVGGLVALR.

In further embodiments, the disclosed methods of determining probability for preeclampsia in a pregnant female comprises detecting a measurable feature of each of at least two isolated biomarkers selected from the group consisting of LDFHFSSDR, TVQAVLTVPK, GPGEDFR, ETLLQDFR, ATVVYQGER, GFQALGDAADIR.

In further embodiments, the disclosed methods of determining probability for preeclampsia in a pregnant female comprises detecting a measurable feature of each of at least two isolated biomarkers selected from the group consisting of FSVVYAK, SPELQAEAK, VNHVTLSQPK, SSNNPHSPIVEEFQVPYNK, VVGGLVALR, LDFHFSSDR, TVQAVLTVPK, GPGEDFR, ETLLQDFR, ATVVYQGER, and GFQALGDAADIR

In additional embodiments, the method of determining probability for preeclampsia in a pregnant female comprises detecting a measurable feature of each of at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (CIS), and retinol binding protein 4 (RBP4 or RET4).

In additional embodiments, the method of determining probability for preeclampsia in a pregnant female comprises detecting a measurable feature of each of at least two isolated biomarkers selected from the group consisting of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

In further embodiments, the disclosed method of determining probability for preeclampsia in a pregnant female comprises detecting a measurable feature of each of at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, subcomponent (CIS), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), plasminogen (PLMN), of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

In additional embodiments, the methods of determining probability for preeclampsia in a pregnant female further encompass detecting a measurable feature for one or more risk indicia associated with preeclampsia. In additional embodiments the risk indicia are selected form the group consisting of history of preeclampsia, first pregnancy, age, obesity, diabetes, gestational diabetes, hypertension, kidney disease, multiple pregnancy, interval between pregnancies, migraine headaches, rheumatoid arthritis, and lupus.

A "measurable feature" is any property, characteristic or aspect that can be determined and correlated with the probability for preeclampsia in a subject. For a biomarker, such a measurable feature can include, for example, the presence, absence, or concentration of the biomarker, or a fragment thereof, in the biological sample, an altered structure, such as, for example, the presence or amount of a post-translational modification, such as oxidation at one or more positions on the amino acid sequence of the biomarker or, for example, the presence of an altered conformation in comparison to the conformation of the biomarker in normal control subjects, and/or the presence, amount, or altered structure of the biomarker as a part of a profile of more than one biomarker. In addition to biomarkers, measurable features can further include risk indicia including, for example, maternal age, race, ethnicity, medical history, past pregnancy history, obstetrical history. For a risk indicium, a measurable feature can include, for example, age, prepregnancy weight, ethnicity, race; the presence, absence or severity of diabetes, hypertension, heart disease, kidney disease; the incidence and/or frequency of prior preeclampsia, prior preeclampsia; the presence, absence, frequency or severity of present or past smoking, illicit drug use, alcohol use; the presence, absence or severity of bleeding after the 12th gestational week; cervical cerclage and transvaginal cervical length.

In some embodiments of the disclosed methods of determining probability for preeclampsia in a pregnant female, the probability for preeclampsia in the pregnant female is calculated based on the quantified amount of each of N biomarkers selected from the biomarkers listed in Tables 2, 3, 4, 5 and 7 through 22. In some embodiments, the disclosed methods for determining the probability of preeclampsia encompass detecting and/or quantifying one or more biomarkers using mass sprectrometry, a capture agent or a combination thereof.

In some embodiments, the disclosed methods of determining probability for preeclampsia in a pregnant female encompass an initial step of providing a biomarker panel comprising N of the biomarkers listed in Tables 2, 3, 4, 5 and 7 through 22. In additional embodiments, the disclosed methods of determining probability for preeclampsia in a pregnant female encompass an initial step of providing a biological sample from the pregnant female.

In some embodiments, the disclosed methods of determining probability for preeclampsia in a pregnant female encompass communicating the probability to a health care provider. In additional embodiments, the communication informs a subsequent treatment decision for the pregnant female.

In some embodiments, the method of determining probability for preeclampsia in a pregnant female encompasses the additional feature of expressing the probability as a risk score.

As used herein, the term "risk score" refers to a score that can be assigned based on comparing the amount of one or more biomarkers in a biological sample obtained from a pregnant female to a standard or reference score that represents an average amount of the one or more biomarkers calculated from biological samples obtained from a random pool of pregnant females. Because the level of a biomarker may not be static throughout pregnancy, a standard or reference score has to have been obtained for the gestational time point that corresponds to that of the pregnant female at the time the sample was taken. The standard or reference score can be predetermined and built into a predictor model such that the comparison is indirect rather than actually performed every time the probability is determined for a subject. A risk score can be a standard (*e.g.,* a number) or a threshold (*e.g.,* a line on a graph). The value of the risk score correlates to the deviation, upwards or downwards, from the average amount of the one or more biomarkers calculated from biological samples obtained from a random pool of pregnant females. In certain embodiments, if a risk score is greater than a standard or reference risk score, the pregnant female can have an increased likelihood of preeclampsia. In some embodiments, the magnitude of a pregnant female's risk score, or the amount by which it exceeds a reference risk score, can be indicative of or correlated to that pregnant female's level of risk.

In the context of the present invention, the term "biological sample," encompasses any sample that is taken from pregnant female and contains one or more of the biomarkers listed in Table 1. Suitable samples in the context of the present invention include, for example, blood, plasma, serum, amniotic fluid, vaginal secretions, saliva, and urine. In some embodiments, the biological sample is selected from the group consisting of whole blood, plasma, and serum. As will be appreciated by those skilled in the art, a biological sample can include any fraction or component of blood, without limitation, T cells, monocytes, neutrophils, erythrocytes, platelets and microvesicles such as exosomes and exosome-like vesicles. In a particular embodiment, the biological sample is serum.

Preeclampsia refers to a condition characterized by high blood pressure and excess protein in the urine (proteinuria) after 20 weeks of pregnancy in a woman who previously had normal blood pressure. Preeclampsia encompasses Eclampsia, a more severe form of preeclampsia that is further characterized by seizures. Preeclampsia can be further classified as mild or severe depending upon the severity of the clinical symptoms. While preeclampsia usually develops during the second half of pregnancy (after 20 weeks), it also can develop shortly after birth or before 20 weeks of pregnancy.

Preeclampsia has been characterized by some investigators as 2 different disease entities: early-onset preeclampsia and late-onset preeclampsia, both of which are intended to be encompassed by reference to preeclampsia herein. Early-onset preeclampsia is usually defined as preeclampsia that develops before 34 weeks of gestation, whereas late-onset preeclampsia develops at or after 34 weeks of gestation. Preclampsia also includes postpartum preeclampsia is a less common condition that occurs when a woman has high blood pressure and excess protein in her urine soon after childbirth. Most cases of postpartum preeclampsia develop within 48 hours of childbirth. However, postpartum preeclampsia sometimes develops up to four to six weeks after childbirth. This is known as late postpartum preeclampsia.

Clinical criteria for diagnosis of preeclampsia are well established, for example, blood pressure of at least 140/90 mm Hg and urinary excretion of at least 0.3 grams of protein in a 24-hour urinary protein excretion (or at least +1 or greater on dipstick testing), each on two occasions 4-6 hours apart. Severe preeclampsia generally refers to a blood pressure of at least 160/110 mm Hg on at least 2 occasions 6 hours apart and greater than 5 grams of protein in a 24-hour urinary protein excretion or persistent +3 proteinuria on dipstick testing. Preeclampsia can include HELLP syndrome (hemolysis, elevated liver enzymes, low platelet count). Other elements of preeclampsia can include in-utero growth restriction (IUGR) in less than the 10% percentile according to the US demographics, persistent neurologic symptoms (headache, visual disturbances), epigastric pain, oliguria (less than 500 mL/24 h), serum creatinine greater than 1.0 mg/dL, elevated liver enzymes (greater than two times normal), thrombocytopenia (<100,000 cells/µL).

In some embodiments, the pregnant female was between 17 and 28 weeks of gestation at the time the biological sample was collected. In other embodiments, the pregnant female was between 16 and 29 weeks, between 17 and 28 weeks, between 18 and 27 weeks, between 19 and 26 weeks, between 20 and 25 weeks, between 21 and 24 weeks, or between 22 and 23 weeks of gestation at the time the biological sample was collected. In further embodiments, the the pregnant female was between about 17 and 22 weeks, between about 16 and 22 weeks between about 22 and 25 weeks, between about 13 and 25 weeks, between about 26 and 28, or between about 26 and 29 weeks of gestation at the time the biological sample was collected. Accordingly, the gestational age of a pregnant female at the time the biological sample is collected can be 15, 16, 17, 18, 19, 20, 21, 22, 23, 24, 25, 26, 27, 28, 29 or 30 weeks.

In some embodiments of the claimed methods the measurable feature comprises fragments or derivatives of each of the N biomarkers selected from the biomarkers listed in Table 1. In additional embodiments of the claimed methods, detecting a measurable feature comprises quantifying an amount of each of N biomarkers selected from the biomarkers listed in Table 1, combinations or portions and/or derivatives thereof in a biological sample obtained from said pregnant female.

The term "amount" or "level" as used herein refers to a quantity of a biomarker that is detectable or measurable in a biological sample and/or control. The quantity of a biomarker can be, for example, a quantity of polypeptide, the quantity of nucleic acid, or the quantity of a fragment or surrogate. The term can alternatively include combinations thereof. The term "amount" or "level" of a biomarker is a measurable feature of that biomarker.

In some embodiments, calculating the probability for preeclampsia in a pregnant female is based on the quantified amount of each of N biomarkers selected from the biomarkers listed in Table 1. Any existing, available or conventional separation, detection and quantification methods can be used herein to measure the presence or absence (*e.g.,* readout being present vs. absent; or detectable amount vs. undetectable amount) and/or quantity (*e.g.,* readout being an absolute or relative quantity, such as, for example, absolute or relative concentration) of biomarkers, peptides, polypeptides, proteins and/or fragments thereof and optionally of the one or more other biomarkers or fragments thereof in samples. In some embodiments, detection and/or quantification of one or more biomarkers comprises an assay that utilizes a capture agent. In further ambodiments, the capture agent is an antibody, antibody fragment, nucleic acid-based protein binding reagent, small molecule or variant thereof. In additional embodiments, the assay is an enzyme immunoassay (EIA), enzyme-linked immunosorbent assay (ELISA), and radioimmunoassay (RIA). In some embodiments, detection and/or quantification of one or more biomarkers further comprises mass spectrometry (MS). In yet further embodiments, the mass spectrometry is co-immunoprecitipation-mass spectrometry (co-IP MS), where coimmunoprecipitation, a technique suitable for the isolation of whole protein complexes is followed by mass spectrometric analysis.

As used herein, the term "mass spectrometer" refers to a device able to volatilize/ionize analytes to form gas-phase ions and determine their absolute or relative molecular masses. Suitable methods of volatilization/ionization are matrix-assisted laser desorption ionization (MALDI), electrospray, laser/light, thermal, electrical, atomized/sprayed and the like, or combinations thereof. Suitable forms of mass spectrometry include, but are not limited to, ion trap instruments, quadrupole instruments, electrostatic and magnetic sector instruments, time of flight instruments, time of flight tandem mass spectrometer (TOF MS/MS), Fourier-transform mass spectrometers, Orbitraps and hybrid instruments composed of various combinations of these types of mass analyzers. These instruments can, in turn, be interfaced with a variety of other instruments that fractionate the samples (for example, liquid chromatography or solid-phase adsorption techniques based on chemical, or biological properties) and that ionize the samples for introduction into the mass spectrometer, including matrix-assisted laser desorption (MALDI), electrospray, or nanospray ionization (ESI) or combinations thereof.

Generally, any mass spectrometric (MS) technique that can provide precise information on the mass of peptides, and preferably also on fragmentation and/or (partial) amino acid sequence of selected peptides (*e.g.,* in tandem mass spectrometry, MS/MS; or in post source decay, TOF MS), can be used in the methods disclosed herein. Suitable peptide MS and MS/MS techniques and systems are well-known *per se* (see, e.g., Methods in Molecular Biology, vol. 146: "Mass Spectrometry of Proteins and Peptides", by Chapman, ed., Humana Press 2000; Biemann 1990. Methods Enzymol 193: 455-79; or Methods in Enzymology, vol. 402: "Biological Mass Spectrometry", by Burlingame, ed., Academic Press 2005) and can be used in practicing the methods disclosed herein. Accordingly, in some embodiments, the disclosed methods comprise performing quantitative MS to measure one or more biomarkers. Such quantitiative methods can be performed in an automated (Villanueva, et al., Nature Protocols (2006) 1(2):880-891) or semi-automated format. In particular embodiments, MS can be operably linked to a liquid chromatography device (LC-MS/MS or LC-MS) or gas chromatography device (GC-MS or GC-MS/MS). Other methods useful in this context include isotope-coded affinity tag (ICAT) followed by chromatography and MS/MS.

As used herein, the terms "multiple reaction monitoring (MRM)" or "selected reaction monitoring (SRM)" refer to an MS-based quantification method that is particularly useful for quantifying analytes that are in low abundance. In an SRM experiment, a predefined precursor ion and one or more of its fragments are selected by the two mass filters of a triple quadrupole instrument and monitored over time for precise quantification. Multiple SRM precursor and fragment ion pairs can be measured within the same experiment on the chromatographic time scale by rapidly toggling between the different precursor/fragment pairs to perform an MRM experiment. A series of transitions (precursor/fragment ion pairs) in combination with the retention time of the targeted analyte (*e.g.,* peptide or small molecule such as chemical entity, steroid, hormone) can constitute a definitive assay. A large number of analytes can be quantified during a single LC-MS experiment. The term "scheduled," or "dynamic" in reference to MRM or SRM, refers to a variation of the assay wherein the transitions for a particular analyte are only acquired in a time window around the expected retention time, significantly increasing the number of analytes that can be detected and quantified in a single LC-MS experiment and contributing to the selectivity of the test, as retention time is a property dependent on the physical nature of the analyte. A single analyte can also be monitored with more than one transition. Finally, included in the assay can be standards that correspond to the analytes of interest (*e.g.,* same amino acid sequence), but differ by the inclusion of stable isotopes. Stable isotopic standards (SIS) can be incorporated into the assay at precise levels and used to quantify the corresponding unknown analyte. An additional level of specificity is contributed by the co-elution of the unknown analyte and its corresponding SIS and properties of their transitions (*e.g.*, the similarity in the ratio of the level of two transitions of the unknown and the ratio of the two transitions of its corresponding SIS).

Mass spectrometry assays, instruments and systems suitable for biomarker peptide analysis can include, without limitation, matrix-assisted laser desorption/ionisation time-of-flight (MALDI-TOF) MS; MALDI-TOF post-source-decay (PSD); MALDI-TOF/TOF; surface-enhanced laser desorption/ionization time-of-flight mass spectrometry (SELDI-TOF) MS; electrospray ionization mass spectrometry (ESI-MS); ESI-MS/MS; ESI-MS/(MS)ₙ (n is an integer greater than zero); ESI 3D or linear (2D) ion trap MS; ESI triple quadrupole MS; ESI quadrupole orthogonal TOF (Q-TOF); ESI Fourier transform MS systems; desorption/ionization on silicon (DIOS); secondary ion mass spectrometry (SIMS); atmospheric pressure chemical ionization mass spectrometry (APCI-MS); APCI-MS/MS; APCI- (MS)ₙ; atmospheric pressure photoionization mass spectrometry (APPI-MS); APPI-MS/MS; and APPI- (MS)ₙ. Peptide ion fragmentation in tandem MS (MS/MS) arrangements can be achieved using manners established in the art, such as, e.g., collision induced dissociation (CID). As described herein, detection and quantification of biomarkers by mass spectrometry can involve multiple reaction monitoring (MRM), such as described among others by Kuhn et al. Proteomics 4: 1175-86 (2004). Scheduled multiple-reaction-monitoring (Scheduled MRM) mode acquisition during LC-MS/MS analysis enhances the sensitivity and accuracy of peptide quantitation. Anderson and Hunter , Molecular and Cellular Proteomics 5(4):573 (2006). As described herein, mass spectrometry-based assays can be advantageously combined with upstream peptide or protein separation or fractionation methods, such as for example with the chromatographic and other methods described herein below.

A person skilled in the art will appreciate that a number of methods can be used to determine the amount of a biomarker, including mass spectrometry approaches, such as MS/MS, LC-MS/MS, multiple reaction monitoring (MRM) or SRM and product-ion monitoring (PIM) and also including antibody based methods such as immunoassays such as Western blots, enzyme-linked immunosorbant assay (ELISA), immunopercipitation, immunohistochemistry, immunofluorescence, radioimmunoassay, dot blotting, and fluorescence-activated cell sorting (FACS). Accordingly, in some embodiments, determining the level of the at least one biomarker comprises using an immunoassay and/or mass spectrometric methods. In additional embodiments, the mass spectrometric methods are selected from MS, MS/MS, LC-MS/MS, SRM, PIM, and other such methods that are known in the art. In other embodiments, LC-MS/MS further comprises ID LC-MS/MS, 2D LC-MS/MS or 3D LC-MS/MS. Immunoassay techniques and protocols are generally known to those skilled in the art (Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and non-competitive immunoassays, can be used (Self et al., Curr. Opin. Biotechnol., 7:60-65 (1996).

In further embodiments, the immunoassay is selected from Western blot, ELISA, immunopercipitation, immunohistochemistry, immunofluorescence, radioimmunoassay (RIA), dot blotting, and FACS. In certain embodiments, the immunoassay is an ELISA. In yet a further embodiment, the ELISA is direct ELISA (enzyme-linked immunosorbent assay), indirect ELISA, sandwich ELISA, competitive ELISA, multiplex ELISA, ELISPOT technologies, and other similar techniques known in the art. Principles of these immunoassay methods are known in the art, for example John R. Crowther, The ELISA Guidebook, 1st ed., Humana Press 2000, ISBN 0896037282. Typically ELISAs are performed with antibodies but they can be performed with any capture agents that bind specifically to one or more biomarkers of the invention and that can be detected. Multiplex ELISA allows simultaneous detection of two or more analytes within a single compartment (*e.g.,* microplate well) usually at a plurality of array addresses (Nielsen and Geierstanger 2004. J Immunol Methods 290: 107-20 (2004) and Ling et al. 2007. Expert Rev Mol Diagn 7: 87-98 (2007)).

In some embodiments, Radioimmunoassay (RIA) can be used to detect one or more biomarkers in the methods of the invention. RIA is a competition-based assay that is well known in the art and involves mixing known quantities of radioactavely-labelled (e.g.,¹²⁵I or ¹³¹I-labelled) target analyte with antibody specific for the analyte, then adding non-labelled analyte from a sample and measuring the amount of labelled analyte that is displaced (see, e.g., An Introduction to Radioimmunoassay and Related Techniques, by Chard T, ed., Elsevier Science 1995, ISBN 0444821198 for guidance).

A detectable label can be used in the assays described herein for direct or indirect detection of the biomarkers in the methods of the invention. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers in the methods of the invention. Suitable detectable labels include, but are not limited to, fluorescent dyes (*e.g.,* fluorescein, fluorescein isothiocyanate (FITC), Oregon Green™, rhodamine, Texas red, tetrarhodimine isothiocynate (TRITC), Cy3, Cy5, etc.), fluorescent markers (*e.g.,* green fluorescent protein (GFP), phycoerythrin, etc.), enzymes (*e.g.,* luciferase, horseradish peroxidase, alkaline phosphatase, etc.), nanoparticles, biotin, digoxigenin, metals, and the like.

For mass-sectrometry based analysis, differential tagging with isotopic reagents, *e.g.,* isotope-coded affinity tags (ICAT) or the more recent variation that uses isobaric tagging reagents, iTRAQ (Applied Biosystems, Foster City, Calif.), or tandem mass tags, TMT, (Thermo Scientific, Rockford, IL), followed by multidimensional liquid chromatography (LC) and tandem mass spectrometry (MS/MS) analysis can provide a further methodology in practicing the methods of the inventon.

A chemiluminescence assay using a chemiluminescent antibody can be used for sensitive, non-radioactive detection of protein levels. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase , beta-galactosidase are well known in the art.

A signal from the direct or indirect label can be analyzed, for example, using a spectrophotometer to detect color from a chromogenic substrate; a radiation counter to detect radiation such as a gamma counter for detection of ¹²⁵I; or a fluorometer to detect fluorescence in the presence of light of a certain wavelength. For detection of enzyme-linked antibodies, a quantitative analysis can be made using a spectrophotometer such as an EMAX Microplate Reader (Molecular Devices; Menlo Park, Calif.) in accordance with the manufacturer's instructions. If desired, assays used to practice the invention can be automated or performed robotically, and the signal from multiple samples can be detected simultaneously.

In some embodiments, the methods described herein encompass quantification of the biomarkers using mass spectrometry (MS). In further embodiments, the mass spectrometry can be liquid chromatography-mass spectrometry (LC-MS), multiple reaction monitoring (MRM) or selected reaction monitoring (SRM). In additional embodiments, the MRM or SRM can further encompass scheduled MRM or scheduled SRM.

As described above, chromatography can also be used in practicing the methods of the invention. Chromatography encompasses methods for separating chemical substances and generally involves a process in which a mixture of analytes is carried by a moving stream of liquid or gas ("mobile phase") and separated into components as a result of differential distribution of the analytes as they flow around or over a stationary liquid or solid phase ("stationary phase"), between the mobile phase and said stationary phase. The stationary phase can be usually a finely divided solid, a sheet of filter material, or a thin film of a liquid on the surface of a solid, or the like. Chromatography is well understood by those skilled in the art as a technique applicable for the separation of chemical compounds of biological origin, such as, *e.g.,* amino acids, proteins, fragments of proteins or peptides, *etc.*

Chromatography can be columnar (*i.e.,* wherein the stationary phase is deposited or packed in a column), preferably liquid chromatography, and yet more preferably high-performance liquid chromatography (HPLC) or ultra high performance/pressure liquid chromatography (UHPLC). Particulars of chromatography are well known in the art (Bidlingmeyer, Practical HPLC Methodology and Applications, John Wiley & Sons Inc., 1993). Exemplary types of chromatography include, without limitation, high-performance liquid chromatography (HPLC), UHPLC, normal phase HPLC (NP-HPLC), reversed phase HPLC (RP-HPLC), ion exchange chromatography (IEC), such as cation or anion exchange chromatography, hydrophilic interaction chromatography (HILIC), hydrophobic interaction chromatography (HIC), size exclusion chromatography (SEC) including gel filtration chromatography or gel permeation chromatography, chromatofocusing, affinity chromatography such as immuno-affinity, immobilised metal affinity chromatography, and the like. Chromatography, including single-, two- or more-dimensional chromatography, can be used as a peptide fractionation method in conjunction with a further peptide analysis method, such as for example, with a downstream mass spectrometry analysis as described elsewhere in this specification.

Further peptide or polypeptide separation, identification or quantification methods can be used, optionally in conjunction with any of the above described analysis methods, for measuring biomarkers in the present disclosure. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

In the context of the invention, the term "capture agent" refers to a compound that can specifically bind to a target, in particular a biomarker. The term includes antibodies, antibody fragments, nucleic acid-based protein binding reagents (*e.g.* aptamers, Slow Off-rate Modified Aptamers (SOMAmer™)), protein-capture agents, natural ligands (*i.e.* a hormone for its receptor or vice versa), small molecules or variants thereof.

Capture agents can be configured to specifically bind to a target, in particular a biomarker. Capture agents can include but are not limited to organic molecules, such as polypeptides, polynucleotides and other non polymeric molecules that are identifiable to a skilled person. In the embodiments disclosed herein, capture agents include any agent that can be used to detect, purify, isolate, or enrich a target, in particular a biomarker. Any art-known affinity capture technologies can be used to selectively isolate and enrich/concentrate biomarkers that are components of complex mixtures of biological media for use in the disclosed methods.

Antibody capture agents that specifically bind to a biomarker can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). Antibody capture agents can be any immunoglobulin or derivative therof, whether natural or wholly or partially synthetically produced. All derivatives thereof which maintain specific binding ability are also included in the term. Antibody capture agents have a binding domain that is homologous or largely homologous to an immunoglobulin binding domain and can be derived from natural sources, or partly or wholly synthetically produced. Antibody capture agents can be monoclonal or polyclonal antibodies. In some embodiments, an antibody is a single chain antibody. Those of ordinary skill in the art will appreciate that antibodies can be provided in any of a variety of forms including, for example, humanized, partially humanized, chimeric, chimeric humanized, *etc.* Antibody capture agents can be antibody fragments including, but not limited to, Fab, Fab', F(ab')2, scFv, Fv, dsFv diabody, and Fd fragments. An antibody capture agent can be produced by any means. For example, an antibody capture agent can be enzymatically or chemically produced by fragmentation of an intact antibody and/or it can be recombinantly produced from a gene encoding the partial antibody sequence. An antibody capture agent can comprise a single chain antibody fragment. Alternatively or additionally, antibody capture agent can comprise multiple chains which are linked together, for example, by disulfide linkages; and, any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

Suitable capture agents useful for practicing the invention also include aptamers. Aptamers are oligonucleotide sequences that can bind to their targets specifically via unique three dimensional (3-D) structures. An aptamer can include any suitable number of nucleotides and different aptamers can have either the same or different numbers of nucleotides. Aptamers can be DNA or RNA or chemically modified nucleic acids and can be single stranded, double stranded, or contain double stranded regions, and can include higher ordered structures. An aptamer can also be a photoaptamer, where a photoreactive or chemically reactive functional group is included in the aptamer to allow it to be covalently linked to its corresponding target. Use of an aptamer capture agent can include the use of two or more aptamers that specifically bind the same biomarker. An aptamer can include a tag. An aptamer can be identified using any known method, including the SELEX (systematic evolution of ligands by exponential enrichment), process. Once identified, an aptamer can be prepared or synthesized in accordance with any known method, including chemical synthetic methods and enzymatic synthetic methods and used in a variety of applications for biomarker detection. Liu et al., Curr Med Chem. 18(27):4117-25 (2011). Capture agents useful in practicing the methods of the invention also include SOMAmers (Slow Off-Rate Modified Aptamers) known in the art to have improved off-rate characteristics. Brody et al., J Mol Biol. 422(5):595-606 (2012). SOMAmers can be generated using using any known method, including the SELEX method.

It is understood by those skilled in the art that biomarkers can be modified prior to analysis to improve their resolution or to determine their identity. For example, the biomarkers can be subject to proteolytic digestion before analysis. Any protease can be used. Proteases, such as trypsin, that are likely to cleave the biomarkers into a discrete number of fragments are particularly useful. The fragments that result from digestion function as a fingerprint for the biomarkers, thereby enabling their detection indirectly. This is particularly useful where there are biomarkers with similar molecular masses that might be confused for the biomarker in question. Also, proteolytic fragmentation is useful for high molecular weight biomarkers because smaller biomarkers are more easily resolved by mass spectrometry. In another example, biomarkers can be modified to improve detection resolution. For instance, neuraminidase can be used to remove terminal sialic acid residues from glycoproteins to improve binding to an anionic adsorbent and to improve detection resolution. In another example, the biomarkers can be modified by the attachment of a tag of particular molecular weight that specifically binds to molecular biomarkers, further distinguishing them. Optionally, after detecting such modified biomarkers, the identity of the biomarkers can be further determined by matching the physical and chemical characteristics of the modified biomarkers in a protein database (*e.g.,* SwissProt).

It is further appreciated in the art that biomarkers in a sample can be captured on a substrate for detection. Traditional substrates include antibody-coated 96-well plates or nitrocellulose membranes that are subsequently probed for the presence of the proteins. Alternatively, protein-binding molecules attached to microspheres, microparticles, microbeads, beads, or other particles can be used for capture and detection of biomarkers. The protein-binding molecules can be antibodies, peptides, peptoids, aptamers, small molecule ligands or other protein-binding capture agents attached to the surface of particles. Each protein-binding molecule can include unique detectable label that is coded such that it can be distinguished from other detectable labels attached to other protein-binding molecules to allow detection of biomarkers in multiplex assays. Examples include, but are not limited to, color-coded microspheres with known fluorescent light intensities (see e.g., microspheres with xMAP technology produced by Luminex (Austin, Tex.); microspheres containing quantum dot nanocrystals, for example, having different ratios and combinations of quantum dot colors (e.g., Qdot nanocrystals produced by Life Technologies (Carlsbad, Calif.); glass coated metal nanoparticles (see *e.g.,* SERS nanotags produced by Nanoplex Technologies, Inc. (Mountain View, Calif.); barcode materials (see e.g., sub-micron sized striped metallic rods such as Nanobarcodes produced by Nanoplex Technologies, Inc.), encoded microparticles with colored bar codes (see e.g., CellCard produced by Vitra Bioscience, vitrabio.com), glass microparticles with digital holographic code images (see *e.g.,* CyVera microbeads produced by Illumina (San Diego, Calif.); chemiluminescent dyes, combinations of dye compounds; and beads of detectably different sizes.

In another aspect, biochips can be used for capture and detection of the biomarkers of the invention. Many protein biochips are known in the art. These include, for example, protein biochips produced by Packard BioScience Company (Meriden Conn.), Zyomyx (Hayward, Calif.) and Phylos (Lexington, Mass.). In general, protein biochips comprise a substrate having a surface. A capture reagent or adsorbent is attached to the surface of the substrate. Frequently, the surface comprises a plurality of addressable locations, each of which location has the capture agent bound there. The capture agent can be a biological molecule, such as a polypeptide or a nucleic acid, which captures other biomarkers in a specific manner. Alternatively, the capture agent can be a chromatographic material, such as an anion exchange material or a hydrophilic material. Examples of protein biochips are well known in the art.

Measuring mRNA in a biological sample can be used as a surrogate for detection of the level of the corresponding protein biomarker in a biological sample. Thus, any of the biomarkers or biomarker panels described herein can also be detected by detecting the appropriate RNA. Levels of mRNA can measured by reverse transcription quantitative polymerase chain reaction (RT-PCR followed with qPCR). RT-PCR is used to create a cDNA from the mRNA. The cDNA can be used in a qPCR assay to produce fluorescence as the DNA amplification process progresses. By comparison to a standard curve, qPCR can produce an absolute measurement such as number of copies of mRNA per cell. Northern blots, microarrays, Invader assays, and RT-PCR combined with capillary electrophoresis have all been used to measure expression levels of mRNA in a sample. See Gene Expression Profiling: Methods and Protocols, Richard A. Shimkets, editor, Humana Press, 2004.

Some embodiments disclosed herein relate to diagnostic and prognostic methods of determining the probability for preeclampsia in a pregnant female. The detection of the level of expression of one or more biomarkers and/or the determination of a ratio of biomarkers can be used to determine the probability for preeclampsia in a pregnant female. Such detection methods can be used, for example, for early diagnosis of the condition, to determine whether a subject is predisposed to preeclampsia, to monitor the progress of preeclampsia or the progress of treatment protocols, to assess the severity of preeclampsia, to forecast the outcome of preeclampsia and/or prospects of recovery or birth at full term, or to aid in the determination of a suitable treatment for preeclampsia.

The quantitation of biomarkers in a biological sample can be determined, without limitation, by the methods described above as well as any other method known in the art. The quantitative data thus obtained is then subjected to an analytic classification process. In such a process, the raw data is manipulated according to an algorithm, where the algorithm has been pre-defined by a training set of data, for example as described in the examples provided herein. An algorithm can utilize the training set of data provided herein, or can utilize the guidelines provided herein to generate an algorithm with a different set of data.

In some embodiments, analyzing a measurable feature to determine the probability for preeclampsia in a pregnant female encompasses the use of a predictive model. In further embodiments, analyzing a measurable feature to determine the probability for preeclampsia in a pregnant female encompasses comparing said measurable feature with a reference feature. As those skilled in the art can appreciate, such comparison can be a direct comparison to the reference feature or an indirect comparison where the reference feature has been incorporated into the predictive model. In further embodiments, analyzing a measurable feature to determine the probability for preeclampsia in a pregnant female encompasses one or more of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, or a combination thereof. In particular embodiments, the analysis comprises logistic regression.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms; etc.

Classification can be made according to predictive modeling methods that set a threshold for determining the probability that a sample belongs to a given class. The probability preferably is at least 50%, or at least 60%, or at least 70%, or at least 80% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, e.g. AUC (area under the curve) or accuracy, of a particular value, or range of values. Area under the curve measures are useful for comparing the accuracy of a classifier across the complete data range. Classifiers with a greater AUC have a greater capacity to classify unknowns correctly between two groups of interest. In some embodiments, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher.

As is known in the art, the relative sensitivity and specificity of a predictive model can be adjusted to favor either the selectivity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about 0.7, at least about 0.75, at least about 0.8, at least about 0.85, at least about 0.9, or higher.

The raw data can be initially analyzed by measuring the values for each biomarker, usually in triplicate or in multiple triplicates. The data can be manipulated, for example, raw data can be transformed using standard curves, and the average of triplicate measurements used to calculate the average and standard deviation for each patient. These values can be transformed before being used in the models, *e.g.* log-transformed, Box-Cox transformed (Box and Cox, Royal Stat. Soc., Series B, 26:211-246(1964). The data are then input into a predictive model, which will classify the sample according to the state. The resulting information can be communicated to a patient or health care provider.

To generate a predictive model for preeclampsia, a robust data set, comprising known control samples and samples corresponding to the preeclampsia classification of interest is used in a training set. A sample size can be selected using generally accepted criteria. As discussed above, different statistical methods can be used to obtain a highly accurate predictive model. Examples of such analysis are provided in Example 2.

In one embodiment, hierarchical clustering is performed in the derivation of a predictive model, where the Pearson correlation is employed as the clustering metric. One approach is to consider a preeclampsia dataset as a "learning sample" in a problem of "supervised learning." CART is a standard in applications to medicine (Singer, Recursive Partitioning in the Health Sciences, Springer(1999)) and can be modified by transforming any qualitative features to quantitative features; sorting them by attained significance levels, evaluated by sample reuse methods for Hotelling's T² statistic; and suitable application of the lasso method. Problems in prediction are turned into problems in regression without losing sight of prediction, indeed by making suitable use of the Gini criterion for classification in evaluating the quality of regressions.

This approach led to what is termed FlexTree (Huang, Proc. Nat. Acad. Sci. U.S.A 101:10529-10534(2004)). FlexTree performs very well in simulations and when applied to multiple forms of data and is useful for practicing the claimed methods. Software automating FlexTree has been developed. Alternatively, LARTree or LART can be used (Turnbull (2005) Classification Trees with Subset Analysis Selection by the Lasso, Stanford University). The name reflects binary trees, as in CART and FlexTree; the lasso, as has been noted; and the implementation of the lasso through what is termed LARS by Efron et al. (2004) Annals of Statistics 32:407-451 (2004). See, also, Huang et al.., Proc. Natl. Acad. Sci. USA. 101(29):10529-34 (2004). Other methods of analysis that can be used include logic regression. One method of logic regression Ruczinski , Journal of Computational and Graphical Statistics 12:475-512 (2003). Logic regression resembles CART in that its classifier can be displayed as a binary tree. It is different in that each node has Boolean statements about features that are more general than the simple "and" statements produced by CART.

Another approach is that of nearest shrunken centroids (Tibshirani, Proc. Natl. Acad. Sci. U.S.A 99:6567-72(2002)). The technology is k-means-like, but has the advantage that by shrinking cluster centers, one automatically selects features, as is the case in the lasso, to focus attention on small numbers of those that are informative. The approach is available as PAM software and is widely used. Two further sets of algorithms that can be used are random forests (Breiman, Machine Learning 45:5-32 (2001)) and MART (Hastie, The Elements of Statistical Learning, Springer (2001)). These two methods are known in the art as "committee methods," that involve predictors that "vote" on outcome.

To provide significance ordering, the false discovery rate (FDR) can be determined. First, a set of null distributions of dissimilarity values is generated. In one embodiment, the values of observed profiles are permuted to create a sequence of distributions of correlation coefficients obtained out of chance, thereby creating an appropriate set of null distributions of correlation coefficients (Tusher et al. , Proc. Natl. Acad. Sci. U.S.A 98, 5116-21 (2001)). The set of null distribution is obtained by: permuting the values of each profile for all available profiles; calculating the pair-wise correlation coefficients for all profile; calculating the probability density function of the correlation coefficients for this permutation; and repeating the procedure for N times, where N is a large number, usually 300. Using the N distributions, one calculates an appropriate measure (mean, median, *etc*.) of the count of correlation coefficient values that their values exceed the value (of similarity) that is obtained from the distribution of experimentally observed similarity values at given significance level.

The FDR is the ratio of the number of the expected falsely significant correlations (estimated from the correlations greater than this selected Pearson correlation in the set of randomized data) to the number of correlations greater than this selected Pearson correlation in the empirical data (significant correlations). This cut-off correlation value can be applied to the correlations between experimental profiles. Using the aforementioned distribution, a level of confidence is chosen for significance. This is used to determine the lowest value of the correlation coefficient that exceeds the result that would have obtained by chance. Using this method, one obtains thresholds for positive correlation, negative correlation or both. Using this threshold(s), the user can filter the observed values of the pair wise correlation coefficients and eliminate those that do not exceed the threshold(s). Furthermore, an estimate of the false positive rate can be obtained for a given threshold. For each of the individual "random correlation" distributions, one can find how many observations fall outside the threshold range. This procedure provides a sequence of counts. The mean and the standard deviation of the sequence provide the average number of potential false positives and its standard deviation.

In an alternative analytical approach, variables chosen in the cross-sectional analysis are separately employed as predictors in a time-to-event analysis (survival analysis), where the event is the occurrence of preeclampsia, and subjects with no event are considered censored at the time of giving birth. Given the specific pregnancy outcome (preeclampsia event or no event), the random lengths of time each patient will be observed, and selection of proteomic and other features, a parametric approach to analyzing survival can be better than the widely applied semi-parametric Cox model. A Weibull parametric fit of survival permits the hazard rate to be monotonically increasing, decreasing, or constant, and also has a proportional hazards representation (as does the Cox model) and an accelerated failure-time representation. All the standard tools available in obtaining approximate maximum likelihood estimators of regression coefficients and corresponding functions are available with this model.

In addition the Cox models can be used, especially since reductions of numbers of covariates to manageable size with the lasso will significantly simplify the analysis, allowing the possibility of a nonparametric or semi-parametric approach to prediction of time to preeclampsia. These statistical tools are known in the art and applicable to all manner of proteomic data. A set of biomarker, clinical and genetic data that can be easily determined, and that is highly informative regarding the probability for preeclampsia and predicted time to a preeclampsia event in said pregnant female is provided. Also, algorithms provide information regarding the probability for preeclampsia in the pregnant female.

In the development of a predictive model, it can be desirable to select a subset of markers, *i.e.* at least 3, at least 4, at least 5, at least 6, up to the complete set of markers. Usually a subset of markers will be chosen that provides for the needs of the quantitative sample analysis, e.g. availability of reagents, convenience of quantitation, etc., while maintaining a highly accurate predictive model. The selection of a number of informative markers for building classification models requires the definition of a performance metric and a user-defined threshold for producing a model with useful predictive ability based on this metric. For example, the performance metric can be the AUROC, the sensitivity and/or specificity of the prediction as well as the overall accuracy of the prediction model.

As will be understood by those skilled in the art, an analytic classification process can use any one of a variety of statistical analytic methods to manipulate the quantitative data and provide for classification of the sample. Examples of useful methods include, without limitation, linear discriminant analysis, recursive feature elimination, a prediction analysis of microarray, a logistic regression, a CART algorithm, a FlexTree algorithm, a LART algorithm, a random forest algorithm, a MART algorithm, and machine learning algorithms.

As described in Example 2, various methods are used in a training model. The selection of a subset of markers can be for a forward selection or a backward selection of a marker subset. The number of markers can be selected that will optimize the performance of a model without the use of all the markers. One way to define the optimum number of terms is to choose the number of terms that produce a model with desired predictive ability (*e.g.* an AUC>0.75, or equivalent measures of sensitivity/specificity) that lies no more than one standard error from the maximum value obtained for this metric using any combination and number of terms used for the given algorithm.

**Table 1. Transitions with p-values less than 0.05 in univariate Cox Proportional Hazards to predict Gestational Age of time to event (preeclampsia).**

| | | |
|---|---|---|
| TSDQIHFFFAK_447.56_512.3 | 0.00 | ANT3_HUMAN |
| DPNGLPPEAQK_583.3_669.4 | 0.00 | RET4_HUMAN |
| SVSLPSLDPASAK_636.35_885.5 | 0.00 | APOB_HUMAN |
| SSNNPHSPIVEEFQVPYNK_729.36_261.2 | 0.00 | C1S_HUMAN |
| IEGNLIFDPNNYLPK_873.96_414.2 | 0.00 | APOB_HUMAN |
| YWGVASFLQK_599.82_849.5 | 0.00 | RET4_HUMAN |
| ITENDIQIALDDAK_779.9_632.3 | 0.00 | APOB HUMAN |
| IEGNLIFDPNNYLPK_873.96_845.5 | 0.00 | APOB_HUMAN |
| GWVTDGFSSLK_598.8_953.5 | 0.00 | APOC3_HUMAN |
| TGISPLALIK_506.82_741.5 | 0.00 | APOB_HUMAN |
| SVSLPSLDPASAK_636.35_473.3 | 0.00 | APOB_HUMAN |
| IIGGSDADIK_494.77_762.4 | 0.00 | C1S_HUMAN |
| TGISPLALIK_506.82_654.5 | 0.00 | APOB_HUMAN |
| TLLIANETLR_572.34_703.4 | 0.00 | IL5_HUMAN |
| YWGVASFLQK_599.82_350.2 | 0.00 | RET4_HUMAN |
| VSALLTPAEQTGTWK_801.43_371.2 | 0.00 | APOB_HUMAN |
| DPNGLPPEAQK_583.3_497.2 | 0.00 | RET4_HUMAN |
| VNHVTLSQPK_561.82_673.4 | 0.00 | B2MG_HUMAN |
| DALSSVQESQVAQQAR_572.96_502.3 | 0.00 | APOC3_HUMAN |
| IAQYYYTFK_598.8_884.4 | 0.00 | F13B_HUMAN |
| IEEIAAK_387.22_531.3 | 0.00 | CO5_HUMAN |
| GWVTDGFSSLK_598.8_854.4 | 0.00 | APOC3_HUMAN |
| VNHVTLSQPK_561.82_351.2 | 0.00 | B2MG_HUMAN |
| ITENDIQIALDDAK_779.9_873.5 | 0.00 | APOB_HUMAN |
| VSALLTPAEQTGTWK_801.43_585.4 | 0.00 | APOB HUMAN |
| VILGAHQEVNLEPHVQEIEVSR_832.78_860.4 | 0.00 | PLMN_HUMAN |
| SPELQAEAK_486.75_788.4 | 0.00 | APOA2_HUMAN |
| SPELQAEAK_486.75_659.4 | 0.00 | APOA2_HUMAN |
| DYWSTVK_449.72_620.3 | 0.00 | APOC3_HUMAN |
| VPLALFALNR_557.34_620.4 | 0.00 | PEPD_HUMAN |
| TSDQIHFFFAK_447.56_659.4 | 0.00 | ANT3_HUMAN |
| DALSSVQESQVAQQAR_572.96_672.4 | 0.00 | APOC3_HUMAN |
| VIAVNEVGR_478.78_284.2 | 0.00 | CHL1_HUMAN |
| LLEVPEGR_456.76_686.3 | 0.00 | C1S_HUMAN |
| VEPLYELVTATDFAYSSTVR_754.38_549.3 | 0.00 | CO8B_HUMAN |
| HHGPTITAK_321.18_275.1 | 0.01 | AMBP_HUMAN |
| ALNFGGIGVVVGHELTHAFDDQGR_837.09_299.2 | 0.01 | ECE1_HUMAN |
| ETLLQDFR 511.27_565.3 | 0.01 | AMBP_HUMAN |
| HHGPTITAK_321.18_432.3 | 0.01 | AMBP_HUMAN |
| IIGGSDADIK_494.77_260.2 | 0.01 | C1S_HUMAN |

**Table 2. Top 40 transitions with p-values less than 0.05 in univariate Cox Proportional Hazards to predict Gestational Age of time to event (preeclampsia), sorted by protein ID.**

| **Transition** | **cox pvalues** | **protein** |
|---|---|---|
| HHGPTITAK_321.18_275.1 | 0.01 | AMBP_HUMAN |
| ETLLQDFR_511.27_565.3 | 0.01 | AMBP_HUMAN |
| HHGPTITAK_321.18_432.3 | 0.01 | AMBP_HUMAN |
| TSDQIHFFFAK_447.56_512.3 | 0.00 | ANT3_HUMAN |
| TSDQIHFFFAK_447.56_659.4 | 0.00 | ANT3_HUMAN |
| SPELQAEAK_486.75_788.4 | 0.00 | APOA2_HUMAN |
| SPELQAEAK_486.75_659.4 | 0.00 | APOA2_HUMAN |
| SVSLPSLDPASAK_636.35_885.5 | 0.00 | APOB_HUMAN |
| IEGNLIFDPNNYLPK_873.96_414.2 | 0.00 | APOB_HUMAN |
| ITENDIQIALDDAK_779.9_632.3 | 0.00 | APOB_HUMAN |
| IEGNLIFDPNNYLPK_873.96_845.5 | 0.00 | APOB_HUMAN |
| TGISPLALIK_506.82_741.5 | 0.00 | APOB_HUMAN |
| SVSLPSLDPASAK_636.35_473.3 | 0.00 | APOB_HUMAN |
| TGISPLALIK_506.82_654.5 | 0.00 | APOB_HUMAN |
| VSALLTPAEQTGTWK_801.43_371.2 | 0.00 | APOB_HUMAN |
| ITENDIQIALDDAK_779.9_873.5 | 0.00 | APOB_HUMAN |
| VSALLTPAEQTGTWK_801.43_585.4 | 0.00 | APOB_HUMAN |
| GWVTDGFSSLK_598.8_953.5 | 0.00 | APOC3_HUMAN |
| DALSSVQESQVAQQAR_572.96_502.3 | 0.00 | APOC3_HUMAN |
| GWVTDGFSSLK_598.8_854.4 | 0.00 | APOC3_HUMAN |
| DYWSTVK_449.72_620.3 | 0.00 | APOC3_HUMAN |
| DALSSVQESQVAQQAR_572.96_672.4 | 0.00 | APOC3_HUMAN |
| VNHVTLSQPK_561.82_673.4 | 0.00 | B2MG_HUMAN |
| VNHVTLSQPK_561.82_351.2 | 0.00 | B2MG_HUMAN |
| SSNNPHSPIVEEFQVPYNK_729.36_261.2 | 0.00 | C1S_HUMAN |
| IIGGSDADIK_494.77_762.4 | 0.00 | C1S_HUMAN |
| LLEVPEGR_456.76_686.3 | 0.00 | C1S_HUMAN |
| IIGGSDADIK_494.77_260.2 | 0.01 | C1S_HUMAN |
| VIAVNEVGR_478.78_284.2 | 0.00 | CHL1_HUMAN |
| IEEIAAK 387.22_531.3 | 0.00 | CO5_HUMAN |
| VEPLYELVTATDFAYSSTVR_754.38_549.3 | 0.00 | CO8B_HUMAN |
| ALNFGGIGVVVGHELTHAFDDQGR_837.09_299.2 | 0.01 | ECE1_HUMAN |
| IAQYYYTFKL_598.8_884.4 | 0.00 | F13B_HUMAN |
| TLLIANETLR_572.34_703.4 | 0.00 | IL5_HUMAN |
| VPLALFALNR_557.34_620.4 | 0.00 | PEPD_HUMAN |
| VILGAHQEYNLEPHV_QEIEVSR_832.78_860.4 | 0.00 | PLMN_HUMAN |
| DPNGLPPEAQK_583.3_669.4 | 0.00 | RET4_HUMAN |
| YWGVASFLQK_599.82_849.5 | 0.00 | RET4_HUMAN |
| YWGVASFLQK_599.82_350.2 | 0.00 | RET4_HUMAN |
| DPNGLPPEAQK_583.3_497.2 | 0.00 | RET4_HUMAN |

**Table 3. Transitions selected by Cox stepwise AIC analysis**

| Transition | coef | exp(coef) | se(coef) | z | Pr(>\|z\|) |
|---|---|---|---|---|---|
| Collection.Window.GA.in.Days | 0.43 | 1.54E+00 | 0.19 | 2.22 | 0.03 |
| IIGGSDADIK 494.77_762.4 | 44.40 | 1.91E+19 | 18.20 | 2.44 | 0.01 |
| GGEGTGYFVDFSVR_745.85_869.5 | 6.91 | 1.00E+03 | 2.76 | 2.51 | 0.01 |
| SPEQQETVLDGNLIIR_906.48_685.4 | 17.28 | 3.21E+07 | 7.49 | 2.31 | 0.02 |
| EPGLCTWQSLR_673.83_790.4 | -2.08 | 1.25E-01 | 1.02 | -2.05 | 0.04 |

**Table 4. Transitions selected by Cox lasso analysis**

| Transition | coef | exp(coef) | se(coef) | z | Pr(>\|z\|) |
|---|---|---|---|---|---|
| Collection.Window.GA.in.Days | 0.05069 | 1.052 | 0.02348 | 2.159 | 0.0309 |
| SPELQAEAK_486.75_788.4 | 0.68781 | 1.98936 | 0.4278 | 1.608 | 0.1079 |
| SSNNPHSPIVEEFQVPYNK_729.36_261.2 | 2.63659 | 13.96553 | 1.69924 | 1.552 | 0.1208 |

**Table 5. Area under the ROC curve for individual analytes to discriminate preeclampsia subjects from non-preeclampsia subjects. The 196 transitions with the highest ROC area are shown.**

| Transition | ROC area |
|---|---|
| SPELQAEAK_486.75_788.4 | 0.92 |
| SSNNPHSPIVEEFQVPYNK_729.36_261.2 | 0.88 |
| VNHVTLSQPK_561.82_673.4 | 0.85 |
| TLLIANETLR_572.34_703.4 | 0.84 |
| SSNNPHSPIVEEFQVPYNK_729.36_521.3 | 0.83 |
| IIGGSDADIK_494.77_762.4 | 0.82 |
| VVGGLVALR_442.29_784.5 | 0.82 |
| ALNFGGIGVVVGHELTHAFDDQGR_837.09_299.2 | 0.81 |
| DYWSTVK_449.72_620.3 | 0.81 |
| FSVVYAK_407.23_579.4 | 0.81 |
| GWVTDGFSSLK 598.8_953.5 | 0.81 |
| IIGGSDADIK_494.77_260.2 | 0.81 |
| LLEVPEGR_456.76_356.2 | 0.81 |
| DALSSVQESQVAQQAR_572.96_672.4 | 0.80 |
| DPNGLPPEAQK_583.3_497.2 | 0.80 |
| FSVVYAK_407.23_381.2 | 0.80 |
| LLEVPEGR_456.76_686.3 | 0.80 |
| SPELQAEAK_486.75_659.4 | 0.80 |
| VVLSSGSGPGLDLPLVLGLPLQLK_791.48_598.4 | 0.79 |
| ETLLQDFR_511.27_565.3 | 0.79 |
| VNHVTLSQPK_561.82_351.2 | 0.79 |
| VVGGLVALR_442.29_685.4 | 0.79 |
| YTTEIIK_434.25_603.4 | 0.79 |
| DPNGLPPEAQK_583.3_669.4 | 0.78 |
| EDTPNSVWEPAK_686.82_315.2 | 0.78 |
| GWVTDGFSSLK_598.8_854.4 | 0.78 |
| HHGPTITAK_321.18_432.3 | 0.78 |
| LHEAFSPVSYQHDLALLR_699.37_251.2 | 0.78 |
| GA.of.Time.to.Event.in.Days | 0.77 |
| DALSSVQESQVAQQAR_572.96_502.3 | 0.77 |
| DYWSTVK_449.72_347.2 | 0.77 |
| IAQYYYTFK_598.8_395.2 | 0.77 |
| YWGVASFLQK_599.82_849.5 | 0.77 |
| AHYDLR_387.7_288.2 | 0.76 |
| EDTPNSVWEPAK_686.82_630.3 | 0.76 |
| GDTYPAELYITGSILR_884.96_922.5 | 0.76 |
| SVSLPSLDPASAK_636.35_885.5 | 0.76 |
| TSESGELHGLTTEEEFVEGIYK_819.06_310.2 | 0.76 |
| ALEQDLPVNIK_620.35_570.4 | 0.75 |
| HHGPTITAK_321.18_275.1 | 0.75 |
| IAQYYYTFK_598.8_884.4 | 0.75 |
| ITENDIQIALDDAK_779.9_632.3 | 0.75 |
| LPNNVLQEK_327.8_844.3 | 0.75 |
| YWGVASFLQK_599.82_350.2 | 0.75 |
| FQLPGQK_409.23_276.1 | 0.75 |
| HTLNQIDEVK_598.82_958.5 | 0.75 |
| VVLSSGSGPGLDLPLVLGLPLQLK_791.48_768.5 | 0.75 |
| DADPDTFFAK_563.76_302.1 | 0.74 |
| DADPDTFFAK_563.76_825.4 | 0.74 |
| FQLPGQK_409.23_429.2 | 0.74 |
| HFQNLGK_422.23_527.2 | 0.74 |
| VIAVNEVGR_478.78_284.2 | 0.74 |
| VPLALFALNR_557.34_620.4 | 0.74 |
| ETLLQDFR_511.27_322.2 | 0.73 |
| FNAVLTNPQGDYDTSTGK_964.46_262.1 | 0.73 |
| SVSLPSLDPASAK_636.35_473.3 | 0.73 |
| AHYDLR_387.7_566.3 | 0.72 |
| ALNHLPLEYNSALYSR_620.99_538.3 | 0.72 |
| AWVAWR_394.71_258.1 | 0.72 |
| AWVAWR_394.71_531.3 | 0.72 |
| ETAASLLQAGYK_626.33_879.5 | 0.72 |
| IALGGLLFPASNLR_481.29_657.4 | 0.72 |
| IAPQLSTEELVSLGEK_857.47_533.3 | 0.72 |
| ITENDIQIALDDAK_779.9_873.5 | 0.72 |
| VAPEEHPVLLTEAPLNPK_652.03_869.5 | 0.71 |
| EPGLCTWQSLR_673.83_375.2 | 0.71 |
| IAPQLSTEELVSLGEK_857.47_333.2 | 0.71 |
| SPEQQETVLDGNLIIR_906.48_699.3 | 0.71 |
| VSALLTPAEQTGTWK_801.43_371.2 | 0.71 |
| VSALLTPAEQTGTWK_801.43_585.4 | 0.71 |
| VSEADSSNADWVTK_754.85_347.2 | 0.71 |
| GDTYPAELYITGSILR_884.96_274.1 | 0.70 |
| IPGIFELGISSQSDR_809.93_849.4 | 0.70 |
| IQTHSTTYR_369.52_540.3 | 0.70 |
| LLDSLPSDTR_558.8_890.4 | 0.70 |
| QLGLPGPPDVPDHAAYHPF_676.67_299.2 | 0.70 |
| SYELPDGQVITIGNER_895.95_251.1 | 0.70 |
| VILGAHQFVNLFPHVQFILVSR_832.78_860.4 | 0.70 |
| WGAAPYR_410.71_577.3 | 0.69 |
| DFHINLFQVLPWLK_885.49_543.3 | 0.69 |
| LLDSLPSDTR_558.8_276.2 | 0.69 |
| VEPLYELVTATDFAYSSTVR_754.38_549.3 | 0.69 |
| VPTADLEDVLPLAEDITNILSK_789.43_841.4 | 0.69 |
| GGEGTGYFVDFSVR_745.85_869.5 | 0.69 |
| HTLNQIDEVK_598.82_951.5 | 0.69 |
| LIENGYFHPVK_439.57_627.4 | 0.69 |
| LPNNVLQEK_527.8_730.4 | 0.69 |
| NKPGVYTDVAYYLAWIR_677.02_545.3 | 0.69 |
| NTVISVNPSTK_580.32_845.5 | 0.69 |
| QLGLPGPPDVPDHAAYHPF_676.67_263.1 | 0.69 |
| YTTEIIK_434.25_704.4 | 0.69 |
| LPDATPK_371.21_628.3 | 0.68 |
| IEGNLIFDPNNYLPK_873.96_845.5 | 0.68 |
| LEQGENVFLQATDK_796.4_822.4 | 0.68 |
| TLYSSSPR_455.74_533.3 | 0.68 |
| TLYSSSPR_455.74_696.3 | 0.68 |
| VSEADSSNADWVTK_754.85_533.3 | 0.68 |
| DGSPDVTTADIGANTPDATK_973.45_844.4 | 0.67 |
| EWVAIESDSVQPVPR_856.44_486.2 | 0.67 |
| IALGGLLFPASNLR_481.29_412.3 | 0.67 |
| IEEIAAK_387.22_531.3 | 0.67 |
| IEGNLIFDPNNYLPK_873.96_414.2 | 0.67 |
| LYYGDDEK_501.72_726.3 | 0.67 |
| TGISPLALIK_506.82_741.5 | 0.67 |
| VPTADLEDVLPLAEDITNILSK_789.43_940.5 | 0.67 |
| ADSQAQLLLSTVVGVFTAPGLHLK_822.46_983.6 | 0.66 |
| AYSDLSR_406.2_577.3 | 0.66 |
| DFHINLFQVLPWLK_885.49_400.2 | 0.66 |
| DLHLSDVFLK_396.22_260.2 | 0.66 |
| EWVAIESDSVQPVPR_856.44_468.3 | 0.66 |
| FNAVLTNPQGDYDTSTGK_964.46_333.2 | 0.66 |
| LSSPAVITDK_515.79_743.4 | 0.66 |
| LYYGDDEK_501.72_563.2 | 0.66 |
| SGFSFGFK_438.72_732.4 | 0.66 |
| IIEVEEEQEDPYLNDR_995.97_777.4 | 0.66 |
| AVYEAVLR_460.76_750.4 | 0.66 |
| WGAAPYR_410.71_634.3 | 0.66 |
| FTFTLHLETPKPSISSSNLNPR_829.44_874.4 | 0.65 |
| DAQYAPGYDK_564.25_315.1 | 0.65 |
| YGLVTYATYPK_638.33_334.2 | 0.65 |
| DGSPDVTTADIGANTPDATK_973.45_531.3 | 0.65 |
| ETAASLLQAGYK_626.33_679.4 | 0.65 |
| ALNHLPLEYNSALYSR_620.99_696.4 | 0.65 |
| DISEVVTPR_508.27_787.4 | 0.65 |
| IS.2_662.3_313.1 | 0.65 |
| IVLGQEQDSYGGK_697.35_261.2 | 0.65 |
| IVLGQEQDSYGGK_697.35_754.3 | 0.65 |
| TLEAQLTPR_514.79_685.4 | 0.65 |
| VPVAVQGEDTVQSLTQGDGVAK_733.38_775.4 | 0.65 |
| VAPEEHPVLLTEAPLNPK_652.03_568.3 | 0.64 |
| ADSQAQLLLSTVVGVFTAPGLHLK_822.46_664.4 | 0.64 |
| AEAQAQYSAAVAK_654.33_908.5 | 0.64 |
| DISEVVTPR_508.27_472.3 | 0.64 |
| ELLESYIDGR_597.8_710.3 | 0.64 |
| TGISPLALIK_506.82_654.5 | 0.64 |
| TNLESILSYPK_632.84_807.5 | 0.64 |
| DAQYAPGYDK_564.25_813.4 | 0.63 |
| LPTAWPLR_483.31_755.5 | 0.63 |
| DSPVLIDFFEDTER_841.9_512.3 | 0.63 |
| FAFNLYR_465.75_712.4 | 0.63 |
| FVFGTTPEDILR_697.87_843.5 | 0.63 |
| GDSGGAFAVQDPNDK_739.33_473.2 | 0.63 |
| SLDFTELDVAAEK_719.36_316.2 | 0.63 |
| SLLQPNK_400.24_599.4 | 0.63 |
| TLLIANETLR_572.34_816.5 | 0.63 |
| VILGAHQEVNLEPHVQEIEVSR_832.78_603.3 | 0.63 |
| VQEAHLTEDQIFYFPK_655.66_701.4 | 0.63 |
| FTFTLHLETPKPSISSSNLNPR_829.44_787.4 | 0.63 |
| AYSDLSR_406.2_375.2 | 0.62 |
| DDLYVSDAFHK_655.31_344.1 | 0.62 |
| DDLYVSDAFHK_655.31_704.3 | 0.62 |
| DPDQTDGLGLSYLSSHIANVER_796.39_456.2 | 0.62 |
| ESDTSYVSLK_564.77_347.2 | 0.62 |
| ESDTSYVSLK_564.77_696.4 | 0.62 |
| FVFGTTPEDILR_697.87_742.4 | 0.62 |
| ILDDLSPR_464.76_587.3 | 0.62 |
| LEQGENVFLQATDK_796.4_675.4 | 0.62 |
| LHEAFSPVSYQHDLALLR_699.37_380.2 | 0.62 |
| LIENGYFHPVK_439.57_343.2 | 0.62 |
| SLPVSDSVLSGFEQR_810.92_836.4 | 0.62 |
| TWDPEGVIFYGDTNPK_919.93_403.2 | 0.62 |
| VGEYSLYIGR_578.8_708.4 | 0.62 |
| VIAVNEVGR_478.78_744.4 | 0.62 |
| VPGTSTSATLTGLTR_731.4_761.5 | 0.62 |
| YEVQGEVFTKPQLWP_910.96_293.1 | 0.62 |
| AFTECCVVASQLR_770.87_673.4 | 0.61 |
| APLTKPLK_289.86_357.3 | 0.61 |
| DSPVLIDFFEDTER_841.9_399.2 | 0.61 |
| ELLESYIDGR_597.8_839.4 | 0.61 |
| FLQEQGHR_338.84_369.2 | 0.61 |
| IQTHSTTYR_369.52_627.3 | 0.61 |
| IS.3_432.6_397.3 | 0.61 |
| IS.4_706.3_780.3 | 0.61 |
| IS.4_706.3_927.4 | 0.61 |
| IS.5_726.3_876.3 | 0.61 |
| ISLLLIESWLEPVR_834.49_500.3 | 0.61 |
| LQGTLPVEAR_542.31_842.5 | 0.61 |
| NKPGVYTDVAYYLAWIR_677.02_821.5 | 0.61 |
| SLDFTELDVAAEK_719.36_874.5 | 0.61 |
| SYTITGLQPGTDYK_772.39_352.2 | 0.61 |
| TASDFITK_441.73_710.4 | 0.61 |
| VLSALQAVQGLLVAQGR_862.02_941.6 | 0.61 |
| VTGWGNLK_437.74_617.3 | 0.61 |
| YEVQGEVFTKPQLWP_910.96_392.2 | 0.61 |
| AFIQLWAFDAVK_704.89_650.4 | 0.60 |
| APLTKPLK_289.86_260.2 | 0.60 |
| GYVIIKPLVWV_643.9_304.2 | 0.60 |
| IITGLLEFEVYLEYLQNR_738.4_822.4 | 0.60 |
| ILDDLSPR_464.76_702.3 | 0.60 |
| LSSPAVITDK_515.79_830.5 | 0.60 |
| TDAPDLPEENQAR_728.34_843.4 | 0.60 |
| TFTLLDPK_467.77_359.2 | 0.60 |
| TFTLLDPK_467.77_686.4 | 0.60 |
| VLEPTLK_400.25_587.3 | 0.60 |
| YEFLNGR_449.72_606.3 | 0.60 |
| YGLVTYATYPK_638.33_843.4 | 0.60 |

**Table 6. AUROCs for random forest, boosting, lasso, and logistic regression models for a specific number of transitions permitted in the model, as estimated by 100 rounds of bootstrap resampling.**

| Number of transitions | rf | boosting | logit | lasso |
|---|---|---|---|---|
| 1 | 0.81 | 0.75 | 0.48 | 0.92 |
| 2 | 0.95 | 0.85 | 0.61 | 0.86 |
| 3 | 0.95 | 0.83 | 0.56 | 0.93 |
| 4 | 0.94 | 0.82 | 0.52 | 0.92 |
| 5 | 0.95 | 0.81 | 0.51 | 0.94 |
| 6 | 0.95 | 0.81 | 0.49 | 0.93 |
| 7 | 0.95 | 0.83 | 0.46 | 0.93 |
| 8 | 0.96 | 0.79 | 0.49 | 0.91 |
| 9 | 0.95 | 0.82 | 0.46 | 0.88 |
| 10 | 0.94 | 0.80 | 0.50 | 0.85 |
| 11 | 0.93 | 0.78 | 0.49 | 0.84 |
| 12 | 0.94 | 0.79 | 0.47 | 0.82 |
| 13 | 0.92 | 0.80 | 0.48 | 0.84 |
| 14 | 0.95 | 0.73 | 0.47 | 0.83 |
| 15 | 0.93 | 0.73 | 0.49 | 0.83 |

**Table 7. Top 15 transitions selected by each multivariate method, ranked by importance for that method.**

| | rf | boosting | lasso | logit |
|---|---|---|---|---|
| 1 | FSVVYAK_407.23_579.4 | DPNGLPPEAQK_583.3_497.2 | SPELQAEAK_486.75_788.4 | AFIQLWAFDAVK_704.89_650.4 |
| 2 | SPELQAEAK_486.75_788.4 | ALNFGGIGVVVGHELTHAFDDQGR_837.09_299.2 | VILGAHQEVNLEPHVQEIEVSR_832.78_860.4 | AFIQLWAFDAVK_704.89_836.4 |
| 3 | VNHVTLSQPK_561.82_673.4 | ALEQDLPVNIK_620.35_570.4 | VVGGLVALR_442.29_784.5 | AEAQAQYSAAVAK_654.33_709.4 |
| 4 | SSNNPHSPIVEEFQVPYNK_729.36 261.2 | DALSSVQESQVAQQAR_572.96_502.3 | TSFSGFLHGLTTEEEFVEGIYK_819.06_310.2 | AFTECCVVASQLR_770.87_574.3 |
| 5 | SSNNPHSPIVEEFQVPYNK_729.36 521.3 | AHYDLR_387.7_288.2 | SSNNPHSPIVEEFQVPYNK_729.3_6_261.2 | ADSQAQLLLSTVVGVFTAPGLHLK_822.46_664.4 |
| 6 | VVGGLVALR_442.29_784.5 | FQLPGQK_409.23_276.1 | VVLSSGSGPGLDLPLVLGLPLQLK_791.48_598.4 | AEAQAQYSAAVAK_654.33_908.5 |
| 7 | FQLPGQK_409.23_276.1 | AFTECCVVASQLR_770.87_673.4 | ALEQDLPVNIK_620.35_570.4 | ADSQAQLLLSTVVGVFTAPGLHLK_822.46_983.6 |
| 8 | TLLIANETLR_572.34_703.4 | ALNHLPLEYNSALYSR_620.99_538.3 | IQTHSTTYR_369.52 540.3 | AFTECCVVASQLR_770.87 673.4 |
| 9 | DYWSTVK_449.72_620.3 | ADSQAQLLLSTVVGVFTAPGLHLK_822.46_664.4 | SSNNPHSPIVEEFQVPYNK_729.36_521.3 | Collection. Window. GA. in.Days |
| 10 | VVGGLVALR_442.29_685.4 | AEAQAQYSAAVAK_654.33_908.5 | FSVVYAK_407.23_579.4 | AHYDLR_387.7_288.2 |
| 11 | DPNGLPPEAQK_583.3_497.2 | ADSQAQLLLSTVVGVFTAPGLHLK_822.46_983.6 | IAQYYYTFK_598.8_884.4 | AHYDLR_387.7_566.3 |
| 12 | LLEVPEGR_456.76_356.2 | AITPPHPASQANIIFDITEGNLR_825.77_459.3 | IAQYYYTFK_598.8_395.2 | AITPPHPASQANIIFDITEGNLR_825.77_459.3 |
| 13 | GWVTDGFSSLK_598.8_953.5 | Collection. Window. G A.in.Days | GDTYPAELYITGSILR_884.96_922.5 | AITPPHPASQANIIFDITEGNLR_825.77_917.5 |
| 14 | VILGAHQEVNLEPHVQEIEVSR_832.78_860.4 | AEAQAQYSAAVAK_654.33_709.4 | SPEQQETVLDGNLIIR_906.48_699.3 | ALEQDLPVNIK_620.35_570.4 |
| 15 | FQLPGQK_409.23_429.2 | AFIQLWAFDAVK_704.89_650.4 | IAPQLSTEELVSLGEK_857.47_533.3 | ALEQDLPVNIK_620.35_798.5 |

In yet another aspect, the invention provides kits for determining probability of preeclampsia, wherein the kits can be used to detect N of the isolated biomarkers listed in Tables 2, 3, 4, 5 and 7 through 22. For example, the kits can be used to detect one or more, two or more, three or more, four or more, or five of the isolated biomarkers selected from the group consisting of SPELQAEAK, SSNNPHSPIVEEFQVPYN, VNHVTLSQPK, VVGGLVALR, and FSVVYAK, LDFHFSSDR, TVQAVLTVPK, GPGEDFR, ETLLQDFR, ATVVYQGER, and GFQALGDAADIR. In another aspect, the kits can be used to detect one or more, two or more, three or more, four or more, five or more, six or more, seven or more, or eight of the isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4), Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

The kit can include one or more agents for detection of biomarkers, a container for holding a biological sample isolated from a pregnant female; and printed instructions for reacting agents with the biological sample or a portion of the biological sample to detect the presence or amount of the isolated biomarkers in the biological sample. The agents can be packaged in separate containers. The kit can further comprise one or more control reference samples and reagents for performing an immunoassay.

In one embodiment, the kit comprises agents for measuring the levels of at least N of the isolated biomarkers listed in Tables 2, 3, 4, 5 and 7 through 22. The kit can include antibodies that specifically bind to these biomarkers, for example, the kit can contain at least one of an antibody that specifically binds to alpha-1-microglobulin (AMBP), an antibody that specifically binds to ADP/ATP translocase 3 (ANT3), an antibody that specifically binds to apolipoprotein A-II (APOA2), an antibody that specifically binds to apolipoprotein C-III (APOC3), an antibody that specifically binds to apolipoprotein B (APOB), an antibody that specifically binds to beta-2-microglobulin (B2MG), an antibody that specifically binds to retinol binding protein 4 (RBP4 or RET4), an antibody that specifically binds to Inhibin beta C chain (INHBC), an antibody that specifically binds to Pigment epithelium-derived factor (PEDF), an antibody that specifically binds to Prostaglandin-H2 D-isomerase (PTGDS), an antibody that specifically binds to alpha-1-microglobulin (AMBP), an antibody that specifically binds to Beta-2-glycoprotein 1 (APOH), an antibody that specifically binds to Metalloproteinase inhibitor 1 (TIMP1), an antibody that specifically binds to Coagulation factor XIII B chain (F13B), an antibody that specifically binds to Alpha-2-HS-glycoprotein (FETUA), and an antibody that specifically binds to Sex hormone-binding globulin (SHBG).

The kit can comprise one or more containers for compositions contained in the kit. Compositions can be in liquid form or can be lyophilized. Suitable containers for the compositions include, for example, bottles, vials, syringes, and test tubes. Containers can be formed from a variety of materials, including glass or plastic. The kit can also comprise a package insert containing written instructions for methods of determining probability of preeclampsia.

From the foregoing description, it will be apparent that variations and modifications can be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

The following examples are provided by way of illustration, not limitation.

### EXAMPLES

### Example 1. Development of Sample Set for Discovery and Validation of Biomarkers for Preeclampsia

A standard protocol was developed governing conduct of the Proteomic Assessment of Preterm Risk (PAPR) clinical study. This protocol also provided the option that the samples and clinical information could be used to study other pregnancy complications. Specimens were obtained from women at 11 Internal Review Board (IRB) approved sites across the United States. After providing informed consent, serum and plasma samples were obtained, as well as pertinent information regarding the patient's demographic characteristics, past medical and pregnancy history, current pregnancy history and concurrent medications. Following delivery, data were collected relating to maternal and infant conditions and complications. Serum and plasma samples were processed according to a protocol that requires standardized refrigerated centrifugation, aliquoting of the samples into 0.5 ml 2-D bar-coded cryovials and subsequent freezing at -80°C.

Following delivery, preeclampsia cases were individually reviewed. Only preterm preeclampsia cases were used for this analysis. For discovery of biomarkers of preeclampsia, 20 samples collected between 17-28 weeks of gestation were analyzed. Samples included 9 cases, 9 term controls matched within one week of sample collection and 2 random term controls. The samples were processed in batches of 24 that included 20 clinical samples and 4 identical human gold standards (HGS). HGS samples are identical aliquots from a pool of human blood and were used for quality control. HGS samples were placed in position 1, 8, 15 and 24 of a batch with patient samples processed in the remaining 20 positions. Matched cases and controls were always processed adjacently.

The samples were subsequently depleted of high abundance proteins using the Human 14 Multiple Affinity Removal System (MARS 14), which removes 14 of the most abundant proteins that are essentially uninformative with regard to the identification for disease-relevant changes in the serum proteome. To this end, equal volumes of each clinical or HGS sample were diluted with column buffer and filtered to remove precipitates. Filtered samples were depleted using a MARS-14 column (4.6 x 100 mm, Cat. #5188-6558, Agilent Technologies). Samples were chilled to 4°C in the autosampler, the depletion column was run at room temperature, and collected fractions were kept at 4°C until further analysis. The unbound fractions were collected for further analysis.

A second aliquot of each clinical serum sample and of each HGS was diluted into ammonium bicarbonate buffer and depleted of the 14 high and approximately 60 additional moderately abundant proteins using an IgY14-SuperMix (Sigma) hand-packed column, comprised of 10 mL of bulk material (50% slurry, Sigma). Shi et al., Methods, 56(2):246-53 (2012). Samples were chilled to 4°C in the autosampler, the depletion column was run at room temperature, and collected fractions were kept at 4°C until further analysis. The unbound fractions were collected for further analysis.

Depleted serum samples were denatured with trifluorethanol, reduced with dithiotreitol, alkylated using iodoacetamide, and then digested with trypsin at a 1:10 trypsin: protein ratio. Following trypsin digestion, samples were desalted on a C18 column, and the eluate lyophilized to dryness. The desalted samples were resolubilized in a reconstitution solution containing five internal standard peptides.

Depleted and trypsin digested samples were analyzed using a scheduled Multiple Reaction Monitoring method (sMRM). The peptides were separated on a 150 mm x 0.32 mm Bio-Basic C18 column (ThermoFisher) at a flow rate of 5 µl/min using a Waters Nano Acquity UPLC and eluted using an acetonitrile gradient into a AB SCIEX QTRAP 5500 with a Turbo V source (AB SCIEX, Framingham, MA). The sMRM assay measured 1708 transitions that correspond to 854 peptides and 236 proteins. Chromatographic peaks were integrated using Rosetta Elucidator software (Ceiba Solutions).

Transitions were excluded from analysis, if their intensity area counts were less than 10000 and if they were missing in more than three samples per batch. Intensity area counts were log transformed and Mass Spectrometry run order trends and depletion batch effects were minimized using a regression analysis.

### Example 2. Analysis of Transitions to Identify PE Biomarkers

The objective of these analyses was to examine the data collected in Example 1 to identify transitions and proteins that predict preeclampsia. The specific analyses employed were (i) Cox time-to-event analyses and (ii) models with preeclampsia as a binary categorical dependent variable. The dependent variable for all the Cox analyses was Gestational Age of time to event (where event is preeclampsia). For the purpose of the Cox analyses, preeclampsia subjects have the event on the day of birth. Non-preeclampsia subjects are censored on the day of birth. Gestational age on the day of specimen collection is a covariate in all Cox analyses.

The assay data obtained in Example 1 were previously adjusted for run order and log transformed. The data was not further adjusted. There were 9 matched non-preeclampsia subjects, and two unmatched non-preeclampsia subjects, where matching was done according to center, gestational age and ethnicity.

### Univariate Cox Proportional Hazards Analyses

Univariate Cox Proportional Hazards analyses was performed to predict Gestational Age of time to event (preeclampsia), including Gestational age on the day of specimen collection as a covariate. Table 1 shows the 40 transitions with p-values less than 0.05. Table 2 shows the same transitions sorted by protein ID. There are 8 proteins that have multiple transitions with p-values less than 0.05: AMBP, ANT3, APOA2, APOB, APOC3, B2MG, CIS, and RET4.

### Multivariate Cox Proportional Hazards Analyses: Stepwise AIC selection

Cox Proportional Hazards analyses was performed to predict Gestational Age of time to event (preeclampsia), including Gestational age on the day of specimen collection as a covariate, using stepwise and lasso models for variable selection. The stepwise variable selection analysis used the Akaike Information Criterion (AIC) as the stopping criterion. Table 3 shows the transitions selected by the stepwise AIC analysis. The coefficient of determination (R²) for the stepwise AIC model is 0.87 of a maximum possible 0.9.

### Multivariate Cox Proportional Hazards Analyses: lasso selection

Lasso variable selection was utilized as the second method of multivariate Cox Proportional Hazards analyses to predict Gestational Age of time to event (preeclampsia), including Gestational age on the day of specimen collection as a covariate. Lasso regression models estimate regression coefficients using penalized optimization methods, where the penalty discourages the model from considering large regression coefficients since we usually believe such large values are not very likely. As a result, some regression coefficients are forced to be zero (i.e., excluded from the model). Here, the resulting model included analytes with non-zero regression coefficients only. The number of these analytes (with non-zero regression coefficients) depends on the severity of the penalty. Cross-validation was used to choose an optimum penalty level. Table 4 shows the results. The coefficient of determination (R²) for the lasso model is 0.53 of a maximum possible 0.9.

### Univariate ROC analysis of preeclampsia as a binary categorical dependent variable

Univariate analyses was used to discriminate preeclampsia subjects from non-preeclampsia subjects (preeclampsia as a binary categorical variable) as estimated by area under the receiver operating characteristic (ROC) curve. Table 5 shows the area under the ROC curve for the 196 transitions with the highest ROC area of 0.6 or greater.

### Multivariate analysis of preeclampsia as a binary categorical dependent variable

Multivariate analyses was performed to predict preeclampsia as a binary categorical dependent variable, using random forest, boosting, lasso, and logistic regression models. Random forest and boosting models grow many classification trees. The trees vote on the assignment of each subject to one of the possible classes. The forest chooses the class with the most votes over all the trees.

For each of the four methods (random forest, boosting, lasso, and logistic regression) each method was allowed to select and rank its own best 15 transitions. We then built models with 1 to 15 transitions. Each method sequentially reduces the number of nodes from 15 to 1 independently. A recursive option was used to reduce the number nodes at each step: To determine which node to be removed, the nodes were ranked at each step based on their importance from a nested cross-validation procedure. The least important node was eliminated. The importance measures for lasso and logistic regression are z-values. For random forest and boosting, the variable importance was calculated from permuting out-of-bag data: for each tree, the classification error rate on the out-of-bag portion of the data was recorded; the error rate was then recalculated after permuting the values of each variable (i.e., transition); if the transition was in fact important, there would have been be a big difference between the two error rates; the difference between the two error rates were then averaged over all trees, and normalized by the standard deviation of the differences. The AUCs for these models are shown in Table 6 and in Figure 1, as estimated by 100 rounds of bootstrap resampling. Table 7 shows the top 15 transitions selected by each multivariate method, ranked by importance for that method. These multivariate analyses suggest that models that combine 2 or more transitions give AUC greater than 0.9, as estimated by bootstrap.

In multivariate models, random forest (rf) and lasso models gave the best area under the ROC curve as estimated by bootstrap. The following transitions were selected by these two models for having high univariate ROC's:.
FSVVYAK_407.23_579.4
SPELQAEAK_486.75_788.4
VNHVTLSQPK 561.82_673.4
SSNNPHSPIVEEFQVPYNK 729.36_261.2
SSNNPHSPIVEEFQVPYNK 729.36_521.3
VVGGLVALR_442.29_784.5

In summary, univariate and multivariate Cox analyses were performed using transitions collected in Example 1 to predict Gestational Age at Birth, including Gestational age on the day of specimen collection as a covariate. In the univariate Cox analyses, 8 proteins were identified with multiple transitions with p-value less than 0.05. In multivariate Cox analyses, stepwise AIC variable analysis selected 4 transitions, while the lasso model selected 2 transitions. Univariate (ROC) and multivariate (random forest, boosting, lasso, and logistic regression) analyses were performed to predict preeclampsia as a binary categorical variable. Univariate analyses identify 78 analytes with AUROC of 0.7 or greater and 196 analytes with AUROC of 0.6 or greater. Multivariate analyses suggest that models that combine 2 or more transitions give AUC greater than 0.9, as estimated by bootstrap.

From the foregoing description, it will be apparent that variations and modifications can be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

### Example 3. Study II Shotgun Identification of Preeclampsia Biomarkers

A further study used a hypothesis-independent shotgun approach to identify and quantify additional biomarkers not present on our multiplexed hypothesis dependent MRM assay. Samples were processed as described in the preceding Examples unless noted below.

Serum samples were depleted of the 14 most abundant serum samples by MARS 14 as described in Example 1. Depleted serum was then reduced with dithiothreitol, alkylated with iodacetamide, and then digested with trypsin at a 1:20 trypsin to protein ratio overnight at 37°C. Following trypsin digestion, the samples were desalted on an Empore C18 96-well Solid Phase Extraction Plate (3M Company) and lyophilized to dryness. The desalted samples were resolubilized in a reconstitution solution containing five internal standard peptides.

Tryptic digests of MARS depleted patient (preeclampsia cases and normal pregnancycontrols) samples were fractionated by two-dimensional liquid chromatography and analyzed by tandem mass spectrometry. Aliquots of the samples, equivalent to 3-4 µl of serum, were injected onto a 6 cm x 75µm self-packed strong cation exchange (Luna SCX, Phenomenex) column. Peptides were eluded from the SCX column with salt (15, 30, 50, 70, and 100% B, where B = 250mM ammonium acetate, 2% acetonitrile, 0.1% formic acid in water) and consecutively for each salt elution, were bound to a 0.5 µl C18 packed stem trap (Optimize Technologies, Inc.) and further fractionated on a 10 cm x 75 µm reversed phase ProteoPep II PicoFrit column (New Objective). Peptides were eluted from the reversed phase column with an acetonitrile gradient containing 0.1% formic acid and directly ionized on an LTQ-Orbitrap (ThermoFisher). For each scan, peptide parent ion masses were obtained in the Orbitrap at 60K resolution and the top seven most abundant ions were fragmented in the LTQ to obtain peptide sequence information.

Parent and fragment ion data were used to search the Human RefSeq database using the Sequest (Eng et al., J. Am. Soc. Mass Spectrom 1994; 5:976-989) and X!Tandem (Craig and Beavis, Bioinformatics 2004; 20:1466-1467) algorithms. For Sequest, data was searched with a 20 ppm tolerance for the parent ion and 1 AMU for the fragment ion. Two missed trypsin cleavages were allowed, and modifications included static cysteine carboxyamidomethylation and methionine oxidation. After searching the data was filtered by charge state vs. Xcorr scores (charge +1 ≥ 1.5 Xcorr, charge +2 ≥ 2.0, charge +3 ≥ 2.5). Similar search parameters were used for X!tandem, except the mass tolerance for the fragment ion was 0.8 AMU and there is no Xcorr filtering. Instead, the PeptideProphet algorithm (Keller et al., Anal. Chem 2002;74:5383-5392) was used to validate each X! Tandem peptide-spectrum assignment and protein assignments were validated using ProteinProphet algorithm (Nesvizhskii et al., Anal. Chem 2002; 74:5383-5392). Data was filtered to include only the peptide-spectrum matches that had PeptideProphet probability of 0.9 or more. After compiling peptide and protein identifications, spectral count data for each peptide were imported into DAnTE software (Polpitiya et al., Bioinformatics. 2008; 24:1556-1558). Log transformed data was mean centered and missing values were filtered, by requiring that a peptide had to be identified in at least 2 cases and 2 controls. To determine the significance of an analyte, Receiver Operating Characteristic (ROC) curves for each analyte were created where the true positive rate (Sensitivity) is plotted as a function of the false positive rate (1-Specificity) for different thresholds that separate the SPTB and Term groups. The area under the ROC curve (AUC) is equal to the probability that a classifier will rank a randomly chosen positive instance higher than a randomly chosen negative one. Peptides with AUC greater than or equal to 0.6 identified by both approaches are found in Table 8 and those found uniquely by Sequest or Xtandem are found in Tables 9 and 10, respectively.

The differentially expressed proteins identified by the hypothesis-independent strategy above, not already present in our MRM-MS assay, were candidates for incorporation into the MRM-MS assay. Candidates were prioritized by AUC and biological function, with preference given for new pathways. Sequences for each protein of interest, were imported into Skyline software which generated a list of tryptic peptides, m/z values for the parent ions and fragment ions, and an instrument-specific collision energy (McLean et al. Bioinformatics (2010) 26 (7): 966-968.McLean et al. Anal. Chem (2010) 82 (24): 10116-10124).

The list was refined by eliminating peptides containing cysteines and methionines, where possible, and by using the shotgun data to select the charge state(s) and a subset of potential fragment ions for each peptide that had already been observed on a mass spectrometer.

After prioritizing parent and fragment ions, a list of transitions was exported with a single predicted collision energy. Approximately 100 transitions were added to a single MRM run. For development, MRM data was collected on either a QTRAP 5500 (AB Sciex) or a 6490 QQQ (Agilent). Commercially available human female serum (from pregnant and non-pregnant donors), was depleted and processed to tryptic peptides, as described above, and used to "scan" for peptides of interest. For development, peptides from the digested serum were separated with a 15 min acetonitrile.e gradient at 100 ul/min on a 2.1 x 50 mM Poroshell 120 EC-C18 column (Agilent) at 40°C.

The MS/MS data was imported back into Skyline, where all chromatograms for each peptide were overlayed and used to identify a concensus peak corresponding to the peptide of interest and the transitions with the highest intensities and the least noise. Table 11, contains a list of the most intensely observed candidate transitions and peptides for transfer to the MRM assay.

Next, the top 2-10 transitions per peptide and up to 7 peptides per protein were selected for collision energy (CE) optimization on the Agilent 6490. Using Skyline or MassHunter Qual software, the optimized CE value for each transition was determined based on the peak area or signal to noise. The two transitions with the largest peak areas per peptide and at least two peptides per protein were chosen for the final MRM method. Substitutions of transitions with lower peak areas were made when a transition with a larger peak area had a high background level or had a low m/z value that has more potential for interference.

Lastly, the retention times of selected peptides were mapped using the same column and gradient as our established sMRM assay. The newly discovered analytes were subsequently added to the sMRM method and used in a further hypothesis-dependent discovery study described in Example 4 below.

The above method was typical for most proteins. However, in some cases, the differentially expressed peptide identified in the shotgun method did not uniquely identify a protein, for example, in protein families with high sequence identity. In these cases, a MRM method was developed for each family member. Also, let it be noted that, for any given protein, peptides in addition to those found to be significant and fragment ions not observed on the Orbitrap may have been included in MRM optimization and added to the final sMRM method if those yielded the best signal intensities. In some cases, transition selection and CEs were re-optmized using purified, synthetic peptides.

**Table 8. Preeclampsia: Peptides significant with AUC > 0.6 by X!Tandem and Sequest**

| Protein description | Uniprot ID (name) | Peptide | XT_AUC | S_AUC |
|---|---|---|---|---|
| afamin | P43652 (AFAM_HUMAN) | R.IVQIYKDLLR.N | 0.67 | 0.63 |
| afamin | P43652 (AFAM_HUMAN) | K.VMNHICSK.Q | 0.73 | 0.74 |
| afamin | P43652 (AFAM_HUMAN) | R.RHPDLSIPELLR.I | 0.86 | 0.83 |
| afamin | P43652 (AFAM_HUMAN) | K.HFQNLGK.D | 0.71 | 0.75 |
| alpha-1-antichymotrypsin | P01011 (AACT_HUMAN) | K.ITLLSALVETR.T | 0.68 | 0.70 |
| alpha-1-antichymotrypsin | P01011 (AACT_HUMAN) | R.LYGSEAFATDFQDSAAA K.K | 0.70 | 0.78 |
| alpha-1-antichymotrypsin | P01011 (AACT_HUMAN) | R.NLAVSQVVHK.A | 0.81 | 0.79 |
| alpha-1B-glycoprotein | P04217 (A1BG_HUMAN) | R.CEGPIPDVTFELLR.E | 0.78 | 0.60 |
| alpha-1B-glycoprotein | P04217 (A1BG_HUMAN) | R.LHDNQNGWSGDSAPVEL ILSDETLPAPEFSPEPESGR. A | 0.72 | 0.66 |
| alpha-1B-glycoprotein | P04217 (A1BG_HUMAN) | R.CEGPIPDVTFELLR.E | 0.64 | 0.60 |
| alpha-1B-glycoprotein | P04217 (A1BG_HUMAN) | R.TPGAAANLELIFVGPQHA GNYR.C | 0.71 | 0.67 |
| alpha-1B-glycoprotein | P04217 (A1BG_HUMAN) | K.LLELTGPK.S | 0.70 | 0.66 |
| alpha-1B-glycoprotein | P04217 (A1BG_HUMAN) | R.ATWSGAVLAGR.D | 0.84 | 0.74 |
| alpha-2-antiplasmin | P08697 (A2AP_HUMAN) | K.HQM*DLVATLSQLGLQE LFQAPDLR.G | 0.67 | 0.67 |
| alpha-2-antiplasmin | P08697 (A2AP_HUMAN) | K.LGNQEPGGQTALK.S | 0.83 | 0.83 |
| alpha-2-antiplasmin | P08697 (A2AP_HUMAN) | K.GFPIKEDFLEQSEQLFGA KPVSLTGK.Q | 0.68 | 0.65 |
| alpha-2-HS-glycoprotein preproprotein | P02765 (FETUA_HUMAN) | R.QPNCDDPETEEAALVAID YINQNLPWGYK.H | 0.61 | 0.61 |
| alpha-2-HS-glycoprotein preproprotein | P02765 (FETUA_HUMAN) | K.VWPQQPSGELFEIEIDTL ETTCHVLDPTPVAR.C | 0.79 | 0.67 |
| alpha-2-HS-glycoprotein preproprotein | P02765 (FETUA_HUMAN) | K.EHAVEGDCDFQLLK.L | 0.90 | 0.77 |
| alpha-2-HS-glycoprotein preproprotein | P02765 (FETUA_HUMAN) | R.QPNCDDPETEEAALVAID YINQNLPWGYK.H | 0.63 | 0.61 |
| alpha-2-HS-glycoprotein preproprotein | P02765 (FETUA_HUMAN) | K.HTLNQIDEVK.V | 0.70 | 0.68 |
| alpha-2-HS-glycoprotein preproprotein | P02765 (FETUA_HUMAN) | R.TVVQPSVGAAAGPVVPP CPGR.I | 0.83 | 0.83 |
| angiotensinogen preproprotein | P01019 (ANGT_HUMAN) | K.TGCSLMGASVDSTLAFN TYVHFQGK.M | 0.75 | 0.67 |
| angiotensinogen preproprotein | P01019 (ANGT_HUMAN) | R.AAM*VGMLANFLGFR.I | 0.65 | 0.63 |
| angiotensinogen preproprotein | P01019 (ANGT_HUMAN) | R.AAMVGMLANFLGFR.I | 0.65 | 0.64 |
| angiotensinogen preproprotein | P01019 (ANGT_HUMAN) | R.AAM*VGM*LANFLGFR.I | 0.65 | 0.65 |
| angiotensinogen preproprotein | P01019 (ANGT_HUMAN) | R.AAMVGM*LANFLGFR.I | 0.65 | 0.74 |
| angiotensinogen preproprotein | P01019 (ANGT_HUMAN) | K.QPFVQGLALYTPVVLPR. S | 0.60 | 0.74 |
| angiotensinogen preproprotein | P01019 (ANGT_HUMAN) | R.AAM*VGMLANFLGFR.I | 0.64 | 0.63 |
| angiotensinogen preproprotein | P01019 (ANGT_HUMAN) | R.AAMVGMLANFLGFR.I | 0.64 | 0.64 |
| angiotensinogen preproprotein | P01019 (ANGT_HUMAN) | R.AAM*VGM*LANFLGFR.I | 0.64 | 0.65 |
| angiotensinogen preproprotein | P01019 (ANGT_HUMAN) | R.AAMVGM*LANFLGFR.I | 0.64 | 0.74 |
| angiotensinogen preproprotein | P01019 (ANGT_HUMAN) | K.VLSALQAVQGLLVAQGR .A | 0.74 | 0.77 |
| angiotensinogen preproprotein | P01019 (ANGT_HUMAN) | K.QPFVQGLALYTPVVLPR. S | 0.75 | 0.74 |
| angiotensinogen preproprotein | P01019 (ANGT_HUMAN) | R.ADSQAQLLLSTVVGVFT APGLHLK.Q | 0.78 | 0.77 |
| antithrombin-III | P01008 (ANT3_HUMAN) | R.ITDVIPSEAINELTVLVLV NTIYFK.G | 0.78 | 0.78 |
| antithrombin-III | P01008 (ANT3_HUMAN) | K.NDNDNIFLSPLSISTAFA MTK.L | 0.87 | 0.83 |
| antithrombin-III | P01008 (ANT3_HUMAN) | R.EVPLNTIIFMGR.V | 0.69 | 0.62 |
| antithrombin-III | P01008 (ANT3_HUMAN) | R.EVPLNTIIFM*GR.V | 0.69 | 0.69 |
| antithrombin-III | P01008 (ANT3_HUMAN) | R.VAEGTQVLELPFKGDDIT M*VLILPKPEK.S | 0.83 | 0.92 |
| antithrombin-III | P01008 (ANT3_HUMAN) | R.VAEGTQVLELPFKGDDIT MVLILPKPEK.S | 0.83 | 0.96 |
| antithrombin-III | P01008 (ANT3_HUMAN) | K.EQLQDMGLVDLFSPEK.S | 0.85 | 0.86 |
| antithrombin-III | P01008 (ANT3_HUMAN) | R.VAEGTQVLELPFKGDDIT M*VLILPKPEK.S | 0.94 | 0.92 |
| antithrombin-III | P01008 (ANT3_HUMAN) | R.VAEGTQVLELPFKGDDIT MVLILPKPEK.S | 0.94 | 0.96 |
| antithrombin-III | P01008 (ANT3_HUMAN) | R.EVPLNTIIFMGR.V | 0.63 | 0.62 |
| antithrombin-III | P01008 (ANT3_HUMAN) | R.EVPLNTIIFM*GR.V | 0.63 | 0.69 |
| antithrombin-III | P01008 (ANT3_HUMAN) | R.DIPMNPMCIYR.S | 0.71 | 0.70 |
| apolipoprotein A-II preproprotein | P02652 (APOA2_HUMAN) | K.EPCVESLVSQYFQTVTD | 0.83 | 0.83 |
| | | YGK.D | | |
| apolipoprotein A-IV | P06727 (APOA4_HUMAN) | K.SLAELGGHLDQQVEEFR. R | 0.67 | 0.67 |
| apolipoprotein A-IV | P06727 (APOA4_HUMAN) | R.LAPLAEDVR.G | 0.67 | 0.90 |
| apolipoprotein A-IV | P06727 (APOA4_HUMAN) | R.VLRENADSLQASLRPHA DELK.A | 0.79 | 0.63 |
| apolipoprotein A-IV | P06727 (APOA4_HUMAN) | R.SLAPYAQDTQEKLNHQL EGLTFQMK.K | 0.90 | 0.65 |
| apolipoprotein A-IV | P06727 (APOA4_HUMAN) | R.SLAPYAQDTQEKLNHQL EGLTFQM*K.K | 0.90 | 0.69 |
| apolipoprotein A-IV | P06727 (APOA4_HUMAN) | K.LGPHAGDVEGHLSFLEK. D | 0.63 | 0.73 |
| apolipoprotein A-IV | P06727 (APOA4_HUMAN) | K.SELTQQLNALFQDKLGE VNTYAGDLQK.K | 0.68 | 0.68 |
| apolipoprotein A-IV | P06727 (APOA4_HUMAN) | R.SLAPYAQDTQEKLNHQL EGLTFQMK.K | 0.71 | 0.65 |
| apolipoprotein A-IV | P06727 (APOA4_HUMAN) | R.SLAPYAQDTQEKLNHQL EGLTFQM*K.K | 0.71 | 0.69 |
| apolipoprotein A-IV | P06727 (APOA4_HUMAN) | R.LLPHANEVSQK.I | 0.62 | 0.79 |
| apolipoprotein A-IV | P06727 (APOA4_HUMAN) | K.SLAELGGHLDQQVEEFR R.R | 0.67 | 0.69 |
| apolipoprotein A-IV | P06727 (APOA4_HUMAN) | K.SELTQQLNALFQDK.L | 0.68 | 0.62 |
| apolipoprotein B-100 | P04114 (APOB_HUMAN) | K.GFEPTLEALFGK.Q | 0.73 | 0.76 |
| apolipoprotein B-100 | P04114 (APOB_HUMAN) | K.ALYWVNGQVPDGVSK.V | 0.78 | 0.67 |
| apolipoprotein B-100 | P04114 (APOB_HUMAN) | K.FIIPSPK.R | 0.90 | 0.90 |
| apolipoprotein B-100 | P04114 (APOB_HUMAN) | R.TPALHFK.S | 0.68 | 0.81 |
| apolipoprotein B-100 | P04114 (APOB_HUMAN) | K.TEVIPPLIENR.Q | 0.62 | 0.64 |
| apolipoprotein B-100 | P04114 (APOB_HUMAN) | R.NLQNNAEWVYQGAIR.Q | 0.65 | 0.60 |
| apolipoprotein B-100 | P04114 (APOB_HUMAN) | K.LPQQANDYLNSFNWER. Q | 0.65 | 0.62 |
| apolipoprotein B-100 | P04114 (APOB_HUMAN) | R.LAAYLMLMR.S | 0.60 | 0.73 |
| apolipoprotein B-100 | P04114 (APOB_HUMAN) | R.VIGNMGQTMEQLTPELK. S | 0.68 | 0.67 |
| apolipoprotein B-100 | P04114 (APOB_HUMAN) | K.LIVAMSSWLQK.A | 0.74 | 0.86 |
| apolipoprotein B-100 | P04114 (APOB_HUMAN) | R.TSSFALNLPTLPEVK.F | 0.79 | 0.70 |
| apolipoprotein B-100 | P04114 (APOB_HUMAN) | K.IADFELPTIIVPEQTIEIPSI K.F | 0.62 | 0.61 |
| apolipoprotein B-100 | P04114 (APOB_HUMAN) | K.IEGNLIFDPNNYLPK.E | 0.63 | 0.62 |
| apolipoprotein B-100 | P04114 (APOB_HUMAN) | R.TSSFALNLPTLPEVKFPE VDVLTK.Y | 0.66 | 0.72 |
| apolipoprotein B-100 | P04114 (APOB_HUMAN) | R.LELELRPTGEIEQYSVSA TYELQR.E | 0.78 | 0.78 |
| apolipoprotein C-II | P02655 (APOC2_HUMAN) | K.STAAMSTYTGIFTDQVLS VLK.G | 0.73 | 0.73 |
| apolipoprotein C-III | P02656 (APOC3_HUMAN) | R.GWVTDGFSSLKDYWST VKDK.F | 1.00 | 1.00 |
| apolipoprotein E | P02649 (APOE_HUMAN) | R.WELALGR.F | 0.60 | 0.63 |
| apolipoprotein E | P02649 (APOE_HUMAN) | R.LAVYQAGAR.E | 0.61 | 0.64 |
| apolipoprotein E | P02649 (APOE_HUMAN) | K.SWFEPLVEDMQR.Q | 0.83 | 0.73 |
| apolipoprotein E | P02649 (APOE_HUMAN) | R.AATVGSLAGQPLQER.A | 0.67 | 0.67 |
| apolipoprotein(a) | P08519 (APOA_HUMAN) | R.TPEYYPNAGLIMNYCR.N | 0.72 | 0.61 |
| beta-2-glycoprotein 1 | P02749 (APOH_HUMAN) | K.TFYEPGEEITYSCKPGYV SR.G | 0.66 | 0.76 |
| beta-2-glycoprotein 1 | P02749 (APOH_HUMAN) | K.FICPLTGLWPINTLK.C | 0.72 | 0.70 |
| bone marrow proteoglycan | P13727 (PRG2_HUMAN) | R.SLQTFSQAWFTCR.R | 0.82 | 0.72 |
| ceruloplasmin | P00450 (CERU_HUMAN) | K.HYYIGIIETTWDYASDHG EKK.L | 0.78 | 0.89 |
| ceruloplasmin | P00450 (CERU_HUMAN) | R.EYTDASFTNRK.E | 0.63 | 0.63 |
| ceruloplasmin | P00450 (CERU_HUMAN) | K.M*YYSAVDPTKDIFTGLI GPMK.I | 0.66 | 0.68 |
| ceruloplasmin | P00450 (CERU_HUMAN) | K.M*YYSAVDPTKDIFTGLI GPM*K.I | 0.66 | 0.76 |
| ceruloplasmin | P00450 (CERU_HUMAN) | R.SGAGTEDSACIPWAYYS TVDQVKDLYSGLIGPLIVC R.R | 0.95 | 0.95 |
| ceruloplasmin | P00450 (CERU_HUMAN) | R.KAEEEHLGILGPQLHAD VGDKVK.I | 0.85 | 0.77 |
| ceruloplasmin | P00450 (CERU_HUMAN) | K.EVGPTNADPVCLAK.M | 0.62 | 0.77 |
| ceruloplasmin | P00450 (CERU_HUMAN) | R.MYSVNGYTFGSLPGLSM CAEDR.V | 0.63 | 0.71 |
| ceruloplasmin | P00450 (CERU_HUMAN) | K.DIASGLIGPLIICK.K | 0.63 | 0.66 |
| ceruloplasmin | P00450 (CERU_HUMAN) | R.QKDVDKEFYLFPTVFDE NESLLLEDNIR.M | 0.64 | 0.66 |
| ceruloplasmin | P00450 (CERU_HUMAN) | R.GPEEEHLGILGPVIWAEV GDTIR.V | 0.65 | 0.61 |
| ceruloplasmin | P00450 (CERU_HUMAN) | K.M*YYSAVDPTKDIFTGLI GPMK.I | 0.67 | 0.68 |
| ceruloplasmin | P00450 (CERU_HUMAN) | K.M*YYSAVDPTKDIFTGLI GPM*K.I | 0.67 | 0.76 |
| ceruloplasmin | P00450 (CERU_HUMAN) | K.M*YYSAVDPTKDIFTGLI GPMK.I | 0.67 | 0.68 |
| ceruloplasmin | P00450 (CERU_HUMAN) | K.M*YYSAVDPTKDIFTGLI GPM*K.I | 0.67 | 0.76 |
| ceruloplasmin | P00450 (CERU_HUMAN) | K.GAYPLSIEPIGVR.F | 0.67 | 0.63 |
| ceruloplasmin | P00450 (CERU_HUMAN) | R.GVYSSDVFDIFPGTYQTL EM*FPR.T | 0.67 | 0.67 |
| ceruloplasmin | P00450 (CERU_HUMAN) | K.DIASGLIGPLIICKK.D | 0.67 | 0.73 |
| ceruloplasmin | P00450 (CERU_HUMAN) | R.SGAGTEDSACIPWAYYS TVDQVK.D | 0.70 | 0.70 |
| ceruloplasmin | P00450 (CERU_HUMAN) | R.IYHSHIDAPK.D | 0.77 | 0.76 |
| ceruloplasmin | P00450 (CERU_HUMAN) | R.ADDKVYPGEQYTYMLL ATEEQSPGEGDGNCVTR.I | 0.77 | 0.80 |
| ceruloplasmin | P00450 (CERU_HUMAN) | K.DLYSGLIGPLIVCR.R | 0.78 | 0.82 |
| ceruloplasmin | P00450 (CERU_HUMAN) | R.TTIEKPVWLGFLGPIIK.A | 0.88 | 0.85 |
| cholinesterase | P06276 (CHLE_HUMAN) | K.IFFPGVSEFGK.E | 0.87 | 0.76 |
| cholinesterase | P06276 (CHLE_HUMAN) | R.AILQSGSFNAPWAVTSLY EAR.N | 1.00 | 0.83 |
| coagulation factor XII | P00748 (FA12_HUMAN) | R.LHEAFSPVSYQHDLALL R.L | 0.72 | 0.76 |
| coagulation factor XIII B chain | P05160 (F13B_HUMAN) | R.GDTYPAELYITGSILR.M | 0.67 | 0.83 |
| coagulation factor XIII B chain | P05160 (F13B_HUMAN) | K.VLHGDLIDFVCK.Q | 0.69 | 0.60 |
| complement C1r subcomponent | P00736 (C1R_HUMAN) | K.LVFQQFDLEPSEGCFYD YVK.I | 0.69 | 0.66 |
| complement C1s subcomponent | P09871 (C1S_HUMAN) | R.VKNYVDWIMK.T | 0.69 | 0.60 |
| complement C1s subcomponent | P09871 (C1S_HUMAN) | K.SNALDIIFQTDLTGQK.K | 0.75 | 0.70 |
| complement C2 | P06681 (CO2_HUMAN) | R.DFHINLFR.M | 0.75 | 0.72 |
| complement C2 | P06681 (CO2_HUMAN) | R.GALISDQWVLTAAHCFR. D | 0.60 | 0.75 |
| complement C2 | P06681 (CO2_HUMAN) | K.KNQGILEFYGDDIALLK. L | 0.62 | 0.67 |
| complement C3 | P01024 (CO3_HUMAN) | R.IHWESASLLR.S | 0.80 | 0.77 |
| complement C4-B-like preproprotein | P0C0L5 (CO4B_HUMAN) | R.VHYTVCIWR.N | 0.67 | 0.65 |
| complement C4-B-like preproprotein | P0C0L5 (CO4B_HUMAN) | K.AEMADQAAAWLTR.Q | 0.78 | 0.89 |
| complement C4-B-like preproprotein | P0C0L5 (CO4B_HUMAN) | K.M*RPSTDTITVMVENSH GLR.V | 0.65 | 0.65 |
| complement C4-B-like preproprotein | P0C0L5 (CO4B_8HUMAN) | K.MRPSTDTITVMVENSHG LR.V | 0.65 | 0.72 |
| complement C4-B-like preproprotein | P0C0L5 (CO4B_HUMAN) | R.VQQPDCREPFLSCCQFAE SLRK.K | 0.67 | 0.60 |
| complement C4-B-like preproprotein | P0C0L5 (CO4B_HUMAN) | K.LVNGQSHISLSK.A | 0.73 | 0.73 |
| complement C4-B-like preproprotein | P0C0L5 (CO4B_HUMAN) | R.GQIVFMNREPK.R | 0.80 | 0.62 |
| complement C4-B-like preproprotein | P0C0L5 (CO4B_HUMAN) | K.VGLSGM*AIADVTLLSGF HALR.A | 0.80 | 0.80 |
| complement C4-B-like preproprotein | P0C0L5 (CO4B_HUMAN) | K.VGLSGMAIADVTLLSGF | 0.80 | 0.83 |
| | | HALR.A | | |
| complement C4-B-like preproprotein | P0C0L5 (CO4B_HUMAN) | R.GHLFLQTDQPIYNPGQR. V | 0.70 | 0.68 |
| complement C4-B-like preproprotein | P0C0L5 (CO4B_HUMAN) | K.M*RPSTDTITVMVENSH GLR.V | 0.75 | 0.65 |
| complement C4-B-like preproprotein | P0C0L5 (CO4B_HUMAN) | K.MRPSTDTITVMVENSHG LR.V | 0.75 | 0.72 |
| complement C4-B-like preproprotein | P0C0L5 (CO4B_HUMAN) | K.SHALQLNNR.Q | 0.76 | 0.70 |
| complement C4-B-like preproprotein | P0C0L5 (CO4B_HUMAN) | R.YVSHFETEGPHVLLYFDS VPTSR.E | 0.88 | 0.89 |
| complement C4-B-like preproprotein | P0C0L5 (CO4B_HUMAN) | R.GSSTWLTAFVLK.V | 0.61 | 0.72 |
| complement C4-B-like preproprotein | P0C0L5 (CO4B_HUMAN) | R.YIYGKPVQGVAYVR.F | 0.63 | 0.73 |
| complement C4-B-like preproprotein | P0C0L5 (CO4B_HUMAN) | K.SCGLHQLLR.G | 0.65 | 0.65 |
| complement C4-B-like preproprotein | P0C0L5 (CO4B_HUMAN) | R.GPEVQLVAHSPWLK.D | 0.69 | 0.73 |
| complement C4-B-like preproprotein | P0C0L5 (CO4B_HUMAN) | R.KKEVYM*PSSIFQDDFVI PDISEPGTWK.I | 0.70 | 0.67 |
| complement C4-B-like preproprotein | P0C0L5 (CO4B_HUMAN) | R.KKEVYMPSSIFQDDFVIP DISEPGTWK.I | 0.70 | 0.69 |
| complement C4-B-like preproprotein | P0C0L5 (CO4B_HUMAN) | R.VQQPDCREPFLSCCQFAE SLR.K | 0.76 | 0.74 |
| complement C4-B-like preproprotein | P0C0L5 (CO4B_HUMAN) | K.VGLSGM*AIADVTLLSGF HALR.A | 0.80 | 0.80 |
| complement C4-B-like preproprotein | P0C0L5 (CO4B_HUMAN) | K.VGLSGMAIADVTLLSGF HALR.A | 0.80 | 0.83 |
| complement C4-B-like preproprotein | P0C0L5 (CO4B_HUMAN) | K.ASAGLLGAHAAAITAYA LTLTK.A | 0.85 | 0.83 |
| complement C5 preproprotein | P01031 (CO5_HUMAN) | K.ITHYNYLILSK.G | 0.73 | 0.73 |
| complement C5 preproprotein | P01031 (CO5_HUMAN) | R.KAFDICPLVK.I | 0.83 | 0.87 |
| complement C5 preproprotein | P01031 (CO5_HUMAN) | R.IPLDLVPK.T | 0.90 | 0.63 |
| complement C5 preproprotein | P01031 (CO5_HUMAN) | R.MVETTAYALLTSLNLKD INYVNPVIK.W | 0.92 | 0.75 |
| complement C5 preproprotein | P01031 (CO5_HUMAN) | K.ALLVGEHLNIIVTPK.S | 1.00 | 0.87 |
| complement C5 preproprotein | P01031 (CO5_HUMAN) | K.LKEGMLSIMSYR.N | 0.62 | 0.75 |
| complement C5 preproprotein | P01031 (CO5_HUMAN) | R.YIYPLDSLTWIEYWPR.D | 0.70 | 0.69 |
| complement C5 preproprotein | P01031 (CO5_HUMAN) | K.GGSASTWLTAFALR.V | 0.63 | 0.83 |
| complement C5 preproprotein | P01031 (CO5_HUMAN) | R.YGGGFYSTQDTINAIEGL TEYSLLVK.Q | 0.73 | 0.74 |
| complement component C6 | P13671 (CO6_HUMAN) | K.AKDLHLSDVFLK.A | 0.63 | 0.62 |
| complement component C6 | P13671 (CO6_HUMAN) | K.ALNHLPLEYNSALYSR.I | 0.60 | 0.62 |
| complement component C7 | P10643 (CO7_HUMAN) | R.LSGNVLSYTFQVK.I | 0.71 | 0.63 |
| complement component C8 alpha chain | P07357 (CO8A_HUMAN) | R.KDDIMLDEGMLQSLMEL PDQYNYGMYAK.F | 0.78 | 0.89 |
| complement component C8 beta chain preproprotein | P07358 (CO8B_HUMAN) | R.DFGTHYITEAVLGGIYEY TLVMNK.E | 0.80 | 0.73 |
| complement component C8 beta chain preproprotein | P07358 (CO8B_HUMAN) | R.DTMVEDLVVLVR.G | 0.88 | 0.76 |
| complement component C8 beta chain preproprotein | P07358 (CO8B_HUMAN) | R.YYAGGCSPHYILNTR.F | 0.70 | 0.71 |
| complement component C8 gamma chain | P07360 (CO8G_HUMAN) | R.SLPVSDSVLSGFEQR.V | 0.79 | 0.81 |
| complement component C8 gamma chain | P07360 (CO8G_HUMAN) | R.VQEAHLTEDQIFYFPK.Y | 0.98 | 0.84 |
| complement component C9 | P02748 (CO9_HUMAN) | R.TAGYGINILGMDPLSTPF DNEFYNGLCNR.D | 0.62 | 0.64 |
| complement component C9 | P02748 (CO9_HUMAN) | R.RPWNVASLIYETK.G | 0.60 | 0.74 |
| complement component C9 | P02748 (CO9_HUMAN) | R.AIEDYINEFSVRK.C | 0.67 | 0.67 |
| complement component C9 | P02748 (CO9_HUMAN) | R.AIEDYINEFSVR.K | 0.77 | 0.79 |
| complement factor B preproprotein | P00751 (CFAB_HUMAN) | R.LEDSVTYHCSR.G | 0.60 | 0.60 |
| complement factor B preproprotein | P00751 (CFAB_HUMAN) | R.FIQVGVISWGVVDVCK.N | 0.67 | 0.79 |
| complement factor B preproprotein | P00751 (CFAB_HUMAN) | R.DFHINLFQVLPWLK.E | 0.78 | 0.76 |
| complement factor B preproprotein | P00751 (CFAB_HUMAN) | K.YGQTIRPICLPCTEGTTR. A | 0.60 | 0.70 |
| complement factor B preproprotein | P00751 (CFAB_HUMAN) | R.LLQEGQALEYVCPSGFY PYPVQTR.T | 0.74 | 0.74 |
| complement factor H | P08603 (CFAH_HUMAN) | R.RPYFPVAVGK.Y | 0.67 | 0.70 |
| complement factor H | P08603 (CFAH_HUMAN) | K.CTSTGWIPAPR.C | 0.70 | 0.66 |
| complement factor H | P08603 (CFAH_HUMAN) | K.CLHPCVISR.E | 0.94 | 0.64 |
| complement factor H | P08603 (CFAH_HUMAN) | R.EIMENYNIALR.W | 0.67 | 0.71 |
| complement factor H | P08603 (CFAH_HUMAN) | K.CLHPCVISR.E | 0.75 | 0.64 |
| complement factor H | P08603 (CFAH_HUMAN) | K.AVYTCNEGYQLLGEINY R.E | 0.73 | 0.62 |
| complement factor H | P08603 (CFAH_HUMAN) | R.SITCIHGVWTQLPQCVAI DK.L | 0.61 | 0.61 |
| complement factor H | P08603 (CFAH_HUMAN) | R.WQSIPLCVEK.I | 0.65 | 0.65 |
| complement factor H | P08603 (CFAH_HUMAN) | K.TDCLSLPSFENAIPMGEK. K | 0.74 | 0.77 |
| complement factor H | P08603 (CFAH_HUMAN) | K.CFEGFGIDGPAIAK.C | 0.76 | 0.69 |
| complement factor H | P08603 (CFAH_HUMAN) | K.CFEGFGIDGPAIAK.C | 0.83 | 0.69 |
| complement factor H | P08603 (CFAH_HUMAN) | K.IDVHLVPDR.K | 0.61 | 0.67 |
| complement factor H | P08603 (CFAH_HUMAN) | K.SSNLIILEEHLK.N | 0.77 | 0.69 |
| complement factor I preproprotein | P05156 (CFAI_HUMAN) | R.AQLGDLPWQVAIK.D | 0.66 | 0.69 |
| complement factor I preproprotein | P05156 (CFAI_HUMAN) | R.VFSLQWGEVK.L | 0.69 | 0.77 |
| corticosteroid-binding globulin | P08185 (CBG_HUMAN) | R.WSAGLTSSQVDLYIPK.V | 0.63 | 0.61 |
| fibrinogen alpha chain | P02671 (FIBA_HUMAN) | K.TFPGFFSPMLGEFVSETE SR.G | 0.80 | 0.78 |
| gelsolin | P06396 (GELS_HUMAN) | R.IEGSNKVPVDPATYGQF YGGDSYIILYNYR.H | 0.78 | 0.78 |
| gelsolin | P06396 (GELS_HUMAN) | R.AQPVQVAEGSEPDGFWE ALGGK.A | 0.62 | 0.65 |
| gelsolin | P06396 (GELS_HUMAN) | K.TPSAAYLWVGTGASEAE KTGAQELLR.V | 0.78 | 0.78 |
| gelsolin | P06396 (GELS_HUMAN) | R.VEKFDLVPVPTNLYGDF FTGDAYVILK.T | 0.61 | 0.63 |
| gelsolin | P06396 (GELS_HUMAN) | R.EVQGFESATFLGYFK.S | 0.87 | 0.88 |
| gelsolin | P06396 (GELS_HUMAN) | K.NWRDPDQTDGLGLSYLS SHIANVER.V | 0.89 | 0.89 |
| gelsolin | P06396 (GELS_HUMAN) | K.TPSAAYLWVGTGASEAE K.T | 0.87 | 0.77 |
| glutathione peroxidase 3 | P22352 (GPX3_HUMAN) | K.FLVGPDGIPIMR.W | 0.85 | 0.77 |
| hemopexin | P02790 (HEMO_HUMAN) | R.LEKEVGTPHGIILDSVDA AFICPGSSR.L | 0.93 | 0.74 |
| hemopexin | P02790 (HEMO_HUMAN) | R.WKNFPSPVDAAFR.Q | 0.64 | 0.82 |
| hemopexin | P02790 (HEMO_HUMAN) | R.GECQAEGVLFFQGDREW FWDLATGTMK.E | 0.60 | 0.64 |
| hemopexin | P02790 (HEMO_HUMAN) | R.GECQAEGVLFFQGDREW FWDLATGTM*K.E | 0.60 | 0.83 |
| hemopexin | P02790 (HEMO_HUMAN) | R.GECQAEGVLFFQGDREW FWDLATGTMK.E | 0.93 | 0.64 |
| hemopexin | P02790 (HEMO_HUMAN) | R.GECQAEGVLFFQGDREW FWDLATGTM*K.E | 0.93 | 0.83 |
| hemopexin | P02790 (HEMO_HUMAN) | K.EVGTPHGIILDSVDAAFI CPGSSR.L | 0.62 | 0.69 |
| hemopexin | P02790 (HEMO_HUMAN) | R.LWWLDLK.S | 0.64 | 0.64 |
| hemopexin | P02790 (HEMO_HUMAN) | K.NFPSPVDAAFR.Q | 0.65 | 0.72 |
| hemopexin | P02790 (HEMO_HUMAN) | R.EWFWDLATGTMK.E | 0.68 | 0.65 |
| hemopexin | P02790 | K.GGYTLVSGYPK.R | 0.69 | 0.65 |
| | (HEMO_HUMAN) | | | |
| hemopexin | P02790 (HEMO_HUMAN) | K.LYLVQGTQVYVFLTK.G | 0.69 | 0.76 |
| heparin cofactor 2 | P05546 (HEP2_HUMAN) | R.EYYFAEAQIADFSDPAFI SK.T | 0.80 | 0.78 |
| heparin cofactor 2 | P05546 (HEP2_HUMAN) | K.QFPILLDFK.T | 0.62 | 1.00 |
| heparin cofactor 2 | P05546 (HEP2_HUMAN) | K.QFPILLDFK.T | 0.64 | 1.00 |
| heparin cofactor 2 | P05546 (HEP2_HUMAN) | K.FAFNLYR.V | 0.70 | 0.60 |
| histidine-rich glycoprotein | P04196 (HRG_HUMAN) | R.DGYLFQLLR.I | 0.65 | 0.65 |
| insulin-like growth factor-binding protein complex acid labile subunit | P35858 (ALS_HUMAN) | R.SFEGLGQLEVLTLDHNQ LQEVK.A | 0.75 | 0.83 |
| insulin-like growth factor-binding protein complex acid labile subunit | P35858 (ALS_HUMAN) | R.TFTPQPPGLER.L | 0.75 | 0.60 |
| insulin-like growth factor-binding protein complex acid labile subunit | P35858 (ALS_HUMAN) | R.AFWLDVSHNR.L | 0.77 | 0.75 |
| insulin-like growth factor-binding protein complex acid labile subunit | P35858 (ALS_HUMAN) | R.LAELPADALGPLQR.A | 0.66 | 0.64 |
| insulin-like growth factor-binding protein complex acid labile subunit | P35858 (ALS_HUMAN) | R.LEALPNSLLAPLGR.L | 0.70 | 0.67 |
| insulin-like growth factor-binding protein complex acid labile subunit | P35858 (ALS_HUMAN) | R.NLIAAVAPGAFLGLK.A | 0.70 | 0.68 |
| inter-alpha-trypsin inhibitor heavy chain H1 | P19827 (ITIH1_HUMAN) | R.QAVDTAVDGVFIR.S | 0.60 | 0.64 |
| inter-alpha-trypsin inhibitor heavy chain H1 | P19827 (ITIH1_HUMAN) | K.TAFISDFAVTADGNAFIG | 0.81 | 0.86 |
| | | DIK.D | | |
| inter-alpha-trypsin inhibitor heavy chain H1 | P19827 (ITIH1_HUMAN) | R.GHMLENHVER.L | 0.63 | 0.61 |
| inter-alpha-trypsin inhibitor heavy chain H1 | P19827 (ITIH1_HUMAN) | R.GHM*LENHVER.L | 0.63 | 0.70 |
| inter-alpha-trypsin inhibitor heavy chain H1 | P19827 (ITIH1_HUMAN) | K.TAFISDFAVTADGNAFIG DIKDKVTAWK.Q | 0.75 | 0.60 |
| inter-alpha-trypsin inhibitor heavy chain H1 | P19827 (ITIH1_HUMAN) | R.GIEILNQVQESLPELSNH ASILIMLTDGDPTEGVTDR. S | 0.80 | 0.80 |
| inter-alpha-trypsin inhibitor heavy chain H1 | P19827 (ITIH1_HUMAN) | K.ILGDM*QPGDYFDLVLF GTR.V | 0.85 | 0.79 |
| inter-alpha-trypsin inhibitor heavy chain H1 | P19827 (ITIH1_HUMAN) | K.LDAQASFLPK.E | 0.88 | 0.75 |
| inter-alpha-trypsin inhibitor heavy chain H1 | P19827 (ITIH1_HUMAN) | R.GFSLDEATNLNGGLLR.G | 0.80 | 0.80 |
| inter-alpha-trypsin inhibitor heavy chain H1 | P19827 (ITIH1_HUMAN) | K.TAFISDFAVTADGNAFIG DIKDK.V | 0.93 | 0.96 |
| inter-alpha-trypsin inhibitor heavy chain H1 | P19827 (ITIH1_HUMAN) | K.GSLVQASEANLQAAQDF VR.G | 0.60 | 0.65 |
| inter-alpha-trypsin inhibitor heavy chain H1 | P19827 (ITIH1_HUMAN) | R.GHMLENHVER.L | 0.64 | 0.61 |
| inter-alpha-trypsin inhibitor heavy chain H1 | P19827 (ITIH1_HUMAN) | R.GHM*LENHVER.L | 0.64 | 0.70 |
| inter-alpha-trypsin inhibitor heavy chain H1 | P19827 (ITIH1_HUMAN) | R.LWAYLTIQELLAK.R | 0.72 | 0.74 |
| inter-alpha-trypsin inhibitor heavy chain H1 | P19827 (ITIH1_HUMAN) | R.EVAFDLEIPK.T | 0.78 | 0.62 |
| inter-alpha-trypsin inhibitor heavy chain H2 | P19823 (ITIH2_HUMAN) | R.SILQMSLDHHIVTPLTSL VIENEAGDER.M | 0.76 | 0.76 |
| inter-alpha-trypsin inhibitor heavy chain H2 | P19823 (ITIH2_HUMAN) | R.SILQM*SLDHHIVTPLTSL VIENEAGDER.M | 0.76 | 0.80 |
| inter-alpha-trypsin inhibitor heavy chain H2 | P19823 (ITIH2_HUMAN) | R.SILQMSLDHHIVTPLTSL VIENEAGDER.M | 0.77 | 0.76 |
| inter-alpha-trypsin inhibitor heavy chain H2 | P19823 (ITIH2_HUMAN) | R.SILQM*SLDHHIVTPLTSL VIENEAGDER.M | 0.77 | 0.80 |
| inter-alpha-trypsin inhibitor heavy chain H2 | P19823 (ITIH2_HUMAN) | K.AGELEVFNGYFVHFFAP DNLDPIPK.N | 0.79 | 0.76 |
| inter-alpha-trypsin inhibitor heavy chain H2 | P19823 (ITIH2_HUMAN) | R.ETAVDGELVVLYDVK.R | 0.94 | 0.97 |
| inter-alpha-trypsin inhibitor heavy chain H2 | P19823 (ITIH2_HUMAN) | R.NVQFNYPHTSVTDVTQN NFHNYFGGSEIVVAGK.F | 0.74 | 0.83 |
| inter-alpha-trypsin inhibitor heavy chain H2 | P19823 (ITIH2_HUMAN) | R.FLHVPDTFEGHFDGVPVI SK.G | 0.81 | 0.81 |
| inter-alpha-trypsin inhibitor heavy chain H4 | Q14624 (ITIH4_HUMAN) | K.YIFHNFM*ER.L | 0.70 | 0.73 |
| inter-alpha-trypsin inhibitor heavy chain H4 | Q14624 (ITIH4_HUMAN) | R.SFAAGIQALGGTNINDA MLMAVQLLDSSNQEER.L | 0.75 | 0.75 |
| inter-alpha-trypsin inhibitor heavy chain H4 | Q14624 (ITIH4_HUMAN) | R.NMEQFQVSVSVAPNAK.I | 1.00 | 1.00 |
| inter-alpha-trypsin inhibitor heavy chain H4 | Q14624 (ITIH4_HUMAN) | R.VQGNDHSATR.E | 0.85 | 0.86 |
| inter-alpha-trypsin inhibitor heavy chain H4 | Q14624 (ITIH4_HUMAN) | K.WKETLFSVMPGLK.M | 0.66 | 0.69 |
| inter-alpha-trypsin inhibitor heavy chain H4 | Q14624 (ITIH4_HUMAN) | K.AGFSWIEVTFK.N | 0.78 | 0.82 |
| inter-alpha-trypsin inhibitor heavy chain H4 | Q14624 (ITIH4_HUMAN) | R.DQFNLIVFSTEATQWRPS LVPASAENVNK.A | 0.61 | 0.60 |
| inter-alpha-trypsin inhibitor heavy chain H4 | Q14624 (ITIH4_HUMAN) | R.LWAYLTIQQLLEQTVSA SDADQQALR.N | 0.66 | 0.66 |
| kallistatin | P29622 (KAIN_HUMAN) | K.FSISGSYVLDQILPR.L | 0.79 | 0.72 |
| kininogen-1 | P01042 (KNG1_HUMAN) | K.AATGECTATVGKR.S | 0.76 | 0.60 |
| kininogen-1 | P01042 (KNG1_HUMAN) | K.ENFLFLTPDCK.S | 0.71 | 0.68 |
| kininogen-1 | P01042 (KNG1_HUMAN) | R.DIPTNSPELEETLTHTITK. L | 0.65 | 0.64 |
| kininogen-1 | P01042 (KNG1_HUMAN) | K.IYPTVNCQPLGM*ISLMK .R | 0.66 | 0.60 |
| kininogen-1 | P01042 (KNG1_HUMAN) | K.IYPTVNCQPLGMISLMK. R | 0.66 | 0.62 |
| kininogen-1 | P01042 (KNG1_HUMAN) | K.IYPTVNCQPLGMISLM*K .R | 0.66 | 0.63 |
| kininogen-1 | P01042 (KNG1_HUMAN) | R.IGEIKEETTSHLR.S | 0.67 | 0.70 |
| kininogen-1 | P01042 (KNG1_HUMAN) | K.YNSQNQSNNQFVLYR.I | 0.76 | 0.65 |
| kininogen-1 | P01042 (KNG1_HUMAN) | K.TVGSDTFYSFK.Y | 0.78 | 0.77 |
| leucine-rich alpha-2-glycoprotein | P02750 (A2GL_HUMAN) | R.DGFDISGNPWICDQNLSD LYR.W | 0.73 | 0.73 |
| leucine-rich alpha-2-glycoprotein | P02750 (A2GL_HUMAN) | R.NALTGLPPGLFQASATLD TLVLK.E | 0.79 | 0.79 |
| leucine-rich alpha-2-glycoprotein | P02750 (A2GL_HUMAN) | K.ALGHLDLSGNR.L | 0.71 | 0.71 |
| leucine-rich alpha-2-glvcoprotein | P02750 (A2GL_HUMAN) | R.VAAGAFQGLR.Q | 0.71 | 0.77 |
| lipopolysacchari de-binding protein | P18428 (LBP_HUMAN) | R.SPVTLLAAVMSLPEEHN K.M | 0.65 | 0.61 |
| lumican | P51884 (LUM_HUMAN) | K.SLEYLDLSFNQIAR.L | 0.93 | 0.96 |
| monocyte differentiation antigen CD14 | P08571 (CD14_HUMAN) | R.LTVGAAQVPAQLLVGAL R.V | 0.68 | 0.63 |
| N-acetylmuramoyl-L-alanine amidase | Q96PD5 (PGRP2_HUMAN) | R.EGKEYGVVLAPDGSTVA VEPLLAGLEAGLQGR.R | 0.64 | 0.64 |
| N-acetylmuramoyl-L-alanine amidase | Q96PD5 (PGRP2_HUMAN) | K.EFTEAFLGCPAIHPR.C | 0.63 | 0.62 |
| N-acetylmuramoyl-L-alanine amidase | Q96PD5 (PGRP2_HUMAN) | R.TDCPGDALFDLLR.T | 0.88 | 0.86 |
| phosphatidylinos itol-glycan-specific phospholipase D | P80108 (PHLD_HUMAN) | K.VAFLTVTLHQGGATR.M | 0.63 | 0.65 |
| pigment epithelium-derived factor | P36955 (PEDF_HUMAN) | R.ALYYDLISSPDIHGTYKE LLDTVTAPQK.N | 0.69 | 0.65 |
| pigment epithelium-derived factor | P36955 (PEDF_HUMAN) | K.TVQAVLTVPK.L | 0.72 | 0.62 |
| pigment epithelium-derived factor | P36955 (PEDF_HUMAN) | R.LDLQEINNWVQAQMK.G | 0.67 | 0.68 |
| plasma kallikrein preproprotein | P03952 (KLKB1_HUMAN) | R.L VGITSWGEGCAR.R | 1.00 | 0.67 |
| plasma protease C1 inhibitor | P05155 (IC1_HUMAN) | K.TNLESILSYPKDFTCVHQ ALK.G | 0.83 | 0.83 |
| plasma protease C1 inhibitor | P05155 (IC1_HUMAN) | R.LVLLNAIYLSAK.W | 0.64 | 0.61 |
| plasma protease C1 inhibitor | P05155 (IC1_HUMAN) | K.FQPTLLTLPR.I | 0.86 | 0.77 |
| plasminogen | P00747 (PLMN_HUMAN) | R.HSIFTPETNPR.A | 0.66 | 0.64 |
| plasminogen | P00747 (PLMN_HUMAN) | R.FVTWIEGVMR.N | 0.65 | 0.74 |
| PREDICTED: complement C4-A | POCOL4 (CO4A_HUMAN) | R.GQIVFMNR.E | 0.75 | 0.61 |
| PREDICTED: complement C4-A | POCOL4 (CO4A_HUMAN) | R.DSSTWLTAFVLK.V | 0.65 | 0.67 |
| PREDICTED: complement C4-A | POCOL4 (CO4A_HUMAN) | R.YLDKTEQWSTLPPETK.D | 0.70 | 0.60 |
| PREDICTED: complement C4-A | POCOL4 (CO4A_HUMAN) | R.DFALLSLQVPLK.D | 0.78 | 0.62 |
| PREDICTED: complement C4-A | POCOL4 (CO4A_HUMAN) | R.TLEIPGNSDPNMIPDGDF NSYVR.V | 0.74 | 0.78 |
| PREDICTED: complement C4-A | POCOL4 (CO4A_HUMAN) | R.EMSGSPASGIPVK.V | 0.88 | 0.88 |
| PREDICTED: complement C4-A | POCOL4 (CO4A_HUMAN) | K.LHLETDSLALVALGALD TALYAAGSK.S | 0.68 | 0.64 |
| PREDICTED: complement C4-A | POCOL4 (CO4A_HUMAN) | R.GCGEQTMIYLAPTLAAS R.Y | 0.71 | 0.67 |
| pregnancy zone protein | P20742 (PZP_HUMAN) | R.NELIPLIYLENPR.R | 1.00 | 0.67 |
| pregnancy zone protein | P20742 (PZP_HUMAN) | K.LEAGINQLSFPLSSEPIQG SYR.V | 1.00 | 0.73 |
| pregnancy zone protein | P20742 (PZP_HUMAN) | R.NQGNTWLTAFVLK.T | 0.73 | 0.78 |
| pregnancy zone protein | P20742 (PZP_HUMAN) | R.AFQPFFVELTMPYSVIR.G | 0.83 | 0.88 |
| pregnancy zone protein | P20742 (PZP_HUMAN) | R.IQHPFTVEEFVLPK.F | 0.65 | 0.79 |
| pregnancy zone protein | P20742 (PZP_HUMAN) | K.ALLAYAFSLLGK.Q | 0.69 | 0.74 |
| pregnancy-specific beta-1-glycoprotein 1 /8/4 | P11464 (PSG1_HUMAN)/Q9UQ74 (PSG8_HUMAN)/Q00888 (PSG4_HUMAN) | R.TLFLLGVTK.Y | 0.74 | 0.83 |
| protein AMBP preproprotein | P02760 (AMBP_HUMAN) | R.TVAACNLPIVR.G | 0.78 | 0.77 |
| protein AMBP preproprotein | P02760 (AMBP_HUMAN) | K.WYNLAIGSTCPWLK.K | 0.80 | 0.80 |
| protein Z-dependent protease inhibitor | Q9UK55 (ZPI_HUMAN) | K.LILVDYILFK.G | 0.69 | 0.62 |
| prothrombin preproprotein | P00734 (THRB_HUMAN) | R.KSPQELLCGASLISDR.W | 0.63 | 0.65 |
| prothrombin preproprotein | P00734 (THRB_HUMAN) | R.TATSEYQTFFNPR.T | 0.79 | 0.61 |
| prothrombin preproprotein | P00734 (THRB_HUMAN) | R.VTGWGNLKETWTANVG K.G | 1.00 | 0.71 |
| prothrombin preproprotein | P00734 (THRB_HUMAN) | R.IVEGSDAEIGMSPWQVM LFR.K | 0.65 | 0.61 |
| prothrombin preproprotein | P00734 (THRB_HUMAN) | K.HQDFNSAVQLVENFCR. N | 0.65 | 0.64 |
| prothrombin preproprotein | P00734 (THRB_HUMAN) | R.IVEGSDAEIGM*SPWQV MLFR.K | 0.65 | 0.80 |
| prothrombin preproprotein | P00734 (THRB_HUMAN) | R.IVEGSDAEIGMSPWQVM *LFR.K | 0.65 | 1.00 |
| prothrombin preproprotein | P00734 (THRB_HUMAN) | R.RQECSIPVCGQDQVTVA MTPR.S | 0.74 | 0.73 |
| prothrombin preproprotein | P00734 (THRB_HUMAN) | R.LAVTTHGLPCLAWASAQ AK.A | 0.76 | 0.80 |
| prothrombin preproprotein | P00734 (THRB_HUMAN) | K.GQPSVLQVVNLPIVERPV | 0.76 | 0.67 |
| | | CK.D | | |
| retinol-binding protein 4 | P02753 (RET4_HUMAN) | R.LLNLDGTCADSYSFVFSR .D | 0.70 | 0.66 |
| sex hormone-binding globulin | P04278 (SHBG_HUMAN) | R.LFLGALPGEDSSTSFCLN GLWAQGQR.L | 0.72 | 0.72 |
| sex hormone-binding globulin | P04278 (SHBG_HUMAN) | R.TWDPEGVIFYGDTNPKD DWFMLGLR.D | 0.75 | 0.76 |
| sex hormone-binding globulin | P04278 (SHBG_HUMAN) | R.IALGGLLFPASNLR.L | 0.62 | 0.72 |
| sex hormone-binding globulin | P04278 (SHBG_HUMAN) | K.VVLSSGSGPGLDLPLVLG LPLQLK.L | 0.65 | 0.68 |
| thyroxine-binding globulin | P05543 (THBG_HUMAN) | K.AVLHIGEK.G | 0.64 | 0.75 |
| thyroxine-binding globulin | P05543 (THBG_HUMAN) | K.GWVDLFVPK.F | 0.60 | 0.61 |
| thyroxine-binding globulin | P05543 (THBG_HUMAN) | K.FSISATYDLGATLLK.M | 0.62 | 0.64 |
| thyroxine-binding globulin | P05543 (THBG_HUMAN) | R.SILFLGK.V | 0.66 | 0.63 |
| transforming growth factor-beta-induced protein ig-h3 | Q15582 (BGH3_HUMAN) | R.LTLLAPLNSVFK.D | 0.78 | 0.65 |
| vitamin D-binding protein | P02774 (VTDB_HUMAN) | K.EYANQFMWEYSTNYGQ APLSLLVSYTK.S | 0.67 | 0.64 |
| vitamin D-binding protein | P02774 (VTDB_HUMAN) | K.EYANQFM*WEYSTNYG QAPLSLLVSYTK.S | 0.67 | 0.67 |
| vitamin D-binding protein | P02774 (VTDB_HUMAN) | K.ELPEHTVK.L | 0.79 | 0.74 |
| vitamin D-binding protein | P02774 (VTDB_HUMAN) | R.RTHLPEVFLSK.V | 0.63 | 0.76 |
| vitamin D-binding protein | P02774 (VTDB_HUMAN) | K.TAMDVFVCTYFMPAAQ LPELPDVELPTNK.D | 0.66 | 0.63 |
| vitamin D-binding protein | P02774 (VTDB_HUMAN) | K.LPDATPTELAK.L | 0.67 | 0.73 |
| vitamin D-binding protein | P02774 (VTDB_HUMAN) | K.EYANQFMWEYSTNYGQ APLSLLVSYTK.S | 0.65 | 0.64 |
| vitamin D-binding protein | P02774 (VTDB_HUMAN) | K.EYANQFM*WEYSTNYG QAPLSLLVSYTK.S | 0.65 | 0.67 |
| vitamin D-binding protein | P02774 (VTDB_HUMAN) | K.ELSSFIDKGQELCADYSE NTFTEYKK.K | 0.71 | 0.73 |
| vitamin D-binding protein | P02774 (VTDB_HUMAN) | K.EDFTSLSLVLYSR.K | 0.71 | 0.75 |
| vitamin D-binding protein | P02774 (VTDB_HUMAN) | K.HQPQEFPTYVEPTNDEIC EAFRK.D | 0.77 | 0.75 |
| vitamin D-binding protein | P02774 (VTDB_HUMAN) | K.HQPQEFPTYVEPTNDEIC EAFR.K | 0.60 | 0.67 |
| vitamin D-binding protein | P02774 (VTDB_HUMAN) | R.KFPSGTFEQVSQLVK.E | 0.62 | 0.61 |
| vitamin D-binding protein | P02774 (VTDB_HUMAN) | K.ELSSFIDKGQELCADYSE NTFTEYK.K | 0.64 | 0.64 |
| vitamin D-binding protein | P02774 (VTDB_HUMAN) | K.EFSHLGKEDFTSLSLVLY SR.K | 0.66 | 0.64 |
| vitamin D-binding protein | P02774 (VTDB_HUMAN) | K.SYLSMVGSCCTSASPTV CFLK.E | 0.68 | 0.77 |
| vitronectin | P04004 (VTNC_HUMAN) | R.IYISGMAPRPSLAK.K | 0.63 | 0.66 |
| vitronectin | P04004 (VTNC_HUMAN) | R.IYISGMAPRPSLAK.K | 0.64 | 0.66 |
| vitronectin | P04004 (VTNC_HUMAN) | K.LIRDVWGIEGPIDAAFTR. I | 0.81 | 0.75 |
| von Willebrand factor preproprotein | P04275 (VWF_HUMAN) | R.IGWPNAPILIQDFETLPR. E | 0.67 | 0.67 |

| | | | | |
|---|---|---|---|---|
| * = Oxidation of Methionine | | | | |

**Table 9. Preeclampsia: Additional peptides significant with AUC > 0.6 by Sequest only**

| Protein description | Uniprot ID (name) | Peptide | S_AUC |
|---|---|---|---|
| afamin | P43652 (AFAM_HUMAN) | R.LCFFYNKK.S | 0.67 |
| afamin | P43652 (AFAM_HUMAN) | R.RPCFESLK.A | 0.81 |
| afamin | P43652 (AFAM_HUMAN) | R.IVQIYK.D | 0.61 |
| afamin | P43652 (AFAM_HUMAN) | R.FLVNLVK.L | 0.60 |
| afamin | P43652 (AFAM_HUMAN) | K.LPNNVLQEK.I | 0.67 |
| alpha-1-antichymotrypsin | P01011 (AACT_HUMAN) | R.LYGSEAFATDFQDSAAAK K.L | 0.61 |
| alpha-1-antichymotrypsin | P01011 (AACT_HUMAN) | K.EQLSLLDRFTEDAKR.L | 0.71 |
| alpha-1-antichymotrypsin | P01011 (AACT_HUMAN) | R.EIGELYLPK.F | 0.68 |
| alpha-1-antichymotrypsin | P01011 (AACT_HUMAN) | R.WRDSLEFR.E | 0.71 |
| alpha-1-antichymotrypsin | P01011 (AACT_HUMAN) | K.RLYGSEAFATDFQDSAAA K.K | 0.89 |
| alpha-1B-glycoprotein | P04217 (A1BG_HUMAN) | R.FALVR.E | 1.00 |
| alpha-1B-glycoprotein | P04217 (A1BG_HUMAN) | R.GVTFLLRR.E | 0.67 |
| alpha-1B-glycoprotein | P04217 (A1BG_HUMAN) | R.RGEKELLVPR.S | 0.71 |
| alpha-1B-glycoprotein | P04217 (A1BG_HUMAN) | K.ELLVPR.S | 0.61 |
| alpha-1B-glycoprotein | P04217 (A1BG_HUMAN) | K.NGVAQEPVHLDSPAIK.H | 0.64 |
| alpha-2-antiplasmin | P08697 (A2AP_HUMAN) | R.NKFDPSLTQR.D | 0.60 |
| alpha-2-antiplasmin | P08697 (A2AP_HUMAN) | R.QLTSGPNQEQVSPLTLLK. L | 0.67 |
| alpha-2-antiplasmin | P08697 (A2AP_HUMAN) | K.HQM*DLVATLSQLGLQEL FQAPDLR.G | 0.67 |
| angiotensinogen preproprotein | P01019 (ANGT_HUMAN) | R.FM*QAVTGWK.T | 0.60 |
| angiotensinogen preproprotein | P01019 (ANGT_HUMAN) | K.PKDPTFIPAPIQAK.T | 0.83 |
| angiotensinogen preproprotein | P01019 (ANGT_HUMAN) | R.SLDFTELDVAAEK.I | 0.60 |
| ankyrin repeat and protein kinase domain-containing protein 1 | Q8NFD2 (ANKK1_HUMAN) | R.KNLVPR.D | 1.00 |
| antithrombin-III | P01008 (ANT3_HUMAN) | R.RVWELSK.A | 0.68 |
| apolipoprotein A-IV | P06727 (APOA4_HUMAN) | K.VKIDQTVEELRR.S | 0.62 |
| apolipoprotein A-IV | P06727 (APOA4_HUMAN) | K.DLRDKVNSFFSTFK.E | 0.92 |
| apolipoprotein A-IV | P06727 (APOA4_HUMAN) | K.LVPFATELHER.L | 0.71 |
| apolipoprotein A-IV | P06727 (APOA4_HUMAN) | R.RVEPYGENFNK.A | 0.86 |
| apolipoprotein A-IV | P06727 (APOA4_HUMAN) | K.VNSFFSTFK.E | 0.87 |
| apolipoprotein B-100 | P04114 (APOB_HUMAN) | K.AVSM*PSFSILGSDVR.V | 0.70 |
| apolipoprotein B-100 | P04114 (APOB_HUMAN) | K.AVSMPSFSILGSDVR.V | 0.66 |
| apolipoprotein B-100 | P04114 (APOB_HUMAN) | K.AVSMPSFSILGSDVR.V | 0.66 |
| apolipoprotein B-100 | P04114 (APOB_HUMAN) | K.AVSM*PSFSILGSDVR.V | 0.70 |
| apolipoprotein B-100 | P04114 (APOB_HUMAN) | K.VNWEEEAASGLLTSLKD NVPK.A | 0.60 |
| apolipoprotein B-100 | P04114 (APOB_HUMAN) | R.DLKVEDIPLAR.I | 0.70 |
| apolipoprotein C-I | P02654 (APOC1_HUMAN) | K.MREWFSETFQK.V | 0.73 |
| apolipoprotein C-II | P02655 (APOC2_HUMAN) | K.STAAMSTYTGIFTDQVLS VLKGEE.- | 0.68 |
| apolipoprotein E | P02649 (APOE_HUMAN) | R.AKLEEQAQQIR.L | 0.67 |
| apolipoprotein E | P02649 (APOE_HUMAN) | R.FWDYLR.W | 0.67 |
| apolipoprotein E | P02649 (APOE_HUMAN) | R.LKSWFEPLVEDMQR.Q | 0.65 |
| beta-2-glycoprotein 1 | P02749 (APOH_HUMAN) | K.VSFFCK.N | 0.67 |
| beta-2-glycoprotein 1 | P02749 (APOH_HUMAN) | R.VCPFAGILENGAVR.Y | 0.63 |
| beta-2-microglobulin | P61769 (B2MG_HUMAN) | K.SNFLNCYVSGFHPSDIEVD LLK.N | 0.60 |
| biotinidase | P43251 (BTD_HUMAN) | R.LSSGLVTAALYGR.L | 1.00 |
| carboxypeptidase B2 preproprotein | Q96IY4 (CBPB2_HUMAN) | K.IAWHVIR.N | 0.90 |
| carboxypeptidase N subunit 2 | P22792 (CPN2_HUMAN) | K.LSNNALSGLPQGVFGK.L | 0.62 |
| carboxypeptidase N subunit 2 | P15169 (CBPN_HUMAN) | R.DHLGFQVTWPDESK.A | 0.93 |
| ceruloplasmin | P00450 (CERU_HUMAN) | K.VYVHLK.N | 0.67 |
| ceruloplasmin | P00450 (CERU_HUMAN) | K.LISVDTEHSNIYLQNGPDR .I | 0.62 |
| ceruloplasmin | P00450 (CERU_HUMAN) | K.M*YYSAVDPTKDIFTGLIG PM*K.I | 0.76 |
| ceruloplasmin | P00450 (CERU_HUMAN) | K.M*YYSAVDPTKDIFTGLIG PMK.I | 0.68 |
| ceruloplasmin | P00450 (CERU_HUMAN) | R.QKDVDKEFYLFPTVFDEN ESLLLEDNIR.M | 0.66 |
| ceruloplasmin | P00450 (CERU_HUMAN) | K.DVDKEFYLFPTVFDENES LLLEDNIR.M | 0.60 |
| ceruloplasmin | P00450 (CERU_HUMAN) | K.DIFTGLIGPMK.I | 0.62 |
| ceruloplasmin | P00450 (CERU_HUMAN) | R.SVPPSASHVAPTETFTYE WTVPK.E | 0.66 |
| ceruloplasmin | P00450 (CERU_HUMAN) | R.GVYSSDVFDIFPGTYQTLE M*FPR.T | 0.67 |
| ceruloplasmin | P00450 (CERU_HUMAN) | K.DIFTGLIGPMK.I | 0.62 |
| ceruloplasmin | P00450 (CERU_HUMAN) | K.VNKDDEEFIESNK.M | 0.78 |
| clusterin preproprotein | P10909 (CLUS_HUMAN) | R.KYNELLK.S | 0.75 |
| coagulation factor XII | P00748 (FA12_HUMAN) | R.TTLSGAPCQPWASEATYR .N | 0.64 |
| complement C1q subcomponent subunit A | P02745 (C1QA_HUMAN) | K.GHIYQGSEADSVFSGFLIF PSA.- | 0.64 |
| complement C1q subcomponent subunit C | P02747 (C1QC_HUMAN) | K.FQSVFTVTR.Q | 0.65 |
| complement C1r subcomponent | P00736 (C1R_HUMAN) | R.WILTAAHTLYPK.E | 0.68 |
| complement C1r subcomponent | P00736 (C1R_HUMAN) | K.VLNYVDWIKK.E | 0.81 |
| complement C1s subcomponent | P09871 (C1S_HUMAN) | R.LPVAPLRK.C | 0.63 |
| complement C2 | P06681 (CO2_HUMAN) | R.PICLPCTMEANLALR.R | 0.78 |
| complement C2 | P06681 (CO2_HUMAN) | R.QHLGDVLNFLPL.- | 0.70 |
| complement C4-B-like preproprotein | P0C0L5 (CO4B_HUMAN) | K.LGQYASPTAKR.C | 0.89 |
| complement C4-B-like preproprotein | P0C0L5 (CO4B_HUMAN) | K.M*RPSTDTITVMVENSHG LR.V | 0.65 |
| complement C4-B-like preproprotein | P0C0L5 (CO4B_HUMAN) | K.MRPSTDTITVMVENSHGL R.V | 0.72 |
| complement C5 preproprotein | P01031 (CO5_HUMAN) | K.EFPYRIPLDLVPK.T | 0.67 |
| complement C5 preproprotein | P01031 (CO5_HUMAN) | R.VFQFLEK.S | 0.60 |
| complement C5 preproprotein | P01031 (CO5_HUMAN) | R.MVETTAYALLTSLNLK.D | 0.61 |
| complement C5 preproprotein | P01031 (CO5_HUMAN) | R.ENSLYLTAFTVIGIR.K | 0.81 |
| complement component C8 alpha chain | P07357 (CO8A_HUMAN) | K.YNPVVIDFEMQPIHEVLR. H | 0.62 |
| complement component C8 beta chain preproprotein | P07358 (CO8B_HUMAN) | K.IPGIFELGISSQSDR.G | 0.61 |
| complement component C8 gamma chain | P07360 (CO8G_HUMAN) | R.RPASPISTIQPK.A | 0.71 |
| complement component C8 gamma chain | P07360 (CO8G_HUMAN) | R.FLQEQGHR.A | 0.87 |
| complement factor B preproprotein | P00751 (CFAB_HUMAN) | K.VSVGGEKR.D | 0.60 |
| complement factor B preproprotein | P00751 (CFAB_HUMAN) | K.CLVNLIEK.V | 0.69 |
| complement factor B preproprotein | P00751 (CFAB_HUMAN) | K.KDNEQHVFK.V | 0.68 |
| complement factor B preproprotein | P00751 (CFAB_HUMAN) | K.ISVIRPSK.G | 0.63 |
| complement factor B preproprotein | P00751 (CFAB_HUMAN) | K.KCLVNLIEK.V | 0.63 |
| complement factor B preproprotein | P00751 (CFAB_HUMAN) | R.LPPTTTCQQQKEELLPAQ DIK.A | 0.64 |
| complement factor B preproprotein | P00751 (CFAB_HUMAN) | K.LQDEDLGFL.- | 0.66 |
| complement factor H | P08603 (CFAH_HUMAN) | K.SCDIPVFMNAR.T | 0.60 |
| complement factor H | P08603 (CFAH_HUMAN) | K.HGGLYHENMR.R | 0.75 |
| complement factor H | P08603 (CFAH_HUMAN) | K.IIYKENER.F | 0.69 |
| complement factor I preproprotein | P05156 (CFAI_HUMAN) | K.RAQLGDLPWQVAIK.D | 0.68 |
| conserved oligomeric Golgi complex subunit 6 isoform | Q9Y2V7 (COG6_HUMAN) | K.ISNLLK.F | 0.71 |
| comulin | Q9UBG3 (CRNN_HUMAN) | R.RYARTEGNCTALTR.G | 0.81 |
| FERM domain-containing protein 8 | Q9BZ67 (FRMD8_HUMAN) | R.VQLGPYQPGRPAACDLR. E | 0.63 |
| gelsolin | P06396 (GELS_HUMAN) | R.VPEARPNSMVVEHPEFLK. A | 0.61 |
| gelsolin | P06396 (GELS_HUMAN) | K.AGKEPGLQIWR.V | 0.70 |
| glucose-induced degradation protein 8 homolog | Q9NWU2 (GID8_HUMAN) | K.VWSEVNQAVLDYENRES TPK.L | 0.83 |
| hemK methyltransferase family member 1 | Q9Y5R4 (HEMK1_HUMAN) | R.M*LWALLSGPGRRGSTR. G | 0.61 |
| hemopexin | P02790 (HEMO_HUMAN) | R.ELISER.W | 0.82 |
| hemopexin | P02790 (HEMO_HUMAN) | R.DVRDYFM*PCPGR.G | 0.70 |
| hemopexin | P02790 (HEMO_HUMAN) | K.GDKVWVYPPEKK.E | 0.71 |
| hemopexin | P02790 (HEMO_HUMAN) | R.DVRDYFMPCPGR.G | 0.60 |
| hemopexin | P02790 (HEMO_HUMAN) | R.EWFWDLATGTMK.E | 0.65 |
| hemopexin | P02790 (HEMO_HUMAN) | R.YYCFQGNQFLR.F | 0.68 |
| hemopexin | P02790 (HEMO_HUMAN) | R.RLWWLDLK.S | 0.65 |
| heparin cofactor 2 | P05546 (HEP2_HUMAN) | R.LNILNAK.F | 0.75 |
| heparin cofactor 2 | P05546 (HEP2_HUMAN) | R.NFGYTLR.S | 0.66 |
| histone deacetylase complex subunit SAP25 | Q8TEE9 (SAP25_HUMAN) | K.LLPPPPIM*SARVLPR.P | 0.63 |
| hyaluronan-binding protein 2 | Q14520 (HABP2_HUMAN) | K.RPGVYTQVTK.F | 0.68 |
| hyaluronan-binding protein 2 | Q14520 (HABP2_HUMAN) | K.FLNWIK.A | 0.62 |
| immediate early response gene 5-like protein | Q5T953 (IER5L_HUMAN) | - .MECALDAQSLISISLRKIHSS R.T | 0.93 |
| inactive caspase-12 | Q6UXS9 (CASPC_HUMAN) | K.AGADTHGRLLQGNICND AVTK.A | 0.60 |
| insulin-like growth factor-binding protein complex acid labile subunit | P35858 (ALS_HUMAN) | K.ANVFVQLPR.L | 0.62 |
| inter-alpha-trypsin inhibitor heavy | P19827 (ITIH1_HUMAN) | K.ELAAQTIKK.S | 0.71 |
| chain H1 | | | |
| inter-alpha-trypsin inhibitor heavy chain H1 | P19827 (ITIH1_HUMAN) | K.ILGDM*QPGDYFDLVLFG TR.V | 0.79 |
| inter-alpha-trypsin inhibitor heavy chain H1 | P19827 (ITIH1_HUMAN) | K.VTFQLTYEEVLKR.N | 0.70 |
| inter-alpha-trypsin inhibitor heavy chain H1 | P19827 (ITIH1_HUMAN) | R.TMEQFTIHLTVNPQSK.V | 0.61 |
| inter-alpha-trypsin inhibitor heavy chain H1 | P19827 (ITIH1_HUMAN) | R.FAHYVVTSQVVNTANEA R.E | 0.63 |
| inter-alpha-trypsin inhibitor heavy chain H2 | P19823 (ITIH2_HUMAN) | R.SSALDMENFRTEVNVLPG AK.V | 0.89 |
| inter-alpha-trypsin inhibitor heavy chain H2 | P19823 (ITIH2_HUMAN) | K.MKQTVEAMK.T | 0.93 |
| inter-alpha-trypsin inhibitor heavy chain H2 | P19823 (ITIH2_HUMAN) | R.IYLQPGR.L | 0.66 |
| inter-alpha-trypsin inhibitor heavy chain H2 | P19823 (ITIH2_HUMAN) | K.HLEVDVWVIEPQGLR.F | 0.61 |
| inter-alpha-trypsin inhibitor heavy chain H2 | P19823 (ITIH2_HUMAN) | K.FYNQVSTPLLR.N | 0.89 |
| inter-alpha-trypsin inhibitor heavy chain H2 | P19823 (ITIH2_HUMAN) | R.KLGSYEHR.I | 0.69 |
| inter-alpha-trypsin | Q14624 | K.GSEMVVAGK.L | 1.00 |
| inhibitor heavy chain H4 | (ITIH4_HUMAN) | | |
| inter-alpha-trypsin inhibitor heavy chain H4 | Q14624 (ITIH4_HUMAN) | R.MNFRPGVLSSR.Q | 0.72 |
| inter-alpha-trypsin inhibitor heavy chain H4 | Q14624 (ITIH4_HUMAN) | K.YIFHNFM*ER.L | 0.73 |
| inter-alpha-trypsin inhibitor heavy chain H4 | Q14624 (ITIH4_HUMAN) | K.ETLFSVMPGLK.M | 0.60 |
| inter-alpha-trypsin inhibitor heavy chain H4 | Q14624 (ITIH4_HUMAN) | R.FKPTLSQQQK.S | 0.64 |
| inter-alpha-trypsin inhibitor heavy chain H4 | Q14624 (ITIH4_HUMAN) | K.WKETLFSVMPGLK.M | 0.69 |
| inter-alpha-trypsin inhibitor heavy chain H4 | Q14624 (ITIH4_HUMAN) | R.RLGVYELLLK.V | 0.65 |
| inter-alpha-trypsin inhibitor heavy chain H4 | Q14624 (ITIH4_HUMAN) | R.DTDRFSSHVGGTLGQFYQ EVLWGSPAASDDGRR.T | 0.69 |
| inter-alpha-trypsin inhibitor heavy chain H4 | Q14624 (ITIH4_HUMAN) | K.VRPQQLVK.H | 0.62 |
| inter-alpha-trypsin inhibitor heavy chain H4 | Q14624 (ITIH4_HUMAN) | R.NVHSAGAAGSR.M | 0.69 |
| kallistatin | P29622 (KAIN_HUMAN) | R.LGFTDLFSK.W | 0.63 |
| kallistatin | P29622 | R.VGSALFLSHNLK.F | 0.62 |
| | (KAIN_HUMAN) | | |
| kininogen-1 | P01042 (KNG1_HUMAN) | R.VQVVAGKK.Y | 0.68 |
| leucine-rich alpha-2-glycoprotein | P02750 (A2GL_HUMAN) | R.LHLEGNKLQVLGK.D | 0.75 |
| lumican | P51884 (LUM_HUMAN) | R.FNALQYLR.L | 0.77 |
| m7GpppX diphosphatase | Q96C86 (DCPS_HUMAN) | R.IVFENPDPSDGFVLIPDLK. W | 0.94 |
| MAGUK p55 subfamily member 5 | Q8N3R9 (MPP5_HUMAN) | K.ILEIEDLFSSLK.H | 0.69 |
| MBT domain-containing protein 1 | Q05BQ5 (MBTD1_HUMAN) | K.WFDYLR.E | 0.63 |
| obscurin | Q5VST9 (OBSCN_HUMAN) | R.CELQIRGLAVEDTGEYLC VCGQERTSATLTVR.A | 0.73 |
| olfactory receptor 1L1 | Q8NH94 (OR1L1_HUMAN) | K.DMKQGLAKLM*HR.M | 0.89 |
| phosphatidylinositol -glycan-specific phospholipase D | P80108 (PHLD_HUMAN) | K.GIVAAFYSGPSLSDKEK.L | 0.79 |
| phosphatidylinositol -glycan-specific phospholipase D | P80108 (PHLD_HUMAN) | R.TLLLVGSPTWK.N | 0.65 |
| phosphatidylinositol -glycan-specific phospholipase D | P80108 (PHLD_HUMAN) | R.WYVPVKDLLGIYEK.L | 0.92 |
| pigment epithelium-derived factor | P36955 (PEDF_HUMAN) | R.SSTSPTTNVLLSPLSVATA LSALSLGAEQR.T | 0.63 |
| plasma protease C 1 inhibitor | P05155 (IC1_HUMAN) | K.GVTSVSQIFHSPDLAIR.D | 0.60 |
| PREDICTED: complement C4-A | P0C0L4 (CO4A_HUMAN) | R.DKGQAGLQR.A | 0.67 |
| PREDICTED: complement C4-A | P0C0L4 (CO4A_HUMAN) | K.SHKPLNMGK.V | 0.87 |
| PREDICTED: complement C4-A | P0C0L4 (CO4A_HUMAN) | R.KKEVYM*PSSIFQDDFVIP DISEPGTWK.I | 0.67 |
| PREDICTED: complement C4-A | P0C0L4 (CO4A_HUMAN) | R.FGLLDEDGKK.T | 0.64 |
| PREDICTED: complement C4-A | P0C0L4 (CO4A_HUMAN) | R.KKEVYMPSSIFQDDFVIPD ISEPGTWK.I | 0.69 |
| PREDICTED: complement C4-A | P0C0L4 (CO4A_HUMAN) | K.GLCVATPVQLR.V | 0.78 |
| PREDICTED: complement C4-A | P0C0L4 (CO4A_HUMAN) | R.YRVFALDQK.M | 0.63 |
| PREDICTED: complement C4-A | P0C0L4 (CO4A_HUMAN) | K.AEFQDALEKLNMGITDLQ GLR.L | 0.60 |
| PREDICTED: complement C4-A | P0C0L4 (CO4A_HUMAN) | R.ECVGFEAVQEVPVGLVQP ASATLYDYYNPERR.C | 0.60 |
| PREDICTED: complement C4-A | P0C0L4 (CO4A_HUMAN) | K.AEFQDALEKLNMGITDLQ GLR.L | 0.60 |
| PREDICTED: complement C4-A | P0C0L4 (CO4A_HUMAN) | R.VTASDPLDTLGSEGALSP GGVASLLR.L | 0.61 |
| pregnancy zone protein | P20742 (PZP_HUMAN) | R.NELIPLIYLENPRR.N | 0.60 |
| pregnancy zone protein | P20742 (PZP_HUMAN) | K.AVGYLITGYQR.Q | 0.67 |
| protein AMBP preproprotein | P02760 (AMBP_HUMAN) | R.AFIQLWAFDAVK.G | 0.70 |
| protein CBFA2T2 | 043439 (MTG8R_HUMAN) | R.LTEREWADEWKHLDHAL NCIMEMVEK.T | 0.61 |
| protein NLRC3 | Q7RTR2 (NLRC3_HUMAN) | K.ALM*DLLAGKGSQGSQA PQALDR.T | 0.83 |
| prothrombin preproprotein | P00734 (THRB_HUMAN) | R.TFGSGEADCGLRPLFEK.K | 0.69 |
| ras-related GTP-binding protein A | Q7L523 (RRAGA_HUMAN) | K.ISNIIK.Q | 0.68 |
| retinol-binding protein 4 | P02753 (RET4_HUMAN) | R.FSGTWYAMAK.K | 0.64 |
| retinol-binding protein 4 | P02753 (RET4_HUMAN) | R.LLNNWDVCADMVGTFTD TEDPAKFK.M | 0.61 |
| retinol-binding protein 4 | P02753 (RET4_HUMAN) | K.YWGVASFLQK.G | 0.63 |
| serum amyloid P-component | P02743 (SAMP_HUMAN) | R.GYVIIKPLVWV.- | 0.60 |
| sex hormone-binding globulin | P04278 (SHBG_HUMAN) | R.LPLVPALDGCLR.R | 0.63 |
| spectrin beta chain, non-erythrocytic 1 | Q13813 (SPTN1_HUMAN) | R.NELIRQEKLEQLAR.R | 0.88 |
| TATA element modulatory factor | P82094 (TMF1_HUMAN) | K.EELATRLNSSETADLLK.E | 0.71 |
| testicular haploid expressed gene protein-like | PODJG4 (THEGL_HUMAN) | R.QCLLNRPFSDNSAR.D | 0.67 |
| thyroxine-binding globulin | P05543 (THBG_HUMAN) | K.NALALFVLPK.E | 0.61 |
| thyroxine-binding globulin | P05543 (THBG_HUMAN) | R.SFMLLILER.S | 0.64 |
| titin | Q8WZ42 (TITIN_HUMAN) | K.TEPKAPEPISSK.P | 0.89 |
| transthyretin | P02766 (TTHY_HUMAN) | R.GSPAINVAVHVFR.K | 0.61 |
| tripartite motif-containing protein 5 | Q9C035 (TRIM5_HUMAN) | R.ELISDLEHRLQGSVM*ELL QGVDGVIK.R | 0.92 |
| vitamin D-binding protein | P02774 (VTDB_HUMAN) | K.TAMDVFVCTYFMPAAQL PELPDVELPTNKDVCDPGN | 0.88 |
| | | TK.V | |
| vitamin D-binding protein | P02774 (VTDB_HUMAN) | K.VM*DKYTFELSR.R | 0.70 |
| vitamin D-binding protein | P02774 (VTDB_HUMAN) | K.LAQKVPTADLEDVLPLAE DITNILSK.C | 0.61 |
| vitamin D-binding protein | P02774 (VTDB_HUMAN) | K.SCESNSPFPVHPGTAECCT K.E | 0.68 |
| vitamin D-binding protein | P02774 (VTDB_HUMAN) | R.KLCMAALK.H | 0.71 |
| vitamin D-binding protein | P02774 (VTDB_HUMAN) | K.LCDNLSTK.N | 0.60 |
| vitamin D-binding protein | P02774 (VTDB_HUMAN) | K.VM*DKYTFELSR.R | 0.70 |
| vitronectin | P04004 (VTNC_HUMAN) | R.IYISGM*APR.P | 0.75 |
| vitronectin | P04004 (VTNC_HUMAN) | R.ERVYFFK.G | 0.67 |
| vitronectin | P04004 (VTNC_HUMAN) | R.IYISGMAPR.P | 0.81 |
| vitronectin | P04004 (VTNC_HUMAN) | K.AVRPGYPK.L | 0.63 |
| zinc finger protein 142 | P52746 (ZN142_HUMAN) | K.TRFLLR.T | 0.67 |

| | | | |
|---|---|---|---|
| * = Oxidation of methionine | | | |

**Table 10. Preeclampsia: Additional peptides significant with AUC > 0.6 by X! Tandem only**

| Protein description | Uniprot ID (name) | Peptide | XT_AUC |
|---|---|---|---|
| afamin | P43652 (AFAM_HUMAN) | K.TYVPPPFSQDLFTFHADMCQSQN EELQR.K | 0.76 |
| afamin | P43652 (AFAM_HUMAN) | K.KSDVGFLPPFPTLDPEEK.C | 0.62 |
| alpha-1-antichymotrypsin | P01011 (AACT_HUMAN) | R.GTHVDLGLASANVDFAFSLYK.Q | 0.69 |
| alpha-1B-glycoprotein | P04217 (A1BG_HUMAN) | K.SLPAPWLSM*APVSWITPGLK.T | 0.67 |
| alpha-1B-glycoprotein | P04217 (A1BG_HUMAN) | K.SLPAPWLSM*APVSWITPGLK.T | 0.67 |
| alpha-1B-glycoprotein | P04217 (A1BG_HUMAN) | R.C^LAPLEGAR.F | 0.62 |
| alpha-2-antiplasmin | P08697 (A2AP_HUMAN) | R.WFLLEQPEIQVAHFPFK.N | 0.60 |
| alpha-2-antiplasmin | P08697 (A2AP_HUMAN) | R.LCQDLGPGAFR.L | 0.92 |
| alpha-2-antiplasmⁱn | P08697 (A2AP_HUMAN) | K.HQMDLVATLSQLGLQELFQAPDL R.G | 0.67 |
| alpha-2-HS-glycoprotein preproprotein | P02765 (FETUA_HUMAN) | R.QLKEHAVEGDCDFQLLK.L | 0.63 |
| alpha-2-HS-glycoprotein preproprotein | P02765 (FETUA_HUMAN) | R.Q^LKEHAVEGDCDFQLLK.L | 0.65 |
| alpha-2-HS-glycoprotein preproprotein | P02765 (FETUA_HUMAN) | K.C^NLLAEK.Q | 0.61 |
| angiotensinogen preproprotein | P01019 (ANGT_HUMAN) | R.SLDFTELDVAAEKIDR.F | 0.62 |
| angiotensinogen preproprotein | P01019 (ANGT_HUMAN) | K.DPTFIPAPIQAK.T | 0.78 |
| apolipoprotein A-II preproprotein | P02652 (APOA2_HUMAN) | K.EPCVESLVSQYFQTVTDYGKDLM EK.V | 0.67 |
| apolipoprotein B-100 | P04114 (APOB_HUMAN) | K.FSVPAGIVIPSFQALTAR.F | 0.66 |
| apolipoprotein B-100 | P04114 (APOB_HUMAN) | K.EQHLFLPFSYK.N | 0.90 |
| apolipoprotein B-100 | P04114 (APOB_HUMAN) | R.GIISALLVPPETEEAK.Q | 0.70 |
| beta-2-glycoprotein 1 | P02749 (APOH_HUMAN) | K.C^FKEHSSLAFWK.T | 0.70 |
| beta-2-glycoprotein 1 | P02749 (APOH_HUMAN) | K.EHSSLAFWK.T | 0.62 |
| ceruloplasmin | P00450 (CERU_HUMAN) | R.FNKNNEGTYYSPNYNPQSR.S | 0.64 |
| ceruloplasmin | P00450 (CERU_HUMAN) | K.HYYIGIIETTWDYASDHGEK.K | 0.63 |
| ceruloplasmin | P00450 (CERU_HUMAN) | K.M*YYSAVDPTKDIFTGLIGPM*K.I | 0.66 |
| ceruloplasmin | P00450 (CERU_HUMAN) | K.M*YYSAVDPTKDIFTGLIGPM*K.I | 0.66 |
| ceruloplasmin | P00450 (CERU_HUMAN) | K.M*YYSAVDPTKDIFTGLIGPMK.I | 0.67 |
| ceruloplasmin | P00450 (CERU_HUMAN) | K.M*YYSAVDPTKDIFTGLIGPMK.I | 0.67 |
| ceruloplasmin | P00450 (CERU_HUMAN) | K.MYYSAVDPTKDIFTGLIGPM*K.I | 0.67 |
| ceruloplasmin | P00450 (CERU_HUMAN) | K.MYYSAVDPTKDIFTGLIGPM*K.I | 0.67 |
| ceruloplasmin | P00450 (CERU_HUMAN) | R.GVYSSDVFDIFPGTYQTLEM*FPR. T | 0.67 |
| coagulation factor XII | P00748 (FA12_HUMAN) | R.VVGGLVALR.G | 0.64 |
| complement C1q subcomponent subunit A | P02745(C1QA_HUMAN) | K.KGHIYQGSEADSVFSGFLIFPSA.- | 0.81 |
| complement C1q subcomponent subunit C | P02747 (CIQC_HUMAN) | R. Q^THQPPAPNSLIR.F | 0.64 |
| complement C1s subcomponent | P09871 (C1S_HUMAN) | R.Q^FGPYCGHGFPGPLNIETK.S | 0.71 |
| complement C2 | P06681 (CO2_HUMAN) | R.QPYSYDFPEDVAPALGTSFSHML GATNPTQK.T | 0.63 |
| complement C2 | P06681 (CO2_HUMAN) | R.LLGMETMAWQEIR.H | 0.70 |
| complement C4-B-like preproprotein | P0C0L5 (CO4B_HUMAN) | R.AVGSGATFSHYYYM*ILSR.G | 0.67 |
| complement C4-B-like preproprotein | P0C0L5 (CO4B_HUMAN) | R.FGLLDEDGKKTFFR.G | 0.61 |
| complement C4-B-like preproprotein | P0C0L5 (CO4B_HUMAN) | K.ITQVLHFTK.D | 0.67 |
| complement C4-B-like preproprotein | P0C0L5 (CO4B_HUMAN) | K.M*RPSTDTITVM*VENSHGLR.V | 0.65 |
| complement C4-B-like preproprotein | P0C0L5 (CO4B_HUMAN) | K.M*RPSTDTITVM*VENSHGLR.V | 0.75 |
| complement C5 preproprotein | P01031 (C05_HUMAN) | R.IVACASYKPSR.E | 0.67 |
| complement C5 preproprotein | P01031 (C05_HUMAN) | R.SYFPESWLWEVHLVPR.R | 0.60 |
| complement C5 preproprotein | P01031 (C05_HUMAN) | K.Q^LPGGQNPVSYVYLEVVSK.H | 0.74 |
| complement C5 preproprotein | P01031 (C05_HUMAN) | K.TLLPVSKPEIR.S | 0.78 |
| complement component C8 beta chain preproprotein | P07358 (CO8B_HUMAN) | R.GGASEHITTLAYQELPTADLMQE WGDAVQYNPAIIK.V | 0.60 |
| complement factor B preproprotein | P00751 (CFAB_HUMAN) | K.GTDYHKQPWQAK.I | 0.89 |
| complement factor B preproprotein | P00751 (CFAB_HUMAN) | K.VKDISEVVTPR.F | 0.64 |
| complement factor B preproprotein | P00751 (CFAB_HUMAN) | K.Q^VPAHAR.D | 0.63 |
| complement factor B preproprotein | P00751 (CFAB_HUMAN) | R.GDSGGPLIVHKR.S | 0.79 |
| complement factor B preproprotein | P00751 (CFAB_HUMAN) | R.FLCTGGVSPYADPNTCR.G | 0.71 |
| complement factor B preproprotein | P00751 (CFAB_HUMAN) | K.KEAGIPEFYDYDVALIK.L | 0.74 |
| complement factor B preproprotein | P00751 (CFAB_HUMAN) | R.YGLVTYATYPK.I | 0.88 |
| complement factor H | P08603 (CFAH_HUMAN) | K.EFDHNSNIR.Y | 1.00 |
| complement factor H | P08603 (CFAH_HUMAN) | K.WSSPPQCEGLPCK.S | 0.71 |
| complement factor H | P08603 (CFAH_HUMAN) | R.KGEWVALNPLR.K | 0.67 |
| complement factor I preproprotein | P05156 (CFAI_HUMAN) | K.SLECLHPGTK.F | 0.60 |
| corticosteroid-binding globulin | P08185 (CBG_HUMAN) | R.GLASANVDFAFSLYK.H | 0.62 |
| fetuin-B | Q9UGM5 (FETUB_HUMAN) | K.LVVLPFPK.E | 0.74 |
| fetuin-B | Q9UGM5 (FETUB_HUMAN) | R.ASSQWVVGPSYFVEYLIK.E | 0.61 |
| ficolin-3 | 075636 (FCN3_HUMAN) | R.LLGEVDHYQLALGK.F | 0.61 |
| gelsolin | P06396 (GELS_HUMAN) | K.QTQVSVLPEGGETPLFK.Q | 0.69 |
| hemopexin | P02790 (HEMO_HUMAN) | K.VDGALCMEK.S | 0.60 |
| hemopexin | P02790 (HEMO_HUMAN) | K.SGAQATWTELPWPHEKVDGALC | 0.66 |
| | | M*EK.S | |
| hemopexin | P02790 (HEMO_HUMAN) | K.SGAQATWTELPWPHEKVDGALC M*EK.S | 0.66 |
| hemopexin | P02790 (HEMO_HUMAN) | R.EWFWDLATGTMK.E | 0.68 |
| hemopexin | P02790 (HEMO_HUMAN) | R.Q^GHNSVFLIK.G | 0.67 |
| heparin cofactor 2 | P05546 (HEP2_HUMAN) | K.TLEAQLTPR.V | 0.67 |
| histidine-rich glycoprotein | P04196 (HRG_HUMAN) | K.DSPVLIDFFEDTER.Y | 0.60 |
| insulin-like growth factor-binding protein complex acid labile subunit | P35858 (ALS_HUMAN) | K.ALRDFALQNPSAVPR.F | 0.89 |
| insulin-like growth factor-binding protein complex acid labile subunit | P35858 (ALS_HUMAN) | R.LWLEGNPWDCGCPLK.A | 0.60 |
| inter-alpha-trypsin inhibitor heavy chain H1 | P19827 (ITIH1_HUMAN) | K.ILGDM*QPGDYFDLVLFGTR.V | 0.85 |
| inter-alpha-trypsin inhibitor heavy chain H2 | P19823 (ITIH2_HUMAN) | R.SSALDMENFR.T | 0.63 |
| inter-alpha-trypsin inhibitor heavy chain H2 | P19823 (ITIH2_HUMAN) | R.SLAPTAAAK.R | 0.83 |
| inter-alpha-trypsin inhibitor heavy chain H2 | P19823 (ITIH2_HUMAN) | R.LSNENHGIAQR.I | 0.76 |
| inter-alpha-trypsin inhibitor heavy chain H2 | P19823 (ITIH2_HUMAN) | R.IYGNQDTSSQLKK.F | 0.63 |
| inter-alpha-trypsin inhibitor heavy chain H4 | Q14624 (ITIH4_HUMAN) | K.TGLLLLSDPDKVTIGLLFWDGR.G | 0.60 |
| inter-alpha-trypsin inhibitor heavy chain H4 | Q14624 (ITIH4_HUMAN) | KYIFHNFM*ER.L | 0.70 |
| inter-alpha-trypsin inhibitor heavy chain H4 | Q14624 (ITIH4_HUMAN) | K.IPKPEASFSPR.R | 0.65 |
| inter-alpha-trypsin inhibitor heavy chain H4 | Q14624 (ITIH4_HUMAN) | R.QGPVNLLSDPEQGVEVTGQYER. E | 0.64 |
| inter-alpha-trypsin inhibitor heavy chain H4 | Q14624 (ITIH4_HUMAN) | R.ANTVQEATFQMELPK.K | 0.61 |
| inter-alpha-trypsin inhibitor heavy chain H4 | Q14624 (ITIH4_HUMAN) | K.WKETLFSVMPGLK.M | 0.66 |
| inter-alpha-trypsin inhibitor heavy chain H4 | Q14624 (ITIH4_HUMAN) | R.RLDYQEGPPGVEISCWSVEL.- | 0.69 |
| inter-alpha-trypsin inhibitor heavy chain H4 | Q14624 (ITIH4_HUMAN) | K.SPEQQETVLDGNLIIR.Y | 0.66 |
| kallistatin | P29622 (KAIN_HUMAN) | K.ALWEKPFISSR.T | 0.65 |
| kininogen-1 | P01042 (KNG1_HUMAN) | R.Q^VVAGLNFR.I | 0.67 |
| kininogen-1 | P01042 (KNG1_HUMAN) | R.QVVAGLNFR.I | 0.71 |
| kininogen-1 | P01042 (KNG1_HUMAN) | K.LGQSLDCNAEVYVVPWEK.K | 0.62 |
| kininogen-1 | P01042 (KNG1_HUMAN) | R.IASFSQNCDIYPGKDFVQPPTK.I | 0.64 |
| leucine-rich alpha-2-glycoprotein | P02750 (A2GL_HUMAN) | R. C^AGPEAVKGQTLLAVAK. S | 0.70 |
| leucine-rich alpha-2-glycoprotein | P02750 (A2GL_HUMAN) | K.GQTLLAVAK.S | 0.67 |
| leucine-rich alpha-2-glycoprotein | P02750 (A2GL_HUMAN) | K.DLLLPQPDLR.Y | 0.71 |
| lumican | P51884 (LUM_HUMAN) | K.ILGPLSYSK.I | 0.83 |
| PREDICTED: complement C4-A | P0C0L4 (C04A_HUMAN) | R.QGSFQGGFR.S | 0.83 |
| PREDICTED: complement C4-A | P0C0L4 (C04A_HUMAN) | K.YVLPNFEVK.I | 0.69 |
| PREDICTED: complement C4-A | P0C0L4 (C04A_HUMAN) | R.LLATLCSAEVCQCAEGK.C | 0.60 |
| PREDICTED: complement C4-A | P0C0L4 (C04A_HUMAN) | R.VGDTLNLNLR.A | 0.66 |
| PREDICTED: complement C4-A | P0C0L4 (C04A_HUMAN) | R.EPFLSCCQFAESLR.K | 0.62 |
| PREDICTED: complement C4-A | P0C0L4 (C04A_HUMAN) | R.EELVYELNPLDHR.G | 0.60 |
| PREDICTED: complement C4-A | P0C0L4 (C04A_HUMAN) | R.GSFEFPVGDAVSK.V | 0.62 |
| PREDICTED: complement C4-A | P0C0L4 (C04A_HUMAN) | R.GCGEQTMIYLAPTLAASR.Y | 0.71 |
| pregnancy zone protein | P20742 (PZP_HUMAN) | K.GSFALSFPVESDVAPIAR.M | 0.63 |
| protein AMBP preproprotein | P02760 (AMBP_HUMAN) | R.VVAQGVGIPEDSIFTMADRGECV PGEQEPEPILIPR.V | 0.62 |
| prothrombin preproprotein | P00734 (THRB_HUMAN) | R.SGIECQLWR.S | 0.65 |
| thyroxine-binding globulin | P05543 (THBG_HUMAN) | K.MSSINADFAFNLYR.R | 0.63 |
| vitronectin | P04004 (VTNC_HUMAN) | R.MDWLVPATCEPIQSVFFFSGDKY YR.V | 1.00 |
| vitronectin | P04004 (VTNC_HUMAN) | R.IYISGM*APRPSLAK.K | 0.64 |
| vitronectin | P04004 (VTNC_HUMAN) | R.IYISGMAPRPSLAK.K | 0.63 |
| vitronectin | P04004 (VTNC_HUMAN) | R.DVWGIEGPIDAAFTR.I | 0.61 |
| zinc finger CCHC domain-containing protein 9 | Q8N567 (ZCHC9_HUMAN) | R.SCPDNPK.G | 0.68 |

| | | | |
|---|---|---|---|
| * = Oxidation of Methionine, ^ = cyclic pyrolidone derivative by the loss of NH3 (-17Da) | | | |

**Table 11. Candidate peptides and transitions for transferring to the MRM assay**

| Protein | Peptide | m/z, charge | fragment ion, m/z, charge, rank | area |
|---|---|---|---|---|
| inter-alpha-trypsin inhibitor heavy chain H1 | K.AAISGENAGLVR.A | 579.3173++ | S [y9] - 902.4690+[1] | 518001 |
| ITIH1_HUMAN | | | G [y8] - 815.4370+[2] | 326256 |
| | | | N [y6] - 629.3729+[3] | 296670 |
| | | | S [b4] - 343.1976+[4] | 258172 |
| inter-alpha-trypsin inhibitor heavy chain H1 | K.GSLVQASEANLQAA QDFVR.G | 668.6763+++ | A [y7] - 806.4155+[1] | 304374 |
| ITIH1_HUMAN | | | V [b4] - 357.2132+[3] | 294094 |
| | | | A [b13] - 635.3253++[7] | 249287 |
| | | | A [y6] - 735.3784+[2] | 193844 |
| | | | F [y3] - 421.2558+[4] | 167816 |
| | | | L [b11] - 535.7775++[6] | 156882 |
| | | | A [b6] - 556.3089+[5] | 149216 |
| | | | A [y14] - 760.3786++[8] | 123723 |
| inter-alpha-trypsin inhibitor heavy chain H1 | K.TAFISDFAVTADGNA FIGDIK.D | 1087.0442++ | G [y4] - 432.2453+[1] | 22362 |
| ITIH1_HUMAN | | | V [b9] - 952.4775+[2] | 9508 |
| | | | I [y5] - 545.3293+[3] | 8319 |
| | | | A [b8] - 853.4090+[4] | 7006 |
| | | | G [y9] - 934.4993+[5] | 6755 |
| | | | F [y6] - 692.3978+[6] | 6193 |
| inter-alpha-trypsin inhibitor heavy chain H1 | K.VTYDVSR.D | 420.2165++ | T [b2] - 201.1234+[1] | 792556 |
| ITIH1_HUMAN | | | Y [y5] - 639.3097+[2] | 609348 |
| | | | V [y3] - 361.2194+[3] | 256946 |
| | | | D [y4] - 476.2463+[4] | 169546 |
| | | | Y [y5] - 320.1585++[5] | 110608 |
| | | | S [y2] - 262.1510+[6] | 50268 |
| | | | D [b4] - 479.2136+[7] | 13662 |
| | | | Y [b3] - 182.5970++[8] | 10947 |
| inter-alpha-trypsin inhibitor heavy chain H1 | R.EVAFDLEIPK.T | 580.8135++ | P [y2] - 244.1656+[1] | 2032509 |
| ITIH1_HUMAN | | | D [y6] - 714.4032+[2] | 672749 |
| | | | A [y8] - 932.5088+[3] | 390837 |
| | | | F [y7] - 861.4716+[4] | 305087 |
| | | | L [y5] - 599.3763+[5] | 255527 |
| inter-alpha-trypsin inhibitor heavy chain H1 | R.LWAYLTIQELLAK.R | 781.4531++ | W [b2] - 300.1707+[1] | 602601 |
| ITIH1_HUMAN | | | A [b3] - 371.2078+[2] | 356967 |
| | | | T [y8] - 915.5510+[3] | 150419 |
| | | | Y [b4] - 534.2711+[4] | 103449 |
| | | | L [b5] - 647.3552+[5] | 99820 |
| | | | I [y7] - 814.5033+[6] | 72044 |
| | | | Q [y6] - 701.4192+[7] | 66989 |
| | | | E [y5] - 573.3606+[8] | 44843 |
| inter-alpha-trypsin inhibitor heavy chain H2 | K.FYNQVSTPLLR.N | 669.3642++ | S [y6] - 686.4196+[1] | 367330 |
| ITIH2_HUMAN | | | V [y7] - 785.4880+[2] | 182396 |
| | | | P [y4] - 498.3398+[3] | 103638 |
| | | | Q [b4] - 553.2405+[4] | 54270 |
| | | | Y [b2] - 311.1390+[5] | 52172 |
| | | | N [b3] - 425.1819+[6] | 34567 |
| inter-alpha-trypsin inhibitor heavy chain H2 | K.HLEVDVWVIEPQGL R.F | 597.3247+++ | P [y5] - 570.3358+[1] | 303693 |
| ITIH2_HUMAN | | | I [y7] - 812.4625+[2] | 206996 |
| | | | E [y6] - 699.3784+[3] | 126752 |
| | | | P [y5] - 285.6715++[4] | 79841 |
| inter-alpha-trypsin inhibitor heavy chain H2 | K.TAGLVR.S | 308.6925++ | G [y4] - 444.2929+[1] | 789068 |
| ITIH2_HUMAN | | | A [b2] - 173.0921+[2] | 460019 |
| | | | V [y2] - 274.1874+[3] | 34333 |
| | | | L [y3] - 387.2714+[4] | 29020 |
| | | | G [b3] - 230.1135+[5] | 15169 |
| inter-alpha-trypsin inhibitor heavy chain H2 | R.IYLQPGR.L | 423.7452++ | L [y5] - 570.3358+[1] | 638209 |
| ITIH2_HUMAN | | | Y [b2] - 277.1547+[2] | 266889 |
| | | | P [y3] - 329.1932+[3] | 235194 |
| | | | Q [y4] - 457.2518+[4] | 171389 |
| inter-alpha-trypsin inhibitor heavy chain H2 | R.LSNENHGIAQR.I | 413.5461+++ | N [y9] - 519.7574++[1] | 325409 |
| ITIH2_HUMAN | | | G [y5] - 544.3202+[2] | 139598 |
| | | | S [b2] - 201.1234+[3] | 54786 |
| | | | N [y7] - 398.2146++[4] | 39521 |
| | | | E [y8] - 462.7359++[5] | 30623 |
| inter-alpha-trypsin inhibitor heavy chain H2 | R.SLAPTAAAKR.R | 415.2425++ | A [y7] - 629.3617+[1] | 582421 |
| ITIH2_HUMAN | | | P [y6] - 558.3246+[2] | 463815 |
| | | | L [b2] - 201.1234+[3] | 430584 |
| | | | A [b3] - 272.1605+[4] | 204183 |
| | | | T [y5] - 461.2718+[5] | 47301 |
| pregnancy-specific beta-1-glycoprotein 1 | K.FQLPGQK.L | 409.2320++ | L [y5] - 542.3297+[3] | 192218 |
| PSG1_HUMAN | | | P [y4] - 429.2456+[2] | 252933 |
| | | | Q [y2] - 275.1714+[6] | 15366 |
| | | | Q [b2] - 276.1343+[1] | 305361 |
| | | | L [b3] - 389.2183+[4] | 27279 |
| | | | G [b5] - 543.2926+[5] | 18416 |
| pregnancy-specific beta-1-glycoprotein 1 | R.DLYHYITSYVVDGEIII YGPAYSGR.E | 955.4762+++ | G [y7] - 707.3471+[1] | 66891 |
| PSG1_HUMAN | | | Y [y8] - 870.4104+[2] | 45076 |
| | | | P [y6] - 650.3257+[3] | 28437 |
| | | | I [y9] - 983.4945+[4] | 20423 |
| | | | V [b10] - 628.3033++[5] | 17864 |
| | | | E [b14] - 828.3830++[6] | 13690 |
| | | | V [b11] - 677.8375++[7] | 12354 |
| | | | I [b6] - 805.3879+[8] | 11186 |
| | | | V [y15] - 805.4147++[9] | 10573 |
| | | | G [b13] - 763.8617++[10] | 10407 |
| pregnancy-specific beta-1-glycoprotein 4 | TLFIFGVTK | 513.3051++ | F [y7] - 811.4713+[1] | 102139 |
| PSG4_HUMAN | | | L [b2] - 215.1390+[2] | 86272 |
| | | | F [y5] - 551.3188+[3] | 49520 |
| | | | I [y6] - 664.4028+[4] | 26863 |
| | | | T [y2] - 248.1605+[5] | 18671 |
| | | | F [b3] - 362.2074+[6] | 17343 |
| | | | G [y4] - 404.2504+[7] | 17122 |
| pregnancy-specific beta-1-glycoprotein 4 | NYTYIWWLNGQSLPV SPR | 1097.5576++ | W [b6] - 841.3879+[1] | 25756 |
| PSG4_HUMAN | | | G [y9] - 940.5211+[2] | 25018 |
| | | | Y [b4] - 542.2245+[3] | 19778 |
| PSG8_HUMAN | LQLSETNR | 480.7591++ | T [y3] - 390.2096+[1] | 185568 |
| pregnancy-specific beta-1-glycoprotein 8 | | | Q [b2] - 242.1499+[2] | 120644 |
| | | | N [y2] - 289.1619+[3] | 95164 |
| | | | S [y5] - 606.2842+[4] | 84314 |
| | | | L [b3] - 355.2340+[5] | 38587 |
| | | | E [y4] - 519.2522+[6] | 34807 |
| | | | L [y6] - 719.3682+[7] | 17482 |
| | | | E [b5] - 571.3086+[8] | 8855 |
| | | | S [b4] - 442.2660+[9] | 7070 |
| Pan-PSG | ILILPSVTR | 506.3317++ | P [y5] - 559.3198+[1] | 484395 |
| | | | L [b2] - 227.1754+[2] | 102774 |
| | | | L [b4] - 227.1754++[3] | 102774 |
| | | | I [y7] - 785.4880+[4] | 90153 |
| | | | I [b3] - 340.2595+[5] | 45515 |
| | | | L [y6] - 672.4039+[6] | 40368 |
| thyroxine-binding globulin | K.ELELQIGNALFIGK.H | 515.6276+++ | E [b3] - 186.5919++[1] | 48549 |
| THBG_HUMAN | | | E [b3] - 372.1765+[2] | 28849 |
| | | | G [y2] - 204.1343+[3] | 27487 |
| | | | F [b11] - 614.8322++[4] | 14892 |
| | | | L [b4] - 485.2606+[5] | 14552 |
| | | | L [b2] - 243.1339+[6] | 10169 |
| | | | L [b4] - 243.1339++[7] | 10169 |
| thyroxine-binding globulin | K.AQWANPFDPSK.T | 630.8040++ | A [b4] - 457.2194+[1] | 48405 |
| THBG_HUMAN | | | S [y2] - 234.1448+[2] | 43781 |
| | | | D [y4] - 446.2245+[3] | 26549 |
| | | | D [y4] - 446.2245+[4] | 25148 |
| thyroxine-binding globulin | K.TEDSSSFLIDK.T | 621.2984++ | E [b2] - 231.0975+[1] | 37113 |
| THBG_HUMAN | | | D [y2] - 262.1397+[2] | 14495 |
| thyroxine-binding globulin | K.AVLHIGEK.G | 433.7584++ | V [b2] - 171.1128+[1] | 151828 |
| THBG_HUMAN | | | L [y6] - 696.4039+[2] | 102903 |
| | | | H [y5] - 583.3198+[3] | 73288 |
| | | | I [y4] - 446.2609+[4] | 54128 |
| | | | G [y3] - 333.1769+[5] | 32717 |
| | | | H [b4] - 421.2558+[6] | 22662 |
| thyroxine-binding globulin | K.AVLHIGEK.G | 289.5080+++ | L [y6] - 348.7056++[1] | 2496283 |
| THBG_HUMAN | | | V [b2] - 171.1128+[2] | 551283 |
| | | | I [y4] - 446.2609+[3] | 229168 |
| | | | H [y5] - 292,1636++[4] | 212709 |
| | | | H [y5] - 583.3198+[5] | 160132 |
| | | | G [y3] - 333.1769+[6] | 117961 |
| | | | H [b4] - 421.2558+[7] | 56579 |
| | | | I [y4] - 223.6341++[8] | 36569 |
| | | | H [b4] - 211.1315++[9] | 19460 |
| | | | L [b3] - 284.1969+[10] | 15758 |
| thyroxine-binding globulin | K.FLNDVK.T | 368.2054++ | N [y4] - 475.2511+[1] | 298227 |
| THBG_HUMAN | | | V [y2] - 246.1812+[2] | 252002 |
| | | | L [b2] - 261.1598+[3] | 98700 |
| | | | D [y3] - 361.2082+[4] | 29215 |
| | | | D [b4] - 490.2296+[5] | 27258 |
| | | | N [b3] - 375.2027+[6] | 10971 |
| thyroxine-binding globulin | K.FSISATYDLGATLLK. M | 800.4351++ | S [b2] - 235.1077+[1] | 50075 |
| THBG_HUMAN | | | G [y6] - 602.3872+[2] | 46373 |
| | | | D [y8] - 830.4982+[3] | 43372 |
| | | | Y [y9] - 993.5615+[4] | 40970 |
| | | | T [y4] - 474.3286+[5] | 22161 |
| | | | L [y7] - 715.4713+[6] | 19710 |
| | | | S [b4] - 435.2238+[7] | 19310 |
| | | | L [y3] - 373.2809+[8] | 14157 |
| | | | I [b3] - 348.1918+[9] | 13207 |
| thyroxine-binding globulin | K.LSNAAHK.A | 370.7061++ | H [y2] - 284.1717+[4] | 19319 |
| THBG_HUMAN | | | S [b2] - 201.1234+[1] | 60611 |
| | | | N [b3] - 315.1663+[2] | 42142 |
| | | | A [b4] - 386.2034+[3] | 31081 |
| thyroxine-binding globulin | K.GWVDLFVPK.F | 530.7949++ | V [y7] - 817.4818+[2] | 297536 |
| THBG_HUMAN | | | D [y6] - 718.4134+[4] | 226951 |
| | | | L [y5] - 603.3865+[8] | 60712 |
| | | | F [y4] - 490.3024+[9] | 45586 |
| | | | V [y3] - 343.2340+[6] | 134588 |
| | | | P [y2] - 244.1656+ [1] | 1619888 |
| | | | V [b3] - 343.1765+[7] | 126675 |
| | | | D [b4] - 458.2034+[10] | 14705 |
| | | | F [b6] - 718.3559+[5] | 208674 |
| | | | V [b7] - 817.4243+[3] | 270156 |
| thyroxine-binding globulin | K.NALALFVLPK.E | 543.3395++ | L [b3] - 299.1714+[1] | 365040 |
| THBG_HUMAN | | | P [y2] - 244.1656+[2] | 274988 |
| | | | A [y7] - 787.5076+[3] | 237035 |
| | | | L [y6] - 716.4705+[4] | 107838 |
| | | | L [y3] - 357.2496+[5] | 103847 |
| | | | L [y8] - 900.5917+[6] | 97265 |
| | | | F [y5] - 603.3865+[7] | 88231 |
| | | | A [b4] - 370.2085+[8] | 82559 |
| | | | V [y4] - 456.3180+[9] | 32352 |
| | | | L [b5] - 483.2926+[10] | 11974 |
| thyroxine-binding globulin | R.SILFLGK.V | 389.2471++ | L [y5] - 577.3708+[1] | 564222 |
| THBG_HUMAN | | | I [b2] - 201.1234+[2] | 384240 |
| | | | G [y2] - 204.1343+[3] | 302557 |
| | | | L [y3] - 317.2183+[4] | 282436 |
| | | | F [y4] - 464.2867+[5] | 194047 |
| | | | L [b3] - 314.2074+[6] | 27878 |
| leucine-rich alpha-2-glycoprotein | R.VLDLTR.N | 358.7187++ | D [y4] - 504.2776+[1] | 629222 |
| A2GL_HUMAN | | | L [y5] - 617.3617+[2] | 236165 |
| | | | L [b2] - 213.1598+[3] | 171391 |
| | | | L [y3] - 389.2507+[4] | 167609 |
| | | | R [yl] - 175.1190+[5] | 41213 |
| | | | T [y2] - 276.1666+[6] | 37194 |
| | | | D [b3] - 328.1867+[7] | 27029 |
| leucine-rich alpha-2-glycoprotein | K.ALGHLDLSGNR.L | 576.8096++ | G [y9] - 484.7490++[1] | 46334 |
| A2GL_HUMAN | | | L [y7] - 774.4104+[2] | 44285 |
| | | | D [y6] - 661.3264+[3] | 40188 |
| | | | H [y8] - 456.2383++[4] | 29392 |
| | | | H [b4] - 379.2088+[5] | 26871 |
| | | | L [y5] - 546.2994+[6] | 17178 |
| | | | L [b5] - 492.2929+[7] | 14578 |
| leucine-rich alpha-2-glycoprotein | K.LPPGLLANFTLLR.T | 712.9348++ | R [yl] - 175.1190+[1] | 34435 |
| A2GL_HUMAN | | | A [b7] - 662.4236+[2] | 25768 |
| | | | G [y10] - 1117.6728+[3] | 11662 |
| leucine-rich alpha-2-glycoprotein | R.TLDLGENQLETLPPD LLR.G | 1019.0468++ | P [y6] - 710.4196+[1] | 232459 |
| A2GL_HUMAN | | | L [y7] - 823.5036+[2] | 16075 |
| | | | E [y9] - 1053.5939+[3] | 15839 |
| | | | D [b3] - 330.1660+[4] | 15524 |
| leucine-rich alpha-2-glycoprotein | R.GPLQLER.L | 406.7349++ | P [b2] - 155.0815+[1] | 144054 |
| A2GL_HUMAN | | | Q [y4] - 545.3042+[2] | 103146 |
| | | | L [y5] - 658.3883+[3] | 77125 |
| | | | L [y3] - 417.2456+[4] | 65928 |
| | | | R [y1] - 175.1190+[5] | 27585 |
| | | | E [y2] - 304.1615+[6] | 22956 |
| leucine-rich alpha-2-glycoprotein | R.LHLEGNK.L | 405.7271++ | H [b2] - 251.1503+[1] | 79532 |
| A2GL_HUMAN | | | L [y5] - 560.3039+[2] | 54272 |
| | | | G [b5] - 550.2984+[3] | 49019 |
| | | | G [y3] - 318.1772+[4] | 18570 |
| | | | L [b3] - 364.2343+[5] | 14068 |
| | | | E [y4] - 447.2198+[6] | 13318 |
| leucine-rich alpha-2-glycoprotein | K.LQVLGK.D | 329.2183++ | V [y4] - 416.2867+[1] | 141056 |
| A2GL_HUMAN | | | G [y2] - 204.1343+[2] | 102478 |
| | | | Q [b2] - 242.1499+[3] | 98414 |
| | | | L [y3] - 317.2183+[4] | 60587 |
| | | | Q [y5] - 544.3453+[5] | 50833 |
| leucine-rich alpha-2-glycoprotein | K.DLLLPQPDLR.Y | 590.3402++ | P [y6] - 725.3941+[1] | 592715 |
| A2GL_HUMAN | | | L [b3] - 342.2023+[2] | 570948 |
| | | | L [b2] - 229.1183+[3] | 403755 |
| | | | P [y6] - 363.2007++[4] | 120157 |
| | | | L [y2] - 288.2030+[5] | 89508 |
| | | | L [y7] - 838.4781+[6] | 76185 |
| | | | L [b4] - 455.2864+[7] | 60422 |
| | | | L [y7] - 419.7427++[8] | 45849 |
| | | | P [y4] - 500.2827+[9] | 45223 |
| | | | L [y8] - 951.5622+[10] | 22393 |
| | | | Q [y5] - 628.3413+[11] | 15450 |
| leucine-rich alpha-2-glycoprotein | R.VAAGAFQGLR.Q | 495.2800++ | A [y8] - 819.4472+[1] | 183637 |
| A2GL_HUMAN | | | G [y7] - 748.4100+[2] | 110920 |
| | | | F [y5] - 620.3515+[3] | 85535 |
| | | | A [y9] - 890.4843+[4] | 45894 |
| | | | G [y3] - 345.2245+[5] | 45644 |
| | | | Q [y4] - 473.2831+[6] | 40579 |
| | | | A [y8] - 410.2272++[7] | 39266 |
| | | | A [b3] - 242.1499+[8] | 35890 |
| | | | A [y6] - 691.3886+[9] | 29637 |
| | | | G [b4] - 299.1714+[10] | 19195 |
| | | | A [b5] - 370.2085+[11] | 14944 |
| | | | A [y9] - 445.7458++[12] | 11567 |
| leucine-rich alpha-2-glycoprotein | R.WLQAQK.D | 387.2189++ | L [y5] - 587.3511+[1] | 80533 |
| A2GL_HUMAN | | | Q [y4] - 474.2671+[2] | 57336 |
| | | | A [y3] - 346.2085+[3] | 35952 |
| | | | L [b2] - 300.1707+[4] | 22509 |
| leucine-rich alpha-2-glycoprotein | K.GQTLLAVAK.S | 450.7793++ | Q [b2] - 186.0873+[1] | 110213 |
| A2GL_HUMAN | | | T [y7] - 715.4713+[2] | 81127 |
| | | | L [y5] - 501.3395+[3] | 52292 |
| | | | L [y6] - 614.4236+[4] | 46349 |
| | | | A [y4] - 388.2554+[5] | 41283 |
| | | | A [y2] - 218.1499+[6] | 38843 |
| | | | V [y3] - 317.2183+[7] | 28961 |
| | | | T [b3] - 287.1350+[8] | 23831 |
| leucine-rich alpha-2-glycoprotein | R.YLFLNGNK.L | 484.7636++ | F [y6] - 692.3726+[1] | 61861 |
| A2GL_HUMAN | | | L [b2] - 277.1547+[2] | 39468 |
| | | | F [b3] - 424.2231+[3] | 21454 |
| | | | L [y5] - 545.3042+[4] | 20016 |
| | | | N [y4] - 432.2201+[5] | 18077 |
| leucine-rich alpha-2-glycoprotein | R.NALTGLPPGLFQASA TLDTLVLK.E | 780.7773+++ | T [y8] - 902.5557+[1] | 44285 |
| A2GL_HUMAN | | | P [y17] - 886.0036++[2] | 39557 |
| | | | D [y6] - 688.4240+[3] | 19464 |
| alpha-1B-glycoprotein | K.NGVAQEPVHLDSPAI K.H | 837.9441++ | P [y10] - 1076.6099+[1] | 130137 |
| A1BG_HUMAN | | | V [b3] - 271.1401+[2] | 110650 |
| | | | A [y13] - 702.8777++[3] | 75803 |
| | | | S [y5] - 515.3188+[4] | 63197 |
| | | | G [b2] - 172.0717+[5] | 57307 |
| | | | E [b6] - 599.2784+[6] | 49765 |
| | | | A [b4] - 342.1772+[7] | 36058 |
| | | | E [y11] - 1205.6525+[8] | 34131 |
| | | | P [y4] - 428.2867+[9] | 31158 |
| | | | H [y8] - 880.4887+[10] | 28296 |
| | | | D [y6] - 630.3457+[11] | 20534 |
| | | | L [y7] - 743.4298+[12] | 17946 |
| alpha-1B-glycoprotein | K.HQFLLTGDTQGR.Y | 686.8520++ | Q [b2] - 266.1248+[1] | 1144372 |
| A1BG_HUMAN | | | F [y10] - 1107.5793+[2] | 725830 |
| | | | T [y7] - 734.3428+[3] | 341528 |
| | | | L [y8] - 847.4268+[4] | 297048 |
| | | | F [b3] - 413.1932+[5] | 230163 |
| | | | G [y6] - 633.2951+[6] | 226694 |
| | | | T [y4] - 461.2467+[7] | 217446 |
| | | | L [y9] - 960.5109+[8] | 215574 |
| | | | L [b4] - 526.2772+[9] | 184306 |
| | | | L [b5] - 639.3613+[10] | 157607 |
| | | | Q [y11] - 1235.6379+[11] | 117366 |
| | | | Q [y11] - 618.3226++[12] | 109274 |
| | | | D [b8] - 912.4574+[13] | 53233 |
| | | | T [b6] - 740.4090+[14] | 49104 |
| | | | D [y5] - 576.2736+[15] | 35232 |
| alpha-1B-glycoprotein | R.SGLSTGWTQLSK.L | 632.8302++ | G [y7] - 819.4359+[1] | 1138845 |
| A1BG_HUMAN | | | L [b3] - 258.1448+[2] | 1128060 |
| | | | S [y9] - 1007.5156+[3] | 877313 |
| | | | S [y2] - 234.1448+[4] | 653032 |
| | | | T [y8] - 920.4836+[5] | 651216 |
| | | | T [y5] - 576.3352+[6] | 538856 |
| | | | W [y6] - 762.4145+[7] | 406137 |
| | | | L [y3] - 347.2289+[8] | 313255 |
| | | | Q [y4] - 475.2875+[9] | 209919 |
| | | | L [y10] - 560.8035++[10] | 103666 |
| | | | W [b7] - 689.3253+[11] | 48587 |
| | | | Q [b9] - 918.4316+[12] | 27677 |
| | | | T [b8] - 790.3730+[13] | 26742 |
| | | | L [b10] - 1031.5156+[14] | 23936 |
| alpha-1B-glycoprotein | K.LLELTGPK.S | 435.7684++ | E [y6] - 644.3614+[1] | 6043967 |
| A1BG_HUMAN | | | L [b2] - 227.1754+[2] | 2185138 |
| | | | L [y7] - 757.4454+[3] | 1878211 |
| | | | L [y5] - 515.3188+[4] | 923148 |
| | | | T [y4] - 402.2347+[5] | 699198 |
| | | | G [y3] - 301.1870+[6] | 666018 |
| | | | P [y2] - 244.1656+[7] | 430183 |
| | | | E [b3] - 356.2180+[8] | 244199 |
| alpha-1B-glycoprotein | R.GVTFLLR.R | 403.2502++ | T [y5] - 649.4032+[1] | 4135468 |
| A1BG_HUMAN | | | L [y3] - 401.2871+[2] | 2868709 |
| | | | V [b2] - 157.0972+[3] | 2109754 |
| | | | F [y4] - 548.3555+[4] | 1895653 |
| | | | R [y1] - 175.1190+[5] | 918856 |
| | | | L [y2] - 288.2030+[6] | 780084 |
| | | | T [b3] - 258.1448+[7] | 478494 |
| | | | T [y5] - 325.2052++[8] | 415711 |
| | | | F [y4] - 274.6814++[9] | 140533 |
| | | | L [b6] - 631.3814+[10] | 129473 |
| alpha-1B-glycoprotein | K.ELLVPR.S | 363.7291++ | P [y2] - 272.1717+[1] | 9969478 |
| A1BG_HUMAN | | | L [y4] - 484.3242+[2] | 3676023 |
| | | | V [y3] - 371.2401+[3] | 2971809 |
| | | | L [b2] - 243.1339+[4] | 809753 |
| | | | L [y5] - 597.4083+[5] | 159684 |
| alpha-1B-glycoprotein | R.SSTSPDR.I | 375.1748++ | S [b2] - 175.0713+[1] | 89016 |
| A1BG_HUMAN | | | R [y1] - 175.1190+[2] | 82740 |
| | | | P [y3] - 387.1987+[3] | 76299 |
| | | | T [y5] - 575.2784+[4] | 75253 |
| | | | D [b6] - 575.2307+[5] | 71180 |
| | | | S [y4] - 474.2307+[6] | 53784 |
| alpha-1B-glycoprotein | R.LELHVDGPPPRPQLR .A | 862.4837++ | D [b6] - 707.3723+[1] | 49322 |
| A1BG_HUMAN | | | G [y9] - 1017.5952+[2] | 32049 |
| | | | G [y9] - 509.3012++[3] | 27715 |
| alpha-1B-glycoprotein | R.LELHVDGPPPRPQLR .A | 575.3249+++ | V [y11] - 616.3489++[1] | 841163 |
| A1BG_HUMAN | | | D [y10] - 566.8147++[2] | 621546 |
| | | | E [b2] - 243.1339+[3] | 581025 |
| | | | H [y12] - 684.8784++[4] | 485731 |
| | | | R [y5] - 669.4155+[5] | 477653 |
| | | | L [y13] - 741.4204++[6] | 369224 |
| | | | H [b4] - 493.2769+[7] | 219485 |
| | | | D [b6] - 707.3723+[8] | 195842 |
| | | | V [b5] - 592.3453+[9] | 170689 |
| | | | R [y1] - 175.1190+[10] | 160049 |
| | | | L [b3] - 356.2180+[11] | 63902 |
| | | | G [b7] - 764.3937+[12] | 62128 |
| | | | P [y4] - 513.3144+[13] | 33888 |
| alpha-1B-glycoprotein | R.ATWSGAVLAGR.D | 544.7960++ | S [y8] - 730.4206+[1] | 1933290 |
| A1BG_HUMAN | | | G [y7] - 643.3886+[2] | 1828931 |
| | | | L [y4] - 416.2616+[3] | 869412 |
| | | | V [y5] - 515.3300+[4] | 615117 |
| | | | A [y3] - 303.1775+[5] | 584118 |
| | | | A [y6] - 586.3671+[6] | 471353 |
| | | | W [y9] - 458.7536++[7] | 466690 |
| | | | W [y9] - 916.4999+[8] | 454934 |
| | | | G [y2] - 232.1404+[9] | 338886 |
| | | | S [b4] - 446.2034+[10] | 165831 |
| | | | W [b3] - 359.1714+[11] | 139166 |
| | | | R [y1] - 175.1190+[12] | 83145 |
| | | | A [b6] - 574.2620+[13] | 65281 |
| | | | G [b5] - 503.2249+[14] | 30473 |
| | | | V [b7] - 673.3304+[15] | 30408 |
| alpha-1B-glycoprotein | R.TPGAAANLELIFVGP QHAGNYR.C | 1148.5953++ | G [y9] - 999.4755+[1] | 39339 |
| A1BG_HUMAN | | | F [y11] - 1245.6123+[2] | 22329 |
| | | | V [y10] - 1098.5439+[3] | 14054 |
| | | | I [b11] - 1051.5782+[4] | 12281 |
| | | | P [y8] - 942.4540+[5] | 10574 |
| alpha-1B-glycoprotein | R.TPGAAANLELIFVGP QHAGNYR.C | 766.0659+++ | G [y9] - 999.4755+[1] | 426098 |
| A1BG_HUMAN | | | P [y8] - 942.4540+[2] | 191245 |
| | | | V [y10] - 1098.5439+[3] | 183889 |
| | | | F [y11] - 1245.6123+[4] | 172790 |
| | | | G [b3] - 256.1292+[5] | 172068 |
| | | | A [y5] - 580.2838+[6] | 170557 |
| | | | A [b4] - 327.1663+[7] | 146455 |
| | | | H [y6] - 717.3427+[8] | 127934 |
| | | | E [b9] - 825.4101+[9] | 119922 |
| | | | G [y4] - 509.2467+[10] | 107378 |
| | | | L [b10] - 938.4942+[11] | 102387 |
| | | | A [b5] - 398.2034+[12] | 86428 |
| | | | L [b10] - 469.7507++[13] | 68959 |
| | | | E [y14] - 800.9152++[14] | 67711 |
| | | | I [y12] - 679.8518++[15] | 65740 |
| | | | N [b7] - 583.2835+[16] | 58648 |
| | | | A [y17] - 949.9972++[17] | 55561 |
| | | | G [y20] - 1049.5451++[18] | 51555 |
| | | | I [b11] - 1051.5782+[19] | 51489 |
| | | | L [y13] - 736.3939++[20] | 49190 |
| | | | L [y15] - 857.4572++[21] | 48534 |
| | | | A [y18] - 985.5158++[22] | 48337 |
| | | | L [b8] - 696.3675+[23] | 47352 |
| | | | N [y16] - 914.4787++[24] | 43280 |
| | | | A [b6] - 469.2405+[25] | 38091 |
| | | | Q [y7] - 845.4013+[26] | 32443 |
| insulin-like growth factor-binding protein complex acid labile subunit | R.SLALGTFAHTPALAS LGLSNNR.L | 737.7342+++ | G [y6] - 660.3424+[1] | 37287 |
| ALS_HUMAN | | | A [b3] - 272.1605+[2] | 21210 |
| | | | S [y8] - 860.4585+[3] | 15266 |
| | | | S [y4] - 490.2368+[4] | 12497 |
| | | | L [y5] - 603.3209+[5] | 9592 |
| insulin-like growth factor-binding protein complex acid labile subunit | R.ELVLAGNR.L | 436.2534++ | A [y4] - 417.2205+[1] | 74710 |
| ALS_HUMAN | | | L [y5] - 530.3045+[2] | 71602 |
| | | | G [y3] - 346.1833+[3] | 39449 |
| | | | V [y6] - 629.3729+[4] | 30127 |
| insulin-like growth factor-binding protein complex acid labile subunit | R.LAYLQPALFSGLAELR .E | 881.4985++ | P [y11] - 1173.6626+[1] | 47285 |
| ALS_HUMAN | | | Y [b3] - 348.1918+[2] | 27425 |
| | | | Q [b5] - 589.3344+[3] | 18779 |
| | | | L [b4] - 461.2758+[4] | 13442 |
| insulin-like growth factor-binding protein complex acid labile subunit | | 588.0014+++ | S [y7] - 745.4203+[1] | 29519 |
| ALS_HUMAN | | | A [y4] - 488.2827+[2] | 23305 |
| | | | G [y6] - 658.3883+[3] | 22089 |
| | | | F [y8] - 892.4887+[4] | 16888 |
| | | | Q [b5] - 589.3344+[5] | 15807 |
| | | | L [y2] - 288.2030+[6] | 15266 |
| | | | Y [b3] - 348.1918+[7] | 12835 |
| | | | L [y5] - 601.3668+[8] | 12024 |
| insulin-like growth factor-binding protein complex acid labile subunit | R.ELDLSR.N | 366.6980++ | S [y2] - 262.1510+[1] | 91447 |
| ALS_HUMAN | | | D [b3] - 358.1609+[2] | 85115 |
| | | | D [y4] - 490.2620+[3] | 75618 |
| | | | L [y3] - 375.2350+[4] | 37835 |
| insulin-like growth factor-binding protein complex acid labile subunit | K.ANVFVQLPR.L | 522.3035++ | N [b2] - 186.0873+[1] | 90097 |
| ALS_HUMAN | | | F [y6] - 759.4512+[2] | 61085 |
| | | | P [y2] - 272.1717+[3] | 46657 |
| | | | V [y5] - 612.3828+[4] | 43595 |
| | | | V [b3] - 285.1557+[5] | 31451 |
| | | | Q [y4] - 513.3144+[6] | 28908 |
| | | | V [y7] - 858.5196+[7] | 15725 |
| | | | L [y3] - 385.2558+[8] | 14324 |
| | | | Q [y4] - 257.1608++[9] | 13753 |
| insulin-like growth factor-binding protein complex acid labile subunit | R.NLIAAVAPGAFLGLK. A | 727.9401++ | L [b2] - 228.1343+[1] | 26729 |
| ALS_HUMAN | | | I [b3] - 341.2183+[2] | 25535 |
| | | | P [y8] - 802.4822+[3] | 25120 |
| | | | A [y9] - 873.5193+[4] | 17542 |
| | | | A [y12] - 1114.6619+[5] | 14895 |
| insulin-like growth factor-binding protein complex acid labile subunit | R.VAGLLEDTFPGLLGL R.V | 835.9774++ | P [y7] - 725.4668+[1] | 22005 |
| ALS_HUMAN | | | L [b4] - 341.2183+[2] | 13753 |
| | | | E [y11] - 1217.6525+[3] | 12611 |
| | | | D [y10] - 1088.6099+[4] | 11003 |
| insulin-like growth factor-binding protein complex acid labile subunit | R.SFEGLGQLEVLTLDH NQLQEVK.A | 833.1026+++ | Q [y4] - 503.2824+[1] | 328959 |
| ALS_HUMAN | | | T [y11] - 662.8464++[2] | 54479 |
| | | | G [b4] - 421.1718+[3] | 24263 |
| insulin-like growth factor-binding protein complex acid labile subunit | R.NLPEQVFR.G | 501.7720++ | P [y6] - 775.4097+[1] | 88417 |
| ALS_HUMAN | | | E [y5] - 678.3570+[2] | 13620 |
| insulin-like growth factor-binding protein complex acid labile subunit | R.IRPHTFTGLSGLR.R | 485.6124+++ | S [y4] - 432.2565+[1] | 82619 |
| ALS_HUMAN | | | L [y5] - 545.3406+[2] | 70929 |
| | | | T [b5] - 303.1795++[3] | 56677 |
| insulin-like growth factor-binding protein complex acid labile subunit | K.LEYLLLSR.N | 503.8002++ | Y [y6] - 764.4665+[1] | 67619 |
| ALS_HUMAN | | | E [b2] - 243.1339+[2] | 56261 |
| | | | L [y4] - 488.3191+[3] | 32890 |
| | | | L [y5] - 601.4032+[4] | 24224 |
| | | | L [y3] - 375.2350+[5] | 21139 |
| insulin-like growth factor-binding protein complex acid labile subunit | R.LAELPADALGPLQR. A | 732.4145++ | E [b3] - 314.1710+[1] | 57859 |
| ALS_HUMAN | | | P [y10] - 1037.5738+[2] | 45907 |
| | | | P [y10] - 519.2905++[3] | 22723 |
| | | | L [b4] - 427.2551+[4] | 14054 |
| insulin-like growth factor-binding protein complex acid labile subunit | R.LEALPNSLLAPLGR.L | 732.4327++ | A [b3] - 314.1710+[1] | 52485 |
| ALS_HUMAN | | | P [y10] - 1037.6102+[2] | 37028 |
| | | | E [b2] - 243.1339+[3] | 24846 |
| | | | P [y10] - 519.3087++[4] | 15601 |
| | | | P [y4] - 442.2772+[5] | 12327 |
| insulin-like growth factor-binding protein complex acid labile subunit | R.TFTPQPPGLER.L | 621.8275++ | P [y6] - 668.3726+[1] | 57877 |
| ALS_HUMAN | | | P [y8] - 447.2456++[2] | 50606 |
| | | | P [b4] - 447.2238+[3] | 50606 |
| | | | F [b2] - 249.1234+[4] | 42083 |
| | | | P [y8] - 893.4839+[5] | 34716 |
| | | | T [y9] - 497.7694++[6] | 24220 |
| | | | T [b3] - 350.1710+[7] | 22053 |
| insulin-like growth factor-binding protein complex acid labile subunit | R.DFALQNPSAVPR.F | 657.8437++ | A [b3] - 334.1397+[1] | 28905 |
| ALS_HUMAN | | | P [y6] - 626.3620+[2] | 23750 |
| | | | P [y2] - 272.1717+[3] | 20860 |
| | | | F [b2] - 263.1026+[4] | 17536 |
| | | | N [y7] - 740.4050+[5] | 15320 |
| | | | Q [y8] - 868.4635+[6] | 12525 |
| beta-2-glycoprotein 1 | K.FICPLTGLWPINTLK. | 886.9920++ | C [b3] - 421.1904+[1] | 546451 |
| APOH_HUMAN | C | | C [y13] - 756.9158++[2] | 438858 |
| | | | P [y6] - 685.4243+[3] | 229375 |
| | | | I [b2] - 261.1598+[4] | 188092 |
| | | | W [y7] - 871.5036+[5] | 143885 |
| | | | G [y9] - 1041.6091+[6] | 143458 |
| | | | T [b13] - 757.3972++[7] | 127058 |
| | | | T [y10] - 1142.6568+[8] | 89126 |
| | | | T [b6] - 732.3749+[9] | 51907 |
| | | | L [b5] - 631.3272+[10] | 43351 |
| | | | L [b8] - 902.4804+[11] | 38788 |
| | | | N [y4] - 475.2875+[12] | 38574 |
| | | | W [b9] - 1088.5597+[13] | 37148 |
| | | | T [y3] - 361.2445+[14] | 34153 |
| | | | G [b7] - 789.3964+[15] | 22460 |
| | | | P [b4] - 518.2432+[16] | 19893 |
| | | | L [y8] - 984.5877+[17] | 19180 |
| beta-2-glycoprotein 1 | K.FICPLTGLWPINTLK. C | 591.6638+++ | P [y6] - 685.4243+[1] | 541745 |
| APOH_HUMAN | | | P [y6] - 343.2158++[2] | 234580 |
| | | | G [b7] - 789.3964+[3] | 99108 |
| | | | W [y7] - 871.5036+[4] | 89126 |
| | | | L [b8] - 902.4804+[5] | 68306 |
| | | | C [b3] - 421.1904+[6] | 58396 |
| | | | N [y4] - 475.2875+[7] | 54474 |
| | | | I [y5] - 588.3715+[8] | 54403 |
| | | | W [y7] - 436.2554++[9] | 44706 |
| | | | I [b2] - 261.1598+[10] | 40214 |
| | | | T [y3] - 361.2445+[11] | 20535 |
| beta-2-glycoprotein 1 | R.VCPFAGILENGAVR. Y | 751.8928++ | P [y12] - 622.3433++[1] | 431648 |
| APOH_HUMAN | | | C [b2] - 260.1063+[2] | 223667 |
| | | | P [y12] - 1243.6793+[3] | 134827 |
| | | | G [y9] - 928.5211+[4] | 89980 |
| | | | L [y7] - 758.4155+[5] | 85773 |
| | | | A [y10] - 999.5582+[6] | 69303 |
| | | | A [b5] - 575.2646+[7] | 47913 |
| | | | E [y6] - 645.3315+[8] | 44705 |
| | | | N [y5] - 516.2889+[9] | 23244 |
| | | | I [y8] - 871.4996+[10] | 20320 |
| | | | G [y4] - 402.2459+[11] | 19180 |
| | | | I [b7] - 745.3702+[12] | 18966 |
| | | | F [b4] - 504.2275+[13] | 16399 |
| beta-2-glycoprotein 1 | R.VCPFAGILENGAVR. Y | 501.5977+++ | E [y6] - 645.3315+[1] | 131191 |
| APOH_HUMAN | | | N [y5] - 516.2889+[2] | 130264 |
| | | | I [b7] - 745.3702+[3] | 112154 |
| | | | G [b6] - 632.2861+[4] | 102743 |
| | | | G [y4] - 402.2459+[5] | 82779 |
| | | | C [b2] - 260.1063+[6] | 65453 |
| | | | L [y7] - 758.4155+[7] | 54330 |
| | | | I [b7] - 373.1887++[8] | 39143 |
| | | | L [y7] - 379.7114++[9] | 29661 |
| | | | V [y2] - 274.1874+[10] | 28377 |
| | | | P [y12] - 622.3433++[11] | 28163 |
| beta-2-glycoprotein 1 | K.CTEEGK.W | 362.1525++ | E [y3] - 333.1769+[1] | 59464 |
| APOH_HUMAN | | | E [b3] - 391.1282+[2] | 21675 |
| beta-2-glycoprotein 1 | K.WSPELPVCAPIICPPP SIPTFATLR.V | 940.4923+++ | P [y12] - 648.8692++[1] | 294510 |
| APOH_HUMAN | | | P [y11] - 600.3428++[2] | 206026 |
| | | | P [y7] - 805.4567+[3] | 122891 |
| | | | P [y10] - 1102.6255+[4] | 75113 |
| | | | L [b5] - 613.2980+[5] | 74578 |
| | | | P [y11] - 1199.6783+[6] | 72855 |
| | | | A [b9] - 1040.4870+[7] | 28643 |
| | | | T [y3] - 195.1290++[8] | 28524 |
| | | | S [b2] - 274.1186+[9] | 23770 |
| | | | P [y10] - 551.8164++[10] | 22284 |
| | | | C [y13] - 728.8845++[11] | 20918 |
| | | | E [b4] - 500.2140+[12] | 17114 |
| beta-2-glycoprotein 1 | K.ATFGCHDGYSLDGP EEIECTK.L | 796.0036+++ | P [y8] - 503.2315++[1] | 67031 |
| APOH_HUMAN | | | E [y4] - 537.2337+[2] | 59841 |
| | | | C [b5] - 537.2126+[3] | 56454 |
| | | | I [y5] - 650.3178+[4] | 55384 |
| | | | C [y3] - 408.1911+[5] | 46946 |
| | | | E [y6] - 779.3604+[6] | 45282 |
| | | | T [b2] - 173.0921+[7] | 37675 |
| | | | G [y9] - 1062.4772+[8] | 36843 |
| | | | C [y17] - 1005.4144++[9] | 35774 |
| | | | P [y8] - 1005.4557+[10] | 33991 |
| | | | D [y10] - 1177.5041+[11] | 30366 |
| | | | E [y7] - 908.4030+[12] | 26503 |
| | | | T [y2] - 248.1605+[13] | 24840 |
| | | | Y [b9] - 1009.3832+[14] | 19491 |
| | | | G [y9] - 531.7422++[15] | 17946 |
| | | | S [b10] - 1096.4153+[16] | 17352 |
| beta-2-glycoprotein 1 | K.ATVVYQGER.V | 511.7669++ | Y [y5] - 652.3049+[1] | 762897 |
| APOH_HUMAN | | | V [y6] - 751.3733+[2] | 548908 |
| | | | T [b2] - 173.0921+[3] | 252556 |
| | | | V [y7] - 850.4417+[4] | 231995 |
| | | | V [b3] - 272.1605+[5] | 223140 |
| | | | Q [y4] - 489.2416+[6] | 165023 |
| | | | G [y3] - 361.1830+[7] | 135013 |
| | | | V [b4] - 371.2289+[8] | 86760 |
| | | | V [y7] - 425.7245++[9] | 54314 |
| beta-2-glycoprotein 1 | K.VSFFCK.N | 394.1940++ | S [y5] - 688.3123+[1] | 384559 |
| APOH_HUMAN | | | F [y4] - 601.2803+[2] | 321951 |
| | | | C [y2] - 307.1435+[3] | 265521 |
| | | | S [b2] - 187.1077+[4] | 237662 |
| | | | F [y3] - 454.2119+[5] | 168104 |
| beta-2-glycoprotein 1 | K.CSYTEDAQCIDGTIE VPK.C | 1043.4588++ | P [y2] - 244.1656+[1] | 34574 |
| APOH_HUMAN | | | V [y3] - 343.2340+[2] | 9173 |
| | | | E [y4] - 472.2766+[3] | 7291 |
| | | | Y [b3] - 411.1333+[4] | 6233 |
| beta-2-glycoprotein 1 | K.CSYTEDAQCIDGTIE VPK.C | 695.9750+++ | D [b11] - 672.2476++[1] | 37044 |
| APOH_HUMAN | | | D [y8] - 858.4567+[2] | 18816 |
| | | | D [b6] - 756.2505+[3] | 12289 |
| | | | V [y3] - 343.2340+[4] | 11348 |
| | | | A [b7] - 414.1474++[5] | 9761 |
| | | | G [y7] - 743.4298+[6] | 8644 |
| beta-2-glycoprotein 1 | K.EHSSLAFWK.T | 552.7773++ | H [b2] - 267.1088+[1] | 237907 |
| APOH_HUMAN | | | S [y7] - 838.4458+[2] | 200568 |
| | | | W [y2] - 333.1921+[3] | 101078 |
| | | | S [y6] - 751.4137+[4] | 54920 |
| | | | A [y4] - 551.2976+[5] | 52920 |
| | | | F [y3] - 480.2605+[6] | 40102 |
| | | | L [y5] - 664.3817+[7] | 30341 |
| | | | F [b7] - 772.3624+[8] | 27871 |
| | | | S [b3] - 354.1408+[9] | 27754 |
| | | | A [b6] - 625.2940+[10] | 25931 |
| beta-2-glycoprotein 1 | K.TDASDVKPC.- | 496.7213++ | D [b2] - 217.0819+[1] | 323810 |
| APOH_HUMAN | | | P [y2] - 276.1013+[2] | 119128 |
| | | | A [y7] - 776.3607+[3] | 86083 |
| | | | S [y6] - 705.3236+[4] | 79262 |
| | | | A [b3] - 288.1190+[5] | 77498 |
| | | | D [y5] - 618.2916+[6] | 70501 |
| | | | K [y3] - 404.1962+[7] | 55801 |
| | | | V [y4] - 503.2646+[8] | 46217 |
| transforming growth factor-beta-induced protein ig-h3 | K.SPYQLVLQHSR.L | 443.2421+++ | Y [y9] - 572.3171++[1] | 560916 |
| BGH3_HUMAN | | | P [b2] - 185.0921+[2] | 413241 |
| | | | H [y3] - 399.2099+[3] | 320572 |
| | | | L [y5] - 640.3525+[4] | 313309 |
| | | | Q [y4] - 527.2685+[5] | 244398 |
| | | | L [y7] - 426.7561++[6] | 215854 |
| | | | V [y6] - 739.4209+[7] | 172897 |
| | | | L [y7] - 852.5050+[8] | 164959 |
| | | | Q [y8] - 490.7854++[9] | 149814 |
| | | | L [y5] - 320.6799++[10] | 127463 |
| | | | L [b5] - 589.2980+[11] | 118061 |
| | | | S [y2] - 262.1510+[12] | 110123 |
| | | | V [y6] - 370.2141++[13] | 97399 |
| | | | P [y10] - 620.8435++[14] | 94640 |
| | | | V [b6] - 688.3665+[15] | 87772 |
| | | | Q [b4] - 476.2140+[16] | 74203 |
| | | | Y [b3] - 348.1554+[17] | 65984 |
| | | | H [y3] - 200.1086++[18] | 55624 |
| | | | Q [y4] - 264.1379++[19] | 41606 |
| | | | L [b7] - 801.4505+[20] | 18241 |
| | | | V [b6] - 344.6869++[21] | 17678 |
| | | | L [b7] - 401.2289++[22] | 14976 |
| transforming growth factor-beta-induced protein ig-h3 | R.VLTDELK.H | 409.2369++ | T [y5] - 605.3141+[1] | 937957 |
| BGH3_HUMAN | | | L [b2] - 213.1598+[2] | 298671 |
| | | | L [y6] - 718.3981+[3] | 244116 |
| | | | L [y2] - 260.1969+[4] | 135739 |
| | | | D [y4] - 504.2664+[5] | 52472 |
| | | | E [y3] - 389.2395+[6] | 50839 |
| transforming growth factor-beta-induced protein ig-h3 | K.VISTITNNIQQIIEIED TFETLR.A | 897.4798+++ | E [y8] - 1010.4789+[1] | 282865 |
| BGH3_HUMAN | | | D [y7] - 881.4363+[2] | 237234 |
| | | | I [y9] - 1123.5630+[3] | 195581 |
| | | | T [y6] - 766.4094+[4] | 186875 |
| | | | I [b2] - 213.1598+[5] | 174492 |
| | | | T [y3] - 389.2507+[6] | 145598 |
| | | | F [y5] - 665.3617+[7] | 143872 |
| | | | E [y4] - 518.2933+[8] | 108148 |
| | | | Q [b11] - 606.8328++[9] | 106647 |
| | | | I [b5] - 514.3235+[10] | 82030 |
| | | | N [b8] - 843.4571+[11] | 75125 |
| | | | T [b4] - 401.2395+[12] | 71448 |
| | | | I [b12] - 663.3748++[13] | 58314 |
| | | | N [b7] - 365.2107++[14] | 54862 |
| | | | I [b9] - 956.5411+[15] | 51034 |
| | | | L [y2] - 288.2030+[16] | 50734 |
| | | | S [b3] - 300.1918+[17] | 48708 |
| | | | Q [b10] - 542.8035++[18] | 43754 |
| | | | Q [b11] - 1212.6583+[19] | 37375 |
| | | | T [b6] - 615.3712+[20] | 33322 |
| | | | I [b9] - 478.7742++[21] | 29570 |
| | | | Q [b10] - 1084.5997+[22] | 25817 |
| | | | T [y6] - 383.7083++[23] | 17187 |
| | | | N [b8] - 422.2322++[24] | 17111 |
| | | | I [b13] - 719.9168++[25] | 16661 |
| transforming growth factor-beta-induced protein ig-h3 | K.IPSETLNR.I | 465.2562++ | S [y6] - 719.3682+[1] | 326570 |
| BGH3_HUMAN | | | P [y7] - 816.4210+[2] | 168951 |
| | | | E [y5] - 632.3362+[3] | 102452 |
| | | | P [b2] - 211.1441+[4] | 85885 |
| | | | T [y4] - 503.2936+[5] | 67650 |
| | | | L [y3] - 402.2459+[6] | 20939 |
| | | | N [y2] - 289.1619+[7] | 13979 |
| transforming growth factor-beta-induced protein ig-h3 | R.ILGDPEALR.D | 492.2796++ | P [y5] - 585.3355+[1] | 1431619 |
| BGH3_HUMAN | | | G [y7] - 757.3839+[2] | 1066060 |
| | | | L [b2] - 227.1754+[3] | 742225 |
| | | | L [y8] - 870.4680+[4] | 254257 |
| | | | D [b4] - 399.2238+[5] | 159932 |
| | | | G [b3] - 284.1969+[6] | 66816 |
| | | | D [y6] - 700.3624+[7] | 65780 |
| | | | A [y3] - 359.2401+[8] | 62730 |
| | | | E [y4] - 488.2827+[9] | 23711 |
| | | | L [y2] - 288.2030+[10] | 16344 |
| transforming growth factor-beta-induced protein ig-h3 | R.DLLNNHILK.S | 360.5451+++ | L [y7] - 426.2585++[1] | 1488651 |
| BGH3_HUMAN | | | L [b2] - 229.1183+[2] | 591961 |
| | | | N [y6] - 369.7165++[3] | 366710 |
| | | | N [y5] - 624.3828+[4] | 103993 |
| | | | L [y2] - 260.1969+[5] | 75103 |
| | | | N [b4] - 228.6263++[6] | 66125 |
| | | | N [y6] - 738.4257+[7] | 49493 |
| | | | H [y4] - 510.3398+[8] | 43681 |
| | | | N [y5] - 312.6950++[9] | 41551 |
| | | | I [y3] - 373.2809+[10] | 40285 |
| | | | L [b3] - 342.2023+[11] | 33494 |
| | | | L [y8] - 482.8006++[12] | 33034 |
| transforming growth factor-beta-induced protein ig-h3 | K.AIISNK.D | 323.2001++ | I [y4] - 461.2718+[1] | 99850 |
| BGH3_HUMAN | | | I [b2] - 185.1285+[2] | 43105 |
| | | | S [y3] - 348.1878+[3] | 39192 |
| | | | N [y2] - 261.1557+[4] | 24516 |
| transforming growth factor-beta-induced protein ig-h3 | K.DILATNGVIHYIDELLI PDSAK.T | 804.1003+++ | P [y5] - 517.2617+[1] | 400251 |
| BGH3_HUMAN | | | I [b2] - 229.1183+[2] | 306709 |
| | | | L [b3] - 342.2023+[3] | 147923 |
| | | | I [y6] - 630.3457+[4] | 91265 |
| | | | S [y3] - 305.1819+[5] | 61472 |
| | | | L [y7] - 743.4298+[6] | 57894 |
| | | | A [b4] - 413.2395+[7] | 52430 |
| | | | H [y13] - 757.3985++[8] | 30183 |
| | | | G [y16] - 891.9855++[9] | 27711 |
| | | | D [y10] - 1100.5834+[10] | 24979 |
| | | | A [y19] - 1035.0493++[11] | 23223 |
| | | | L [y8] - 856.5138+[12] | 22507 |
| | | | L [y20] - 1091.5913++[13] | 16783 |
| transforming growth factor-beta-induced protein ig-h3 | K.TLFELAAESDVSTAID LFR.Q | 1049.5388++ | D [y4] - 550.2984+[1] | 64464 |
| BGH3_HUMAN | | | S [y8] - 922.4993+[2] | 47291 |
| | | | S [y11] - 1223.6266+[3] | 44234 |
| | | | A [b6] - 675.3712+[4] | 35972 |
| | | | L [b5] - 604.3341+[5] | 34997 |
| | | | A [b7] - 746.4083+[6] | 33045 |
| | | | E [b4] - 491.2500+[7] | 31744 |
| | | | D [ylO] - 1136.5946+[8] | 30183 |
| | | | E [b8] - 875.4509+[9] | 26475 |
| | | | F [y2] - 322.1874+[10] | 25044 |
| | | | T [y7] - 835.4672+[11] | 21596 |
| | | | I [y5] - 663.3824+[12] | 21011 |
| | | | L [y3] - 435.2714+[13] | 20295 |
| | | | L [b2] - 215.1390+[14] | 20295 |
| | | | V [y9] - 1021.5677+[15] | 18929 |
| | | | A [y6] - 734.4196+[16] | 17694 |
| | | | F [b3] - 362.2074+[17] | 14441 |
| transforming growth factor-beta-induced protein ig-h3 | R.QAGLGNHLSGSER.L | 442.5567+++ | G [y9] - 478.7309++[1] | 180677 |
| BGH3_HUMAN | | | L [y10] - 535.2729++[2] | 147807 |
| | | | S [y5] - 535.2471+[3] | 129825 |
| | | | G [y11] - 563.7836++[4] | 84584 |
| | | | L [y6] - 648.3311+[5] | 51642 |
| | | | A [b2] - 200.1030+[6] | 26469 |
| | | | G [y4] - 448.2150+[7] | 26397 |
| | | | H [y7] - 393.1987++[8] | 25390 |
| | | | A [y12] - 599.3022++[9] | 21434 |
| | | | N [y8] - 450.2201++[10] | 19276 |
| transforming growth factor-beta-induced protein ig-h3 | R.LTLLAPLNSVFK.D | 658.4028++ | P [y7] - 804.4614+[1] | 1635673 |
| BGH3_HUMAN | | | A [y8] - 875.4985+[2] | 869779 |
| | | | L [b3] - 328.2231+[3] | 516429 |
| | | | T [b2] - 215.1390+[4] | 415472 |
| | | | L [y9] - 988.5826+[5] | 334225 |
| | | | L [b4] - 441.3071+[6] | 209200 |
| | | | L [y10] - 1101.6667+[7] | 174268 |
| | | | A [b5] - 512.3443+[8] | 160217 |
| | | | A [y8] - 438.2529++[9] | 83264 |
| | | | N [y5] - 594.3246+[10] | 54512 |
| | | | F [y2] - 294.1812+[11] | 51649 |
| | | | L [y9] - 494.7949++[12] | 34541 |
| | | | L [y6] - 707.4087+[13] | 34086 |
| | | | S [y4] - 480.2817+[14] | 30053 |
| | | | T [y11] - 1202.7143+[15] | 16653 |
| transforming growth factor-beta-induced protein ig-h3 | K.DGTPPIDAHTR.N | 393.8633+++ | P [y8] - 453.7432++[1] | 355240 |
| BGH3_HUMAN | | | P [y7] - 405.2169++[2] | 88181 |
| | | | T [b3] - 274.1034+[3] | 81204 |
| | | | G [b2] - 173.0557+[4] | 40062 |
| | | | D [y5] - 599.2896+[5] | 37689 |
| | | | A [y4] - 242.6350++[6] | 29633 |
| | | | P [y7] - 809.4264+[7] | 22153 |
| | | | I [y6] - 712.3737+[8] | 16327 |
| transforming growth factor-beta-induced protein ig-h3 | K.YLYHGQTLETLGGK. K | 527.2753+++ | E [y6] - 604.3301+[1] | 483222 |
| BGH3_HUMAN | | | Y [y12] - 652.3357++[2] | 264640 |
| | | | T [y5] - 475.2875+[3] | 239600 |
| | | | G [y3] - 261.1557+[4] | 206272 |
| | | | L [b2] - 277.1547+[5] | 134992 |
| | | | L [y13] - 708.8777++[6] | 119379 |
| | | | T [b7] - 863.4046+[7] | 104307 |
| | | | L [y4] - 374.2398+[8] | 100344 |
| | | | H [y11] - 570.8040++[9] | 93318 |
| | | | L [y7] - 717.4141+[10] | 91276 |
| | | | G [b13] - 717.3566++[11] | 80707 |
| | | | T [y8] - 818.4618+[12] | 57888 |
| | | | Q [b6] - 762.3570+[13] | 54766 |
| | | | G [ylO] - 1003.5419+[14] | 51523 |
| | | | T [b7] - 432.2060++[15] | 49121 |
| | | | G [y2] - 204.1343+[16] | 45518 |
| | | | T [y8] - 409.7345++[17] | 44437 |
| | | | L [y7] - 359.2107++[18] | 33028 |
| | | | T [b10] - 603.7931++[19] | 26902 |
| | | | G [b5] - 634.2984+[20] | 21858 |
| | | | Q [b6] - 381.6821++[21] | 17595 |
| | | | H [b4] - 577.2769+[22] | 16093 |
| | | | L [b8] - 488.7480++[23] | 15133 |
| | | | T [y5] - 238.1474++[24] | 15013 |
| | | | E [b9] - 553.2693++[25] | 12370 |
| transforming growth factor-beta-induced protein ig-h3 | R.EGVYTVFAPTNEAFR .A | 850.9176++ | P [y7] - 834.4104+[1] | 364143 |
| BGH3_HUMAN | | | F [y9] - 1052.5160+[2] | 269144 |
| | | | A [y8] - 905.4476+[3] | 176007 |
| | | | V [b3] - 286.1397+[4] | 107490 |
| | | | V [y10] - 1151.5844+[5] | 74822 |
| | | | T [b5] - 550.2508+[6] | 47560 |
| | | | V [b6] - 649.3192+[7] | 45398 |
| | | | G [b2] - 187.0713+[8] | 43056 |
| | | | Y [b4] - 449.2031+[9] | 33148 |
| | | | F [b7] - 796.3876+[10] | 24440 |
| | | | A [b8] - 867.4247+[11] | 24020 |
| | | | E [y4] - 522.2671+[12] | 17174 |
| | | | A [y3] - 393.2245+[13] | 14712 |
| | | | F [y2] - 322.1874+[14] | 12611 |
| transforming growth factor-beta-induced protein ig-h3 | R.LLGDAK.E | 308.6869++ | A [y2] - 218.1499+[1] | 206606 |
| BGH3_HUMAN | | | G [y4] - 390.1983+[2] | 204445 |
| | | | L [y5] - 503.2824+[3] | 117829 |
| | | | L [b2] - 227.1754+[4] | 43998 |
| transforming growth factor-beta-induced protein ig-h3 | K.ELANILK.Y | 400.7475++ | A [y5] - 558.3610+[1] | 963502 |
| BGH3_HUMAN | | | L [y2] - 260.1969+[2] | 583986 |
| | | | N [y4] - 487.3239+[3] | 326252 |
| | | | I [y3] - 373.2809+[4] | 302352 |
| | | | I [b5] - 541.2980+[5] | 179670 |
| | | | L [b2] - 243.1339+[6] | 74642 |
| | | | L [y6] - 671.4450+[7] | 38792 |
| | | | N [b4] - 428.2140+[8] | 14952 |
| transforming growth factor-beta-induced protein ig-h3 | K.YHIGDEILVSGGIGAL VR.L | 935.0151++ | H [b2] - 301.1295+[1] | 24601 |
| BGH3_HUMAN | | | S [y9] - 829.4890+[2] | 15456 |
| transforming growth factor-beta-induced protein ig-h3 | K.YHIGDEILVSGGIGAL VR.L | 623.6791+++ | S [y9] - 829.4890+[1] | 917445 |
| BGH3_HUMAN | | | G [y5] - 515.3300+[2] | 654048 |
| | | | I [b7] - 828.3886+[3] | 553713 |
| | | | G [y8] - 742.4570+[4] | 467481 |
| | | | L [b8] - 941.4727+[5] | 322194 |
| | | | G [y7] - 685.4355+[6] | 228428 |
| | | | E [b6] - 715.3046+[7] | 199383 |
| | | | V [y10] - 928.5574+[8] | 141616 |
| | | | G [b4] - 471.2350+[9] | 126224 |
| | | | L [b8] - 471.2400++[10] | 117080 |
| | | | H [b2] - 301.1295+[11] | 107162 |
| | | | I [y6] - 628.4141+[12] | 105488 |
| | | | A [y4] - 458.3085+[13] | 103491 |
| | | | L [y3] - 387.2714+[14] | 73094 |
| | | | I [b3] - 414.2136+[15] | 72515 |
| | | | S [y9] - 415.2482++[16] | 65044 |
| | | | V [b9] - 1040.5411+[17] | 61760 |
| | | | V [y2] - 274.1874+[19] | 56093 |
| | | | I [b7] - 414.6980++[18] | 56093 |
| | | | V [b9] - 520.7742++[20] | 39413 |
| | | | L [y11] - 1041.6415+[21] | 38962 |
| | | | D [b5] - 586.2620+[22] | 36257 |
| | | | S [b10] - 564.2902++[23] | 32329 |
| | | | I [y6] - 314.7107++[24] | 30526 |
| | | | A [b15] - 741.8830++[25] | 27692 |
| | | | V [y10] - 464.7824++[26] | 26340 |
| | | | L [y11] - 521.3244++[27] | 20415 |
| | | | G [b12] - 621.3117++[28] | 18612 |
| | | | G [b12] - 1241.6161+[29] | 13073 |
| transforming growth factor-beta-induced protein ig-h3 | K.LEVSLK.N | 344.7156++ | V [y4] - 446.2973+[1] | 120860 |
| BGH3_HUMAN | | | E [y5] - 575.3399+[2] | 82786 |
| | | | E [b2] - 243.1339+[3] | 76794 |
| | | | S [y3] - 347.2289+[4] | 36335 |
| | | | L [y2] - 260.1969+[5] | 24932 |
| transforming growth factor-beta-induced protein ig-h3 | K.NNVVSVNK.E | 437.2431++ | V [y5] - 546.3246+[1] | 17073 |
| BGH3_HUMAN | | | N [b2] - 229.0931+[2] | 14045 |
| transforming growth factor-beta-induced protein ig-h3 | R.GDELADSALEIFK.Q | 704.3537++ | E [b3] - 302.0983+[1] | 687754 |
| BGH3_HUMAN | | | A [y9] - 993.5251+[2] | 431716 |
| | | | D [y8] - 922.4880+[3] | 368670 |
| | | | D [b2] - 173.0557+[4] | 358545 |
| | | | F [y2] - 294.1812+[5] | 200930 |
| | | | L [b4] - 415.1823+[6] | 197364 |
| | | | S [y7] - 807.4611+[7] | 187412 |
| | | | I [y3] - 407.2653+[8] | 129601 |
| | | | A [b5] - 486.2195+[9] | 121605 |
| | | | E [y4] - 536.3079+[10] | 108432 |
| | | | A [y6] - 720.4291+[11] | 107627 |
| | | | L [y5] - 649.3919+[12] | 95662 |
| | | | L [y10] - 1106.6092+[13] | 79325 |
| | | | D [b6] - 601.2464+[14] | 42625 |
| | | | A [b8] - 759.3155+[15] | 28647 |
| | | | S [b7] - 688.2784+[16] | 20709 |
| transforming growth factor-beta-induced protein ig-h3 | K.QASAFSR.A | 383.6958++ | F [y3] - 409.2194+[1] | 64604 |
| BGH3_HUMAN | | | S [y5] - 567.2885+[2] | 60496 |
| | | | S [y2] - 262.1510+[3] | 42825 |
| | | | A [y4] - 480.2565+[4] | 25211 |
| transforming growth factor-beta-induced protein ig-h3 | R.LAPVYQK.L | 409.7422++ | P [y5] - 634.3559+[1] | 416225 |
| BGH3_HUMAN | | | Y [y3] - 438.2347+[2] | 171715 |
| | | | V [y4] - 537.3031+[3] | 98187 |
| | | | Q [y2] - 275.1714+[4] | 42056 |
| | | | A [y6] - 705.3930+[5] | 32429 |
| ceruloplasmin | K.LISVDTEHSNIYLQNG PDR.I | 724.3624+++ | I [b2] - 227.1754+[1] | 168111 |
| CERU_HUMAN | | | N [y5] - 558.2630+[2] | 87133 |
| | | | G [y4] - 444.2201+[3] | 86682 |
| | | | L [y7] - 799.4057+[4] | 84956 |
| | | | Q [y6] - 686.3216+[5] | 79928 |
| | | | Y [y8] - 962.4690+[6] | 64167 |
| | | | S [b3] - 314.2074+[7] | 39476 |
| | | | N [y10] - 1189.5960+[8] | 24691 |
| | | | P [y3] - 387.1987+[9] | 22065 |
| | | | I [y18] - 1029.4980++[10] | 20714 |
| | | | N [b10] - 1096.5269+[11] | 18087 |
| | | | I [y9] - 1075.5531+[12] | 15460 |
| ceruloplasmin | K.ALYLQYTDETFR.T | 760.3750++ | Y [b3] - 348.1918+[1] | 681082 |
| CERU_HUMAN | | | Y [y7] - 931.4156+[2] | 405797 |
| | | | Q [y8] - 1059.4742+[3] | 343430 |
| | | | T [y6] - 768.3523+[4] | 279638 |
| | | | L [b2] - 185.1285+[5] | 229654 |
| | | | L [y9] - 1172.5582+[6] | 164660 |
| | | | L [b4] - 461.2758+[7] | 142145 |
| | | | D [y5] - 667.3046+[8] | 107547 |
| | | | Y [y10] - 668.3144++[9] | 91862 |
| | | | E [y4] - 552.2776+[10] | 76852 |
| | | | Q [b5] - 589.3344+[11] | 75200 |
| | | | T [y3] - 423.2350+[12] | 64168 |
| | | | F [y2] - 322.1874+[13] | 47807 |
| | | | Y [b6] - 752.3978+[14] | 40377 |
| | | | L [y9] - 586.7828++[15] | 40227 |
| ceruloplasmin | R.TTIEKPVWLGFLGPII K.A | 956.5690++ | E [b4] - 445.2293+[1] | 92012 |
| CERU_HUMAN | | | K [b5] - 573.3243+[2] | 45856 |
| | | | L [y9] - 957.6132+[3] | 32272 |
| | | | G [y8] - 844.5291+[4] | 29044 |
| | | | K [y13] - 734.4579++[5] | 26118 |
| | | | G [y5] - 527.3552+[6] | 24917 |
| | | | L [y6] - 640.4392+[7] | 19738 |
| | | | I [b3] - 316.1867+[8] | 18838 |
| | | | P [y4] - 470.3337+[9] | 18012 |
| | | | W [y10] - 1143.6925+[10] | 17412 |
| | | | I [y15] - 855.5213++[11] | 14785 |
| | | | V [b7] - 769.4454+[12] | 14710 |
| ceruloplasmin | R.TTIEKPVWLGFLGPII K.A | 638.0484+++ | G [y8] - 844.5291+[1] | 1645779 |
| CERU_HUMAN | | | G [y5] - 527.3552+[2] | 1180842 |
| | | | L [y6] - 640.4392+[3] | 920117 |
| | | | T [b2] - 203.1026+[4] | 775570 |
| | | | F [y7] - 787.5076+[5] | 416229 |
| | | | P [y4] - 470.3337+[6] | 285341 |
| | | | W [b8] - 955.5247+[7] | 275960 |
| | | | I [y2] - 260.1969+[8] | 256597 |
| | | | V [b7] - 769.4454+[9] | 230104 |
| | | | E [b4] - 445.2293+[10] | 117754 |
| | | | W [b8] - 478.2660++[11] | 105521 |
| | | | P [y12] - 670.4105++[13] | 104020 |
| | | | P [b6] - 670.3770+[12] | 104020 |
| | | | G [b10] - 1125.6303+[14] | 93363 |
| | | | F [y7] - 394.2575++[15] | 76176 |
| | | | K [b5] - 573.3243+[16] | 63718 |
| | | | I [b3] - 316.1867+[17] | 52986 |
| | | | L [b9] - 1068.6088+[18] | 33548 |
| | | | I [y3] - 373.2809+[19] | 20864 |
| ceruloplasmin | K.VYVHLK.N | 379.7316++ | V [y4] - 496.3242+[1] | 228979 |
| CERU_HUMAN | | | Y [y5] - 659.3875+[2] | 196857 |
| | | | H [y3] - 397.2558+[3] | 89610 |
| | | | Y [b2] - 263.1390+[4] | 88034 |
| | | | L [y2] - 260.1969+[5] | 85482 |
| | | | Y [y5] - 330.1974++[6] | 31821 |
| ceruloplasmin | R.IYHSHIDAPK.D | 590.8091++ | H [y8] - 452.7354++[1] | 167209 |
| CERU_HUMAN | | | P [y2] - 244.1656+[2] | 84831 |
| | | | A [y3] - 315.2027+[3] | 78036 |
| | | | S [y7] - 767.4046+[4] | 75864 |
| | | | H [b3] - 414.2136+[5] | 67808 |
| | | | Y [y9] - 534.2671++[6] | 50296 |
| | | | H [y8] - 904.4635+[7] | 42801 |
| | | | D [b7] - 866.4155+[8] | 28721 |
| | | | H [y6] - 680.3726+[9] | 23817 |
| | | | A [b8] - 937.4526+[10] | 19964 |
| | | | D [y4] - 430.2296+[11] | 17653 |
| | | | Y [b2] - 277.1547+[12] | 16742 |
| ceruloplasmin | R.IYHSHIDAPK.D | 394.2085+++ | H [y8] - 452.7354++[1] | 402227 |
| CERU_HUMAN | | | Y [y9] - 534.2671++[2] | 305348 |
| | | | P [y2] - 244.1656+[5] | 101993 |
| | | | A [y3] - 315.2027+[3] | 97580 |
| | | | Y [b2] - 277.1547+[4] | 93377 |
| | | | D [y4] - 430.2296+[6] | 89734 |
| | | | S [y7] - 767.4046+[7] | 88263 |
| | | | S [y7] - 384.2060++[8] | 60663 |
| | | | I [y5] - 543.3137+[9] | 44692 |
| | | | H [y6] - 680.3726+[11] | 38528 |
| | | | A [b8] - 469.2300++[10] | 37547 |
| | | | H [b5] - 638.3045+[12] | 36146 |
| | | | H [b3] - 414.2136+[13] | 23467 |
| ceruloplasmin | R.HYYIAAEEIIWNYAPS GIDIFTK.E | 905.4549+++ | P [y9] - 977.5302+[1] | 253794 |
| CERU_HUMAN | | | E [b8] - 977.4363+[2] | 233479 |
| | | | Y [b2] - 301.1295+[3] | 128823 |
| | | | I [b9] - 1090.5204+[4] | 103955 |
| | | | A [y10] - 1048.5673+[5] | 78247 |
| | | | P [y9] - 489.2687++[6] | 76005 |
| | | | E [b8] - 489.2218++[7] | 76005 |
| | | | I [b10] - 1203.6045+[8] | 56671 |
| | | | F [y3] - 395.2289+[9] | 49456 |
| | | | Y [b3] - 464.1928+[10] | 46864 |
| | | | E [b7] - 848.3937+[11] | 44622 |
| | | | A [b5] - 648.3140+[12] | 42451 |
| | | | A [b6] - 719.3511+[13] | 40629 |
| | | | I [b4] - 577.2769+[14] | 39999 |
| | | | D [y5] - 623.3399+[15] | 29631 |
| | | | I [y4] - 508.3130+[16] | 28581 |
| | | | T [y2] - 248.1605+[17] | 27040 |
| | | | I [b10] - 602.3059++[18] | 24448 |
| | | | Y [y11] - 1211.6307+[19] | 24238 |
| | | | G [y7] - 793.4454+[20] | 21926 |
| | | | W [b11] - 695.3455++[21] | 18704 |
| | | | S [y8] - 880.4775+[22] | 18633 |
| ceruloplasmin | R.IGGSYK.K | 312.6712++ | G [y5] - 511.2511+[1] | 592392 |
| CERU_HUMAN | | | G [y4] - 454.2296+[2] | 89266 |
| | | | G [b2] - 171.1128+[3] | 71261 |
| | | | Y [y2] - 310.1761+[4] | 52498 |
| | | | S [y3] - 397.2082+[5] | 22364 |
| ceruloplasmin | R.EYTDASFTNR.K | 602.2675++ | S [y5] - 624.3100+[1] | 163623 |
| CERU_HUMAN | | | F [y4] - 537.2780+[2] | 83580 |
| | | | T [y8] - 911.4217+[3] | 83391 |
| | | | A [y6] - 695.3471+[4] | 82886 |
| | | | D [y7] - 810.3741+[5] | 76315 |
| | | | T [y3] - 390.2096+[6] | 66018 |
| | | | Y [b2] - 293.1132+[7] | 50224 |
| | | | N [y2] - 289.1619+[8] | 29376 |
| ceruloplasmin | R.GPEEEHLGILGPVIW AEVGDTIR.V | 829.7675+++ | A [y8] - 860.4472+[1] | 259776 |
| CERU_HUMAN | | | W [y9] - 1046.5265+[2] | 210032 |
| | | | E [y7] - 789.4101+[3] | 201448 |
| | | | G [y5] - 561.2991+[4] | 189809 |
| | | | V [y6] - 660.3675+[5] | 121142 |
| | | | T [y3] - 389.2507+[6] | 80306 |
| | | | P [b2] - 155.0815+[7] | 65806 |
| | | | V [b13] - 664.8459++[8] | 65676 |
| | | | G [b11] - 1132.5633+[9] | 64765 |
| | | | I [y10] - 1159.6106+[10] | 58783 |
| | | | L [b10] - 1075.5419+[11] | 56702 |
| | | | I [b9] - 962.4578+[12] | 54101 |
| | | | L [b7] - 792.3523+[13] | 48509 |
| | | | P [b12] - 615.3117++[14] | 37715 |
| | | | D [y4] - 504.2776+[15] | 34528 |
| | | | G [b8] - 849.3737+[16] | 34008 |
| | | | I [b14] - 721.3879++[17] | 23669 |
| | | | H [b6] - 679.2682+[18] | 22174 |
| | | | W [b15] - 814.4276++[19] | 21979 |
| | | | E [b3] - 284.1241+[20] | 18272 |
| | | | G [b11] - 566.7853++[21] | 17882 |
| | | | A [b16] - 849.9461++[22] | 15476 |
| ceruloplasmin | R.VTFHNK.G | 373.2032++ | T [y5] - 646.3307+[1] | 178952 |
| CERU_HUMAN | | | F [y4] - 545.2831+[2] | 175829 |
| | | | T [b2] - 201.1234+[3] | 127758 |
| | | | N [y2] - 261.1557+[4] | 107852 |
| | | | H [y3] - 398.2146+[5] | 103754 |
| ceruloplasmin | K.GAYPLSIEPIGVR.F | 686.3852++ | S [y8] - 870.5043+[1] | 970541 |
| CERU_HUMAN | | | P [y5] - 541.3457+[2] | 966508 |
| | | | P [y10] - 1080.6412+[3] | 590391 |
| | | | E [y6] - 670.3883+[4] | 493076 |
| | | | I [y7] - 783.4723+[5] | 391013 |
| | | | Y [b3] - 292.1292+[6] | 265598 |
| | | | L [y9] - 983.5884+[7] | 217591 |
| | | | P [b4] - 389.1819+[8] | 188839 |
| | | | S [b6] - 589.2980+[9] | 95623 |
| | | | G [y3] - 331.2088+[10] | 85605 |
| | | | L [b5] - 502.2660+[11] | 76628 |
| | | | V [y2] - 274.1874+[12] | 52365 |
| | | | I [b7] - 702.3821+[13] | 39225 |
| | | | E [b8] - 831.4247+[14] | 26866 |
| ceruloplasmin | K.NNEGTYYSPNYNPQ SR.S | 952.4139++ | P [y4] - 487.2623+[1] | 37339 |
| CERU_HUMAN | | | S [y9] - 1062.4963+[2] | 33696 |
| | | | P [y8] - 975.4643+[3] | 29467 |
| | | | N [y5] - 601.3052+[4] | 24068 |
| | | | N [b2] - 229.0931+[5] | 19060 |
| | | | Y [y10] - 1225.5596+[6] | 16718 |
| | | | E [b3] - 358.1357+[7] | 16523 |
| ceruloplasmin | R.SVPPSASHVAPTETF TYEWTVPK.E | 844.4199+++ | P [y2] - 244.1656+[1] | 579331 |
| CERU_HUMAN | | | T [y8] - 1023.5146+[2] | 126817 |
| | | | W [y5] - 630.3610+[3] | 101524 |
| | | | V [y3] - 343.2340+[4] | 99970 |
| | | | Y [y7] - 922.4669+[5] | 95448 |
| | | | E [y6] - 759.4036+[6] | 88030 |
| | | | T [y4] - 444.2817+[7] | 55884 |
| | | | F [y9] - 1170.5830+[8] | 55743 |
| | | | V [b2] - 187.1077+[9] | 46982 |
| | | | P [y20] - 1124.5497++[10] | 37303 |
| | | | P [b3] - 284.1605+[11] | 21690 |
| | | | E [b18] - 951.4494++[12] | 18652 |
| | | | P [b4] - 381.2132+[13] | 16956 |
| | | | T [b14] - 681.3384++[14] | 15543 |
| ceruloplasmin | K.GSLHANGR.Q | 271.1438+++ | L [y6] - 334.1854++[1] | 154779 |
| CERU_HUMAN | | | A [y4] - 417.2205+[2] | 41628 |
| | | | S [y7] - 377.7014++[3] | 35762 |
| | | | H [y5] - 277.6433++[4] | 29542 |
| ceruloplasmin | R.QSEDSTFYLGER.T | 716.3230++ | G [y3] - 361.1830+[1] | 157040 |
| CERU_HUMAN | | | Y [y5] - 637.3304+[2] | 126155 |
| | | | F [y6] - 784.3988+[3] | 97814 |
| | | | L [y4] - 474.2671+[4] | 80146 |
| | | | T [y7] - 443.2269++[5] | 70746 |
| | | | T [y7] - 885.4465+[6] | 54844 |
| | | | S [y8] - 972.4785+[7] | 44101 |
| | | | S [b2] - 216.0979+[8] | 42193 |
| | | | D [y9] - 1087.5055+[9] | 36186 |
| | | | E [y10] - 1216.5481+[10] | 35055 |
| | | | E [b3] - 345.1405+[11] | 20778 |
| | | | E [y2] - 304.1615+[12] | 19153 |
| ceruloplasmin | R.TYYIAAVEVEWDYSP QR.E | 1045.4969++ | P [y3] - 400.2303+[1] | 64887 |
| CERU_HUMAN | | | Y [b3] - 428.1816+[2] | 49716 |
| | | | S [y4] - 487.2623+[3] | 37369 |
| | | | Y [b2] - 265.1183+[4] | 35596 |
| | | | E [y8] - 1080.4745+[5] | 28569 |
| | | | W [y7] - 951.4319+[6] | 26204 |
| | | | V [b7] - 782.4083+[7] | 23577 |
| | | | A [b6] - 683.3399+[8] | 23512 |
| | | | V [y9] - 1179.5429+[10] | 22526 |
| | | | D [y6] - 765.3526+[9] | 22526 |
| | | | Y [y5] - 650.3257+[11] | 19965 |
| | | | A [b5] - 612.3028+[12] | 18520 |
| ceruloplasmin | K.ELHHLQEQNVSNAF LDK.G | 674.6728+++ | N [y6]- 707.3723+[1] | 22715 |
| CERU_HUMAN | | | L [y3] - 188.1155++[2] | 21336 |
| | | | S [y7] - 794.4043+[3] | 10176 |
| ceruloplasmin | K.GEFYIGSK.Y | 450.7267++ | E [b2] - 187.0713+[1] | 53262 |
| CERU_HUMAN | | | F [y6] - 714.3821+[2] | 50438 |
| | | | I [y4] - 404.2504+[3] | 39602 |
| | | | Y [y5] - 567.3137+[4] | 34020 |
| | | | G [y3] - 291.1663+[5] | 33100 |
| ceruloplasmin | R.QYTDSTFR.V | 509.2354++ | T [y6] - 726.3417+[1] | 164056 |
| CERU_HUMAN | | | S [y4] - 510.2671+[2] | 155584 |
| | | | D [y5] - 625.2940+[3] | 136472 |
| | | | T [y3] - 423.2350+[4] | 54313 |
| | | | F [y2] - 322.1874+[5] | 47220 |
| | | | Y [b2] - 292.1292+[6] | 27846 |
| | | | Y [y7] - 889.4050+[7] | 16550 |
| ceruloplasmin | K.AEEEHLGILGPQLHA DVGDK.V | 710.0272+++ | E [b2] - 201.0870+[1] | 60743 |
| CERU_HUMAN | | | V [y4] - 418.2296+[2] | 23296 |
| | | | E [y17] - 899.9759++[3] | 14619 |
| ceruloplasmin | K.LEFALLFLVFDENES WYLDDNIK.T | 945.1372+++ | L [y6] - 359.1925++[1] | 19544 |
| CERU_HUMAN | | | L [b5] - 574.3235+[2] | 17902 |
| ceruloplasmin | K.TYSDHPEK.V | 488.7222++ | S [y6] - 712.3260+[1] | 93810 |
| CERU_HUMAN | | | P [y3] - 373.2082+[2] | 43778 |
| | | | Y [b2] - 265.1183+[3] | 35960 |
| | | | H [y4] - 510.2671+[4] | 16651 |
| ceruloplasmin | K.TYSDHPEK.V | 326.1505+++ | S [y6] - 356.6667++[1] | 539251 |
| CERU_HUMAN | | | Y [y7] - 438.1983++[2] | 180506 |
| | | | Y [b2] - 265.1183+[3] | 109445 |
| | | | P [y3] - 373.2082+[4] | 84742 |
| | | | H [y4] - 255.6372++[5] | 27596 |
| | | | P [y3] - 187.1077++[6] | 25016 |
| | | | D [y5] - 625.2940+[7] | 24000 |
| | | | H [y4] - 510.2671+[8] | 20795 |
| hepatocyte growth factor activator | R.YEYLEGGDR.W | 551.2460++ | E [b2] - 293.1132+[1] | 229354 |
| HGFA_HUMAN | | | Y [y7] - 809.3788+[2] | 204587 |
| | | | L [y6] - 646.3155+[3] | 96740 |
| | | | Y [b3] - 456.1765+[4] | 54186 |
| | | | E [y8] - 938.4214+[5] | 22065 |
| hepatocyte growth factor activator | R.VQLSPDLLATLPEPA SPGR.Q | 981.0387++ | P [y8] - 810.4104+[1] | 51109 |
| HGFA_HUMAN | | | Q [b2] - 228.1343+[2] | 19063 |
| hepatocyte growth factor activator | R.TTDVTQTFGIEK.Y | 670.3406++ | D [b3] - 318.1296+[1] | 104844 |
| HGFA_HUMAN | | | T [y8] - 923.4833+[2] | 93287 |
| | | | T [b2] - 203.1026+[3] | 72498 |
| | | | D [y10] - 1137.5786+[4] | 53886 |
| | | | I [y3] - 389.2395+[5] | 53811 |
| | | | Q [y7] - 822.4356+[6] | 42253 |
| | | | V [b4] - 417.1980+[7] | 38726 |
| | | | T [y6] - 694.3770+[8] | 36474 |
| | | | F [y5] - 593.3293+[9] | 26793 |
| | | | E [y2] - 276.1554+[10] | 24616 |
| | | | G [y4] - 446.2609+[11] | 22215 |
| | | | V [y9] - 1022.5517+[12] | 20564 |
| hepatocyte growth factor activator | R.EALVPLVADHK.C | 596.3402++ | P [y7] - 779.4410+[1] | 57992 |
| HGFA_HUMAN | | | L [b3] - 314.1710+[2] | 42740 |
| hepatocyte growth factor activator | R.EALVPLVADHK.C | 397.8959+++ | P [y7] - 390.2241++[1] | 502380 |
| HGFA_HUMAN | | | V [y5] - 569.3042+[2] | 108586 |
| | | | V [y8] - 439.7584++[3] | 100001 |
| | | | H [y2] - 284.1717+[4] | 71234 |
| | | | L [y9] - 496.3004++[5] | 65572 |
| | | | A [y4] - 470.2358+[6] | 62284 |
| hepatocyte growth factor activator | R.LHKPGVYTR.V | 357.5417+++ | P [y6] - 692.3726+[1] | 104812 |
| HGFA_HUMAN | | | H [y8] - 479.2669++[2] | 49302 |
| | | | K [y7] - 410.7374++[3] | 30859 |
| | | | Y [y3] - 439.2300+[4] | 23829 |
| hepatocyte growth factor activator | R.VANYVDWINDR.I | 682.8333++ | D [y6] - 818.3791+[1] | 132314 |
| HGFA_HUMAN | | | V [y7] - 917.4476+[2] | 81805 |
| | | | N [b3] - 285.1557+[3] | 70622 |
| | | | W [y5] - 703.3522+[4] | 53586 |
| | | | N [y3] - 404.1888+[5] | 37675 |
| | | | A [b2] - 171.1128+[6] | 36474 |
| alpha-1-antichymotrypsin | R.GTHVDLGLASANVD FAFSLYK.Q | 1113.0655++ | L [b6] - 623.3148+[1] | 244118 |
| AACT_HUMAN | | | L [b8] - 793.4203+[2] | 211429 |
| | | | H [b3] - 296.1353+[3] | 204581 |
| | | | D [b5] - 510.2307+[4] | 200032 |
| | | | S [y4] - 510.2922+[5] | 195904 |
| | | | V [b4] - 395.2037+[6] | 187415 |
| | | | A [b9] - 864.4574+[7] | 167905 |
| | | | G [b7] - 680.3362+[8] | 87564 |
| | | | Y [y2] - 310.1761+[9] | 74385 |
| | | | F [y7] - 875.4662+[10] | 50794 |
| | | | F [y5] - 657.3606+[11] | 44462 |
| | | | S [b10] - 951.4894+[12] | 43899 |
| | | | D [y8] - 990.4931+[13] | 39866 |
| | | | A [y6] - 728.3978+[14] | 33300 |
| | | | A [b11] - 1022.5265+[15] | 32502 |
| | | | L [y3] - 423.2602+[16] | 29829 |
| | | | V [y9] - 1089.5615+[17] | 22043 |
| | | | N [b12] - 1136.5695+[18] | 17353 |
| alpha-1-antichymotrypsin | R.GTHVDLGLASANVD FAFSLYK.Q | 742.3794+++ | D [y8] - 990.4931+[1] | 830612 |
| AACT_HUMAN | | | L [b8] - 793.4203+[2] | 635646 |
| | | | G [b7] - 680.3362+[3] | 582273 |
| | | | S [y4] - 510.2922+[4] | 548645 |
| | | | D [b5] - 510.2307+[5] | 471071 |
| | | | F [y7] - 875.4662+[6] | 420278 |
| | | | A [b9] - 864.4574+[7] | 411366 |
| | | | A [y6] - 728.3978+[8] | 391668 |
| | | | Y [y2] - 310.1761+[9] | 390214 |
| | | | F [y5] - 657.3606+[10] | 358134 |
| | | | T [b2] - 159.0764+[11] | 288721 |
| | | | H [b3] - 296.1353+[12] | 251998 |
| | | | L [b6] - 623.3148+[13] | 240742 |
| | | | V [y9] - 1089.5615+[14] | 197218 |
| | | | V [b4] - 395.2037+[15] | 186055 |
| | | | L [y3] - 423.2602+[16] | 173673 |
| | | | S [b10] - 951.4894+[17] | 103651 |
| | | | N [b12] - 1136.5695+[18] | 97976 |
| | | | A [b11] - 1022.5265+[19] | 76448 |
| alpha-1-antichymotrypsin | K.FNLTETSEAEIHQSFQ HLLR.T | 800.7363+++ | A [b9] - 993.4524+[1] | 75792 |
| AACT_HUMAN | | | L [b3] - 375.2027+[2] | 59001 |
| | | | H [y9] - 1165.6225+[3] | 57829 |
| | | | L [y2] - 288.2030+[4] | 55343 |
| | | | T [b4] - 476.2504+[5] | 19323 |
| alpha-1-antichymotrypsin | K.EQLSLLDR.F | 487.2693++ | S [y5] - 603.3461+[1] | 4247034 |
| AACT_HUMAN | | | L [y3] - 403.2300+[2] | 2094711 |
| | | | L [y6] - 716.4301+[3] | 1465135 |
| | | | L [y4] - 516.3140+[4] | 1365427 |
| | | | Q [b2] - 258.1084+[5] | 1222196 |
| | | | D [y2] - 290.1459+[6] | 957403 |
| | | | L [b3] - 371.1925+[7] | 114810 |
| alpha-1-antichymotrypsin | K.EQLSLLDR.F | 325.1819+++ | L [y3] - 403.2300+[1] | 57123 |
| AACT_HUMAN | | | D [y2] - 290.1459+[2] | 52105 |
| alpha-1-antichymotrypsin | K.YTGNASALFILPDQD K.M | 876.9438++ | L [y9] - 1088.5986+[1] | 39933 |
| AACT_HUMAN | | | A [b5] - 507.2198+[2] | 20117 |
| | | | D [y4] - 505.2253+[3] | 19937 |
| alpha-1-antichymotrypsin | R.EIGELYLPK.F | 531.2975++ | P [y2] - 244.1656+[1] | 8170395 |
| AACT_HUMAN | | | G [y7] - 819.4611+[2] | 3338199 |
| | | | L [y5] - 633.3970+[3] | 2616703 |
| | | | L [y3] - 357.2496+[4] | 1922561 |
| | | | Y [y4] - 520.3130+[5] | 1527792 |
| | | | G [b3] - 300.1554+[6] | 1417240 |
| | | | I [b2] - 243.1339+[7] | 1097654 |
| | | | E [y6] - 762.4396+[8] | 302412 |
| | | | E [b4] - 429.1980+[9] | 81633 |
| | | | Y [b6] - 705.3454+[10] | 36795 |
| | | | L [b5] - 542.2821+[11] | 31993 |
| alpha-1-antichymotrypsin | R.EIGELYLPK.F | 354.5341+++ | P [y2] - 244.1656+[1] | 189758 |
| AACT_HUMAN | | | L [y3] - 357.2496+[2] | 86952 |
| | | | G [b3] - 300.1554+[3] | 49661 |
| | | | Y [y4] - 520.3130+[4] | 45518 |
| | | | E [b4] - 429.1980+[5] | 19576 |
| | | | I [b2] - 243.1339+[6] | 18375 |
| | | | L [b5] - 542.2821+[7] | 13091 |
| alpha-1-antichymotrypsin | R.DYNLNDILLQLGIEEA FTSK.A | 1148.5890++ | G [y9] - 981.4888+[1] | 378153 |
| AACT_HUMAN | | | F [b17] - 981.4964++[2] | 378153 |
| | | | N [b3] - 393.1405+[3] | 338897 |
| | | | L [y10] - 1094.5728+[4] | 283255 |
| | | | E [y7] - 811.3832+[5] | 180253 |
| | | | I [b7] - 848.3785+[6] | 172510 |
| | | | T [y3] - 335.1925+[7] | 162966 |
| | | | D [b6] - 735.2944+[8] | 135235 |
| | | | L [b4] - 506.2245+[9] | 131573 |
| | | | A [y5] - 553.2980+[10] | 129232 |
| | | | F [y4] - 482.2609+[11] | 124490 |
| | | | Y [b2] - 279.0975+[12] | 115367 |
| | | | L [b9] - 1074.5466+[13] | 106363 |
| | | | L [b8] - 961.4625+[14] | 101621 |
| | | | E [y6] - 682.3406+[15] | 98740 |
| | | | S [y2] - 234.1448+[16] | 75991 |
| | | | N [b5] - 620.2675+[17] | 66387 |
| | | | I [y8] - 924.4673+[18] | 61465 |
| alpha-1-antichymotrypsin | R.DYNLNDILLQLGIEEA FTSK.A | 766.0618+++ | G [y9] - 981.4888+[1] | 309485 |
| AACT_HUMAN | | | F [b17] - 981.4964++[2] | 309485 |
| | | | E [y7] - 811.3832+[3] | 262306 |
| | | | N [b3] - 393.1405+[4] | 212306 |
| | | | T [y3] - 335.1925+[5] | 199100 |
| | | | F [y4] - 482.2609+[6] | 164346 |
| | | | A [y5] - 553.2980+[7] | 161405 |
| | | | Y [b2] - 279.0975+[8] | 149220 |
| | | | E [y6] - 682.3406+[9] | 138836 |
| | | | L [y10] - 1094.5728+[10] | 137336 |
| | | | S [y2] - 234.1448+[11] | 134094 |
| | | | I [b7] - 848.3785+[12] | 80072 |
| | | | I [y8] - 924.4673+[13] | 77791 |
| | | | L [b4] - 506.2245+[14] | 70889 |
| | | | D [b6] - 735.2944+[15] | 64706 |
| | | | L [b8] - 961.4625+[16] | 51201 |
| | | | N [b5] - 620.2675+[17] | 42677 |
| | | | L [b9] - 1074.5466+[18] | 21609 |
| alpha-1-antichymotrypsin | K.ADLSGITGAR.N | 480.7591++ | S [y7] - 661.3628+[1] | 4360743 |
| AACT_HUMAN | | | G [y6] - 574.3307+[2] | 3966462 |
| | | | T [y4] - 404.2252+[3] | 1937824 |
| | | | D [b2] - 187.0713+[4] | 799907 |
| | | | G [y3] - 303.1775+[5] | 647883 |
| | | | I [y5] - 517.3093+[6] | 612145 |
| | | | L [b3] - 300.1554+[7] | 606995 |
| | | | S [b4] - 387.1874+[8] | 544408 |
| | | | L [y8] - 774.4468+[9] | 348247 |
| | | | G [b5] - 444.2089+[10] | 232083 |
| | | | I [b6] - 557.2930+[11] | 132531 |
| | | | A [y2] - 246.1561+[12] | 113896 |
| alpha-1-antichymotrypsin | K.ADLSGITGAR.N | 320.8418+++ | T [y4] - 404.2252+[1] | 218597 |
| AACT_HUMAN | | | G [y3] - 303.1775+[2] | 159381 |
| | | | G [b5] - 444.2089+[3] | 46527 |
| | | | A [y2] - 246.1561+[4] | 26911 |
| | | | D [b2] - 187.0713+[5] | 22497 |
| | | | S [b4] - 387.1874+[6] | 14589 |
| alpha-1-antichymotrypsin | R.NLAVSQVVHK.A | 547.8195++ | L [b2] - 228.1343+[1] | 1872233 |
| AACT_HUMAN | | | A [y8] - 867.5047+[2] | 1133381 |
| | | | A [b3] - 299.1714+[3] | 1126331 |
| | | | V [y7] - 796.4676+[4] | 672341 |
| | | | S [y6] - 697.3991+[5] | 650028 |
| | | | H [y2] - 284.1717+[6] | 582720 |
| | | | V [y3] - 383.2401+[7] | 211547 |
| | | | V [b4] - 398.2398+[8] | 163917 |
| | | | Q [y5] - 610.3671+[9] | 100778 |
| | | | V [y4] - 482.3085+[10] | 88456 |
| | | | S [b5] - 485.2718+[11] | 64488 |
| | | | V [b7] - 712.3988+[12] | 36045 |
| alpha-1-antichymotrypsin | R.NLAVSQVVHK.A | 365.5487+++ | L [b2] - 228.1343+[1] | 1175923 |
| AACT_HUMAN | | | V [y3] - 383.2401+[2] | 593693 |
| | | | S [y6] - 697.3991+[3] | 587502 |
| | | | H [y2] - 284.1717+[4] | 440259 |
| | | | V [y4] - 482.3085+[5] | 375955 |
| | | | Q [y5] - 610.3671+[6] | 349044 |
| | | | A [b3] - 299.1714+[7] | 339236 |
| | | | V [b4] - 398.2398+[8] | 172805 |
| | | | S [b5] - 485.2718+[9] | 84594 |
| alpha-1-antichymotrypsin | K.AVLDVFEEGTEASAA TAVK.I | 954.4835++ | D [b4] - 399.2238+[1] | 1225699 |
| AACT_HUMAN | | | G [y11] - 1005.5211+[2] | 812780 |
| | | | V [b5] - 498.2922+[3] | 741243 |
| | | | E [y12] - 1134.5637+[4] | 651070 |
| | | | V [b2] - 171.1128+[5] | 634335 |
| | | | A [y8] - 718.4094+[6] | 416106 |
| | | | S [y7] - 647.3723+[7] | 360507 |
| | | | F [b6] - 645.3606+[8] | 293935 |
| | | | T [y4] - 418.2660+[9] | 281736 |
| | | | E [y9] - 847.4520+[10] | 247592 |
| | | | A [y3] - 317.2183+[11] | 246550 |
| | | | E [b7] - 774.4032+[12] | 234044 |
| | | | T [y10] - 948.4997+[13] | 221478 |
| | | | A [y6] - 560.3402+[14] | 212344 |
| | | | A [y5] - 489.3031+[15] | 195364 |
| | | | E [b8] - 903.4458+[16] | 183901 |
| | | | L [b3] - 284.1969+[17] | 176116 |
| | | | V [y2] - 246.1812+[18] | 157419 |
| | | | T [b10] - 1061.5150+[19] | 52841 |
| | | | E [b11] - 1190.5576+[20] | 34757 |
| | | | G [b9] - 960.4673+[21] | 25807 |
| alpha-1-antichymotrypsin | K.AVLDVFEEGTEASAA TAVK.I | 636.6581+++ | V [b2] - 171.1128+[1] | 659591 |
| AACT_HUMAN | | | S [y7] - 647.3723+[2] | 630596 |
| | | | A [y8] - 718.4094+[3] | 509467 |
| | | | D [b4] - 399.2238+[4] | 353335 |
| | | | A [y6] - 560.3402+[5] | 306747 |
| | | | A [y5] - 489.3031+[6] | 280878 |
| | | | E [y9] - 847.4520+[7] | 247347 |
| | | | T [y4] - 418.2660+[8] | 197203 |
| | | | A [y3] - 317.2183+[9] | 128853 |
| | | | V [b5] - 498.2922+[10] | 120271 |
| | | | V [y2] - 246.1812+[11] | 115428 |
| | | | L [b3] - 284.1969+[12] | 102984 |
| | | | G [y11] - 1005.5211+[13] | 91215 |
| | | | F [b6] - 645.3606+[14] | 79016 |
| | | | E [y12] - 1134.5637+[15] | 72947 |
| | | | E [b7] - 774.4032+[16] | 58358 |
| | | | T [y10] - 948.4997+[17] | 41071 |
| | | | E [b8] - 903.4458+[18] | 32918 |
| | | | G [b9] - 960.4673+[19] | 24275 |
| alpha-1-antichymotrypsin | K.ITLLSALVETR.T | 608.3690++ | S [y7] - 775.4308+[1] | 7387615 |
| AACT_HUMAN | | | T [b2] - 215.1390+[2] | 3498457 |
| | | | L [y8] - 888.5149+[3] | 2684639 |
| | | | L [b3] - 328.2231+[4] | 2164246 |
| | | | A [y6] - 688.3988+[5] | 2045853 |
| | | | L [y5] - 617.3617+[6] | 2027311 |
| | | | L [y9] - 1001.5990+[7] | 1949318 |
| | | | V [y4] - 504.2776+[8] | 1598519 |
| | | | T [y2] - 276.1666+[9] | 1416847 |
| | | | E [y3] - 405.2092+[10] | 967259 |
| | | | A [b6] - 599.3763+[11] | 579420 |
| | | | L [b4] - 441.3071+[12] | 431556 |
| | | | S [b5] - 528.3392+[13] | 107634 |
| | | | L [b7] - 712.4604+[14] | 71104 |
| | | | V [b8] - 811.5288+[15] | 24197 |
| alpha-1-antichymotrypsin | K.ITLLSALVETR.T | 405.9151+++ | E [y3] - 405.2092+[1] | 738128 |
| AACT_HUMAN | | | T [y2] - 276.1666+[2] | 368830 |
| | | | V [y4] - 504.2776+[3] | 328133 |
| | | | A [b6] - 599.3763+[4] | 132469 |
| | | | T [b2] - 215.1390+[5] | 126898 |
| | | | L [y5] - 617.3617+[6] | 124559 |
| | | | S [y7] - 775.4308+[7] | 54263 |
| | | | L [b3] - 328.2231+[8] | 37891 |
| | | | A [y6] - 688.3988+[9] | 29853 |
| | | | L [b4] - 441.3071+[10] | 25558 |
| | | | L [b7] - 712.4604+[11] | 13353 |
| | | | S [b5] - 528.3392+[12] | 12290 |
| Pigment epithelium-derived factor | K.LAAAVSNFGYDLYR. V | 780.3963++ | D [b11] - 1109.5262+[1] | 136227 |
| PEDF_HUMAN* | | | F [b8] - 774.4145+[2] | 61248 |
| | | | N [b7] - 314.1767++[3] | 55532 |
| | | | A [y12] - 1375.6641+[4] | 53268 |
| | | | V [b5] - 213.6392++[5] | 35818 |
| | | | L [b12] - 1222.6103+[6] | 34918 |
| | | | G [b9] - 831.4359+[7] | 33934 |
| | | | Y [b10] - 994.4993+[8] | 32923 |
| | | | G [b9] - 416.2216++[9] | 32650 |
| | | | V [b5] - 426.2711+[10] | 15646 |
| | | | A [b2] - 185.1285+[11] | 14964 |
| | | | D [b11] - 555.2667++[12] | 13922 |
| | | | L [y3] - 226.1368++[13] | 13027 |
| | | | A [b4] - 327.2027+[14] | 12782 |
| | | | A [y12] - 688.3357++[15] | 12446 |
| | | | V [y10] - 1233.5899+[16] | 12400 |
| | | | A [y11] - 652.8171++[17] | 10793 |
| Pigment epithelium-derived factor | K.LAAAVSNFGYDLYR. V | 520.5999+++ | G [y6] - 786.3781+[1] | 42885 |
| PEDF_HUMAN* | | | D [y4] - 566.2933+[2] | 32080 |
| | | | Y [y5] - 729.3566+[3] | 17494 |
| | | | L [y3] - 451.2663+[5] | 12304 |
| | | | Y [y2] - 338.1823+[6] | 7780 |
| Pigment epithelium-derived factor | R.ALYYDLISSPDIHGTY K.E | 652.6632+++ | Y [y15] - 886.4305++[1] | 12278 |
| PEDF_HUMAN* | | | L [b2] - 185.1285+[2] | 7601 |
| | | | S [y10] - 1104.5320+[3] | 7345 |
| | | | Y [y14] - 804.8988++[4] | 5976 |
| Pigment epithelium-derived factor | K.ELLDTVTAPQK.N | 607.8350++ | T [y5] - 272.6581++[1] | 59670 |
| PEDF_HUMAN* | | | Q [y2] - 275.1714+[2] | 11954 |
| Pigment epithelium-derived factor | K.ELLDTVTAPQK.N | 405.5591+++ | L [b2] - 243.1339+[1] | 16428 |
| PEDF_HUMAN* | | | T [b7] - 386.7080++[2] | 7918 |
| | | | Q [y2] - 275.1714+[3] | 7043 |
| | | | T [y5] - 272.6581++[4] | 5237 |
| Pigment epithelium-derived factor | K.SSFVAPLEK.S | 489.2687++ | A [y5] - 557.3293+[1] | 20068 |
| PEDF_HUMAN* | | | A [y5] - 279.1683++[2] | 5059 |
| | | | S [b2] - 175.0713+[3] | 4883 |
| Pigment epithelium-derived factor | K.SSFVAPLEK.S | 326.5149+++ | A [y5] - 279.1683++[1] | 70240 |
| PEDF_HUMAN* | | | A [y5]- 557.3293+[2] | 63329 |
| | | | S [b2] - 175.0713+[3] | 39662 |
| | | | L [b7] - 351.6947++[4] | 5393 |
| Pigment epithelium-derived factor | K.EIPDEISILLLGVAHFK .G | 632.0277+++ | P [y15] - 826.4745++[1] | 37871 |
| PEDF_HUMAN* | | | G [y6] - 658.3671+[2] | 20077 |
| | | | L [y7] - 771.4512+[3] | 8952 |
| Pigment epithelium-derived factor | K.TSLEDFYLDEER.T | 758.8437++ | R [y1] - 175.1190+[1] | 8206 |
| PEDF_HUMAN* | | | D [b9] - 1084.4833+[2] | 4591 |
| | | | F [b6] - 693.3090+[3] | 4498 |
| Pigment epithelium-derived factor | K.TSLEDFYLDEER.T | 506.2316+++ | F [b6] - 693.3090+[1] | 3526 |
| PEDF_HUMAN* | | | D [y4] - 548.2311+[2] | 3208 |
| Pigment epithelium-derived factor | K.VTQNLTLIEESLTSEFI HDIDR.E | 858.4413+++ | T [b13] - 721.8905++[1] | 11072 |
| PEDF_HUMAN* | | | T [y17] - 1009.5075++[2] | 8442 |
| | | | D [y4] - 518.2569+[3] | 6522 |
| Pigment epithelium-derived factor | K.TVQAVLTVPK.L | 528.3266++ | Q [y8] - 855.5298+[1] | 83536 |
| PEDF_HUMAN* | | | V [b2] - 201.1234+[2] | 64729 |
| | | | A [b4] - 200.6132++[3] | 58198 |
| | | | P [y2] - 244.1656+[4] | 43347 |
| | | | Q [y8] - 428.2686++[5] | 38398 |
| | | | A [y7] - 727.4713+[6] | 33770 |
| | | | Q [b3] - 329.1819+[7] | 17809 |
| | | | L [y5] - 557.3657+[8] | 17518 |
| | | | V [y6] - 656.4341+[9] | 17029 |
| | | | V [y6] - 328.7207++[10] | 15839 |
| | | | T [y4] - 444.2817+[11] | 13859 |
| | | | V [y3] - 343.2340+[12] | 10717 |
| | | | A [b4] - 400.2191+[13] | 9695 |
| Pigment epithelium-derived factor | K.TVQAVLTVPK.L | 352.5535+++ | P [y2] - 244.1656+[1] | 8295 |
| PEDF_HUMAN* | | | T [y4] - 444.2817+[2] | 2986 |
| | | | A [b4] - 400.2191+[3] | 2848 |
| Pigment epithelium-derived factor | K.LSYEGEVTK.S | 513.2611++ | V [b7] - 389.6845++[1] | 60831 |
| PEDF_HUMAN* | | | E [b6] - 679.2933+[2] | 34857 |
| | | | Y [y7] - 413.2031++[3] | 10075 |
| | | | V [b7] - 778.3618+[4] | 8920 |
| | | | Y [b3] - 364.1867+[5] | 8008 |
| Pigment epithelium-derived factor | K.LQSLFDSPDFSK.I | 692.3432++ | S [y2] - 234.1448+[1] | 49594 |
| PEDF_HUMAN* | | | L [y9] - 1055.5044+[2] | 48160 |
| | | | P [b8] - 888.4462+[3] | 23566 |
| | | | S [b7] - 791.3934+[4] | 13766 |
| | | | P [y5] - 297.1501++[5] | 12305 |
| | | | P [y5] - 593.2930+[6] | 10702 |
| | | | F [b5] - 589.3344+[7] | 8929 |
| | | | D [b9] - 1003.4731+[8] | 8742 |
| Pigment epithelium-derived factor | K.LQSLFDSPDFSK.I | 461.8979+++ | P [y5] - 593.2930+[1] | 9154 |
| PEDF_HUMAN* | | | P [y5] - 297.1501++[2] | 5479 |
| Pigment epithelium-derived factor | R.DTDTGALLFIGK.I | 625.8350++ | G [y2] - 204.1343+[1] | 32092 |
| PEDF_HUMAN* | | | G [y8] - 818.5135+[2] | 29707 |
| | | | T [b2] - 217.0819+[4] | 28172 |
| | | | T [b4] - 217.0819++[3] | 28172 |
| | | | F [y4] - 464.2867+[5] | 22160 |
| | | | D [y10] - 1034.5881+[6] | 20267 |
| | | | T [y9] - 919.5611+[7] | 17083 |
| | | | L [y6] - 690.4549+[8] | 14854 |
| | | | L [y5] - 577.3708+[9] | 12349 |
| | | | T [b4] - 433.1565+[10] | 11773 |
| | | | I [y3] - 317.2183+[11] | 11575 |
| | | | D [b3] - 332.1088+[12] | 8968 |
| | | | A [y7] - 761.4920+[13] | 8598 |

| | | | | |
|---|---|---|---|---|
| * Transition scan on Agilent 6490 | | | | |

### Example 4. Study III to Identify and Confirm Preeclampsia Biomarkers

A further hypothesis-dependent study was performed using essentially the same methods described in the preceding Examples unless noted below. The scheduled MRM assay used in Examples 1 and 2 but now augmented with newly discovered analytes from the Example 3 and related studies was used. Less robust transitions (from the original 1708 described in Example 1) were removed to improve analytical performance and make room for the newly discovered analytes.

Thirty subjects with preeclampsia who delivered preterm (<37 weeks 0 days) were selected for analyses. Twenty-three subjects were available with isolated preeclampsia; thus, eight subjects were selected with additional findings as follows: 5 subjects with gestational diabetes, one subject with pre-existing type 2 diabetes, and one subject with chronic hypertension. Subjects were classified as having severe preeclampsia if it was indicated in the Case Report Form as severe or if the pregnancy was complicated by HELLP syndrome. All other cases were classified as mild preeclampsia. Cases were matched to term controls (>/= 37 weeks 0 days) without preeclampsia at a 2:1 control-to-case ratio.

The samples were processed in 4 batches with each containing 3 HGS controls. All serum samples were depleted of the 14 most abundant serum proteins using MARS 14 (Agilent), digested with trypsin, desalted, and resolubilized with reconstitution solution containing 5 internal standard peptides as described in previous examples.

The LC-MS/MS analysis was performed with an Agilent Poroshell 120 EC-C18 column (2.1x50mm, 2.7 µm) at a flow rate of 400 µl/min and eluted with an acetonitrile gradient into an AB Sciex QTRAP5500 mass spectrometer. The sMRM assay measured 750 transitions that correspond to 349 peptides and 164 proteins. Chromatographic peaks were integrated using MultiQuantTM software (AB Sciex).

Transitions were excluded from analysis if they were missing in more than 20% of the samples. Log transformed peak areas for each transition were corrected for run order and batch effects by regression. The ability of each analyte to separate cases and controls was determined by calculating univariate AUC values from ROC curves. Ranked univariate AUC values (0.6 or greater) are reported for individual gestational age window sample sets or various combinations (Tables 12-15). Multivariate classifiers were built by Lasso and Random Forest methods. 1000 rounds of bootstrap resampling were performed and the nonzero Lasso coefficients or Random Forest Gini importance values were summed for each analyte amongst panels with AUCs of 0.85 or greater. For summed Random Forest Gini Importance values an Empirical Cumulative Distribution Function was fitted and probabilities (P) were calculated. The nonzero Lasso summed coefficients calculated from the different window combinations are shown in Tables 16-19. Summed Random Forest Gini values, with P >0.9 are found in Tables 20-22.

**Table 12. Univariate AUC values all windows**

| Transition | Protein | AUC |
|---|---|---|
| LDFHFSSDR_375.2_611.3 | INHBC_HUMAN | 0.785 |
| TVQAVLTVPK_528.3_428.3 | PEDF_HUMAN | 0.763 |
| TVQAVLTVPK_528.3_855.5 | PEDF_HUMAN | 0.762 |
| ETLLQDFR_511.3_565.3 | AMBP_HUMAN | 0.756 |
| DTDTGALLFIGK_625.8_818.5 | PEDF_HUMAN | 0.756 |
| DTDTGALLFIGK_625.8_217.1 | PEDF_HUMAN | 0.756 |
| IQTHSTTYR_369.5_627.3 | F13B_HUMAN | 0.755 |
| IQTHSTTYR_369.5_540.3 | F13B_HUMAN | 0.753 |
| ETLLQDFR_511.3_322.2 | AMBP_HUMAN | 0.751 |
| LDFHFSSDR_375.2_464.2 | INHBC_HUMAN | 0.745 |
| HHGPTITAK_321.2_275.1 | AMBP_HUMAN | 0.743 |
| VNHVTLSQPK_374.9_244.2 | B2MG_HUMAN | 0.733 |
| VEHSDLSFSK_383.5_468.2 | B2MG_HUMAN | 0.732 |
| ALALPPLGLAPLLNLWAKPQGR_770.5_256.2 | SHBG_HUMAN | 0.728 |
| HHGPTITAK_321.2_432.3 | AMBP_HUMAN | 0.728 |
| FLYHK_354.2_447.2 | AMBP_HUMAN | 0.722 |
| FLYHK_354.2_284.2 | AMBP_HUMAN | 0.721 |
| IALGGLLFPASNLR_481.3_657.4 | SHBG_HUMAN | 0.719 |
| GDTYPAELYITGSILR_885.0_274.1 | F13B_HUMAN | 0.716 |
| VEHSDLSFSK_383.5_234.1 | B2MG_HUMAN | 0.714 |
| GPGEDFR_389.2_623.3 | PTGDS_HUMAN | 0.714 |
| IALGGLLFPASNLR_481.3_412.3 | SHBG_ HUMAN | 0.712 |
| EVFSKPISWEELLQ_852.9_260.2 | FA40A_HUMAN | 0.708 |
| FICPLTGLWPINTLK_887.0_685.4 | APOH_HUMAN | 0.707 |
| GFQALGDAADIR_617.3_717.4 | TIMP1_HUMAN | 0.707 |
| DVLLLVHNLPQNLTGHIWYK_791.8_310.2 | PSG7_HUMAN | 0.704 |
| VVLSSGSGPGLDLPLVLGLPLQLK_791.5_598.4 | SHBG_HUMAN | 0.704 |
| ATVVYQGER_511.8_652.3 | APOH_HUMAN | 0.702 |
| ALALPPLGLAPLLNLWAKPQGR_770.5_457.3 | SHBG_HUMAN | 0.702 |
| VVLSSGSGPGLDLPLVLGLPLQLK_791.5_768.5 | SHBG_HUMAN | 0.702 |
| DVLLLVHNLPQNLTGHIWYK_791.8_883.0 | PSG7_HUMAN | 0.702 |
| AHYDLR_387.7_566.3 | FETUA_HUMAN | 0.701 |
| GPGEDFR_389.2_322.2 | PTGDS_HUMAN | 0.701 |
| FSVVYAK_407.2_579.4 | FETUA_HUMAN | 0.701 |
| TLAFVR_353.7_274.2 | FA7_HUMAN | 0.699 |
| IAPQLSTEELVSLGEK_857.5_533.3 | AFAM_HUMAN | 0.698 |
| HFQNLGK_422.2_527.2 | AFAM_HUMAN | 0.696 |
| GDTYPAELYITGSILR_885.0_922.5 | F13B_HUMAN | 0.694 |
| FICPLTGLWPINTLK_887.0_756.9 | APOH_HUMAN | 0.694 |
| EVFSKPISWEELLQ_852.9_376.2 | FA40A_HUMAN | 0.692 |
| ATVVYQGER_511.8_751.4 | APOH_HUMAN | 0.690 |
| EL IEEL VNITQNQK_557.6_618.3 | IL13_HUMAN | 0.690 |
| VNHVTLSQPK_374.9_459.3 | B2MG_HUMAN | 0.687 |
| IAQYYYTFK_598.8_395.2 | F13B_HUMAN | 0.685 |
| IAPQLSTEELVSLGEK_857.5_333.2 | AFAM_HUMAN | 0.685 |
| LIENGYFHPVK_439.6_627.4 | F13B_HUMAN | 0.684 |
| FSVVYAK_407.2_381.2 | FETUA_HUMAN | 0.684 |
| HFQNLGK_422.2_285.1 | AFAM_HUMAN | 0.684 |
| AHYDLR_387.7_288.2 | FETUA_HUMAN | 0.684 |
| ELPQSIVYK_538.8_417.7 | FBLN3_HUMAN | 0.683 |
| DADPDTFFAK_563.8_825.4 | AFAM_HUMAN | 0.679 |
| DADPDTFFAK_563.8_302.1 | AFAM_HUMAN | 0.676 |
| IAQYYYTFK_598.8_884.4 | F13B_HUMAN | 0.673 |
| VVESLAK_373.2_646.4 | IBP1_HUMAN | 0.673 |
| YGIEEHGK_311.5_599.3 | CXA1_HUMAN | 0.673 |
| GFQALGDAADIR_617.3_288.2 | TIMP1_HUMAN | 0.673 |
| YTTEIIK_434.2_704.4 | C1R_HUMAN | 0.671 |
| LPDTPQGLLGEAR_683.87_427.2 | EGLN_HUMAN | 0.666 |
| TLAFVR_353.7_492.3 | FA7_HUMAN | 0.666 |
| LIENGYFHPVK_439.6_343.2 | F13B_HUMAN | 0.665 |
| ELIEELVNITQNQK_557.6_517.3 | IL13_HUMAN | 0.665 |
| DPNGLPPEAQK_583.3_669.4 | RET4_HUMAN | 0.664 |
| TNTNEFLIDVDK_704.85_849.5 | TF_HUMAN | 0.663 |
| NTVISVNPSTK_580.3_845.5 | VCAM1_HUMAN | 0.662 |
| YEFLNGR_449.7_293.1 | PLMN_HUMAN | 0.662 |
| AIGLPEELIQK_605.86_856.5 | FABPL_HUMAN | 0.662 |
| YTTEIIK_434.2_603.4 | C1R_HUMAN | 0.661 |
| AEHPTWGDEQLFQTTR_639.3_765.4 | PGH1_HUMAN | 0.658 |
| HTLNQIDEVK_598.8_951.5 | FETUA_HUMAN | 0.658 |
| HTLNQIDEVK_598.8 958.5 | FETUA_HUMAN | 0.656 |
| LPNNVLQEK_527.8_730.4 | AFAM_HUMAN | 0.655 |
| DPNGLPPEAQK_583.3 497.2 | RET4_HUMAN | 0.655 |
| TFLTVYWTPER_706.9_401.2 | ICAM1_HUMAN | 0.653 |
| TFLTVYWTPER_706.9_502.3 | ICAM1_HUMAN | 0.653 |
| SEPRPGVLLR_375.2_454.3 | FA7_HUMAN | 0.652 |
| FTFTLHLETPKPSISSSNLNPR_829.4_787.4 | PSG1_HUMAN | 0.652 |
| DAQYAPGYDK_564.3_813.4 | CFAB_HUMAN | 0.651 |
| ALDLSLK_380.2_185.1 | ITIH3_HUMAN | 0.651 |
| NCSFSIIYPVVIK_770.4_555.4 | CRHBP_HUMAN | 0.650 |
| NTVISVNPSTK_580.3_732.4 | VCAM1_HUMAN | 0.649 |
| IPSNPSHR_ 303.2_610.3 | FBLN3_HUMAN | 0.649 |
| DAQYAPGYDK_564.3_315.1 | CFAB_HUMAN | 0.647 |
| TLPFSR_360.7_506.3 | LYAM1_HUMAN | 0.647 |
| LPNNVLQEK_527.8_844.5 | AFAM_HUMAN | 0.644 |
| AALAAFNAQNNGSNFQLEEISR_789.1_746.4 | FETUA_HUMAN | 0.644 |
| AEHPTWGDEQLFQTTR_639.3_569.3 | PGH1_HUMAN | 0.644 |
| NNQLVAGYLQGPNVNLEEK_700.7_999.5 | IL1RA_HUMAN | 0.642 |
| EHSSLAFWK_552.8_267.1 | APOH_HUMAN | 0.642 |
| ALNHLPLEYNSALYSR_621.0_696.4 | CO6_HUMAN | 0.641 |
| VSEADSSNADWVTK_754.9_347.2 | CFAB_HUMAN | 0.641 |
| NFPSPVDAAFR_610.8_959.5 | HEMO_HUMAN | 0.641 |
| WNFAYWAAHQPWSR_607.3_545.3 | PRG2_HUMAN | 0.638 |
| WNFAYWAAHQPWSR_607.3_673.3 | PRG2_HUMAN | 0.638 |
| TAVTANLDIR_537.3_802.4 | CHL1_HUMAN | 0.638 |
| IPSNPSHR_303.2_496.3 | FBLN3_HUMAN | 0.637 |
| YWGVASFLQK_599.8_849.5 | RET4_HUMAN | 0.637 |
| ALDLSLK_380.2_575.3 | ITIH3_HUMAN | 0.636 |
| YNSQLLSFVR_613.8_508.3 | TFR1_HUMAN | 0.636 |
| EHSSLAFWK_ 552.8_838.4 | APOH_HUMAN | 0.635 |
| YWGVASFLQK_599.8_350.2 | RET4_HUMAN | 0.635 |
| ALNHLPLEYNSALYSR_621.0_538.3 | CO6_HUMAN | 0.633 |
| DLYHYITSYVVDGEIIIYGPAYSGR_955.5_707.3 | PSG1_HUMAN | 0.633 |
| FTFTLHLETPKPSISSSNLNPR_829.4_874.4 | PSG1_HUMAN | 0.633 |
| YQISVNK_426.2_560.3 | FIBB_HUMAN | 0.632 |
| YEFLNGR_449.7_606.3 | PLMN_HUMAN | 0.632 |
| LNIGYIEDLK_589.3_950.5 | PAI2_HUMAN | 0.631 |
| LLEVPEGR_456.8_356.2 | C1S_HUMAN | 0.630 |
| ENPAVIDFELAPIVDLVR_670.7_811.5 | CO6_HUMAN | 0.630 |
| YYLQGAK_421.7_516.3 | ITIH4_HUMAN | 0.630 |
| ITGFLKPGK_320.9_301.2 | LBP_HUMAN | 0.629 |
| DLHLSDVFLK_396.2_260.2 | CO6_HUMAN | 0.629 |
| HELTDEELQSLFTNFANVVDK_817.1_854.4 | AFAM_HUMAN | 0.629 |
| YYLQGAK_421.7_327.1 | ITIH4_HUMAN | 0.628 |
| NCSFSIIYPVVIK_770.4_831.5 | CRHBP HUMAN | 0.627 |
| FLNWIK_410.7_560.3 | HABP2 HUMAN | 0.627 |
| ITGFLKPGK_320.9_429.3 | LBP_HUMAN | 0.627 |
| VVESLAK_373.2_547.3 | IBP1_HUMAN | 0.627 |
| NFPSPVDAAFR_610.8_775.4 | HEMO_HUMAN | 0.627 |
| AEIEYLEK_ 497.8_552.3 | LYAM1_HUMAN | 0.627 |
| ENPAVIDFELAPIVDLVR_670.7_601.4 | CO6_HUMAN | 0.627 |
| VQEVLLK_414.8_373.3 | HYOU1_HUMAN | 0.626 |
| TQIDSPLSGK_523.3_703.4 | VCAM1_HUMAN | 0.626 |
| VSEADSSNADWVTK_754.9_533.3 | CFAB_HUMAN | 0.625 |
| DFNQFSSGEK_386.8_189.1 | FETA_HUMAN | 0.624 |
| LPDTPQGLLGEAR_683.87_940.5 | EGLN_HUMAN | 0.623 |
| DLYHYITSYVVDGEIIIYGPAYSGR_955.5_650.3 | PSG1_HUMAN | 0.623 |
| FAFNLYR_465.8_712.4 | HEP2_HUMAN | 0.623 |
| LLELTGPK_435.8_644.4 | A1BG_HUMAN | 0.623 |
| NEIVFPAGILQAPFYTR_968.5_357.2 | ECE1_HUMAN | 0.623 |
| EFDDDTYDNDIALLQLK_1014.48_501.3 | TPA_HUMAN | 0.621 |
| FSLVSGWGQLLDR_493.3_403.2 | FA7_HUMAN | 0.621 |
| LLELTGPK_435.8_227.2 | A1BG_HUMAN | 0.621 |
| LIQDAVTGLTVNGQITGDK_972.0_640.4 | ITIH3_HUMAN | 0.621 |
| QGHNSVFLIK_381.6_520.4 | HEMO_HUMAN | 0.620 |
| ILPSVPK_377.2_244.2 | PGH1_HUMAN | 0.620 |
| STLFVPR_410.2_272.2 | PEPD_HUMAN | 0.620 |
| TLEAQLTPR_514.8_685.4 | HEP2_HUMAN | 0.619 |
| QGHNSVFLIK_381.6_260.2 | HEMO_HUMAN | 0.619 |
| LSSPAVITDK_515.8_743.4 | PLMN_HUMAN | 0.618 |
| LLEVPEGR_456.8_686.4 | C1S_HUMAN | 0.617 |
| GVTGYFTFNLYLK_508.3_260.2 | PSG5_HUMAN | 0.617 |
| EALVPLVADHK_397.9_390.2 | HGFA_HUMAN | 0.616 |
| SFRPFVPR_335.9_272.2 | LBP_HUMAN | 0.616 |
| DFNQFSSGEK_386.8_333.2 | FETA_HUMAN | 0.616 |
| GSLVQASEANLQAAQDFVR_668.7_735.4 | ITIH1_HUMAN | 0.616 |
| ITLPDFTGDLR_624.3_920.5 | LBP_HUMAN | 0.615 |
| LIQDAVTGLTVNGQITGDK_972.0_798.4 | ITIH3_HUMAN | 0.615 |
| ILPSVPK_377.2_227.2 | PGH1_HUMAN | 0.614 |
| DIIKPDPPK_511.8_342.2 | IL12B_HUMAN | 0.613 |
| QGFGNVATNTDGK_654.81_319.2 | FIBB_HUMAN | 0.613 |
| AVLHIGEK_289.5_348.7 | THBG_HUMAN | 0.613 |
| YENYTSSFFIR_713.8_756.4 | IL12B_HUMAN | 0.613 |
| LSSPAVITDK_515.8_830.5 | PLMN_HUMAN | 0.613 |
| SFRPFVPR_335.9_635.3 | LBP_HUMAN | 0.613 |
| GLQYAAQEGLLALQSELLR_1037.1_858.5 | LBP_HUMAN | 0.612 |
| VELAPLPSWQPVGK_760.9_400.3 | ICAM1_HUMAN | 0.612 |
| CRPINATLAVEK_457.9_559.3 | CGB1_HUMAN | 0.610 |
| GIVEECCFR_585.3_771.3 | IGF2_HUMAN | 0.610 |
| AVLHIGEK_289.5_292.2 | THBG_HUMAN | 0.610 |
| TLEAQLTPR_514.8_814.4 | HEP2_HUMAN | 0.610 |
| SILFLGK_389.2_577.4 | THBG_HUMAN | 0.609 |
| HVVQLR_376.2_614.4 | IL6RA_HUMAN | 0.609 |
| TQILEWAAER_608.8_761.4 | EGLN_HUMAN | 0.609 |
| NSDQEIDFK_548.3_409.2 | S10A5_HUMAN | 0.609 |
| SGAQATWTELPWPHEK_613.3_510.3 | HEMO_HUMAN | 0.607 |
| EDTPNSVWEPAK_686.8_630.3 | C1S_ HUMAN | 0.607 |
| ITLPDFTGDLR_624.3_288.2 | LBP_HUMAN | 0.607 |
| TLPFSR_360.7_409.2 | LYAM1_HUMAN | 0.607 |
| GIVEECCFR_585.3_900.3 | IGF2_HUMAN | 0.606 |
| SGAQATWTELPWPHEK_613.3_793.4 | HEMO_HUMAN | 0.606 |
| VRPQQLVK_484.3_609.4 | ITIH4_HUMAN | 0.605 |
| SEYGAALAWEK_612.8_788.4 | CO6_HUMAN | 0.605 |
| LEEHYELR_363.5_288.2 | PAI2_HUMAN | 0.605 |
| FQLPGQK_409.2_275.1 | PSG1_HUMAN | 0.605 |
| IHWESASLLR_606.3_437.2 | CO3_HUMAN | 0.604 |
| NAVVQGLEQPHGLVVHPLR_688.4_890.6 | LRP1_HUMAN | 0.604 |
| VTGLDFIPGLHPILTLSK_641.04_771.5 | LEP_HUMAN | 0.603 |
| YNSQLLSFVR_613.8_734.5 | TFR1_HUMAN | 0.603 |
| ALVLELAK_428.8_672.4 | INHBE_HUMAN | 0.603 |
| FAFNLYR_465.8_565.3 | HEP2_HUMAN | 0.603 |
| VRPQQLVK_484.3_722.4 | ITIH4_HUMAN | 0.602 |
| SLQAFVAVAAR_566.8_487.3 | IL23A_HUMAN | 0.602 |
| AGFAGDDAPR_488.7_701.3 | ACTB_HUMAN | 0.601 |
| EDTPNSVWEPAK_686.8_315.2 | C1S_HUMAN | 0.601 |
| VQEVLLK_414.8_601.4 | HYOU1_HUMAN | 0.601 |
| SEYGAALAWEK_612.8_845.5 | CO6_HUMAN | 0.601 |
| TLFIFGVTK_513.3_215.1 | PSG4_HUMAN | 0.601 |
| YNQLLR_403.7_288.2 | ENOA_HUMAN | 0.600 |
| TQIDSPLSGK_523.3_816.5 | VCAM1_HUMAN | 0.600 |

**Table 13. Univariate AUC values early window**

| Transition | Protein | AUC |
|---|---|---|
| LDFHFSSDR_375.2_611.3 | INHBC_HUMAN | 0.858 |
| LDFHFSSDR_375.2_464.2 | INHBC_HUMAN | 0.838 |
| ELPQSIVYK_538.8_417.7 | FBLN3_HUMAN | 0.815 |
| VNHVTLSQPK_374.9_244.2 | B2MG_HUMAN | 0.789 |
| GFQALGDAADIR_617.3_717.4 | TIMP1_HUMAN | 0.778 |
| VEHSDLSFSK_383.5_234.1 | B2MG_HUMAN | 0.778 |
| TVQAVLTVPK_528.3_428.3 | PEDF_HUMAN | 0.775 |
| TVQAVLTVPK_528.3_855.5 | PEDF_HUMAN | 0.775 |
| DTDTGALLFIGK_625.8_217.1 | PEDF_HUMAN | 0.772 |
| ETLLQDFR_511.3_565.3 | AMBP_HUMAN | 0.772 |
| DTDTGALLFIGK_625.8_818.5 | PEDF_HUMAN | 0.769 |
| VVESLAK_373.2_646.4 | IBP1_HUMAN | 0.766 |
| FSVVYAK_407.2_381.2 | FETUA_HUMAN | 0.764 |
| HHGPTITAK_321.2_275.1 | AMBP_HUMAN | 0.764 |
| ETLLQDFR_511.3_322.2 | AMBP_HUMAN | 0.761 |
| FLYHK_354.2_447.2 | AMBP_HUMAN | 0.758 |
| GPGEDFR_389.2_623.3 | PTGDS HUMAN | 0.755 |
| HHGPTITAK_321.2_432.3 | AMBP_HUMAN | 0.755 |
| VEHSDLSFSK_383.5_468.2 | B2MG_HUMAN | 0.752 |
| FLYHK_354.2_284.2 | AMBP_HUMAN | 0.749 |
| FSVVYAK_407.2_579.4 | FETUA_HUMAN | 0.749 |
| VNHVTLSQPK_374.9_459.3 | B2MG_HUMAN | 0.749 |
| IPSNPSHR_303.2_610.3 | FBLN3_HUMAN | 0.746 |
| VVESLAK_373.2_547.3 | IBP1_HUMAN | 0.746 |
| IPSNPSHR_303.2_496.3 | FBLN3_HUMAN | 0.746 |
| NCSFSIIYPVVIK_770.4_555.4 | CRHBP_HUMAN | 0.746 |
| GFQALGDAADIR_617.3_288.2 | TIMP1_HUMAN | 0.744 |
| IQTHSTTYR_369.5_627.3 | F13B_HUMAN | 0.744 |
| AALAAFNAQNNGSNFQLEEISR_789.1_746.4 | FETUA_HUMAN | 0.738 |
| AHYDLR_387.7_566.3 | FETUA_HUMAN | 0.738 |
| IQTHSTTYR_369.5_540.3 | F13B_HUMAN | 0.738 |
| AIGLPEELIQK_605.86_856.5 | FABPL_HUMAN | 0.735 |
| ATVVYQGER_511.8_751.4 | APOH_HUMAN | 0.735 |
| FICPLTGLWPINTLK_887.0_685.4 | APOH_HUMAN | 0.735 |
| FICPLTGLWPINTLK_887.0_756.9 | APOH_HUMAN | 0.735 |
| HTLNQIDEVK_598.8_958.5 | FETUA_HUMAN | 0.735 |
| AQETSGEEISK_589.8_979.5 | IBP1_HUMAN | 0.732 |
| DSPSVWAAVPGK_607.31_301.2 | PROF1_HUMAN | 0.732 |
| GPGEDFR_389.2_322.2 | PTGDS_HUMAN | 0.732 |
| ATVVYQGER_511.8_652.3 | APOH_HUMAN | 0.729 |
| NFPSPVDAAFR_610.8_959.5 | HEMO_HUMAN | 0.729 |
| LIENGYFHPVK_439.6_627.4 | F13B_HUMAN | 0.726 |
| AHYDLR_387.7_288.2 | FETUA_HUMAN | 0.726 |
| EL IEEL VNITQNQK_557.6_618.3 | IL13_HUMAN | 0.724 |
| ETPEGAEAKPWYEPIYLGGVFQLEK_951.14_877.5 | TNFA_HUMAN | 0.724 |
| ALDLSLK_380.2_185.1 | ITIH3_HUMAN | 0.721 |
| IHWESASLLR_606.3_437.2 | CO3_HUMAN | 0.721 |
| DAQYAPGYDK_564.3_813.4 | CFAB_HUMAN | 0.718 |
| NFPSPVDAAFR_610.8_775.4 | HEMO_HUMAN | 0.718 |
| AVGYLITGYQR_620.8_523.3 | PZP_HUMAN | 0.715 |
| AVGYLITGYQR_620.8_737.4 | PZP_HUMAN | 0.712 |
| DIPHWLNPTR_416.9_600.3 | PAPP1_HUMAN | 0.712 |
| ALDLSLK_380.2 575.3 | ITIH3_HUMAN | 0.709 |
| IEGNLIFDPNNYLPK_874.0_845.5 | APOB_HUMAN | 0.709 |
| LIENGYFHPVK_439.6_343.2 | F13B_HUMAN | 0.709 |
| QTLSWTVTPK_580.8_818.4 | PZP_HUMAN | 0.709 |
| DAQYAPGYDK_564.3_315.1 | CFAB_HUMAN | 0.707 |
| GLQYAAQEGLLALQSELLR_1037.1_858.5 | LBP_HUMAN | 0.707 |
| IEGNLIFDPNNYLPK_874.0_414.2 | APOB_HUMAN | 0.707 |
| IQHPFTVEEFVLPK_562.0_861.5 | PZP_HUMAN | 0.707 |
| QTLSWTVTPK_580.8_545.3 | PZP_HUMAN | 0.707 |
| VSEADSSNADWVTK_754.9_347.2 | CFAB_HUMAN | 0.707 |
| ILPSVPK_377.2_244.2 | PGH1_HUMAN | 0.704 |
| IQHPFTVEEFVLPK_562.0 603.4 | PZP_HUMAN | 0.704 |
| NCSFSIIYPVVIK_770.4_831.5 | CRHBP_HUMAN | 0.704 |
| YNSQLLSFVR_613.8_508.3 | TFR1_HUMAN | 0.704 |
| HTLNQIDEVK_598.8_951.5 | FETUA_HUMAN | 0.701 |
| NEIWYR_440.7_637.4 | FA12_HUMAN | 0.701 |
| QGHNSVFLIK_381.6_260.2 | HEMO_HUMAN | 0.701 |
| YTTEIIK_434.2_603.4 | C1R_HUMAN | 0.701 |
| STLFVPR_410.2_272.2 | PEPD_HUMAN | 0.699 |
| EVFSKPISWEELLQ_852.9_260.2 | FA40A_HUMAN | 0.698 |
| TGISPLALIK_506.8_741.5 | APOB_HUMAN | 0.698 |
| TSESGELHGLTTEEEFVEGIYK_819.06_310.2 | TTHY_HUMAN | 0.698 |
| AEHPTWGDEQLFQTTR_639.3_569.3 | PGH1_HUMAN | 0.695 |
| AEHPTWGDEQLFQTTR_639.3_765.4 | PGH1_HUMAN | 0.695 |
| HFQNLGK_422.2_527.2 | AFAM_HUMAN | 0.695 |
| SVSLPSLDPASAK_636.4_473.3 | APOB_HUMAN | 0.695 |
| ILPSVPK_377.2_227.2 | PGH1_HUMAN | 0.692 |
| LIQDAVTGLTVNGQITGDK_972.0_640.4 | ITIH3_HUMAN | 0.692 |
| QGHNSVFLIK_381.6_520.4 | HEMO_HUMAN | 0.692 |
| TGISPLALIK_506.8_654.5 | APOB_HUMAN | 0.692 |
| YGIEEHGK_311.5_599.3 | CXA1_HUMAN | 0.692 |
| ELIEELVNITQNQK_557.6_517.3 | IL13_HUMAN | 0.689 |
| IHWESASLLR_606.3_251.2 | CO3_HUMAN | 0.689 |
| LIQDAVTGLTVNGQITGDK_972.0_798.4 | ITIH3_HUMAN | 0.689 |
| ALALPPLGLAPLLNLWAKPQGR_770.5_256.2 | SHBG_HUMAN | 0.687 |
| ALNFGGIGVVVGHELTHAFDDQGR_837.1_299.2 | ECE1_HUMAN | 0.687 |
| AQETSGEEISK_589.8_850.4 | IBP1_HUMAN | 0.687 |
| GVTGYFTFNLYLK_508.3_683.9 | PSG5_HUMAN | 0.687 |
| ITLPDFTGDLR_624.3_288.2 | LBP_HUMAN | 0.687 |
| LPDTPQGLLGEAR_683.87_427.2 | EGLN_HUMAN | 0.687 |
| SVSLPSLDPASAK_636.4_885.5 | APOB_HUMAN | 0.687 |
| TLAFVR_353.7_274.2 | FA7_HUMAN | 0.687 |
| YTTEIIK_434.2_704.4 | C1R_HUMAN | 0.687 |
| EFDDDTYDNDIALLQLK_1014.48_388.3 | TPA_HUMAN | 0.684 |
| IALGGLLFPASNLR_481.3 657.4 | SHBG_HUMAN | 0.684 |
| DFNQFSSGEK_386.8_189.1 | FETA_HUMAN | 0.681 |
| EHSSLAFWK_552.8_838.4 | APOH_HUMAN | 0.681 |
| ELPQSIVYK_538.8_409.2 | FBLN3_HUMAN | 0.681 |
| ITGFLKPGK_320.9_301.2 | LBP_HUMAN | 0.681 |
| ITGFLKPGK_320.9_429.3 | LBP_HUMAN | 0.681 |
| AFQVWSDVTPLR_709.88_385.3 | MMP2_HUMAN | 0.678 |
| GLQYAAQEGLLALQSELLR_1037.1_929.5 | LBP_HUMAN | 0.678 |
| HYINLITR_515.3_301.1 | NPY_HUMAN | 0.678 |
| NAVVQGLEQPHGLVVHPLR_688.4_890.6 | LRP1_HUMAN | 0.675 |
| WWGGQPLWITATK_772.4_929.5 | ENPP2_HUMAN | 0.675 |
| YNQLLR_403.7_288.2 | ENOA_HUMAN | 0.675 |
| LDGSTHLNIFFAK_488.3_852.5 | PAPP1_HUMAN | 0.672 |
| VVGGLVALR_442.3_784.5 | FA12_HUMAN | 0.672 |
| WNFAYWAAHQPWSR_607.3_673.3 | PRG2_HUMAN | 0.672 |
| NHYTESISVAK_624.8_252.1 | NEUR1_HUMAN | 0.670 |
| NSDQEIDFK_548.3_409.2 | S10A5_HUMAN | 0.670 |
| SGAQATWTELPWPHEK_613.3_510.3 | HEMO_HUMAN | 0.670 |
| WNFAYWAAHQPWSR_607.3_545.3 | PRG2_HUMAN | 0.670 |
| SFRPFVPR_335.9_272.2 | LBP_HUMAN | 0.670 |
| AFQVWSDVTPLR_709.88_347.2 | MMP2_HUMAN | 0.667 |
| DADPDTFFAK_563.8_825.4 | AFAM_HUMAN | 0.667 |
| EHSSLAFWK_552.8_267.1 | APOH_HUMAN | 0.667 |
| ITENDIQIALDDAK_779.9_632.3 | APOB_HUMAN | 0.667 |
| ITLPDFTGDLR_624.3_920.5 | LBP_HUMAN | 0.667 |
| VQEVLLK_414.8_373.3 | HYOU1_HUMAN | 0.667 |
| VSFSSPLVAISGVALR_802.0_715.4 | PAPP1_HUMAN | 0.667 |
| HFQNLGK_422.2_285.1 | AFAM_HUMAN | 0.664 |
| ITENDIQIALDDAK_779.9_873.5 | APOB_HUMAN | 0.664 |
| ALQDQLVLVAAK_634.9_289.2 | ANGT_HUMAN | 0.661 |
| DLHLSDVFLK_396.2_260.2 | CO6_HUMAN | 0.661 |
| DLHLSDVFLK_396.2_366.2 | CO6_HUMAN | 0.661 |
| TAVTANLDIR_537.3_802.4 | CHL1_HUMAN | 0.661 |
| DADPDTFFAK_563.8_302.1 | AFAM_HUMAN | 0.658 |
| DPTFIPAPIQAK_433.2_461.2 | ANGT_HUMAN | 0.658 |
| FAFNLYR_465.8_712.4 | HEP2_HUMAN | 0.658 |
| IALGGLLFPASNLR_481.3_412.3 | SHBG_HUMAN | 0.658 |
| IAQYYYTFK_598.8_395.2 | F13B_HUMAN | 0.658 |
| LPNNVLQEK_527.8_730.4 | AFAM_HUMAN | 0.658 |
| SLDFTELDVAAEK_719.4_874.5 | ANGT_HUMAN | 0.658 |
| VELAPLPSWQPVGK_760.9_400.3 | ICAM1_HUMAN | 0.658 |
| DIIKPDPPK_511.8_342.2 | IL12B_HUMAN | 0.655 |
| EVFSKPISWEELLQ_852.9_376.2 | FA40A_HUMAN | 0.655 |
| LSETNR_360.2_330.2 | PSG1_HUMAN | 0.655 |
| NEIWYR_440.7_357.2 | FA12_HUMAN | 0.655 |
| SFRPFVPR_335.9_635.3 | LBP_HUMAN | 0.655 |
| SGAQATWTELPWPHEK_613.3_793.4 | HEMO_HUMAN | 0.655 |
| TGAQELLR_444.3_530.3 | GELS HUMAN | 0.655 |
| VSEADSSNADWVTK_754.9_533.3 | CFAB_HUMAN | 0.655 |
| VVGGLVALR_442.3_685.4 | FA12_HUMAN | 0.655 |
| DISEVVTPR_508.3_787.4 | CFAB_HUMAN | 0.652 |
| IHPSYTNYR_575.8_598.3 | PSG2_HUMAN | 0.652 |
| VSFSSPLVAISGVALR_802.0_602.4 | PAPP1_HUMAN | 0.652 |
| YNQLLR_403.7_529.3 | ENOA_HUMAN | 0.652 |
| ALQDQLVLVAAK_634.9_956.6 | ANGT_HUMAN | 0.650 |
| IHPSYTNYR_575.8_813.4 | PSG2_HUMAN | 0.650 |
| TFLTVYWTPER_706.9_401.2 | ICAM1_HUMAN | 0.650 |
| VQEVLLK_414.8_601.4 | HYOU1_HUMAN | 0.650 |
| GDTYPAELYITGSILR_885.0_274.1 | F13B_HUMAN | 0.647 |
| GVTGYFTFNLYLK_508.3_260.2 | PSG5_HUMAN | 0.647 |
| SLDFTELDVAAEK_719.4_316.2 | ANGT_HUMAN | 0.647 |
| VVLSSGSGPGLDLPLVLGLPLQLK_791.5_598.4 | SHBG_HUMAN | 0.647 |
| YEFLNGR_449.7_293.1 | PLMN_HUMAN | 0.647 |
| AQPVQVAEGSEPDGFWEALGGK_758.0_623.4 | GELS_HUMAN | 0.644 |
| FLNWIK_410.7_561.3 | HABP2_HUMAN | 0.644 |
| IAPQLSTEELVSLGEK_857.5_533.3 | AFAM_HUMAN | 0.644 |
| NTVISVNPSTK_580.3_732.4 | VCAM1_ HUMAN | 0.644 |
| SFEGLGQLEVLTLDHNQLQEVK_833.1_503.3 | ALS_HUMAN | 0.644 |
| TFLTVYWTPER_706.9_502.3 | ICAM1_HUMAN | 0.644 |
| AGFAGDDAPR_488.7_701.3 | ACTB_HUMAN | 0.641 |
| AIGLPEELIQK_605.86_355.2 | FABPL_HUMAN | 0.641 |
| DISEVVTPR_508.3_472.3 | CFAB_HUMAN | 0.641 |
| DPTFIPAPIQAK_433.2_556.3 | ANGT_HUMAN | 0.641 |
| ENPAVIDFELAPIVDLVR_670.7_811.5 | CO6_HUMAN | 0.641 |
| FAFNLYR_465.8_565.3 | HEP2_HUMAN | 0.641 |
| IAPQLSTEELVSLGEK_857.5_333.2 | AFAM_HUMAN | 0.641 |
| TNTNEFLIDVDK_704.85_849.5 | TF_HUMAN | 0.639 |
| DVLLLVHNLPQNLTGHIWYK_791.8_883.0 | PSG7_HUMAN | 0.638 |
| LDGSTHLNIFFAK_488.3_739.4 | PAPP1_HUMAN | 0.638 |
| LPDTPQGLLGEAR_683.87_940.5 | EGLN_HUMAN | 0.638 |
| VVLSSGSGPGLDLPLVLGLPLQLK_791.5_768.5 | SHBG_HUMAN | 0.638 |
| ALALPPLGLAPLLNLWAKPQGR_770.5_457.3 | SHBG_HUMAN | 0.635 |
| LPNNVLQEK_527.8_844.5 | AFAM_HUMAN | 0.635 |
| QINSYVK_426.2_496.3 | CBG_HUMAN | 0.635 |
| QINSYVK_426.2_610.3 | CBG HUMAN | 0.635 |
| TGAQELLR_444.3_658.4 | GELS_HUMAN | 0.635 |
| TLEAQLTPR_514.8_685.4 | HEP2_HUMAN | 0.635 |
| WILTAAHTLYPK_471.9_621.4 | C1R_HUMAN | 0.635 |
| SEPRPGVLLR_375.2_454.3 | FA7_HUMAN | 0.632 |
| AGFAGDDAPR_488.7_630.3 | ACTB_HUMAN | 0.632 |
| DFNQFSSGEK_386.8_333.2 | FETA_HUMAN | 0.632 |
| DVLLLVHNLPQNLTGHIWYK_791.8_310.2 | PSG7_HUMAN | 0.632 |
| NKPGVYTDVAYYLAWIR_677.0_545.3 | FA12_HUMAN | 0.632 |
| SEYGAALAWEK_612.8_788.4 | CO6_HUMAN | 0.632 |
| YNSQLLSFVR_613.8_734.5 | TFR1_HUMAN | 0.632 |
| ALVLELAK_428.8_672.4 | INHBE_HUMAN | 0.630 |
| ENPAVIDFELAPIVDLVR_670.7_601.4 | CO6_HUMAN | 0.630 |
| NNQLVAGYLQGPNVNLEEK_700.7_999.5 | IL1RA_HUMAN | 0.630 |
| WGAAPYR_410.7_577.3 | PGRP2_HUMAN | 0.630 |
| HELTDEELQSLFTNFANVVDK_817.1_854.4 | AFAM_HUMAN | 0.627 |
| AKPALEDLR_506.8_288.2 | APOA1_HUMAN | 0.624 |
| AVLHIGEK_289.5_348.7 | THBG_HUMAN | 0.624 |
| EDTPNSVWEPAK_686.8_630.3 | C1S_HUMAN | 0.624 |
| SPELQAEAK_486.8_788.4 | APOA2_HUMAN | 0.624 |
| YENYTSSFFIR_713.8_756.4 | IL12B_HUMAN | 0.624 |
| NEIVFPAGILQAPFYTR_968.5_456.2 | ECE1_HUMAN | 0.621 |
| TAVTANLDIR_537.3_288.2 | CHL1_HUMAN | 0.621 |
| WWGGQPLWITATK_772.4_373.2 | ENPP2_HUMAN | 0.621 |
| AVDIPGLEAATPYR_736.9_399.2 | TENA_HUMAN | 0.618 |
| ALNFGGIGVVVGHELTHAFDDQGR_837.1_360.2 | ECE1_HUMAN | 0.618 |
| ALNHLPLEYNSALYSR_621.0_696.4 | CO6_HUMAN | 0.618 |
| FNAVLTNPQGDYDTSTGK_964.5_262.1 | C1QC_HUMAN | 0.618 |
| GDTYPAELYITGSILR_885.0 922.5 | F13B_HUMAN | 0.618 |
| IAQYYYTFK_598.8_884.4 | F13B_HUMAN | 0.618 |
| LEQGENVFLQATDK_796.4_822.4 | C1QB_HUMAN | 0.618 |
| LSITGTYDLK_555.8_696.4 | A1AT_HUMAN | 0.618 |
| NTVISVNPSTK_580.3_845.5 | VCAM1_HUMAN | 0.618 |
| TLAFVR_353.7_492.3 | FA7_HUMAN | 0.618 |
| TLEAQLTPR_514.8_814.4 | HEP2_HUMAN | 0.618 |
| TQIDSPLSGK_523.3_703.4 | VCAM1_HUMAN | 0.618 |
| AVLHIGEK_289.5_292.2 | THBG_HUMAN | 0.615 |
| FLIPNASQAESK_652.8_931.4 | 1433Z_HUMAN | 0.615 |
| FNAVLTNPQGDYDTSTGK_964.5_333.2 | C1QC_HUMAN | 0.615 |
| FQSVFTVTR_542.8_722.4 | C1QC_HUMAN | 0.615 |
| INPASLDK_429.2_630.4 | C163A_HUMAN | 0.615 |
| IPKPEASFSPR_410.2_506.3 | ITIH4_HUMAN | 0.615 |
| ITQDAQLK_458.8_803.4 | CBG_HUMAN | 0.615 |
| TSYQVYSK_488.2_397.2 | C163A_HUMAN | 0.615 |
| WGAAPYR_410.7_634.3 | PGRP2_HUMAN | 0.615 |
| AVDIPGLEAATPYR_736.9_286.1 | TENA_HUMAN | 0.613 |
| DVLLLVHNLPQNLPGYFWYK_810.4_328.2 | PSG9_HUMAN | 0.613 |
| SFEGLGQLEVLTLDHNQLQEVK_833.1_662.8 | ALS_HUMAN | 0.613 |
| TASDFITK_441.7_710.4 | GELS_HUMAN | 0.613 |
| AGPLQAR_356.7_584.4 | DEF4_HUMAN | 0.610 |
| DYWSTVK_449.7_347.2 | APOC3_HUMAN | 0.610 |
| FQSVFTVTR_542.79_623.4 | C1QC_HUMAN | 0.610 |
| FQSVFTVTR_542.79_722.4 | C1QC_HUMAN | 0.610 |
| SYTITGLQPGTDYK_772.4_352.2 | FINC_HUMAN | 0.610 |
| FQLSETNR_497.8_476.3 | PSG2_HUMAN | 0.607 |
| IPKPEASFSPR_410.2_359.2 | ITIH4_HUMAN | 0.607 |
| LIEIANHVDK_384.6_498.3 | ADA12_HUMAN | 0.607 |
| SILFLGK_389.2_201.1 | THBG_HUMAN | 0.607 |
| SLLQPNK_400.2_358.2 | CO8A_HUMAN | 0.607 |
| VFQFLEK_455.8_811.4 | CO5_HUMAN | 0.607 |
| VPGLYYFTYHASSR_554.3_720.3 | C1QB_HUMAN | 0.607 |
| VSAPSGTGHLPGLNPL_506.3_860.5 | PSG3_HUMAN | 0.607 |
| AGITIPR_364.2_486.3 | IL17_HUMAN | 0.604 |
| FLIPNASQAESK_652.8_261.2 | 1433Z_HUMAN | 0.604 |
| FQSVFTVTR_542.8_623.4 | C1QC_HUMAN | 0.604 |
| IRPFFPQQ_516.79_661.4 | FIBB_HUMAN | 0.604 |
| LLELTGPK_435.8_644.4 | A1BG_HUMAN | 0.604 |
| SETEIHQGFQHLHQLFAK_717.4_318.1 | CBG_HUMAN | 0.604 |
| SILFLGK_389.2_577.4 | THBG_HUMAN | 0.604 |
| STLFVPR_410.2_518.3 | PEPD_HUMAN | 0.604 |
| TEQAAVAR_423.2_487.3 | FA12_HUMAN | 0.604 |
| EDTPNSVWEPAK_686.8_315.2 | C1S_HUMAN | 0.601 |
| FLNWIK_410.7_560.3 | HABP2_HUMAN | 0.601 |
| ITQDAQLK_458.8_702.4 | CBG_HUMAN | 0.601 |
| SPELQAEAK_486.8 659.4 | APOA2_HUMAN | 0.601 |
| TLLPVSKPEIR_418.3_288.2 | CO5_HUMAN | 0.601 |
| VFQFLEK_455.8_276.2 | CO5_HUMAN | 0.601 |
| YGLVTYATYPK_638.3_843.4 | CFAB_HUMAN | 0.601 |

**Table 14. Univariate AUC values early-middle combined windows**

| Transition | Protein | AUC |
|---|---|---|
| LDFHFSSDR_375.2_611.3 | INHBC_HUMAN | 0.809 |
| ETLLQDFR_511.3_565.3 | AMBP_HUMAN | 0.802 |
| HHGPTITAK_321.2_275.1 | AMBP_HUMAN | 0.801 |
| ATVVYQGER_511.8_652.3 | APOH_HUMAN | 0.799 |
| ETLLQDFR_511.3_322.2 | AMBP_HUMAN | 0.796 |
| ATVVYQGER_511.8_751.4 | APOH_HUMAN | 0.795 |
| HHGPTITAK_321.2_432.3 | AMBP_HUMAN | 0.794 |
| TVQAVLTVPK_528.3_855.5 | PEDF_HUMAN | 0.791 |
| AHYDLR_387.7_566.3 | FETUA_HUMAN | 0.789 |
| TVQAVLTVPK_528.3_428.3 | PEDF_HUMAN | 0.787 |
| FICPLTGLWPINTLK_887.0_685.4 | APOH_HUMAN | 0.785 |
| VNHVTLSQPK_374.9_244.2 | B2MG_HUMAN | 0.783 |
| AHYDLR_387.7_288.2 | FETUA_HUMAN | 0.781 |
| ELIEELVNITQNQK_557.6_618.3 | IL13_HUMAN | 0.780 |
| FSVVYAK_407.2_381.2 | FETUA_HUMAN | 0.777 |
| IQTHSTTYR_369.5_627.3 | F13B_HUMAN | 0.777 |
| DTDTGALLFIGK_625.8_818.5 | PEDF_HUMAN | 0.774 |
| FICPLTGLWPINTLK_887.0_756.9 | APOH_HUMAN | 0.773 |
| DTDTGALLFIGK_625.8_217.1 | PEDF_HUMAN | 0.771 |
| FSVVYAK_407.2_579.4 | FETUA_HUMAN | 0.770 |
| IQTHSTTYR_369.5_540.3 | F13B_HUMAN | 0.769 |
| LDFHFSSDR_375.2_464.2 | INHBC_HUMAN | 0.769 |
| TLAFVR_353.7_274.2 | FA7_HUMAN | 0.769 |
| FLYHK_354.2_447.2 | AMBP_HUMAN | 0.766 |
| VNHVTLSQPK_374.9_459.3 | B2MG_HUMAN | 0.762 |
| AIGLPEELIQK_605.86_856.5 | FABPL_HUMAN | 0.752 |
| FLYHK_354.2_284.2 | AMBP_HUMAN | 0.752 |
| ELIEELVNITQNQK_557.6_517.3 | IL13_HUMAN | 0.751 |
| ETPEGAEAKPWYEPIYLGGVFQLEK_951.14_877.5 | TNFA_HUMAN | 0.751 |
| HFQNLGK_422.2_527.2 | AFAM_HUMAN | 0.749 |
| LIQDAVTGLTVNGQITGDK_972.0_640.4 | ITIH3_HUMAN | 0.749 |
| LIQDAVTGLTVNGQITGDK_972.0_798.4 | ITIH3_HUMAN | 0.747 |
| IAPQLSTEELVSLGEK_857.5_533.3 | AFAM_HUMAN | 0.745 |
| HFQNLGK_422.2_285.1 | AFAM_HUMAN | 0.740 |
| NNQLVAGYLQGPNVNLEEK_700.7_999.5 | IL1RA_HUMAN | 0.738 |
| VVESLAK_373.2_646.4 | IBP1_HUMAN | 0.738 |
| IAPQLSTEELVSLGEK_857.5_333.2 | AFAM_HUMAN | 0.737 |
| IALGGLLFPASNLR_481.3_657.4 | SHBG_HUMAN | 0.734 |
| ALALPPLGLAPLLNLWAKPQGR_770.5_256.2 | SHBG_HUMAN | 0.731 |
| ELPQSIVYK_538.8_417.7 | FBLN3_HUMAN | 0.724 |
| TFLTVYWTPER_706.9_401.2 | ICAM1_HUMAN | 0.723 |
| GVTGYFTFNLYLK_508.3_260.2 | PSG5_HUMAN | 0.717 |
| DVLLLVHNLPQNLTGHIWYK_791.8_310.2 | PSG7_HUMAN | 0.716 |
| WNFAYWAAHQPWSR_607.3_545.3 | PRG2_HUMAN | 0.716 |
| YTTEIIK_434.2_603.4 | C1R_HUMAN | 0.716 |
| YTTEIIK_434.2_704.4 | C1R_HUMAN | 0.716 |
| DIPHWLNPTR_416.9_600.3 | PAPP1_HUMAN | 0.715 |
| WNFAYWAAHQPWSR_607.3_673.3 | PRG2_HUMAN | 0.715 |
| IALGGLLFPASNLR_481.3_412.3 | SHBG_HUMAN | 0.713 |
| VVLSSGSGPGLDLPLVLGLPLQLK_791.5_598.4 | SHBG_HUMAN | 0.713 |
| GFQALGDAADIR_617.3_717.4 | TIMP1_HUMAN | 0.711 |
| VVLSSGSGPGLDLPLVLGLPLQLK_791.5_768.5 | SHBG_HUMAN | 0.711 |
| DVLLLVHNLPQNLTGHIWYK_791.8_883.0 | PSG7_HUMAN | 0.708 |
| YGIEEHGK_311.5_599.3 | CXA1_HUMAN | 0.706 |
| AEHPTWGDEQLFQTTR_639.3_765.4 | PGH1_HUMAN | 0.705 |
| VVESLAK_373.2_547.3 | IBP1_HUMAN | 0.705 |
| DADPDTFFAK_563.8_825.4 | AFAM_HUMAN | 0.704 |
| DAQYAPGYDK_564.3_813.4 | CFAB_HUMAN | 0.704 |
| GFQALGDAADIR_617.3_288.2 | TIMP1_HUMAN | 0.704 |
| AEHPTWGDEQLFQTTR_639.3_569.3 | PGH1_HUMAN | 0.702 |
| NFPSPVDAAFR_610.8_959.5 | HEMO_HUMAN | 0.702 |
| ALALPPLGLAPLLNLWAKPQGR_770.5_457.3 | SHBG_HUMAN | 0.701 |
| GVTGYFTFNLYLK_508.3_683.9 | PSG5_HUMAN | 0.701 |
| DFNQFSSGEK_386.8_189.1 | FETA_HUMAN | 0.699 |
| GDTYPAELYITGSILR_885.0_274.1 | F13B_HUMAN | 0.699 |
| TLEAQLTPR_514.8_685.4 | HEP2_HUMAN | 0.699 |
| VEHSDLSFSK_383.5_468.2 | B2MG_HUMAN | 0.699 |
| DAQYAPGYDK_564.3_315.1 | CFAB_HUMAN | 0.698 |
| VSEADSSNADWVTK_754.9_347.2 | CFAB_HUMAN | 0.698 |
| ILPSVPK_377.2_244.2 | PGH1_HUMAN | 0.695 |
| DADPDTFFAK_563.8_302.1 | AFAM_HUMAN | 0.694 |
| EVFSKPISWEELLQ_852.9_260.2 | FA40A_HUMAN | 0.694 |
| HTLNQIDEVK_598.8_5958.5 | FETUA_HUMAN | 0.694 |
| NFPSPVDAAFR_610.8_775.4 | HEMO_HUMAN | 0.694 |
| VSFSSPLVAISGVALR_802.0_715.4 | PAPP1_HUMAN | 0.694 |
| TLAFVR_353.7_492.3 | FA7_HUMAN | 0.693 |
| ILPSVPK_377.2_227.2 | PGH1_HUMAN | 0.691 |
| LLEVPEGR_456.8_356.2 | C1S_HUMAN | 0.691 |
| TLEAQLTPR_514.8_814.4 | HEP2_HUMAN | 0.691 |
| IPSNPSHR_303.2_610.3 | FBLN3_HUMAN | 0.690 |
| LPNNVLQEK_527.8_730.4 | AFAM_HUMAN | 0.690 |
| NCSFSIIYPVVIK_770.4_555.4 | CRHBP_HUMAN | 0.690 |
| NCSFSIIYPVVIK_770.4_831.5 | CRHBP_HUMAN | 0.690 |
| VEHSDLSFSK_383.5_234.1 | B2MG_HUMAN | 0.690 |
| ALDLSLK_380.2_185.1 | ITIH3_HUMAN | 0.688 |
| IHWESASLLR_606.3_437.2 | CO3_HUMAN | 0.688 |
| IPSNPSHR_303.2_496.3 | FBLN3_HUMAN | 0.688 |
| LDGSTHLNIFFAK_488.3_852.5 | PAPP1_HUMAN | 0.687 |
| QGHNSVFLIK_381.6_260.2 | HEMO_HUMAN | 0.687 |
| AVLHIGEK_289.5_348.7 | THBG_HUMAN | 0.686 |
| VSEADSSNADWVTK_754.9_533.3 | CFAB_HUMAN | 0.686 |
| TNTNEFLIDVDK_704.85_849.5 | TF_HUMAN | 0.685 |
| AVLHIGEK_289.5_292.2 | THBG_HUMAN | 0.683 |
| HTLNQIDEVK_598.8_951.5 | FETUA_HUMAN | 0.683 |
| VSFSSPLVAISGVALR_802.0_602.4 | PAPP1_HUMAN | 0.683 |
| IAQYYYTFK_598.8_395.2 | F13B_HUMAN | 0.681 |
| ALDLSLK_380.2_575.3 | ITIH3_HUMAN | 0.680 |
| LLEVPEGR_456.8_686.4 | C1S_HUMAN | 0.680 |
| QGHNSVFLIK_381.6_520.4 | HEMO_HUMAN | 0.680 |
| SEPRPGVLLR_375.2_454.3 | FA7_HUMAN | 0.680 |
| SFRPFVPR_335.9_272.2 | LBP_HUMAN | 0.680 |
| AFQVWSDVTPLR_709.88_385.3 | MMP2_HUMAN | 0.679 |
| FAFNLYR_465.8_712.4 | HEP2_HUMAN | 0.679 |
| IAQYYYTFK_598.8_884.4 | F13B_HUMAN | 0.679 |
| ITGFLKPGK_320.9_429.3 | LBP_HUMAN | 0.679 |
| EHSSLAFWK_552.8 838.4 | APOH_HUMAN | 0.677 |
| GLQYAAQEGLLALQSELLR_1037.1_858.5 | LBP_HUMAN | 0.676 |
| YYLQGAK_ 421.7_327.1 | ITIH4_HUMAN | 0.676 |
| LIENGYFHPVK_439.6 627.4 | F13B_HUMAN | 0.675 |
| SFRPFVPR_335.9_635.3 | LBP_HUMAN | 0.675 |
| AALAAFNAQNNGSNFQLEEISR_789.1 746.4 | FETUA_HUMAN | 0.674 |
| ITGFLKPGK_320.9_301.2 | LBP_HUMAN | 0.673 |
| VQEVLLK_414.8_373.3 | HYOU1_HUMAN | 0.673 |
| YNSQLLSFVR_613.8_508.3 | TFR1_HUMAN | 0.673 |
| EHSSLAFWK_552.8_267.1 | APOH_HUMAN | 0.672 |
| FAFNLYR_465.8_565.3 | HEP2_HUMAN | 0.672 |
| GDTYPAELYITGSILR_885.0_922.5 | F13B_HUMAN | 0.672 |
| ITLPDFTGDLR_624.3_920.5 | LBP_HUMAN | 0.672 |
| NSDQEIDFK_548.3_409.2 | S10A5_HUMAN | 0.672 |
| TAVTANLDIR_537.3_802.4 | CHL1_HUMAN | 0.672 |
| YYLQGAK_421.7_516.3 | ITIH4_HUMAN | 0.672 |
| ITLPDFTGDLR_624.3_288.2 | LBP_HUMAN | 0.670 |
| AIGLPEELIQK_605.86_355.2 | FABPL_HUMAN | 0.669 |
| ALNFGGIGVVVGHELTHAFDDQGR_837.1_299.2 | ECE1_HUMAN | 0.668 |
| AQETSGEEISK_589.8_979.5 | IBP1_HUMAN | 0.668 |
| LPNNVLQEK_527.8_844.5 | AFAM_HUMAN | 0.668 |
| TGISPLALIK_506.8_654.5 | APOB_HUMAN | 0.666 |
| DFHINLFQVLPWLK_885.5_543.3 | CFAB_HUMAN | 0.665 |
| VQEVLLK_414.8_601.4 | HYOU1_HUMAN | 0.665 |
| YENYTSSFFIR_713.8_756.4 | IL12B_HUMAN | 0.665 |
| CRPINATLAVEK_457.9_559.3 | CGB1_HUMAN | 0.663 |
| LDGSTHLNIFFAK_488.3_739.4 | PAPP1_HUMAN | 0.663 |
| TGISPLALIK_506.8_741.5 | APOB_HUMAN | 0.663 |
| EVFSKPISWEELLQ_852.9_376.2 | FA40A_HUMAN | 0.662 |
| SLDFTELDVAAEK_719.4_874.5 | ANGT_HUMAN | 0.662 |
| TFLTVYWTPER_706.9_502.3 | ICAM1_HUMAN | 0.662 |
| VRPQQLVK_484.3_609.4 | ITIH4_HUMAN | 0.662 |
| GLQYAAQEGLLALQSELLR_1037.1_929.5 | LBP_HUMAN | 0.661 |
| NAVVQGLEQPHGLWHPLR_688.4_890.6 | LRP1_HUMAN | 0.661 |
| SILFLGK_389.2_201.1 | THBG_HUMAN | 0.661 |
| DFNQFSSGEK_386.8_333.2 | FETA_HUMAN | 0.659 |
| IHWESASLLR_606.3_251.2 | CO3_ HUMAN | 0.659 |
| SILFLGK_389.2_577.4 | THBG_HUMAN | 0.658 |
| SVSLPSLDPASAK_636.4 473.3 | APOB_HUMAN | 0.658 |
| WWGGQPLWITATK_772.4_929.5 | ENPP2_HUMAN | 0.658 |
| LNIGYIEDLK_ 589.3_950.5 | PAI2_HUMAN | 0.657 |
| DFHINLFQVLPWLK_885.5_400.2 | CFAB_HUMAN | 0.657 |
| YSHYNER_323.48_418.2 | HABP2_HUMAN | 0.657 |
| STLFVPR_410.2_272.2 | PEPD_HUMAN | 0.656 |
| AFQVWSDVTPLR_709.88_347.2 | MMP2_HUMAN | 0.655 |
| FQSVFTVTR_542.8_722.4 | C1QC_HUMAN | 0.655 |
| GPGEDFR_389.2_623.3 | PTGDS_HUMAN | 0.655 |
| LEEHYELR_363.5_288.2 | PAI2_HUMAN | 0.655 |
| LPDTPQGLLGEAR_683.87_427.2 | EGLN_HUMAN | 0.655 |
| FQSVFTVTR_542.79_722.4 | C1QC_HUMAN | 0.654 |
| FTFTLHLETPKPSISSSNLNPR_829.4_787.4 | PSG1_HUMAN | 0.654 |
| NHYTESISVAK_624.8_252.1 | NEUR1_HUMAN | 0.654 |
| YSHYNER_323.48_581.3 | HABP2_HUMAN | 0.654 |
| FQSVFTVTR_542.79_623.4 | C1QC_HUMAN | 0.652 |
| IEGNLIFDPNNYLPK_874.0_845.5 | APOB_HUMAN | 0.652 |
| VRPQQLVK_484.3_722.4 | ITIH4_HUMAN | 0.652 |
| WILTAAHTLYPK_471.9_621.4 | C1R_HUMAN | 0.652 |
| ITQDAQLK_458.8_803.4 | CBG_ HUMAN | 0.651 |
| SVSLPSLDPASAK_636.4_885.5 | APOB_HUMAN | 0.651 |
| ESDTSYVSLK_564.8_347.2 | CRP_HUMAN | 0.650 |
| ESDTSYVSLK_564.8_696.4 | CRP_HUMAN | 0.650 |
| FQSVFTVTR_542.8_623.4 | C1QC_HUMAN | 0.650 |
| HELTDEELQSLFTNFANVVDK_817.1_854.4 | AFAM_HUMAN | 0.650 |
| IEGNLIFDPNNYLPK_874.0_414.2 | APOB_HUMAN | 0.650 |
| DIIKPDPPK_511.8_342.2 | IL12B_ HUMAN | 0.648 |
| SPELQAEAK_486.8_788.4 | APOA2_HUMAN | 0.648 |
| VELAPLPSWQPVGK_760.9_400.3 | ICAM1_HUMAN | 0.648 |
| AQETSGEEISK_589.8_850.4 | IBP1_HUMAN | 0.647 |
| QTLSWTVTPK_580.8 545.3 | PZP_HUMAN | 0.647 |
| DISEVVTPR_508.3_787.4 | CFAB_ HUMAN | 0.645 |
| DVLLLVHNLPQNLPGYFWYK_810.4_328.2 | PSG9_HUMAN | 0.645 |
| QTLSWTVTPK_580.8_818.4 | PZP_HUMAN | 0.645 |
| SGAQATWTELPWPHEK_613.3_510.3 | HEMO_HUMAN | 0.645 |
| SLDFTELDVAAEK_719.4_316.2 | ANGT_HUMAN | 0.645 |
| AVGYLITGYQR_620.8_523.3 | PZP_HUMAN | 0.644 |
| DISEVVTPR_508.3_472.3 | CFAB_ HUMAN | 0.644 |
| FLNWIK_410.7_560.3 | HABP2_HUMAN | 0.644 |
| IQHPFTVEEFVLPK_562.0_861.5 | PZP_HUMAN | 0.644 |
| ALQDQLVLVAAK_634.9_289.2 | ANGT_HUMAN | 0.643 |
| AVGYLITGYQR_620.8_737.4 | PZP_HUMAN | 0.643 |
| FLNWIK_410.7_561.3 | HABP2_HUMAN | 0.643 |
| LEQGENVFLQATDK_796.4_822.4 | C1QB_HUMAN | 0.643 |
| LSITGTYDLK_555.8_797.4 | A1AT_HUMAN | 0.641 |
| SEPRPGVLLR_375.2_654.4 | FA7_HUMAN | 0.641 |
| VPGLYYFTYHASSR_554.3_720.3 | C1QB_HUMAN | 0.641 |
| APLTKPLK_289.9_357.2 | CRP_HUMAN | 0.639 |
| FNAVLTNPQGDYDTSTGK_964.5_333.2 | C1QC_HUMAN | 0.639 |
| IQHPFTVEEFVLPK_562.0_603.4 | PZP_HUMAN | 0.639 |
| LSSPAVITDK_515.8_743.4 | PLMN_HUMAN | 0.639 |
| ALNFGGIGVVVGHELTHAFDDQGR_837.1_360.2 | ECE1_HUMAN | 0.637 |
| FNAVLTNPQGDYDTSTGK_964.5_262.1 | C1QC_HUMAN | 0.637 |
| LLELTGPK_435.8_227.2 | A1BG_HUMAN | 0.637 |
| YNSQLLSFVR_613.8_734.5 | TFR1_HUMAN | 0.636 |
| DLYHYITSYVVDGEIIIYGPAYSGR_955.5_707.3 | PSG1_HUMAN | 0.634 |
| GPGEDFR_389.2_322.2 | PTGDS_HUMAN | 0.634 |
| IHPSYTNYR_575.8_813.4 | PSG2_HUMAN | 0.634 |
| SGAQATWTELPWPHEK_613.3_793.4 | HEMO_HUMAN | 0.634 |
| SPELQAEAK_486.8_659.4 | APOA2_HUMAN | 0.634 |
| ALQDQLVLVAAK_634.9_956.6 | ANGT_HUMAN | 0.633 |
| ITENDIQIALDDAK_779.9_632.3 | APOB_HUMAN | 0.632 |
| ITQDAQLK_458.8_702.4 | CBG_HUMAN | 0.632 |
| LSSPAVITDK_515.8_830.5 | PLMN_HUMAN | 0.632 |
| SLLQPNK_400.2_358.2 | CO8A_HUMAN | 0.632 |
| VPGLYYFTYHASSR_554.3_420.2 | C1QB_HUMAN | 0.632 |
| YGLVTYATYPK_638.3_843.4 | CFAB_HUMAN | 0.632 |
| AGITIPR_364.2_486.3 | IL17_HUMAN | 0.630 |
| IHPSYTNYR_575.8_598.3 | PSG2_HUMAN | 0.630 |
| QINSYVK_426.2_610.3 | CBG_HUMAN | 0.630 |
| SSNNPHSPIVEEFQVPYNK_729.4_261.2 | C1S_HUMAN | 0.630 |
| ANDQYLTAAALHNLDEAVK_686.3_317.2 | ILIA_HUMAN | 0.629 |
| ATWSGAVLAGR_544.8_730.4 | A1BG_HUMAN | 0.629 |
| TLPFSR_360.7_506.3 | LYAM1_HUMAN | 0.629 |
| TYLHTYESEI_628.3_515.3 | ENPP2_HUMAN | 0.629 |
| EFDDDTYDNDIALLQLK_1014.48_388.3 | TPA_HUMAN | 0.627 |
| EFDDDTYDNDIALLQLK_1014.48_501.3 | TPA_HUMAN | 0.627 |
| VTGLDFIPGLHPILTLSK_641.04_771.5 | LEP_HUMAN | 0.627 |
| HVVQLR_376.2_614.4 | IL6RA_HUMAN | 0.626 |
| LIENGYFHPVK_439.6_343.2 | F13B_HUMAN | 0.626 |
| LLELTGPK_435.8_644.4 | A1BG_HUMAN | 0.626 |
| YEVQGEVFTKPQLWP_911.0_392.2 | CRP_HUMAN | 0.626 |
| DPNGLPPEAQK_583.3_497.2 | RET4_HUMAN | 0.625 |
| FTFTLHLETPKPSISSSNLNPR_829.4_874.4 | PSG1_HUMAN | 0.625 |
| YGLVTYATYPK_638.3_334.2 | CFAB_HUMAN | 0.625 |
| APLTKPLK_289.9_398.8 | CRP_HUMAN | 0.623 |
| DSPSVWAAVPGK_607.31_301.2 | PROF1_HUMAN | 0.623 |
| ENPAVIDFELAPIVDLVR_670.7_811.5 | CO6_HUMAN | 0.623 |
| ILILPSVTR_506.3_559.3 | PSGx_HUMAN | 0.623 |
| SFEGLGQLEVLTLDHNQLQEVK_833.1_503.3 | ALS_HUMAN | 0.623 |
| TSESGELHGLTTEEEFVEGIYK_819.06_310.2 | TTHY_HUMAN | 0.623 |
| AGITIPR_364.2_272.2 | IL17_HUMAN | 0.622 |
| DPDQTDGLGLSYLSSHIANVER_796.4_328.1 | GELS_HUMAN | 0.622 |
| ATWSGAVLAGR_544.8_643.4 | A1BG_HUMAN | 0.620 |
| HVVQLR_376.2_515.3 | IL6RA_HUMAN | 0.620 |
| QINSYVK_426.2_496.3 | CBG_HUMAN | 0.620 |
| TLFIFGVTK_513.3_215.1 | PSG4_HUMAN | 0.620 |
| YEVQGEVFTKPQLWP_911.0_293.1 | CRP_HUMAN | 0.620 |
| YYGYTGAFR_549.3_771.4 | TRFL_HUMAN | 0.620 |
| AALAAFNAQNNGSNFQLEEISR_789.1_633.3 | FETUA_HUMAN | 0.619 |
| ALNHLPLEYNSALYSR_621.0_696.4 | CO6_HUMAN | 0.619 |
| EDTPNSVWEPAK_686.8_630.3 | C1S_HUMAN | 0.619 |
| NNQLVAGYLQGPNVNLEEK_700.7_357.2 | IL1RA_HUMAN | 0.619 |
| ELANTIK_394.7_475.3 | S10AC_HUMAN | 0.618 |
| ENPAVIDFELAPIVDLVR_670.7_601.4 | CO6_HUMAN | 0.618 |
| GEVTYTTSQVSK_650.3_913.5 | EGLN_HUMAN | 0.616 |
| NEIWYR_440.7 637.4 | FA12_HUMAN | 0.616 |
| TLFIFGVTK_513.3_811.5 | PSG4_HUMAN | 0.616 |
| DLYHYITSYVVDGEIIIYGPAYSGR_955.5_650.3 | PSG1_HUMAN | 0.615 |
| DPTFIPAPIQAK_433.2_556.3 | ANGT_HUMAN | 0.615 |
| VELAPLPSWQPVGK_760.9_342.2 | ICAM1_HUMAN | 0.615 |
| DPNGLPPEAQK_583.3_669.4 | RET4_HUMAN | 0.614 |
| GIVEECCFR_585.3_900.3 | IGF2_HUMAN | 0.614 |
| ITENDIQIALDDAK_779.9_873.5 | APOB_HUMAN | 0.614 |
| LSETNR_360.2_330.2 | PSG1_HUMAN | 0.614 |
| LSNENHGIAQR_413.5_519.8 | ITIH2_HUMAN | 0.614 |
| YEFLNGR_449.7_293.1 | PLMN_HUMAN | 0.614 |
| AEIEYLEK_497.8_552.3 | LYAM1_HUMAN | 0.612 |
| GIVEECCFR_585.3_771.3 | IGF2_HUMAN | 0.612 |
| ILDDLSPR_464.8_587.3 | ITIH4_HUMAN | 0.611 |
| IRPHTFTGLSGLR_485.6_545.3 | ALS_HUMAN | 0.611 |
| VVGGLVALR_442.3_784.5 | FA12_HUMAN | 0.609 |
| LEEHYELR_363.5_417.2 | PAI2_HUMAN | 0.609 |
| LSNENHGIAQR_413.5_544.3 | ITIH2_HUMAN | 0.609 |
| TYLHTYESEI_628.3_908.4 | ENPP2_HUMAN | 0.609 |
| VLEPTLK_400.3_587.3 | VTDB_HUMAN | 0.609 |
| ILILPSVTR_506.3_785.5 | PSGx_HUMAN | 0.608 |
| TAVTANLDIR_537.3_288.2 | CHL1_HUMAN | 0.608 |
| WWGGQPLWITATK_772.4_373.2 | ENPP2_HUMAN | 0.607 |
| ALVLELAK_428.8_672.4 | INHBE_HUMAN | 0.605 |
| EAQLPVIENK_570.8_329.2 | PLMN_HUMAN | 0.605 |
| QRPPDLDTSSNAVDLLFFTDESGDSR_961.5_866.3 | C1R_HUMAN | 0.605 |
| TDAPDLPEENQAR_728.3_613.3 | CO5_HUMAN | 0.605 |
| TLPFSR_360.7_409.2 | LYAM1_HUMAN | 0.605 |
| VQTAHFK_277.5_502.3 | CO8A_HUMAN | 0.605 |
| ANLINNIFELAGLGK_793.9_299.2 | LCAP_HUMAN | 0.604 |
| FQLPGQK_409.2_275.1 | PSG1_HUMAN | 0.604 |
| NTVISVNPSTK_580.3_845.5 | VCAM1_HUMAN | 0.604 |
| VLEPTLK_400.3_458.3 | VTDB_HUMAN | 0.604 |
| YWGVASFLQK_599.8_849.5 | RET4_HUMAN | 0.604 |
| AGPLQAR_356.7_584.4 | DEF4_HUMAN | 0.602 |
| AHQLAIDTYQEFEETYIPK_766.0_521.3 | CSH_HUMAN | 0.602 |
| DLHLSDVFLK_396.2_366.2 | CO6_HUMAN | 0.602 |
| SSNNPHSPIVEEFQVPYNK_729.4_521.3 | C1S_HUMAN | 0.602 |
| YWGVASFLQK_599.8_350.2 | RET4_HUMAN | 0.602 |
| AGPLQAR_356.7_487.3 | DEF4 HUMAN | 0.601 |
| ALNHLPLEYNSALYSR_621.0_538.3 | CO6_HUMAN | 0.601 |
| EAQLPVIENK_570.8_699.4 | PLMN_HUMAN | 0.601 |
| EDTPNSVWEPAK_686.8_315.2 | C1S_HUMAN | 0.601 |
| NTVISVNPSTK_580.3_732.4 | VCAM1_HUMAN | 0.601 |

**Table 15. Univariate AUC values middle-late combined windows**

| Transition | Protein | AUC |
|---|---|---|
| GDTYPAELYITGSILR_885.0_274.1 | F13B_HUMAN | 0.7750 |
| TVQAVLTVPK_528.3_428.3 | PEDF_HUMAN | 0.7667 |
| IQTHSTTYR_369.5_627.3 | F13B_HUMAN | 0.7667 |
| DVLLLVHNLPQNLTGHIWYK_791.8_310.2 | PSG7_HUMAN | 0.7667 |
| IQTHSTTYR_369.5_540.3 | F13B_HUMAN | 0.7646 |
| ALALPPLGLAPLLNLWAKPQGR_770.5_256.2 | SHBG_HUMAN | 0.7646 |
| VVLSSGSGPGLDLPLVLGLPLQLK_791.5_768.5 | SHBG_HUMAN | 0.7625 |
| VVLSSGSGPGLDLPLVLGLPLQLK_791.5_98.4 | SHBG_HUMAN | 0.7625 |
| TVQAVLTVPK_528.3_855.5 | PEDF_HUMAN | 0.7604 |
| GDTYPAELYITGSILR_885.0_922.5 | F13B_HUMAN | 0.7604 |
| DVLLLVHNLPQNLTGHIWYK_791.8_883.0 | PSG7_HUMAN | 0.7604 |
| TLPFSR_360.7_506.3 | LYAM1_ HUMAN | 0.7563 |
| ALALPPLGLAPLLNLWAKPQGR_770.5_457.3 | SHBG_ HUMAN | 0.7563 |
| IALGGLLFPASNLR_481.3_657.4 | SHBG_ HUMAN | 0.7542 |
| IALGGLLFPASNLR_481.3_412.3 | SHBG_ HUMAN | 0.7542 |
| DTDTGALLFIGK_625.8_217.1 | PEDF_HUMAN | 0.7500 |
| QGFGNVATNTDGK_654.81_706.3 | FIBB_HUMAN | 0.7438 |
| ETLLQDFR_511.3_565.3 | AMBP_HUMAN | 0.7438 |
| ETLLQDFR_511.3_322.2 | AMBP_HUMAN | 0.7417 |
| IAQYYYTFK_598.8_884.4 | F13B_HUMAN | 0.7396 |
| DTDTGALLFIGK_625.8_818.5 | PEDF_HUMAN | 0.7396 |
| AEIEYLEK_497.8_552.3 | LYAM1_ HUMAN | 0.7396 |
| LDFHFSSDR_375.2_611.3 | INHBC_ HUMAN | 0.7354 |
| YQISVNK_426.2_560.3 | FIBB_HUMAN | 0.7333 |
| IAPQLSTEELVSLGEK_857.5_533.3 | AFAM_HUMAN | 0.7313 |
| EVFSKPISWEELLQ_852.9_376.2 | FA40A_HUMAN | 0.7292 |
| TLAFVR_353.7_274.2 | FA7_HUMAN | 0.7229 |
| HHGPTITAK_321.2_275.1 | AMBP_HUMAN | 0.7229 |
| SLQAFVAVAAR_566.8_487.3 | IL23A_HUMAN | 0.7208 |
| IAQYYYTFK_598.8_395.2 | F13B_HUMAN | 0.7208 |
| EVFSKPISWEELLQ_852.9_260.2 | FA40A_HUMAN | 0.7208 |
| DPNGLPPEAQK_583.3_669.4 | RET4_HUMAN | 0.7208 |
| DPNGLPPEAQK_583.3_497.2 | RET4_HUMAN | 0.7167 |
| VEHSDLSFSK_383.5_468.2 | B2MG_HUMAN | 0.7146 |
| YQISVNK_426.2_292.1 | FIBB_HUMAN | 0.7125 |
| TLAFVR_353.7_492.3 | FA7_HUMAN | 0.7125 |
| IAPQLSTEELVSLGEK_857.5_333.2 | AFAM_HUMAN | 0.7125 |
| AEIEYLEK_497.8_389.2 | LYAM1_ HUMAN | 0.7125 |
| YWGVASFLQK_599.8_849.5 | RET4_HUMAN | 0.7104 |
| TLPFSR_360.7_409.2 | LYAM1_ HUMAN | 0.7104 |
| HFQNLGK_422.2_527.2 | AFAM_HUMAN | 0.7104 |
| TQILEWAAER_608.8_761.4 | EGLN_HUMAN | 0.7083 |
| HFQNLGK_422.2_285.1 | AFAM_HUMAN | 0.7063 |
| FTFTLHLETPKPSISSSNLNPR_829.4_787.4 | PSG1_HUMAN | 0.7063 |
| DPDQTDGLGLSYLSSHIANVER_796.4_456.2 | GELS_ HUMAN | 0.7063 |
| DADPDTFFAK_563.8_825.4 | AFAM_HUMAN | 0.7042 |
| YWGVASFLQK_599.8_350.2 | RET4_HUMAN | 0.7021 |
| DADPDTFFAK_563.8_302.1 | AFAM_HUMAN | 0.7021 |
| HHGPTITAK_321.2_432.3 | AMBP_HUMAN | 0.6979 |
| NTVISVNPSTK_580.3_845.5 | VCAM1_HUMAN | 0.6958 |
| FLYHK_354.2_447.2 | AMBP HUMAN | 0.6958 |
| FICPLTGLWPINTLK_887.0_685.4 | APOH_HUMAN | 0.6958 |
| FTFTLHLETPKPSISSSNLNPR_829.4_874.4 | PSG1_HUMAN | 0.6938 |
| FLYHK_354.2_284.2 | AMBP_HUMAN | 0.6938 |
| EALVPLVADHK_397.9_390.2 | HGFA_HUMAN | 0.6938 |
| LNIGYIEDLK_589.3_837.4 | PAI2_HUMAN | 0.6917 |
| QGFGNVATNTDGK_654.81_319.2 | FIBB_HUMAN | 0.6896 |
| EALVPLVADHK_397.9_439.8 | HGFA_HUMAN | 0.6896 |
| TNTNEFLIDVDK_704.85_849.5 | TF_HUMAN | 0.6875 |
| DTYVSSFPR_357.8_272.2 | TCEA1_HUMAN | 0.6813 |
| VNHVTLSQPK_374.9_244.2 | B2MG_HUMAN | 0.6771 |
| GPGEDFR_389.2_623.3 | PTGDS_HUMAN | 0.6771 |
| GEVTYTTSQVSK_650.3_913.5 | EGLN_HUMAN | 0.6771 |
| GEVTYTTSQVSK_650.3_750.4 | EGLN_HUMAN | 0.6771 |
| FICPLTGLWPINTLK_887.0_756.9 | APOH_HUMAN | 0.6771 |
| YEFLNGR_449.7_606.3 | PLMN_HUMAN | 0.6750 |
| YEFLNGR_449.7_293.1 | PLMN_HUMAN | 0.6750 |
| TLFIFGVTK_513.3_215.1 | PSG4_HUMAN | 0.6750 |
| LNIGYIEDLK_589.3_950.5 | PAI2_HUMAN | 0.6750 |
| LLELTGPK_435.8_227.2 | A1BG_HUMAN | 0.6750 |
| TPSAAYLWVGTGASEAEK_919.5_849.4 | GELS_HUMAN | 0.6729 |
| FQLPGQK_409.2_275.1 | PSG1_HUMAN | 0.6729 |
| ELIEELVNITQNQK_557.6_618.3 | IL13_HUMAN | 0.6729 |
| DLYHYITSYVVDGEIIIYGPAYSGR_955.5_707.3 | PSG1_HUMAN | 0.6729 |
| AHYDLR_387.7_566.3 | FETUA_HUMAN | 0.6729 |
| LLEVPEGR_456.8_356.2 | C1S_HUMAN | 0.6708 |
| TLFIFGVTK_513.3_811.5 | PSG4_HUMAN | 0.6688 |
| FQLPGQK_409.2_429.2 | PSG1_HUMAN | 0.6667 |
| DLYHYITSYVVDGEIIIYGPAYSGR_955.5_650.3 | PSG1_HUMAN | 0.6667 |
| YYLQGAK_421.7_516.3 | ITIH4_HUMAN | 0.6646 |
| FSVVYAK_407.2_579.4 | FETUA_HUMAN | 0.6646 |
| EQLGEFYEALDCLR_871.9_747.4 | A1AG1_HUMAN | 0.6646 |
| LDFHFSSDR_375.2_464.2 | INHBC_HUMAN | 0.6625 |
| ALNHLPLEYNSALYSR_621.0_696.4 | CO6_HUMAN | 0.6625 |
| YYLQGAK_421.7_327.1 | ITIH4_HUMAN | 0.6604 |
| YTTEIIK_434.2_704.4 | C1R_HUMAN | 0.6604 |
| VEHSDLSFSK_383.5_234.1 | B2MG_HUMAN | 0.6604 |
| SNPVTLNVLYGPDLPR_585.7_654.4 | PSG6_HUMAN | 0.6604 |
| LWAYLTIQELLAK_781.5_300.2 | ITIH1_HUMAN | 0.6604 |
| FSLVSGWGQLLDR_493.3_403.2 | FA7_HUMAN | 0.6604 |
| ATVVYQGER_511.8_652.3 | APOH_HUMAN | 0.6604 |
| TPSAAYLWVGTGASEAEK_919.5_428.2 | GELS_HUMAN | 0.6583 |
| SEPRPGVLLR_375.2_454.3 | FA7_HUMAN | 0.6583 |
| LSSPAVITDK_515.8_830.5 | PLMN_HUMAN | 0.6583 |
| GPGEDFR_389.2_322.2 | PTGDS_HUMAN | 0.6583 |
| EFDDDTYDNDIALLQLK_1014.48_501.3 | TPA_HUMAN | 0.6583 |
| TFLTVYWTPER_706.9_502.3 | ICAM1_HUMAN | 0.6563 |
| NTVISVNPSTK_580.3_732.4 | VCAM1_HUMAN | 0.6563 |
| LPNNVLQEK_527.8_730.4 | AFAM_HUMAN | 0.6563 |
| LPDTPQGLLGEAR_683.87_427.2 | EGLN_HUMAN | 0.6563 |
| VANYVDWINDR_682.8_818.4 | HGFA_HUMAN | 0.6542 |
| LSSPAVITDK_515.8_743.4 | PLMN_HUMAN | 0.6542 |
| LPNNVLQEK_527.8_844.5 | AFAM_HUMAN | 0.6542 |
| IPGIFELGISSQSDR_809.9_849.4 | CO8B_HUMAN | 0.6542 |
| GAVHVVVAETDYQSFAVLYLER_822.8_580.3 | CO8G_HUMAN | 0.6542 |
| FLNWIK_410.7_560.3 | HABP2_HUMAN | 0.6542 |
| TFLTVYWTPER_706.9_401.2 | ICAM1_HUMAN | 0.6521 |
| NKPGVYTDVAYYLAWIR_677.0_821.5 | FA12_HUMAN | 0.6521 |
| AHYDLR_387.7_288.2 | FETUA_HUMAN | 0.6521 |
| LLEVPEGR_456.8_686.4 | C1S_HUMAN | 0.6500 |
| LIENGYFHPVK_439.6_627.4 | F13B_HUMAN | 0.6500 |
| GFQALGDAADIR_617.3_717.4 | TIMP1_HUMAN | 0.6500 |
| ELIEELVNITQNQK_557.6_517.3 | IL13_HUMAN | 0.6500 |
| EAQLPVIENK_570.8_329.2 | PLMN_HUMAN | 0.6479 |
| CRPINATLAVEK_457.9_559.3 | CGB1_HUMAN | 0.6479 |
| ATVVYQGER_511.8_751.4 | APOH_HUMAN | 0.6479 |
| ALNHLPLEYNSALYSR_621.0_538.3 | CO6_HUMAN | 0.6479 |
| AHQLAIDTYQEFEETYIPK_766.0_634.4 | CSH_HUMAN | 0.6479 |
| VTGLDFIPGLHPILTLSK_641.04_771.5 | LEP_HUMAN | 0.6458 |
| VANYVDWINDR_682.8_917.4 | HGFA_HUMAN | 0.6458 |
| SSNNPHSPIVEEFQVPYNK_729.4_261.2 | C1S_HUMAN | 0.6458 |
| NKPGVYTDVAYYLAWIR_677.0_545.3 | FA12_HUMAN | 0.6458 |
| GSLVQASEANLQAAQDFVR_668.7_735.4 | ITIH1_HUMAN | 0.6458 |
| YTTEIIK_434.2_603.4 | C1R_HUMAN | 0.6438 |
| NEIVFPAGILQAPFYTR_968.5_357.2 | ECE1_HUMAN | 0.6438 |
| IPGIFELGISSQSDR_809.9_679.3 | CO8B_HUMAN | 0.6438 |
| SNPVTLNVLYGPDLPR_585.7_817.4 | PSG6_HUMAN | 0.6417 |
| LLELTGPK_435.8_644.4 | A1BG_HUMAN | 0.6417 |
| EAQLPVIENK_570.8_699.4 | PLMN_HUMAN | 0.6417 |
| AEHPTWGDEQLFQTTR_639.3_765.4 | PGH1_HUMAN | 0.6417 |
| YGIEEHGK_311.5_599.3 | CXA1_HUMAN | 0.6396 |
| TQIDSPLSGK_523.3_703.4 | VCAM1_HUMAN | 0.6396 |
| YHFEALADTGISSEFYDNANDLLSK_940.8_301.1 | CO8A_HUMAN | 0.6375 |
| SCDLALLETYCATPAK_906.9_315.2 | IGF2_HUMAN | 0.6375 |
| NAVVQGLEQPHGLWHPLR_688.4_285.2 | LRP1_HUMAN | 0.6375 |
| HVVQLR_376.2_614.4 | IL6RA_HUMAN | 0.6375 |
| NNQLVAGYLQGPNVNLEEK_700.7_999.5 | IL1RA_HUMAN | 0.6354 |
| GIVEECCFR_585.3_771.3 | IGF2_HUMAN | 0.6354 |
| DGSPDVTTADIGANTPDATK_973.5_531.3 | PGRP2_HUMAN | 0.6354 |
| AEHPTWGDEQLFQTTR_639.3_569.3 | PGH1_HUMAN | 0.6354 |
| YVVISQGLDKPR_458.9_400.3 | LRP1_HUMAN | 0.6333 |
| WGAAPYR_410.7_577.3 | PGRP2_HUMAN | 0.6333 |
| VRPQQLVK_484.3_609.4 | ITIH4_HUMAN | 0.6333 |
| AVYEAVLR_460.8_750.4 | PEPD_HUMAN | 0.6333 |
| TQIDSPLSGK_523.3_816.5 | VCAM1_HUMAN | 0.6313 |
| IPKPEASFSPR_410.2_359.2 | ITIH4_HUMAN | 0.6313 |
| HELTDEELQSLFTNFANVVDK_817.1_854.4 | AFAM_HUMAN | 0.6313 |
| GSLVQASEANLQAAQDFVR_668.7_806.4 | ITIH1_HUMAN | 0.6313 |
| GAVHVVVAETDYQSFAVLYLER_822.8_863.5 | CO8G_HUMAN | 0.6313 |
| ENPAVIDFELAPIVDLVR_670.7_811.5 | CO6_HUMAN | 0.6313 |
| VRPQQLVK_484.3_722.4 | ITIH4_HUMAN | 0.6292 |
| IRPFFPQQ_516.79_372.2 | FIBB_HUMAN | 0.6292 |
| LWAYLTIQELLAK_781.5_371.2 | ITIH1_HUMAN | 0.6271 |
| EQLGEFYEALDCLR_871.9_563.3 | A1AG1_HUMAN | 0.6271 |
| LLDFEFSSGR_585.8_553.3 | G6PE_HUMAN | 0.6250 |
| LIENGYFHPVK_439.6_343.2 | F13B_HUMAN | 0.6250 |
| ENPAVIDFELAPIVDLVR_670.7_601.4 | CO6_HUMAN | 0.6250 |
| WNFAYWAAHQPWSR_607.3_545.3 | PRG2_HUMAN | 0.6229 |
| TAVTANLDIR_537.3_802.4 | CHL1_HUMAN | 0.6229 |
| WNFAYWAAHQPWSR_607.3_673.3 | PRG2_HUMAN | 0.6208 |
| HTLNQIDEVK_598.8_951.5 | FETUA_HUMAN | 0.6208 |
| DPDQTDGLGLSYLSSHIANVER_796.4_328.1 | GELS_HUMAN | 0.6208 |
| WGAAPYR_410.7_634.3 | PGRP2_HUMAN | 0.6188 |
| TEQAAVAR_423.2_487.3 | FA12_HUMAN | 0.6188 |
| LEEHYELR_363.5_288.2 | PAI2_HUMAN | 0.6188 |
| GIVEECCFR_585.3_900.3 | IGF2_HUMAN | 0.6188 |
| YHFEALADTGISSEFYDNANDLLSK_940.8_874.5 | CO8A_HUMAN | 0.6167 |
| TQILEWAAER_608.8_632.3 | EGLN_HUMAN | 0.6167 |
| DSPSVWAAVPGK_607.31_301.2 | PROF1_HUMAN | 0.6167 |
| DLHLSDVFLK_396.2_260.2 | CO6_HUMAN | 0.6167 |
| AQPVQVAEGSEPDGFWEALGGK_758.0_574.3 | GELS_HUMAN | 0.6167 |
| YSHYNER_323.48_581.3 | HABP2_HUMAN | 0.6146 |
| YSHYNER_323.48_418.2 | HABP2_HUMAN | 0.6146 |
| VNHVTLSQPK_374.9_459.3 | B2MG_HUMAN | 0.6146 |
| EHSSLAFWK_552.8_267.1 | APOH_HUMAN | 0.6146 |
| TATSEYQTFFNPR_781.4_386.2 | THRB_HUMAN | 0.6104 |
| SGFSFGFK_438.7_732.4 | CO8B_HUMAN | 0.6104 |
| GFQALGDAADIR_617.3_288.2 | TIMP1_HUMAN | 0.6104 |
| FSVVYAK_407.2_381.2 | FETUA_HUMAN | 0.6104 |
| QTLSWTVTPK_580.8_545.3 | PZP_HUMAN | 0.6083 |
| QLGLPGPPDVPDHAAYHPF_676.7_263.1 | ITIH4_HUMAN | 0.6083 |
| LSITGTYDLK_555.8_797.4 | A1AT_HUMAN | 0.6083 |
| LPDTPQGLLGEAR_683.87_940.5 | EGLN_HUMAN | 0.6083 |
| VVESLAK_373.2_646.4 | IBP1_HUMAN | 0.6063 |
| VSEADSSNADWVTK_754.9_347.2 | CFAB_HUMAN | 0.6063 |
| TEQAAVAR_423.2_615.4 | FA12_HUMAN | 0.6063 |
| SEPRPGVLLR_375.2_654.4 | FA7_HUMAN | 0.6063 |
| QTLSWTVTPK_580.8_818.4 | PZP_HUMAN | 0.6063 |
| HYINLITR_515.3_301.1 | NPY_HUMAN | 0.6063 |
| DPTFIPAPIQAK_433.2_461.2 | ANGT_HUMAN | 0.6063 |
| VSEADSSNADWVTK_754.9_533.3 | CFAB_HUMAN | 0.6042 |
| VQEVLLK_414.8_373.3 | HYOU1_HUMAN | 0.6042 |
| SILFLGK_389.2_577.4 | THBG_HUMAN | 0.6042 |
| IQHPFTVEEFVLPK_562.0_603.4 | PZP_HUMAN | 0.6042 |
| ELPQSIVYK_538.8_417.7 | FBLN3_HUMAN | 0.6042 |
| AVGYLITGYQR_620.8_737.4 | PZP_HUMAN | 0.6042 |
| ATWSGAVLAGR_544.8_643.4 | A1BG_HUMAN | 0.6042 |
| AKPALEDLR_506.8_288.2 | APOA1_HUMAN | 0.6042 |
| SEYGAALAWEK_612.8_845.5 | CO6_HUMAN | 0.6021 |
| NVNQSLLELHK_432.2_656.3 | FRIH_HUMAN | 0.6021 |
| IQHPFTVEEFVLPK_562.0_861.5 | PZP_HUMAN | 0.6021 |
| IPKPEASFSPR_410.2_506.3 | ITIH4_HUMAN | 0.6021 |
| GVTGYFTFNLYLK_508.3_260.2 | PSG5_HUMAN | 0.6021 |
| DGSPDVTTADIGANTPDATK_973.5_844.4 | PGRP2_HUMAN | 0.6021 |
| AVGYLITGYQR_620.8_523.3 | PZP_HUMAN | 0.6021 |
| ANDQYLTAAALHNLDEAVK_686.3_317.2 | ILIA_HUMAN | 0.6021 |
| TLYSSSPR_455.7_696.3 | IC1_HUMAN | 0.6000 |
| LHKPGVYTR_357.5_479.3 | HGFA_HUMAN | 0.6000 |
| IIGGSDADIK_494.8_260.2 | C1S_HUMAN | 0.6000 |
| HELTDEELQSLFTNFANVVDK_817.1_906.5 | AFAM_HUMAN | 0.6000 |
| GGEGTGYFVDFSVR_745.9_869.5 | HRG_HUMAN | 0.6000 |
| AVLHIGEK_289.5_348.7 | THBG_HUMAN | 0.6000 |
| ALVLELAK_428.8_672.4 | INHBE_HUMAN | 0.6000 |

**Table 16. Lasso Summed Coefficients All Windows**

| Transition | Protein | SumBestCoefs_All |
|---|---|---|
| TQILEWAAER_608.8_761.4 | EGLN_HUMAN | 26.4563 |
| GFQALGDAADIR_617.3_717.4 | TIMP1_HUMAN | 17.6447 |
| AVDIPGLEAATPYR_736.9_399.2 | TENA_HUMAN | 16.2270 |
| TVQAVLTVPK_528.3_428.3 | PEDF_HUMAN | 15.1166 |
| LDFHFSSDR_375.2_611.3 | INHBC_HUMAN | 15.0029 |
| ATVVYQGER_511.8_652.3 | APOH__HUMAN | 13.2314 |
| ETLLQDFR_511.3_565.3 | AMBP_HUMAN | 13.1219 |
| GFQALGDAADIR_617.3_288.2 | TIMP1_HUMAN | 12.1693 |
| IQTHSTTYR_369.5_627.3 | F13B_HUMAN | 9.4737 |
| GDTYPAELYITGSILR_885.0_274.1 | F13B_HUMAN | 6.1820 |
| ELPQSIVYK_538.8_417.7 | FBLN3_HUMAN | 6.1607 |
| NEIVFPAGILQAPFYTR_968.5_357.2 | ECE1_HUMAN | 5.5493 |
| AHYDLR_387.7_566.3 | FETUA_HUMAN | 5.4415 |
| HHGPTITAK_321.2_275.1 | AMBP_HUMAN | 5.0751 |
| SERPPIFEIR_415.2_564.3 | LRP1_HUMAN | 4.5620 |
| ALDLSLK_380.2_185.1 | ITIH3_HUMAN | 4.4275 |
| DTDTGALLFIGK_625.8_217.1 | PEDF_HUMAN | 4.3562 |
| ALNHLPLEYNSALYSR_621.0_696.4 | CO6_HUMAN | 3.9022 |
| ETLLQDFR_511.3_322.2 | AMBP_HUMAN | 3.3017 |
| YGIEEHGK_311.5_599.3 | CXA1_HUMAN | 2.8410 |
| IHWESASLLR_606.3_437.2 | CO3_HUMAN | 2.6618 |
| GEVTYTTSQVSK_650.3_750.4 | EGLN_HUMAN | 2.5328 |
| ELIEELVNITQNQK_557.6_618.3 | IL13_HUMAN | 2.5088 |
| DLHLSDVFLK_396.2_260.2 | CO6_HUMAN | 2.4010 |
| SYTITGLQPGTDYK_772.4_352.2 | FINC_HUMAN | 2.3304 |
| SPELQAEAK_486.8_788.4 | APOA2_HUMAN | 2.2657 |
| VNHVTLSQPK_374.9_459.3 | B2MG_HUMAN | 2.1480 |
| DTDTGALLFIGK_625.8_818.5 | PEDF_HUMAN | 2.0051 |
| LLDFEFSSGR_585.8_944.4 | G6PE_HUMAN | 1.7763 |
| GPGEDFR_389.2_623.3 | PTGDS_HUMAN | 1.6782 |
| DPNGLPPEAQK_583.3_669.4 | RET4_HUMAN | 1.6581 |
| IQTHSTTYR_369.5_540.3 | F13B_HUMAN | 1.6107 |
| VNHVTLSQPK_374.9_244.2 | B2MG_HUMAN | 1.4779 |
| STLFVPR_410.2_518.3 | PEPD_HUMAN | 1.3961 |
| GEVTYTTSQVSK_650.3_913.5 | EGLN_HUMAN | 1.3306 |
| ALVLELAK_428.8_672.4 | INHBE _HUMAN | 1.2973 |
| ANDQYLTAAALHNLDEAVK_686.3_317.2 | ILIA_HUMAN | 1.1850 |
| STLFVPR_410.2_272.2 | PEPD_HUMAN | 1.1842 |
| GPGEDFR_389.2_322.2 | PTGDS_HUMAN | 1.1742 |
| IPSNPSHR_303.2_610.3 | FBLN3_HUMAN | 1.0868 |
| HHGPTITAK_321.2_432.3 | AMBP_HUMAN | 1.0813 |
| TLAFVR_353.7_274.2 | FA7_HUMAN | 1.0674 |
| DLHLSDVFLK_396.2_366.2 | CO6_HUMAN | 0.9887 |
| EFDDDTYDNDIALLQLK_1014.48_501.3 | TPA_HUMAN | 0.9468 |
| AIGLPEELIQK_605.86_856.5 | FABPL_HUMAN | 0.7740 |
| LIENGYFHPVK_439.6_343.2 | F13B_HUMAN | 0.7740 |
| LPDTPQGLLGEAR_683.87_427.2 | EGLN_HUMAN | 0.6748 |
| EHSSLAFWK_552.8_267.1 | APOH_HUMAN | 0.6035 |
| NCSFSIIYPVVIK_770.4_831.5 | CRHBP_HUMAN | 0.6014 |
| ALNSIIDVYHK_424.9_661.3 | S10A8_HUMAN | 0.5987 |
| WGAAPYR_410.7_577.3 | PGRP2_HUMAN | 0.5699 |
| TQILEWAAER_608.8 632.3 | EGLN_HUMAN | 0.5395 |
| IPSNPSHR_303.2_496.3 | FBLN3_HUMAN | 0.4845 |
| VEHSDLSFSK_383.5_234.1 | B2MG_HUMAN | 0.4398 |
| VEHSDLSFSK_383.5_468.2 | B2MG_HUMAN | 0.3883 |
| FLYHK_354.2_284.2 | AMBP_HUMAN | 0.3410 |
| LPDTPQGLLGEAR_683.87_940.5 | EGLN_HUMAN | 0.3282 |
| EALVPLVADHK_397.9_390.2 | HGFA_HUMAN | 0.3091 |
| IEGNLIFDPNNYLPK_874.0_845.5 | APOB_HUMAN | 0.2933 |
| LIENGYFHPVK_439.6_627.4 | F13B_HUMAN | 0.2896 |
| VPLALFALNR_557.3_620.4 | PEPD_HUMAN | 0.2875 |
| FICPLTGLWPINTLK_887.0_685.4 | APOH_HUMAN | 0.2823 |
| NAVVQGLEQPHGLWHPLR_688.4_890.6 | LRP1_HUMAN | 0.2763 |
| ALNFGGIGVVVGHELTHAFDDQGR_837.1_299.2 | ECE1_HUMAN | 0.2385 |
| SPELQAEAK_486.8_659.4 | APOA2_HUMAN | 0.2232 |
| EVFSKPISWEELLQ_852.9_260.2 | FA40A_HUMAN | 0.1608 |
| VANYVDWINDR_682.8_917.4 | HGFA_HUMAN | 0.1507 |
| EVFSKPISWEELLQ_852.9_376.2 | FA40A_HUMAN | 0.1487 |
| HVVQLR_376.2_614.4 | IL6RA_HUMAN | 0.1256 |
| TVQAVLTVPK_528.3_855.5 | PEDF_HUMAN | 0.1170 |
| FTTFFTVNITQNQK_557.6_517.3 | IL13_HUMAN | 0.1159 |
| EALVPLVADHK_397.9_439.8 | HGFA_HUMAN | 0.0979 |
| AITPPHPASQANIIFDITEGNLR_825.8_917.5 | FBLN1_HUMAN | 0.0797 |
| FLYHK_354.2_447.2 | AMBP_HUMAN | 0.0778 |
| SLLQPNK_400.2_358.2 | CO8A_HUMAN | 0.0698 |
| TGISPLALIK_506.8_654.5 | APOB_HUMAN | 0.0687 |
| ALNFGGIGVVVGHELTHAFDDQGR_837.1_360.2 | ECE1_HUMAN | 0.0571 |
| DYWSTVK_449.7_347.2 | APOC3_HUMAN | 0.0357 |
| AITPPHPASQANIIFDITEGNLR_825.8 459.3 | FBLN1_HUMAN | 0.0313 |
| AALAAFNAQNNGSNFQLEEISR_789.1_633.3 | FETUA_HUMAN | 0.0279 |
| DPNGLPPEAQK_583.3_497.2 | RET4_HUMAN | 0.0189 |
| TLAFVR_353.7_492.3 | FA7_HUMAN | 0.0087 |

**Table 17. Lasso Summed Coefficients Early Window**

| Transition | Protein | SumBestCoefs Early |
|---|---|---|
| LDFHFSSDR_375.2_611.3 | INHBC_HUMAN | 40.2030 |
| ELPQSIVYK_538.8_417.7 | FBLN3_HUMAN | 22.6926 |
| GFQALGDAADIR_617.3_288.2 | TIMP1_HUMAN | 17.4169 |
| GFQALGDAADIR_617.3_717.4 | TIMP1_HUMAN | 3.4083 |
| VNHVTLSQPK_374.9_459.3 | B2MG_HUMAN | 3.2559 |
| EFDDDTYDNDIALLQLK_1014.48_388.3 | TPA_HUMAN | 2.4073 |
| STLFVPR_410.2_272.2 | PEPD_HUMAN | 2.3984 |
| WGAAPYR_410.7_634.3 | PGRP2_HUMAN | 2.3564 |
| LDFHFSSDR_375.2_464.2 | INHBC_HUMAN | 1.9038 |
| VNHVTLSQPK_374.9_244.2 | B2MG_HUMAN | 1.7999 |
| DTDTGALLFIGK_625.8_217.1 | PEDF_HUMAN | 1.5802 |
| GPGEDFR_389.2_623.3 | PTGDS_HUMAN | 1.4223 |
| IHWESASLLR_606.3_437.2 | CO3_HUMAN | 1.2735 |
| FTIEELVNITQNQK_557.6_618.3 | IL13_HUMAN | 1.2652 |
| AQPVQVAEGSEPDGFWEALGGK_758.0_623.4 | GELS_HUMAN | 1.2361 |
| FAFNLYR_465.8_565.3 | HEP2_HUMAN | 1.0876 |
| SGFSFGFK_438.7_732.4 | CO8B_HUMAN | 1.0459 |
| VVGGLVALR_442.3_784.5 | FA12_HUMAN | 0.9572 |
| IEGNLIFDPNNYLPK_874.0845.5 | APOB_HUMAN | 0.9571 |
| ETLLQDFR_511.3_565.3 | AMBP_HUMAN | 0.7851 |
| LSIPQITTK_500.8_687.4 | PSG5_HUMAN | 0.7508 |
| TASDFITK_441.7_710.4 | GELS_HUMAN | 0.6549 |
| YGIEEHGK_311.5_599.3 | CXA1_ HUMAN | 0.6179 |
| AFQVWSDVTPLR_709.88_347.2 | MMP2_HUMAN | 0.6077 |
| TVQAVLTVPK_528.3_855.5 | PEDF_HUMAN | 0.5889 |
| LSITGTYDLK_555.8_696.4 | A1AT_HUMAN | 0.5857 |
| ELIEELVNITQNQK_557.6_517.3 | IL13_HUMAN | 0.5334 |
| LIENGYFHPVK_439.6_627.4 | F13B_HUMAN | 0.5257 |
| NEIVFPAGILQAPFYTR_968.5_357.2 | ECE1_HUMAN | 0.4601 |
| SLLQPNK_400.2_358.2 | CO8A_HUMAN | 0.4347 |
| LSIPQITTK_500.8_800.5 | PSG5_HUMAN | 0.4329 |
| GVTGYFTFNLYLK_508.3_683.9 | PSG5_HUMAN | 0.4302 |
| IQTHSTTYR_369.5_627.3 | F13B_HUMAN | 0.4001 |
| ATVVYQGER_511.8_652.3 | APOH_HUMAN | 0.3909 |
| LPDTPQGLLGEAR_683.87_427.2 | EGLN_HUMAN | 0.3275 |
| NNQLVAGYLQGPNVNLEEK_700.7_999.5 | IL1RA_HUMAN | 0.3178 |
| SERPPIFEIR_415.2_564.3 | LRP1_HUMAN | 0.3112 |
| AHYDLR_387.7_566.3 | FETUA_HUMAN | 0.2900 |
| NEIWYR_440.7_637.4 | FA12_HUMAN | 0.2881 |
| ALDLSLK_380.2_575.3 | ITIH3_HUMAN | 0.2631 |
| NKPGVYTDVAYYLAWIR_677.0_545.3 | FA12_HUMAN | 0.2568 |
| SYTITGLQPGTDYK_772.4_352.2 | FINC_HUMAN | 0.2277 |
| LFIPQITPK_528.8_683.4 | PSG11_HUMAN | 0.2202 |
| IIGGSDADIK_494.8_260.2 | C1S_HUMAN | 0.2182 |
| AVDIPGLEAATPYR_736.9_399.2 | TENA_HUMAN | 0.2113 |
| DTDTGALLFIGK_625.8_818.5 | PEDF_HUMAN | 0.2071 |
| AEIEYLEK_497.8_389.2 | LYAM1_HUMAN | 0.1925 |
| EHSSLAFWK_552.8 838.4 | APOH_HUMAN | 0.1899 |
| LPDTPQGLLGEAR_683.87_940.5 | EGLN_HUMAN | 0.1826 |
| WGAAPYR_410.7_577.3 | PGRP2_HUMAN | 0.1669 |
| LFIPQITPK_528.8_261.2 | PSG11_HUMAN | 0.1509 |
| WWGGQPLWITATK_772.4_929.5 | ENPP2_HUMAN | 0.1446 |
| DSPSVWAAVPGK_607.31_301.2 | PROF1_HUMAN | 0.1425 |
| LIQDAVTGLTVNGQITGDK_972.0_798.4 | ITIH3_HUMAN | 0.1356 |
| ALDLSLK_380.2_185.1 | ITIH3_HUMAN | 0.1305 |
| TVQAVLTVPK_528.3_428.3 | PEDF_HUMAN | 0.1249 |
| NAVVQGLEQPHGLWHPLR_688.4_890.6 | LRP1_HUMAN | 0.1092 |
| NSDQEIDFK_548.3_409.2 | S10A5 _HUMAN | 0.0937 |
| YNSQLLSFVR_613.8_508.3 | TFR1_HUMAN | 0.0905 |
| LLDFEFSSGR_585.8 553.3 | G6PE_HUMAN | 0.0904 |
| ALNFGGIGVVVGHELTHAFDDQGR_837.1_2 99.2 | ECE1_HUMAN | 0.0766 |
| STLFVPR_410.2_518.3 | PEPD_HUMAN | 0.0659 |
| DLHLSDVFLK_396.2_260.2 | CO6_HUMAN | 0.0506 |
| EHSSLAFWK_552.8_267.1 | APOH_HUMAN | 0.0452 |
| TQIDSPLSGK_523.3_703.4 | VCAM1_HUMAN | 0.0447 |
| HHGPTITAK_321.2_432.3 | AMBP_HUMAN | 0.0421 |
| AFQVWSDVTPLR_709.88_385.3 | MMP2_HUMAN | 0.0417 |
| TGISPLALIK_506.8_741.5 | APOB_HUMAN | 0.0361 |
| DLHLSDVFLK_396.2_366.2 | CO6_HUMAN | 0.0336 |
| NTVISVNPSTK_580.3_845.5 | VCAM1_HUMAN | 0.0293 |
| DIIKPDPPK_511.8_342.2 | IL12B_HUMAN | 0.0219 |
| TGISPLALIK_506.8_654.5 | APOB_HUMAN | 0.0170 |
| GAVHVVVAFTDYQSFAVLYLER_822.8_580.3 | CO8G_HUMAN | 0.0151 |
| LNIGYIEDLK_589.3_837.4 | PAI2_HUMAN | 0.0048 |
| GPGEDFR_389.2_322.2 | PTGDS_HUMAN | 0.0008 |

**Table 18. Lasso Summed Coefficients Early Middle Combined Windows**

| Transition | Protein | SumBestCoefs EM |
|---|---|---|
| ELPQSIVYK_538.8_417.7 | FBLN3_HUMAN | 24.8794 |
| AHYDLR_387.7_566.3 | FETUA_HUMAN | 20.8397 |
| LDFHFSSDR_375.2_611.3 | INHBC_HUMAN | 18.6630 |
| GFQALGDAADIR_617.3_288.2 | TIMP1_HUMAN | 14.7270 |
| HHGPTITAK_321.2_432.3 | AMBP_HUMAN | 11.1473 |
| VNHVTLSQPK_374.9_459.3 | B2MG_HUMAN | 10.9421 |
| NNQLVAGYLQGPNVNLEEK_700.7_999.5 | IL1RA_HUMAN | 10.4646 |
| HHGPTITAK_321.2_275.1 | AMBP_HUMAN | 7.7034 |
| ETLLQDFR_511.3_565.3 | AMBP_HUMAN | 6.7435 |
| TVQAVLTVPK_528.3_428.3 | PEDF_HUMAN | 5.7356 |
| SLQAFVAVAAR_566.8_487.3 | IL23A_HUMAN | 4.8684 |
| YGIEEHGK_311.5_599.3 | CXA1_HUMAN | 4.4936 |
| ATVVYQGER_511.8_652.3 | APOH_HUMAN | 3.9524 |
| VNHVTLSQPK_374.9_244.2 | B2MG_HUMAN | 3.8937 |
| ET IEEL VNITQNQK_557.6_618.3 | IL13_HUMAN | 3.8022 |
| ALNFGGIGVVVGHELTHAFDDQGR_837.1_299.2 | ECE1_HUMAN | 3.7603 |
| ETLLQDFR_511.3_322.2 | AMBP_HUMAN | 3.1792 |
| TVQAVLTVPK_528.3_855.5 | PEDF_HUMAN | 3.1046 |
| AALAAFNAQNNGSNFQLEEISR_789.1_633.3 | FETUA_HUMAN | 3.0021 |
| AVDIPGLEAATPYR_736.9_399.2 | TENA_HUMAN | 2.6899 |
| DLHLSDVFLK_396.2_366.2 | CO6_HUMAN | 2.5525 |
| DTDTGALLFIGK_625.8_818.5 | PEDF_HUMAN | 2.4794 |
| SYTITGLQPGTDYK_772.4_352.2 | FINC_HUMAN | 2.4535 |
| IQTHSTTYR_369.5_627.3 | F13B_HUMAN | 2.3395 |
| AHYDLR_387.7_288.2 | FETUA_HUMAN | 2.1058 |
| NCSFSIIYPVVIK_770.4_831.5 | CRHBP_HUMAN | 2.0427 |
| AIGLPEELIQK_605.86_856.5 | FABPL_HUMAN | 1.5354 |
| GFQALGDAADIR_617.3_717.4 | TIMP1_HUMAN | 1.4175 |
| TGISPLALIK_506.8_654.5 | APOB_HUMAN | 1.3562 |
| YTTEIIK_434.2_603.4 | C1R_HUMAN | 1.2855 |
| ETPEGAEAKPWYEPIYLGGVFQLEK_951.14_8 77.5 | TNFA_HUMAN | 1.1198 |
| ANDQYLTAAALHNLDEAVK_686.3_317.2 | ILIA_HUMAN | 1.0574 |
| ILPSVPK_377.2_244.2 | PGH1_HUMAN | 1.0282 |
| ALDLSLK_380.2_185.1 | ITIH3_HUMAN | 1.0057 |
| NAVVQGLEQPHGLWHPLR_688.4_890.6 | LRP1_HUMAN | 0.9884 |
| IEGNLIFDPNNYLPK_874.0_845.5 | APOB_HUMAN | 0.9846 |
| ALDLSLK_380.2_575.3 | ITIH3_HUMAN | 0.9327 |
| LDFHFSSDR_375.2_464.2 | INHBC_HUMAN | 0.8852 |
| LSIPQITTK_500.8_800.5 | PSG5_HUMAN | 0.7740 |
| SERPPIFEIR_415.2_564.3 | LRP1_HUMAN | 0.7013 |
| AEAQAQYSAAVAK_654.3_709.4 | ITIH4_HUMAN | 0.6752 |
| IHWESASLLR_606.3_437.2 | CO3_HUMAN | 0.6176 |
| LFIPQITPK_528.8_261.2 | PSG11_HUMAN | 0.5345 |
| FICPLTGLWPINTLK_887.0_685.4 | APOH_HUMAN | 0.5022 |
| DFNQFSSGEK_386.8_189.1 | FETA_HUMAN | 0.4932 |
| TATSEYQTFFNPR_781.4_272.2 | THRB_HUMAN | 0.4725 |
| SPELQAEAK_486.8_788.4 | APOA2_HUMAN | 0.4153 |
| FIVGFTR_420.2_261.2 | CCL20_HUMAN | 0.4111 |
| TLLPVSKPEIR_418.3_288.2 | CO5_HUMAN | 0.3409 |
| DIIKPDPPK_511.8_342.2 | IL12B_HUMAN | 0.3403 |
| DTDTGALLFIGK_625.8 217.1 | PEDF_HUMAN | 0.3073 |
| YTTEIIK_434.2_704.4 | C1R_HUMAN | 0.3050 |
| SPELQAEAK_486.8_659.4 | APOA2_HUMAN | 0.3047 |
| TGISPLALIK_506.8_741.5 | APOB_HUMAN | 0.3031 |
| VVGGLVALR_442.3_784.5 | FA12_HUMAN | 0.2960 |
| WWGGQPLWITATK_772.4_373.2 | ENPP2_HUMAN | 0.2498 |
| TQILEWAAER_608.8_632.3 | EGLN_HUMAN | 0.2342 |
| STLFVPR_410.2_272.2 | PEPD_HUMAN | 0.2035 |
| DYWSTVK_449.7_347.2 | APOC3_HUMAN | 0.2018 |
| WWGGQPLWITATK_772.4_929.5 | ENPP2_HUMAN | 0.1614 |
| SILFLGK_389.2_201.1 | THBG_HUMAN | 0.1593 |
| AFQVWSDVTPLR_709.88 385.3 | MMP2_HUMAN | 0.1551 |
| IQTHSTTYR_369.5_540.3 | F13B_HUMAN | 0.1434 |
| AFQVWSDVTPLR_709.88_347.2 | MMP2_HUMAN | 0.1420 |
| LSITGTYDLK_555.8_797.4 | A1AT_HUMAN | 0.1395 |
| LSITGTYDLK_555.8_696.4 | A1AT_HUMAN | 0.1294 |
| WGAAPYR_410.7_634.3 | PGRP2_HUMAN | 0.1259 |
| IAPQLSTEELVSLGEK_857.5_533.3 | AFAM_HUMAN | 0.1222 |
| FICPLTGLWPINTLK_887.0_756.9 | APOH_HUMAN | 0.1153 |
| QINSYVK_426.2_496.3 | CBG_HUMAN | 0.1055 |
| TATSEYQTFFNPR_781.4_386.2 | THRB_HUMAN | 0.0921 |
| AFLEVNEEGSEAAASTAVVIAGR_764.4_685.4 | ANT3_HUMAN | 0.0800 |
| AKPALEDLR_506.8_288.2 | APOA1_HUMAN | 0.0734 |
| GPGEDFR_389.2_623.3 | PTGDS_HUMAN | 0.0616 |
| SLLQPNK_400.2_358.2 | CO8A_HUMAN | 0.0565 |
| ESDTSYVSLK_564.8_347.2 | CRP_HUMAN | 0.0497 |
| FFQYDTWK_567.8_712.3 | IGF2_HUMAN | 0.0475 |
| FSVVYAK_407.2_579.4 | FETUA_HUMAN | 0.0437 |
| TQIDSPLSGK_523.3_703.4 | VCAM1_HUMAN | 0.0401 |
| LNIGYIEDLK_589.3_837.4 | PAI2_HUMAN | 0.0307 |
| IPSNPSHR_303.2_496.3 | FBLN3_HUMAN | 0.0281 |
| NEIVFPAGILQAPFYTR_968.5_456.2 | ECE1_HUMAN | 0.0276 |
| TLAFVR_353.7_274.2 | FA7_HUMAN | 0.0220 |
| AEAQAQYSAAVAK_654.3_908.5 | ITIH4_HUMAN | 0.0105 |
| AQPVQVAEGSEPDGFWEALGGK_758.0_623.4 | GELS_HUMAN | 0.0103 |
| QINSYVK_426.2_610.3 | CBG_HUMAN | 0.0080 |
| NSDQEIDFK_548.3_409.2 | S10A5_HUMAN | 0.0017 |

**Table 19. Lasso Summed Coefficients Middle-Late Combined Windows**

| Transition | Protein | SumBestCoefs ML |
|---|---|---|
| TQILEWAAER_608.8_761.4 | EGLN_HUMAN | 45.0403 |
| GDTYPAELYITGSILR_885.0_274.1 | F13B_HUMAN | 31.4888 |
| GEVTYTTSQVSK_650.3_750.4 | EGLN_HUMAN | 22.3322 |
| GEVTYTTSQVSK_650.3_913.5 | EGLN_HUMAN | 17.0298 |
| AVDIPGLEAATPYR_736.9_286.1 | TENA_HUMAN | 8.6029 |
| AVDIPGLEAATPYR_736.9_399.2 | TENA_HUMAN | 7.9874 |
| NEIVFPAGILQAPFYTR_968.5_357.2 | ECE1_HUMAN | 7.8773 |
| ALNHLPLEYNSALYSR_621.0_696.4 | CO6_HUMAN | 6.8534 |
| DPNGLPPEAQK_583.3_669.4 | RET4_HUMAN | 5.0045 |
| GFQALGDAADIR_617.3_717.4 | TIMP1_HUMAN | 4.6191 |
| ATVVYQGER_511.8_652.3 | APOH_HUMAN | 4.2522 |
| IAQYYYTFK_598.8_395.2 | F13B_HUMAN | 3.5721 |
| NAVVQGLEQPHGLWHPLR_688.4_285.2 | LRP1_HUMAN | 3.2886 |
| IAQYYYTFK_598.8_884.4 | F13B_HUMAN | 2.9205 |
| SERPPIFEIR_415.2_564.3 | LRP1_HUMAN | 2.4237 |
| TLAFVR_353.7_274.2 | FA7_HUMAN | 2.1925 |
| EVFSKPISWEELLQ_852.9_260.2 | FA40A_HUMAN | 2.1591 |
| EVFSKPISWEELLQ_852.9_376.2 | FA40A_HUMAN | 2.1586 |
| EFDDDTYDNDIALLQLK_1014.48_501.3 | TPA_HUMAN | 2.0892 |
| TLAFVR_353.7_492.3 | FA7_HUMAN | 2.0399 |
| EALVPLVADHK_397.9_439.8 | HGFA_HUMAN | 1.8856 |
| ETLLQDFR_511.3_565.3 | AMBP_HUMAN | 1.7809 |
| ALNSIIDVYHK_424.9_661.3 | S10A8_HUMAN | 1.6114 |
| AITPPHPASQANIIFDITEGNLR_825.8_917.5 | FBLN1_HUMAN | 1.3423 |
| EQLGEFYEALDCLR_871.9_747.4 | A1AG1_HUMAN | 1.2473 |
| TFLTVYWTPER_706.9_502.3 | ICAM1_HUMAN | 0.9851 |
| NTVISVNPSTK_580.3_845.5 | VCAM1_HUMAN | 0.9845 |
| FLNWIK_410.7_560.3 | HABP2_HUMAN | 0.9798 |
| ETPEGAEAKPWYEPIYLGGVFQLEK_951.14_99 0.6 | TNFA_HUMAN | 0.9679 |
| NVNQSLLELHK_432.2_656.3 | FRIH_HUMAN | 0.8280 |
| VPLALFALNR_557.3_620.4 | PEPD_HUMAN | 0.7851 |
| IAPQLSTEELVSLGEK_857.5_533.3 | AFAM_HUMAN | 0.7731 |
| AVYEAVLR_460.8_750.4 | PEPD_HUMAN | 0.7452 |
| LPDTPQGLLGEAR_683.87_427.2 | EGLN_HUMAN | 0.7145 |
| TVQAVLTVPK_528.3_428.3 | PEDF_HUMAN | 0.6584 |
| YSHYNER_323.48_418.2 | HABP2_HUMAN | 0.5244 |
| LLELTGPK_435.8_644.4 | A1BG_HUMAN | 0.5072 |
| DTDTGALLFIGK_625.8_818.5 | PEDF_HUMAN | 0.5010 |
| DPNGLPPEAQK_583.3_497.2 | RET4_HUMAN | 0.4803 |
| AHYDLR_387.7_566.3 | FETUA_HUMAN | 0.4693 |
| LPNNVLQEK_527.8_844.5 | AFAM_HUMAN | 0.4640 |
| VTGLDFIPGLHPILTLSK_641.04_771.5 | LEP_HUMAN | 0.4584 |
| LLELTGPK_435.8_227.2 | A1BG_HUMAN | 0.4515 |
| YTTEIIK_434.2_704.4 | C1R_HUMAN | 0.4194 |
| SSNNPHSPIVEEFQVPYNK_729.4_261.2 | C1S_ HUMAN | 0.3886 |
| ALNHLPLEYNSALYSR_621.0_538.3 | CO6_HUMAN | 0.3405 |
| HFQNLGK_422.2_527.2 | AFAM_HUMAN | 0.3368 |
| EQLGEFYEALDCLR_871.9_563.3 | A1AG1_HUMAN | 0.3348 |
| TQILEWAAER_608.8_632.3 | EGLN_HUMAN | 0.2943 |
| ALVLELAK_428.8_672.4 | INHBE_HUMAN | 0.2895 |
| LSNENHGIAQR_413.5_519.8 | ITIH2_HUMAN | 0.2835 |
| LPNNVLQEK_527.8_730.4 | AFAM_HUMAN | 0.2764 |
| DTDTGALLFIGK_625.8_217.1 | PEDF_HUMAN | 0.2694 |
| GDTYPAELYITGSILR_885.0_922.5 | F13B_HUMAN | 0.2594 |
| GPITSAAELNDPQSILLR_632.3_601.4 | EGLN_HUMAN | 0.2388 |
| ANLINNIFELAGLGK_793.9_834.5 | LCAP_HUMAN | 0.2158 |
| SEPRPGVLLR_375.2_454.3 | FA7_HUMAN | 0.1921 |
| EQSLNVSQDLDTIR_539.9_557.8 | SYNE2_HUMAN | 0.1836 |
| FICPLTGLWPINTLK_887.0_685.4 | APOH_HUMAN | 0.1806 |
| ALNFGGIGVVVGHELTHAFDDQGR_837.1_360.2 | ECE1_HUMAN | 0.1608 |
| ANDQYLTAAALHNLDEAVK_686.3_317.2 | IL1A_HUMAN | 0.1607 |
| AQETSGEEISK_589.8_979.5 | IBP1_HUMAN | 0.1598 |
| QINSYVK_426.2_610.3 | CBG_HUMAN | 0.1592 |
| SILFLGK_389.2577.4 | THBG_HUMAN | 0.1412 |
| DAVVYPILVEFTR_761.4_286.1 | HYOU1_HUMAN | 0.1298 |
| LIEIANHVDK_384.6_683.3 | ADA12_HUMAN | 0.1297 |
| LSSPAVITDK_515.8_830.5 | PLMN_HUMAN | 0.1272 |
| LIENGYFHPVK_439.6_343.2 | F13B_HUMAN | 0.1176 |
| AALAAFNAQNNGSNFQLEEISR_789.1_633.3 | FETUA_HUMAN | 0.1160 |
| IQTHSTTYR_369.5_540.3 | F13B_HUMAN | 0.1146 |
| IPKPEASFSPR_410.2_506.3 | ITIH4_HUMAN | 0.1001 |
| LLDFEFSSGR_585.8_944.4 | G6PE_HUMAN | 0.0800 |
| YYLQGAK_421.7_516.3 | ITIH4_HUMAN | 0.0793 |
| VRPQQLVK_484.3_722.4 | ITIH4_HUMAN | 0.0744 |
| GPGEDFR_389.2_322.2 | PTGDS_HUMAN | 0.0610 |
| ITQDAQLK_458.8_803.4 | CBG_HUMAN | 0.0541 |
| TATSEYQTFFNPR_781.4_272.2 | THRB_HUMAN | 0.0511 |
| ETLLQDFR_511.3_322.2 | AMBP_HUMAN | 0.0472 |
| YEFLNGR_449.7_293.1 | PLMN_HUMAN | 0.0345 |
| TLYSSSPR_455.7_696.3 | IC1_HUMAN | 0.0316 |
| SLLQPNK_400.2_599.4 | CO8A_HUMAN | 0.0242 |
| LLEVPEGR_456.8_686.4 | C1S_HUMAN | 0.0168 |
| GGEGTGYFVDFSVR_745.9_722.4 | HRG_HUMAN | 0.0110 |
| IQTHSTTYR_369.5_627.3 | F13B_HUMAN | 0.0046 |

**Table 20. Random Forest SummedGini All Windows**

| Transition | Protein | SumBestGini | Probability |
|---|---|---|---|
| TVQAVLTVPK_528.3_428.3 | PEDF_HUMAN | 12.6521 | 1.0000 |
| DTDTGALLFIGK_625.8_818.5 | PEDF_HUMAN | 11.9585 | 0.9985 |
| ALALPPLGLAPLLNLWAKPQGR_770.5_2 56.2 | SHBG_HUMAN | 10.5229 | 0.9971 |
| DVLLLVHNLPQNLTGHIWYK_791.8_883. 0 | PSG7_HUMAN | 10.2666 | 0.9956 |
| ETLLQDFR_511.3_565.3 | AMBP_HUMAN | 8.9862 | 0.9941 |
| ALALPPLGLAPLLNLWAKPQGR_770.5_4 57.3 | SHBG_HUMAN | 8.6349 | 0.9927 |
| IALGGLLFPASNL_481.3_657.4 | SHBG_HUMAN | 8.5838 | 0.9912 |
| DTDTGALLFIGK_625.8_217.1 | PEDF_HUMAN | 8.2463 | 0.9897 |
| IQTHSTTYR_369.5_627.3 | F13B_HUMAN | 8.1199 | 0.9883 |
| DVLLLVHNLPQNLTGHIWYK_791.8_310. 2 | PSG7_HUMAN | 7.7393 | 0.9868 |
| IALGGLLFPASNLR_481.3_412.3 | SHBG_HUMAN | 7.5601 | 0.9853 |
| HHGPTITAK_321.2_432.3 | AMBP_HUMAN | 7.5181 | 0.9838 |
| ETLLQDFR_511.3_322.2 | AMBP_HUMAN | 7.4043 | 0.9824 |
| FICPLTGLWPINTLK_887.0_685.4 | APOH_HUMAN | 7.2072 | 0.9809 |
| GPGEDFR_389.2_623.3 | PTGDS_HUMAN | 7.1422 | 0.9794 |
| IQTHSTTYR_369.5_540.3 | F13B_HUMAN | 6.9809 | 0.9780 |
| TVQAVLTVPK_528.3_855.5 | PEDF_HUMAN | 6.6191 | 0.9765 |
| ATVVYQGER_511.8_652.3 | APOH_HUMAN | 6.5813 | 0.9750 |
| VVLSSGSGPGLDLPLVLGLPLQLK_791.5_ 598.4 | SHBG_HUMAN | 6.3244 | 0.9736 |
| HHGPTITAK_321.2_275.1 | AMBP_HUMAN | 6.3081 | 0.9721 |
| VVLSSGSGPGLDLPLVLGLPLQLK_791.5 | SHBG_HUMAN | 6.0654 | 0.9706 |
| 768.5 | | | |
| GDTYPAELYITGSILR_885.0_274.1 | F13B_HUMAN | 5.9580 | 0.9692 |
| ATVVYQGER_511.8_751.4 | APOH_HUMAN | 5.9313 | 0.9677 |
| LDFHFSSDR_375.2_611.3 | INHBC_HUMAN | 5.8533 | 0.9662 |
| LDFHFSSDR_375.2_464.2 | INHBC_HUMAN | 5.8010 | 0.9648 |
| EVFSKPISWEELLQ_852.9260.2 | FA40A HUMAN | 5.6648 | 0.9633 |
| DTYVSSFPR_357.8_272.2 | TCEA1_HUMAN | 5.6549 | 0.9618 |
| LPDTPQGLLGEAR_683.87_427.2 | EGLN_HUMAN | 5.3806 | 0.9604 |
| FLYHK_354.2_447.2 | AMBP_HUMAN | 5.3764 | 0.9589 |
| SPELQAEAK_486.8_659.4 | APOA2_HUMAN | 5.1896 | 0.9574 |
| GPGEDFR_389.2_322.2 | PTGDS_HUMAN | 5.1876 | 0.9559 |
| SGVDLADSNQK_567.3_662.3 | VGFR3_HUMAN | 5.1159 | 0.9545 |
| TNTNEFLIDVDK_704.85_849.5 | TF_HUMAN | 4.7216 | 0.9530 |
| FICPLTGLWPINTLK_887.0_756.9 | APOH_HUMAN | 4.6421 | 0.9515 |
| LNIGYIEDLK_589.3 950.5 | PAI2_HUMAN | 4.6250 | 0.9501 |
| EVFSKPISWEELLQ_852.9_376.2 | FA40A_HUMAN | 4.4215 | 0.9486 |
| SYTITGLQPGTDYK_772.4 680.3 | FINC_HUMAN | 4.4103 | 0.9471 |
| TLPFSR_360.7_409.2 | LYAM1_HUMAN | 4.2148 | 0.9457 |
| SPELQAEAK_486.8_788.4 | APOA2_HUMAN | 4.2081 | 0.9442 |
| GDTYPAELYITGSILR_885.0_922.5 | F13B_HUMAN | 4.0672 | 0.9427 |
| AEIEYLEK_497.8_552.3 | LYAM1_HUMAN | 3.9248 | 0.9413 |
| FSLVSGWGQLLDR_493.3_403.2 | FA7_HUMAN | 3.9034 | 0.9398 |
| FLYHK_354.2_284.2 | AMBP_HUMAN | 3.8982 | 0.9383 |
| SGVDLADSNQK_567.3_591.3 | VGFR3_HUMAN | 3.8820 | 0.9369 |
| LDGSTHLNIFFAK_488.3_739.4 | PAPP1_HUMAN | 3.8770 | 0.9354 |
| HFQNLGK_422.2_527.2 | AFAM_HUMAN | 3.7628 | 0.9339 |
| IAQYYYTFK_598.8_884.4 | F13B_HUMAN | 3.7040 | 0.9325 |
| GFQALGDAADIR_617.3_717.4 | TIMP1_HUMAN | 3.6538 | 0.9310 |
| ELPQSIVYK_538.8_417.7 | FBLN3_HUMAN | 3.6148 | 0.9295 |
| IAQYYYTFK_598.8_395.2 | F13B_HUMAN | 3.5820 | 0.9280 |
| GSLVQASEANLQAAQDFVR_668.7_735.4 | ITIH1_HUMAN | 3.5283 | 0.9266 |
| TLPFSR_360.7_506.3 | LYAM1_HUMAN | 3.5064 | 0.9251 |
| VNHVTLSQPK_374.9_244.2 | B2MG_HUMAN | 3.5045 | 0.9236 |
| IAPQLSTEELVSLGEK_857.5_533.3 | AFAM_HUMAN | 3.4990 | 0.9222 |
| VEHSDLSFSK_383.5_468.2 | B2MG_HUMAN | 3.4514 | 0.9207 |
| TQILEWAAER_608.8_761.4 | EGLN_HUMAN | 3.4250 | 0.9192 |
| AHQLAIDTYQEFEETYIPK_766.0_521.3 | CSH_HUMAN | 3.3634 | 0.9178 |
| TEFLSNYLTNVDDITLVPGTLGR_846.8_600.3 | ENPP2_HUMAN | 3.3512 | 0.9163 |
| HFQNLGK_422.2_285.1 | AFAM_HUMAN | 3.3375 | 0.9148 |
| VEHSDLSFSK_383.5_234.1 | B2MG_HUMAN | 3.3371 | 0.9134 |
| TELRPGETLNVNFLLR_624.68_875.5 | CO3_HUMAN | 3.1889 | 0.9119 |
| YQISVNK_426.2_292.1 | FIBB_HUMAN | 3.1668 | 0.9104 |
| YGFYTHVFR_397.2_659.4 | THRB_HUMAN | 3.1188 | 0.9075 |
| SEPRPGVLLR_375.2_454.3 | FA7_HUMAN | 3.1068 | 0.9060 |
| IAPQLSTEELVSLGEK_857.5_333.2 | AFAM_HUMAN | 3.0917 | 0.9046 |
| ILILPSVTR_506.3_785.5 | PSGx_HUMAN | 3.0346 | 0.9031 |
| TLAFVR_353.7_492.3 | FA7_HUMAN | 3.0237 | 0.9016 |
| AKPALEDLR_506.8_288.2 | APOA1_HUMAN | 3.0189 | 0.9001 |

**Table 21. Random Forest SummedGini Early Window**

| Transition | Protein | SumBestGini | Probability |
|---|---|---|---|
| LSETNR_360.2_330.2 | PSG1_HUMAN | 26.3610 | 1.0000 |
| ALNFGGIGVVVGHELTHAFDDQGR_837.1_299.2 | ECE1_HUMAN | 24.8946 | 0.9985 |
| ELPQSIVYK_538.8_417.7 | FBLN3_HUMAN | 24.8817 | 0.9971 |
| LDFHFSSDR_375.2_464.2 | INHBC_HUMAN | 24.3229 | 0.9956 |
| LDFHFSSDR_375.2_611.3 | INHBC_HUMAN | 22.2162 | 0.9941 |
| FSLVSGWGQLLDR_493.3_403.2 | FA7_HUMAN | 19.6528 | 0.9927 |
| TSESGELHGLTTEEEFVEGIYK_819.06_31 0.2 | TTHY_HUMAN | 19.2430 | 0.9912 |
| ATVVYQGER_511.8_751.4 | APOH_HUMAN | 19.1321 | 0.9897 |
| IQTHSTTYR_369.5_627.3 | F13B_HUMAN | 17.1528 | 0.9883 |
| ATVVYQGER_511.8_652.3 | APOH_HUMAN | 17.0214 | 0.9868 |
| HYINLITR_515.3_301.1 | NPY_HUMAN | 16.6713 | 0.9853 |
| FICPLTGLWPINTLK_887.0_685.4 | APOH_HUMAN | 15.0826 | 0.9838 |
| AFLEVNEEGSEAAASTAVVIAGR_764.4_ 614.4 | ANT3_HUMAN | 14.6110 | 0.9824 |
| IQTHSTTYR_369.5_540.3 | F13B_HUMAN | 14.5473 | 0.9809 |
| AHQLAIDTYQEFEETYIPK_766.0_521.3 | CSH_HUMAN | 14.0287 | 0.9794 |
| TGAQELLR_444.3_530.3 | GELS_HUMAN | 13.1389 | 0.9780 |
| DSPSVWAAVPGK_607.31_301.2 | PROF1_HUMAN | 12.9571 | 0.9765 |
| NCSFSIIYPVVIK_770.4_555.4 | CRHBP_HUMAN | 12.5867 | 0.9750 |
| ALALPPLGLAPLLNLWAKPQGR_770.5_256.2 | SHBG_HUMAN | 12.1138 | 0.9721 |
| DTDTGALLFIGK_625.8_818.5 | PEDF_HUMAN | 11.7054 | 0.9706 |
| TSDQIHFFFAK_447.6_512.3 | ANT3_HUMAN | 11.4261 | 0.9692 |
| IALGGLLFPASNLR_481.3_657.4 | SHBG_HUMAN | 11.0968 | 0.9677 |
| DTDTGALLFIGK_625.8_217.1 | PEDF_HUMAN | 10.9040 | 0.9662 |
| EQSLNVSQDLDTIR_539.9_758.4 | SYNE2_HUMAN | 10.6572 | 0.9648 |
| IALGGLLFPASNLR_481.3_412.3 | SHBG_HUMAN | 10.0629 | 0.9633 |
| FGFGGSTDSGPIR_649.3_745.4 | ADA12_HUMAN | 10.0449 | 0.9618 |
| ETPEGAEAKPWYEPIYLGGVFQLEK_951.14_877.5 | TNFA_HUMAN | 10.0286 | 0.9604 |
| LPDTPQGLLGEAR_683.87_427.2 | EGLN_HUMAN | 9.8980 | 0.9589 |
| FSVVYAK_407.2_381.2 | FETUA_HUMAN | 9.7971 | 0.9574 |
| YGIEEHGK_311.5_599.3 | CXA1_HUMAN | 9.7850 | 0.9559 |
| GFQALGDAADIR_617.3_717.4 | TIMP1_HUMAN | 9.7587 | 0.9545 |
| VVLSSGSGPGLDLPLVLGLPLQLK_791.5_598.4 | SHBG_HUMAN | 9.3421 | 0.9530 |
| HHGPTITAK_321.2_275.1 | AMBP_HUMAN | 9.2728 | 0.9515 |
| ALALPPLGLAPLLNLWAKPQGR_770.5_457.3 | SHBG_HUMAN | 9.2431 | 0.9501 |
| LIEIANHVDK_384.6_498.3 | ADA12_HUMAN | 9.1368 | 0.9486 |
| AFQVWSDVTPLR_709.88_347.2 | MMP2_HUMAN | 8.6789 | 0.9471 |
| AFQVWSDVTPLR_709.88_385.3 | MMP2_HUMAN | 8.6339 | 0.9457 |
| ETLLQDFR_511.3_322.2 | AMBP_HUMAN | 8.6252 | 0.9442 |
| ETLLQDFR_511.3_565.3 | AMBP_HUMAN | 8.3957 | 0.9427 |
| VNHVTLSQPK_374.9_459.3 | B2MG_HUMAN | 8.3179 | 0.9413 |
| HHGPTITAK_321.2_432.3 | AMBP_HUMAN | 8.2567 | 0.9398 |
| DTYVSSFPR_357.8_272.2 | TCEA1_HUMAN | 8.2028 | 0.9383 |
| GGEGTGYFVDFSVR_745.9_722.4 | HRG_HUMAN | 8.0751 | 0.9369 |
| DFNQFSSGEK_ 386.8_189.1 | FETA_HUMAN | 8.0401 | 0.9354 |
| DVLLLVHNLPQNLTGHIWYK_791.8_883.0 | PSG7_HUMAN | 7.9924 | 0.9339 |
| VSEADSSNADWVTK_754.9_347.2 | CFAB_HUMAN | 7.8630 | 0.9325 |
| QGHNSVFLIK_381.6_260.2 | HEMO_HUMAN | 7.8588 | 0.9310 |
| AQETSGEEISK_589.8_979.5 | IBP1_HUMAN | 7.7787 | 0.9295 |
| DIPHWLNPTR_416.9_600.3 | PAPP1_HUMAN | 7.6393 | 0.9280 |
| SPELQAEAK_486.8_788.4 | APOA2_HUMAN | 7.6248 | 0.9266 |
| QGHNSVFLIK_381.6_520.4 | HEMO_HUMAN | 7.6042 | 0.9251 |
| LIENGYFHPVK_439.6_343.2 | F13B_HUMAN | 7.5771 | 0.9236 |
| DIIKPDPPK_511.8_342.2 | IL12B_HUMAN | 7.5523 | 0.9222 |
| VNHVTLSQPK_374.9_244.2 | B2MG_HUMAN | 7.5296 | 0.9207 |
| TELRPGETLNVNFLLR_624.68_875.5 | CO3_HUMAN | 7.4484 | 0.9178 |
| QINSYVK_426.2_496.3 | CBG_HUMAN | 7.3266 | 0.9163 |
| YNSQLLSFVR_613.8_734.5 | TFR1_HUMAN | 7.3262 | 0.9148 |
| TVQAVLTVPK_528.3_855.5 | PEDF_HUMAN | 7.1408 | 0.9134 |
| QTLSWTVTPK_580.8_818.4 | PZP_HUMAN | 6.9764 | 0.9119 |
| DVLLLVHNLPQNLPGYFWYK_810.4_328.2 | PSG9_HUMAN | 6.9663 | 0.9104 |
| FICPLTGLWPINTLK_887.0_756.9 | APOH_HUMAN | 6.8924 | 0.9090 |
| TSYQVYSK_488.2_397.2 | C163A_ HUMAN | 6.5617 | 0.9075 |
| VVLSSGSGPGLDLPLVLGLPLQLK_791.5_768.5 | SHBG_ HUMAN | 6.4615 | 0.9060 |
| QINSYVK_426.2_610.3 | CBG_ HUMAN | 6.4595 | 0.9046 |
| LHKPGVYTR_357.5_479.3 | HGFA_HUMAN | 6.4062 | 0.9031 |
| ALVLELAK_428.8_672.4 | INHBE_HUMAN | 6.3684 | 0.9016 |
| YNSQLLSFVR_613.8_508.3 | TFR1_HUMAN | 6.3628 | 0.9001 |

**Table 22. Random Forest SummedGini Early-Middle Combined Windows**

| Transition | Protein | SumBestGini | Probability |
|---|---|---|---|
| ATVVYQGER_511.8_652.3 | APOH_HUMAN | 120.6132 | 1.0000 |
| ATVVYQGER_511.8_751.4 | APOH_HUMAN | 99.7548 | 0.9985 |
| IQTHSTTYR_369.5_627.3 | F13B_HUMAN | 57.5339 | 0.9971 |
| IQTHSTTYR_369.5_540.3 | F13B_HUMAN | 55.0267 | 0.9956 |
| FICPLTGLWPINTLK_887.0_685.4 | APOH_HUMAN | 49.9116 | 0.9941 |
| AHQLAIDTYQEFEETYIPK_766.0_521.3 | CSH_HUMAN | 48.9796 | 0.9927 |
| HHGPTITAK_321.2_432.3 | AMBP_HUMAN | 45.7432 | 0.9912 |
| SPELQAEAK_486.8_659.4 | APOA2_HUMAN | 42.1848 | 0.9897 |
| AHYDLR_387.7_566.3 | FETUA_HUMAN | 41.4591 | 0.9883 |
| ETLLQDFR_511.3_565.3 | AMBP_HUMAN | 39.7301 | 0.9868 |
| HHGPTITAK_321.2_275.1 | AMBP_HUMAN | 39.2096 | 0.9853 |
| ETLLQDFR_511.3_322.2 | AMBP_HUMAN | 36.8033 | 0.9838 |
| FICPLTGLWPINTLK_887.0_756.9 | APOH_HUMAN | 31.8246 | 0.9824 |
| TVQAVLTVPK_528.3_855.5 | PEDF_HUMAN | 31.1356 | 0.9809 |
| IALGGLLFPASNLR_481.3_657.4 | SHBG_HUMAN | 30.5805 | 0.9794 |
| DVLLLVHNLPQNLTGHIWYK_791.8_883.0 | PSG7_HUMAN | 29.5729 | 0.9780 |
| AHYDLR_387.7_288.2 | FETUA_HUMAN | 29.0239 | 0.9765 |
| SPELQAEAK_486.8_788.4 | APOA2_HUMAN | 28.6741 | 0.9750 |
| ETPEGAEAKPWYEPIYLGGVFQLEK_951. 14_877.5 | TNFA_HUMAN | 26.8117 | 0.9736 |
| LDFHFSSDR_375.2_611.3 | INHBC_HUMAN | 26.0001 | 0.9721 |
| DFNQFSSGEK_386.8_189.1 | FETA_HUMAN | 25.9113 | 0.9706 |
| HFQNLGK_422.2_527.2 | AFAM_HUMAN | 25.7497 | 0.9692 |
| DPDQTDGLGLSYLSSHIANVER_796.4_328.1 | GELS_HUMAN | 25.7418 | 0.9677 |
| VVLSSGSGPGLDLPLVLGLPLQLK_ 791.5_598.4 | SHBG_HUMAN | 25.6425 | 0.9662 |
| IALGGLLFPASNLR_481.3_412.3 | SHBG_HUMAN | 25.1737 | 0.9648 |
| LDFHFSSDR_375.2_464.2 | INHBC_HUMAN | 25.0674 | 0.9633 |
| LIQDAVTGLTVNGQITGDK_972.0_640.4 | ITIH3_HUMAN | 24.5613 | 0.9618 |
| VVLSSGSGPGLDLPLVLGLPLQLK_791.5_768.5 | SHBG_HUMAN | 23.2995 | 0.9604 |
| DIPHWLNPTR_416.9_600.3 | PAPP1_HUMAN | 22.9504 | 0.9589 |
| VNHVTLSQPK_374.9_459.3 | B2MG_HUMAN | 22.2821 | 0.9574 |
| QINSYVK_426.2_496.3 | CBG_ HUMAN | 22.2233 | 0.9559 |
| ALALPPLGLAPLLNLWAKPQGR_770.5_256.2 | SHBG_HUMAN | 22.1160 | 0.9545 |
| TELRPGETLNVNFLLR_624.68_875.5 | CO3_HUMAN | 21.9043 | 0.9530 |
| ITQDAQLK_458.8_803.4 | CBG_HUMAN | 21.8933 | 0.9515 |
| IAPQLSTEELVSLGEK_857.5_533.3 | AFAM_HUMAN | 21.4577 | 0.9501 |
| QINSYVK_426.2_610.3 | CBG_HUMAN | 21.3414 | 0.9486 |
| LIQDAVTGLTVNGQITGDK_972.0_798.4 | ITIH3_HUMAN | 21.2843 | 0.9471 |
| DTDTGALLFIGK_625.8_818.5 | PEDF_HUMAN | 21.2631 | 0.9457 |
| DVLLLVHNLPQNLPGYFWYK_810.4_328.2 | PSG9_HUMAN | 21.2547 | 0.9442 |
| HFQNLGK_422.2_285.1 | AFAM_HUMAN | 20.8051 | 0.9427 |
| DTDTGALLFIGK_625.8_217.1 | PEDF_HUMAN | 20.2572 | 0.9413 |
| FLYHK_354.2447.2 | AMBP_HUMAN | 19.6822 | 0.9398 |
| NNQLVAGYLQGPNVNLEEK_700.7_999.5 | IL1RA_HUMAN | 19.2156 | 0.9383 |
| VSFSSPLVAISGVALR_802.0_715.4 | PAPP1_HUMAN | 18.9721 | 0.9369 |
| TVQAVLTVPK_528.3_428.3 | PEDF_HUMAN | 18.9392 | 0.9354 |
| TFVNITPAEVGVLVGK_822.47_968.6 | PROF1_HUMAN | 18.9351 | 0.9339 |
| LQVLGK_329.2_416.3 | A2GL_HUMAN | 18.6613 | 0.9325 |
| TLAFVR_353.7_274.2 | FA7_HUMAN | 18.5095 | 0.9310 |
| ITQDAQLK_458.8_702.4 | CBG_HUMAN | 18.5046 | 0.9295 |
| DVLLLVHNLPQNLTGHIWYK_791.8_310.2 | PSG7_HUMAN | 18.4015 | 0.9280 |
| VSFSSPLVAISGVALR_802.0_602.4 | PAPP1_HUMAN | 17.5397 | 0.9266 |
| IAPQLSTEELVSLGEK_857.5_333.2 | AFAM_HUMAN | 17.5338 | 0.9251 |
| TLFIFGVTK_513.3_215.1 | PSG4_HUMAN | 17.5245 | 0.9236 |
| ALNFGGIGVVVGHEL_THAFDDQGR_837.1_299.2 | ECE1_HUMAN | 17.1108 | 0.9222 |
| FLYHK_354.2_284.2 | AMBP_HUMAN | 16.9237 | 0.9207 |
| LDGSTHLNIFFAK_488.3_739.4 | PAPP1_HUMAN | 16.8260 | 0.9192 |
| ELIEELVNITQNQK_557.6_618.3 | IL13_HUMAN | 16.5607 | 0.9178 |
| YNSQLLSFVR_613.8_734.5 | TFR1_HUMAN | 16.5425 | 0.9163 |
| AFQVWSDVTPLR_709.88_385.3 | MMP2_HUMAN | 16.3293 | 0.9148 |
| LDGSTHLNIFFAK_488.3_852.5 | PAPP1_HUMAN | 15.9820 | 0.9134 |
| TPSAAYLWVGTGASEAEK_919.5_428.2 | GELS_HUMAN | 15.9084 | 0.9119 |
| YTTEIIK_434.2_603.4 | C1R_HUMAN | 15.7998 | 0.9104 |
| FSVVYAK_407.2_381.2 | FETUA_HUMAN | 15.4991 | 0.9090 |
| VNHVTLSQPK_374.9_244.2 | B2MG_HUMAN | 15.2938 | 0.9075 |
| SYTITGLQPGTDYK_772.4_680.3 | FINC_HUMAN | 14.9898 | 0.9060 |
| DIPHWLNPTR_416.9_373.2 | PAPP1_HUMAN | 14.6923 | 0.9046 |
| AFQVWSDVTPLR_709.88_347.2 | MMP2_HUMAN | 14.4361 | 0.9031 |
| IAQYYYTFK_598.8_884.4 | F13B_HUMAN | 14.4245 | 0.9016 |
| FSLVSGWGQLLDR_493.3_403.2 | FA7_HUMAN | 14.3848 | 0.9001 |

From the foregoing description, it will be apparent that variations and modifications can be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

The application discloses *inter alia* the following items:
1. A panel of isolated biomarkers comprising N of the biomarkers listed in Tables 2, 3, 4, 5 and 7 through 22.
2. The panel of item 1, wherein N is a number selected from the group consisting of 2 to 24.
3. The panel of item 2, wherein said panel comprises at least two of the isolated biomarkers selected from the group consisting of FSVVYAK, SPELQAEAK, VNHVTLSQPK, SSNNPHSPIVEEFQVPYNK, and VVGGLVALR.
4. The panel of item 2, wherein said panel comprises alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4).
5. The panel of item 2, wherein said panel comprises at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4).
6. The panel of item 2, wherein said panel comprises at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), and plasminogen (PLMN).
7. A method of determining probability for preeclampsia in a pregnant female, the method comprising detecting a measurable feature of each of N biomarkers selected from the biomarkers listed in Tables 2, 3, 4, 5 and 7 through 22 in a biological sample obtained from said pregnant female, and analyzing said measurable features to determine the probability for preeclampsia in said pregnant female.
8. The method of item 7, wherein said measurable feature comprises fragments or derivatives of each of said N biomarkers selected from the biomarkers listed in Tables 2, 3, 4, 5 and 7 through 22.
9. The method of item 7, wherein said detecting a measurable feature comprises quantifying an amount of each of N biomarkers selected from the biomarkers listed in Tables 2, 3, 4, 5 and 7 through 22, combinations or portions and/or derivatives thereof in a biological sample obtained from said pregnant female.
10. The method of item 9, further comprising calculating the probability for preeclampsia in said pregnant female based on said quantified amount of each of N biomarkers selected from the biomarkers listed in Tables 2, 3, 4, 5 and 7 through 22.
11. The method of item 7, further comprising an initial step of providing a biomarker panel comprising N of the biomarkers listed in Tables 2, 3, 4, 5 and 7 through 22.
12. The method of item 7, further comprising an initial step of providing a biological sample from the pregnant female.
13. The method of item 7, further comprising communicating said probability to a health care provider.
14. The method of item 13, wherein said communication informs a subsequent treatment decision for said pregnant female.
15. The method of item 7, wherein N is a number selected from the group consisting of 2 to 24.
16. The method of item 15, wherein said N biomarkers comprise at least two of the isolated biomarkers selected from the group consisting of FSVVYAK, SPELQAEAK, VNHVTLSQPK, SSNNPHSPIVEEFQVPYNK, and VVGGLVALR.
17. The method of item 7, wherein said analysis comprises a use of a predictive model.
18. The method of item 17, wherein said analysis comprises comparing said measurable feature with a reference feature.
19. The method of item 18, wherein said analysis comprises using one or more selected from the group consisting of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, and a combination thereof.
20. The method of item 19, wherein said analysis comprises logistic regression.
21. The method of item 7, wherein said probability is expressed as a risk score.
22. The method of item 7, wherein the biological sample is selected from the group consisting of whole blood, plasma, and serum.
23. The method of item 22, wherein the biological sample is serum.
24. The method of item 7, wherein said quantifying comprises mass spectrometry (MS).
25. The method of item 24, wherein said MS comprises liquid chromatography-mass spectrometry (LC-MS).
26. The method of item 24, wherein said MS comprises multiple reaction monitoring (MRM) or selected reaction monitoring (SRM).
27. The method of item 26, wherein said MRM (or SRM) comprises scheduled MRM (SRM).
28. The method of item 7, wherein said quantifying comprises an assay that utilizes a capture agent.
29. The method of item 28, wherein said capture agent is selected from the group consisting of and antibody, antibody fragment, nucleic acid-based protein binding reagent, small molecule or variant thereof.
30. The method of item 28, wherein said assay is selected from the group consisting of enzyme immunoassay (EIA), enzyme-linked immunosorbent assay (ELISA), and radioimmunoassay (RIA).
31. The method of item 30, wherein said quantifying further comprises mass spectrometry (MS).
32. The method of item 31, wherein said quantifying comprises co-immunoprecitipation-mass spectrometry (co-IP MS).
33. The method of item 7, further comprising detecting a measurable feature for one or more risk indicia.
34. The method of item 33, wherein the one or more risk indicia are selected from the group consisting of history of preeclampsia, first pregnancy, age, obesity, diabetes, gestational diabetes, hypertension, kidney disease, multiple pregnancy, interval between pregnancies, new paternity, migraine headaches, rheumatoid arthritis, and lupus.
35. A method of determining probability for preeclampsia in a pregnant female, the method comprising: (a) quantifying in a biological sample obtained from said pregnant female an amount of each of N biomarkers selected from the biomarkers listed in Tables 2, 3, 4, 5 and 7 through 22; (b) multiplying said amount by a predetermined coefficient, (c) determining the probability for preeclampsia in said pregnant female comprising adding said individual products to obtain a total risk score that corresponds to said probability.
36. The panel of item 2, wherein said panel comprises at least two of the isolated biomarkers selected from the group consisting of LDFHFSSDR, TVQAVLTVPK, GPGEDFR, ETLLQDFR, ATVVYQGER, and GFQALGDAADIR.
37. The panel of item 2, wherein said panel comprises at least two of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), and Sex hormone-binding globulin (SHBG).
38. The panel of item 2, wherein said panel comprises at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), plasminogen (PLMN), of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), and Sex hormone-binding globulin (SHBG).
39. The method of item 7, wherein said N biomarkers comprise at least two of the isolated biomarkers selected from the group consisting of LDFHFSSDR, TVQAVLTVPK, GPGEDFR, ETLLQDFR, ATVVYQGER, and GFQALGDAADIR.
40. The method of item 7, wherein said N biomarkers comprise at least two of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), and Sex hormone-binding globulin (SHBG).
41. The method of item 7, wherein said N biomarkers comprise at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), plasminogen (PLMN), of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), and Sex hormone-binding globulin (SHBG).
42. The method of item 35 wherein said N biomarkers comprise at least two of the isolated biomarkers selected from the group consisting of LDFHFSSDR, TVQAVLTVPK, GPGEDFR, ETLLQDFR, ATVVYQGER, and GFQALGDAADIR.
43. The method of item 35 wherein said N biomarkers comprise at least two of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), and Sex hormone-binding globulin (SHBG).
44. The method of item 35, herein said N biomarkers comprise at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), plasminogen (PLMN), of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), and Sex hormone-binding globulin (SHBG).

## Claims

1. An isolated biomarker consisting of the sequence RLPLVPALDGCLRR.
